# EUROPEAN PATENT APPLICATION

(11) **EP 2 738 265 A2**
(43) Date of publication of application: **04.06.2014**
(21) Application number: 14152339.9
(22) Date of filing: 14.12.2011
(51) Int. Cl.: C12Q 1/68

(54) **MHC genes and risk of graft versus host disease**

(30) Priority: 14.12.2010 GB 201021149
(62) Divisional of application: 11794504.8
(71) Applicant: Georg-August-Universität Göttingen Stiftung Öffentlichen Rechts Universitätsmedizin, 37099 Göttingen (DE); University of Newcastle-Upon Tyne, Newcastle-upon-Tyne Tyne and Wear NE1 7RU (GB); Deutsches Primatenzentrum GmbH, 37077 Göttingen (DE)
(72) Inventor: Dressel, Ralf, 37085 Göttingen (DE); Dickson, Anne, New castle upon Tyne Tyne and Wear NE1 7RU (GB); Rolstad, Bent, 0316 Oslo (NO); Walter, Lutz, 37077 Göttingen (DE)
(74) Representative: Wichmann, Hendrik

(57) **Abstract**

The invention relates to the novel use of gene markers in a method of predicting the risk of or diagnosing a subject to develop graft versus host reaction (GvHR) or graft versus host disease (GvHD). In other aspects the invention also relates to methods of monitoring the efficacy of treatment of GvHR or GvHD, and methods of screening a candidate substance for the treatment of GvHR or GvHD.

SEQUENCE LISTING

<110>  Universität Goettingen

<120>  MHC Genes and risk of graft versus host disease

<130>  EPA-124840

<160>  77

<170>  PatentIn version 3.5

<210>  1
<211>  4100
<212>  DNA
<213>  Homo sapiens

<220>
<221>  CDS
<222>  (134)..(1129)
<223>  Ctss

<400>  1
gtacctcatg tgacaagttc caatttcttt tcaagtcaat tgaactgaaa tctccttgtt      60

gctttgaaat cttagaagag agcccactaa ttcaaggact cttactgtgg gagcaactgc     120

tggttctatc aca atg aaa cgg ctg gtt tgt gtg ctc ttg gtg tgc tcc        169
               Met Lys Arg Leu Val Cys Val Leu Leu Val Cys Ser
               1               5                   10

tct gca gtg gca cag ttg cat aaa gat cct acc ctg gat cac cac tgg       217
Ser Ala Val Ala Gln Leu His Lys Asp Pro Thr Leu Asp His His Trp
        15                  20                  25

cat ctc tgg aag aaa acc tat ggc aaa caa tac aag gaa aag aat gaa       265
His Leu Trp Lys Lys Thr Tyr Gly Lys Gln Tyr Lys Glu Lys Asn Glu
    30                  35                  40

gaa gca gta cga cgt ctc atc tgg gaa aag aat cta aag ttt gtg atg       313
Glu Ala Val Arg Arg Leu Ile Trp Glu Lys Asn Leu Lys Phe Val Met
45                  50                  55                  60

ctt cac aac ctg gag cat tca atg gga atg cac tca tac gat ctg ggc       361
Leu His Asn Leu Glu His Ser Met Gly Met His Ser Tyr Asp Leu Gly
                65                  70                  75

atg aac cac ctg gga gac atg acc agt gaa gaa gtg atg tct ttg atg       409
Met Asn His Leu Gly Asp Met Thr Ser Glu Glu Val Met Ser Leu Met
            80                  85                  90

agt tcc ctg aga gtt ccc agc cag tgg cag aga aat atc aca tat aag       457
Ser Ser Leu Arg Val Pro Ser Gln Trp Gln Arg Asn Ile Thr Tyr Lys
        95                  100                 105

tca aac cct aat cgg ata ttg cct gat tct gtg gac tgg aga gag aaa       505
Ser Asn Pro Asn Arg Ile Leu Pro Asp Ser Val Asp Trp Arg Glu Lys
    110                 115                 120

ggg tgt gtt act gaa gtg aaa tat caa ggt tct tgt ggt gct tgc tgg       553
Gly Cys Val Thr Glu Val Lys Tyr Gln Gly Ser Cys Gly Ala Cys Trp
125                 130                 135                 140

gct ttc agt gct gtg ggg gcc ctg gaa gca cag ctg aag ctg aaa aca     601
Ala Phe Ser Ala Val Gly Ala Leu Glu Ala Gln Leu Lys Leu Lys Thr
                145                 150                 155

gga aag ctg gtg tct ctc agt gcc cag aac ctg gtg gat tgc tca act     649
Gly Lys Leu Val Ser Leu Ser Ala Gln Asn Leu Val Asp Cys Ser Thr
            160                 165                 170

gaa aaa tat gga aac aaa ggc tgc aat ggt ggc ttc atg aca acg gct     697
Glu Lys Tyr Gly Asn Lys Gly Cys Asn Gly Gly Phe Met Thr Thr Ala
        175                 180                 185

ttc cag tac atc att gat aac aag ggc atc gac tca gac gct tcc tat     745
Phe Gln Tyr Ile Ile Asp Asn Lys Gly Ile Asp Ser Asp Ala Ser Tyr
    190                 195                 200

ccc tac aaa gcc atg gat cag aaa tgt caa tat gac tca aaa tat cgt     793
Pro Tyr Lys Ala Met Asp Gln Lys Cys Gln Tyr Asp Ser Lys Tyr Arg
205                 210                 215                 220

gct gcc aca tgt tca aag tac act gaa ctt cct tat ggc aga gaa gat     841
Ala Ala Thr Cys Ser Lys Tyr Thr Glu Leu Pro Tyr Gly Arg Glu Asp
                225                 230                 235

gtc ctg aaa gaa gct gtg gcc aat aaa ggc cca gtg tct gtt ggt gta     889
Val Leu Lys Glu Ala Val Ala Asn Lys Gly Pro Val Ser Val Gly Val
            240                 245                 250

gat gcg cgt cat cct tct ttc ttc ctc tac aga agt ggt gtc tac tat     937
Asp Ala Arg His Pro Ser Phe Phe Leu Tyr Arg Ser Gly Val Tyr Tyr
        255                 260                 265

gaa cca tcc tgt act cag aat gtg aat cat ggt gta ctt gtg gtt ggc     985
Glu Pro Ser Cys Thr Gln Asn Val Asn His Gly Val Leu Val Val Gly
    270                 275                 280

tat ggt gat ctt aat ggg aaa gaa tac tgg ctt gtg aaa aac agc tgg     1033
Tyr Gly Asp Leu Asn Gly Lys Glu Tyr Trp Leu Val Lys Asn Ser Trp
285                 290                 295                 300

ggc cac aac ttt ggt gaa gaa gga tat att cgg atg gca aga aat aaa     1081
Gly His Asn Phe Gly Glu Glu Gly Tyr Ile Arg Met Ala Arg Asn Lys
                305                 310                 315

gga aat cat tgt ggg att gct agc ttt ccc tct tac cca gaa atc tag     1129
Gly Asn His Cys Gly Ile Ala Ser Phe Pro Ser Tyr Pro Glu Ile
            320                 325                 330

aggatctctc ctttttataa caaatcaaga aatatgaagc actttctctt aacttaattt     1189

ttcctgctgt atccagaaga aataattgtg tcatgattaa tgtgtattta ctgtactaat     1249

tagaaaatat agtttgaggc cgggcacggt ggctcacgcc tgtaatccca gtacttggga     1309

ggccaaggca ggcatatcaa cttgaggcca ggagttaaag agcagcctgg ctaacatggt     1369

gaaaccccat ctctactaaa aatacaaaaa attagccgag cacggtggtg catgcctgta     1429

atcccagcta cttgggaggc tgaggcacga gattccttga acccaagagg ttgaggctat     1489

gttgagctga gatcacacca ctgtactcca gcctggatga cagagtggag actctgtttc     1549

aaaaaaacag aaaagaaaat atagtttgat tcttcatttt tttaaatttg caaatctcag 1609

gataaagttt gctaagtaaa ttagtaatgt actatagata taactgtaca aaaattgttc 1669

aacctaaaac aatctgtaat tgcttattgt tttattgtat actctttgtc tttttaagac 1729

ccctaatagc cttttgtaac ttgatggctt aaaaatactt aataaatctg ccatttcaaa 1789

tttctatcat tgccacatac cattcttatt cctaggcaac tattaataat ctatcctgag 1849

aatattaatt gtggtattct ggtgatgggg tttagcaact ttgatggaag aaaatattag 1909

gctataaatg tcctaaggac tcagattgta tctttgtaca gaagaggatt caaaacgcca 1969

cgtgtagtgg ctcatgcctg taatcccaac actttgggag gctgaagtag gaggatcgtc 2029

ttgagcccag gagttcaaga ccagcctgga caacatagtg agaccttgtc tccacaaaaa 2089

taaaaaagaa actatccagg agtggtggtg tgtgcctgtg gtccctgcta tgcagatgtc 2149

taagacagga ggatcacaag agcccaggag gttgagaatg cagtgagctt gtaattgcac 2209

cactgcactc cagcctgggt gacagagcaa gaccctgtct taaaaaaaga ggattcaaca 2269

catattttta tattatgtta aagtaaagaa atgcataaaa gacaagcact ttggaagaat 2329

tattttaatg atcaacaatt taatgtatta gtccaaatta tttttacgta gtcatcaaca 2389

atttgaccag ggcctttatt tggcaaataa ctgagccaac cagaataaaa taaccaatac 2449

tccactgctc atatttttat ctaattcaga tggatcttcc ttacaactgc tctagattag 2509

tagatgcatc taagcaggca gcaggaactt taaatttttt aagttcatgt ctatgacatg 2569

aacaatgtgt gggataatgt cattaatata tcctaaatta acctaaacgt atttcactaa 2629

ctctggctcc ttctccataa agcacatttt aaggaacaag aattgctaaa tataaaaaca 2689

taaataatac cataatacat ggctatcatc aaaagtgtat agaatattat agtttaaaag 2749

tatttagttg attacttttc agttttgttt tgttttttga gacggagtct cactctgttg 2809

cccaggctgg agtgcagtgg caccatctca gttcactgca acttctgcct cccgagttca 2869

agcgattctc ctgcctcagc ctcccgagta gctggaatta taggcgtgca ccaccacgcc 2929

cagctaattt ttgtattttt agtaaagaca gggttttgcc acattagcca ggctggtctc 2989

aaactcctga cctcaggtga tccacccacc ccagcctccc aaagtgctaa gattacaggc 3049

gtgagccact gagcccagcc tacttttcag tttttaacat aatttttgtt ttatccacaa 3109

cttttcaagt attgaaagta gaataaaaac atgggttctt agtctttagc tatctgttaa 3169

agcctatgaa tgccttctta aaatcatgtt tttaaatgca taaaatatat aggattacaa 3229

aggaatctaa ttatatcgaa atacagttat taaaatgtta aaagataagt ttgttatata 3289

ttaatatgca tgcttcttta taaatgcatt aaataagagt taatagctat cctaaatttg 3349

aaatagtgat aagcataatg aaaatagatg caaaaaacta atgtgatatg aaaatatctg 3409

ggtttttctt ttgatgatga agtattgcta atattaccgt ggtttatgaa ctatgttcag 3469

aattgaagaa aatcctaact ttcagttaga ggttagtgac ggggttcagg acaccctaca 3529

caaaatacag cactttgaca tattgaatat tttaagctga aggcatttga ggaaattgca 3589

gaagcaggaa ggtgactctg accttctgcc tgctgttctc cccagaagca gccataaaac 3649

ctgggaagga ttttctgacc ttcccctgaa gtagatcata agactgtcat gtaagaggtg 3709

ctctcctggc acccagagaa aaggagcatc cttacctcca aaagcacagg gacacaaaga 3769

ggaatctaaa caaacaggcc tctcagtttc cccagtttta ttacatttag cttgttcaca 3829

ctttgcccta tgacatttct acatcactgg ctgctcttca tcaaacctac tataaaaaac 3889

attcaagttc aactgtttct ttgggccttt atttccttat ggagcccctc gtgtcgtgta 3949

aaacttatat taaataaatg tgcatgcttt tctcttgcta atctctcttt tgttatagag 4009

atctcagccc taaacctagg atggatagaa ggaaacatat gttctcccct acattagtaa 4069

aaataaaaat ggaatttttt acccatacaa a 4100

<210> 2
<211> 331
<212> PRT
<213> Homo sapiens

<400> 2

Met Lys Arg Leu Val Cys Val Leu Leu Val Cys Ser Ser Ala Val Ala
1               5                   10                  15

Gln Leu His Lys Asp Pro Thr Leu Asp His His Trp His Leu Trp Lys
            20                  25                  30

Lys Thr Tyr Gly Lys Gln Tyr Lys Glu Lys Asn Glu Glu Ala Val Arg
        35                  40                  45

Arg Leu Ile Trp Glu Lys Asn Leu Lys Phe Val Met Leu His Asn Leu
    50                  55                  60

Glu His Ser Met Gly Met His Ser Tyr Asp Leu Gly Met Asn His Leu
65                  70                  75                  80

Gly Asp Met Thr Ser Glu Glu Val Met Ser Leu Met Ser Ser Leu Arg
                85                  90                  95

Val Pro Ser Gln Trp Gln Arg Asn Ile Thr Tyr Lys Ser Asn Pro Asn
            100                 105                 110

Arg Ile Leu Pro Asp Ser Val Asp Trp Arg Glu Lys Gly Cys Val Thr
        115                 120                 125

Glu Val Lys Tyr Gln Gly Ser Cys Gly Ala Cys Trp Ala Phe Ser Ala
    130                 135                 140

Val Gly Ala Leu Glu Ala Gln Leu Lys Leu Lys Thr Gly Lys Leu Val
145                 150                 155                 160

Ser Leu Ser Ala Gln Asn Leu Val Asp Cys Ser Thr Glu Lys Tyr Gly
                165                 170                 175

Asn Lys Gly Cys Asn Gly Gly Phe Met Thr Thr Ala Phe Gln Tyr Ile
            180                 185                 190

Ile Asp Asn Lys Gly Ile Asp Ser Asp Ala Ser Tyr Pro Tyr Lys Ala
        195                 200                 205

Met Asp Gln Lys Cys Gln Tyr Asp Ser Lys Tyr Arg Ala Ala Thr Cys
    210                 215                 220

Ser Lys Tyr Thr Glu Leu Pro Tyr Gly Arg Glu Asp Val Leu Lys Glu
225                 230                 235                 240

Ala Val Ala Asn Lys Gly Pro Val Ser Val Gly Val Asp Ala Arg His
                245                 250                 255

Pro Ser Phe Phe Leu Tyr Arg Ser Gly Val Tyr Tyr Glu Pro Ser Cys
            260                 265                 270

Thr Gln Asn Val Asn His Gly Val Leu Val Val Gly Tyr Gly Asp Leu
        275                 280                 285

Asn Gly Lys Glu Tyr Trp Leu Val Lys Asn Ser Trp Gly His Asn Phe
    290                 295                 300

Gly Glu Glu Gly Tyr Ile Arg Met Ala Arg Asn Lys Gly Asn His Cys
305                 310                 315                 320

Gly Ile Ala Ser Phe Pro Ser Tyr Pro Glu Ile
                325                 330

<210>  3
<211>  3231
<212>  DNA
<213>  Homo sapiens

<220>
<221>  CDS
<222>  (272)..(1564)
<223>  Pbx2

<400> 3
   ctctctcaca cacacccccg cttgggcctc ctctctctct ccggctccat tttctccgcc 60
   gccgggggcc ggggtctcct gtggggggcc cagccggtat cccaggtctc ccttcagtgc 120
   cggggtgaac ccccggggga gccgggagcc gggggcagac gggcgggggt tggggcggag 180
   ggagcagcgg ccccagcgag tttgggggga gaagtaacca ggcgggggga ggggcggagc 240
   agggaggggg cctcagggcc cccccccagc t atg gac gaa cgg cta ctg ggg 292
   Met Asp Glu Arg Leu Leu Gly
   1 5
   ccg ccc cct cca ggc ggg ggc cgg ggg ggc ctg gga ttg gtg agt ggg 340
   Pro Pro Pro Pro Gly Gly Gly Arg Gly Gly Leu Gly Leu Val Ser Gly
   10 15 20
   gag cct ggg ggc cct ggc gag cct ccc ggt ggc gga gac ccc ggt ggg 388
   Glu Pro Gly Gly Pro Gly Glu Pro Pro Gly Gly Gly Asp Pro Gly Gly
   25 30 35
   ggt agc ggg ggg gtc ccg gga ggc cga ggg aag caa gac atc ggg gac 436
   Gly Ser Gly Gly Val Pro Gly Gly Arg Gly Lys Gln Asp Ile Gly Asp
   40 45 50 55
   att ctg cag cag ata atg acc atc acc gac cag agc ctg gac gag gcc 484
   Ile Leu Gln Gln Ile Met Thr Ile Thr Asp Gln Ser Leu Asp Glu Ala
   60 65 70
   cag gcc aag aaa cac gcc cta aac tgc cac cga atg aag cct gct ctc 532
   Gln Ala Lys Lys His Ala Leu Asn Cys His Arg Met Lys Pro Ala Leu
   75 80 85
   ttt agc gtc ctg tgt gaa atc aag gag aaa act ggc ctc agc att cgg 580
   Phe Ser Val Leu Cys Glu Ile Lys Glu Lys Thr Gly Leu Ser Ile Arg
   90 95 100
   agc tcc cag gag gag gag ccg gtg gac cca cag ctg atg cgc ttg gac 628
   Ser Ser Gln Glu Glu Glu Pro Val Asp Pro Gln Leu Met Arg Leu Asp
   105 110 115
   aac atg ctt ctg gca gag ggt gtg gct ggg ccc gag aaa ggg ggc ggc 676
   Asn Met Leu Leu Ala Glu Gly Val Ala Gly Pro Glu Lys Gly Gly Gly
   120 125 130 135
   tca gca gca gca gct gca gcc gct gca gcc tct ggt ggt ggt gtg tcc 724
   Ser Ala Ala Ala Ala Ala Ala Ala Ala Ala Ser Gly Gly Gly Val Ser
   140 145 150
   cct gac aac tcc atc gaa cac tcg gac tat cgc agc aaa ctt gcc cag 772
   Pro Asp Asn Ser Ile Glu His Ser Asp Tyr Arg Ser Lys Leu Ala Gln
   155 160 165
   atc cgt cac ata tac cac tcg gag ctg gag aag tat gag cag gca tgt 820
   Ile Arg His Ile Tyr His Ser Glu Leu Glu Lys Tyr Glu Gln Ala Cys
   170 175 180
   aat gag ttc acg acc cat gtc atg aac ctg ctg agg gag cag agc cgc 868
   Asn Glu Phe Thr Thr His Val Met Asn Leu Leu Arg Glu Gln Ser Arg
   185 190 195
   acc agg ccc gtg gcc ccc aaa gag atg gaa cgc atg gtg agc atc atc 916
   Thr Arg Pro Val Ala Pro Lys Glu Met Glu Arg Met Val Ser Ile Ile
   200                 205                 210                 215
   cat cga aag ttc agc gcc atc cag atg cag ctg aag cag agc acc tgc       964
   His Arg Lys Phe Ser Ala Ile Gln Met Gln Leu Lys Gln Ser Thr Cys
                   220                 225                 230
   gag gct gtg atg atc ctg cgc tcc cgt ttc ctg gat gcc aga cga aag      1012
   Glu Ala Val Met Ile Leu Arg Ser Arg Phe Leu Asp Ala Arg Arg Lys
               235                 240                 245
   cgc cgt aac ttc agc aaa cag gcc act gag gtc cta aat gag tat ttc      1060
   Arg Arg Asn Phe Ser Lys Gln Ala Thr Glu Val Leu Asn Glu Tyr Phe
           250                 255                 260
   tac tcc cac ctg agt aac cca tat cct agt gag gag gcc aag gag gag      1108
   Tyr Ser His Leu Ser Asn Pro Tyr Pro Ser Glu Glu Ala Lys Glu Glu
       265                 270                 275
   ctt gcc aag aag tgt ggc atc acc gtg tct cag gtc tcc aac tgg ttt      1156
   Leu Ala Lys Lys Cys Gly Ile Thr Val Ser Gln Val Ser Asn Trp Phe
   280                 285                 290                 295
   ggc aac aag agg att cgc tat aag aaa aac atc gga aag ttc caa gag      1204
   Gly Asn Lys Arg Ile Arg Tyr Lys Lys Asn Ile Gly Lys Phe Gln Glu
                   300                 305                 310
   gag gca aac atc tat gct gtc aag acc gcc gtg tca gtc acc cag ggg      1252
   Glu Ala Asn Ile Tyr Ala Val Lys Thr Ala Val Ser Val Thr Gln Gly
               315                 320                 325
   ggc cac agc cgc acc agc tcc ccg aca ccc cct tcc tct gca ggc tct      1300
   Gly His Ser Arg Thr Ser Ser Pro Thr Pro Pro Ser Ser Ala Gly Ser
           330                 335                 340
   ggc ggc tct ttc aat ctc tca gga tct gga gac atg ttt ctg ggg atg      1348
   Gly Gly Ser Phe Asn Leu Ser Gly Ser Gly Asp Met Phe Leu Gly Met
       345                 350                 355
   cct ggg ctc aac gga gat tcc tat tct gct tcc cag gtg gaa tca ctc      1396
   Pro Gly Leu Asn Gly Asp Ser Tyr Ser Ala Ser Gln Val Glu Ser Leu
   360                 365                 370                 375
   cga cac tcg atg ggg cca ggg ggc tat ggg gat aac ctc ggg gga ggc      1444
   Arg His Ser Met Gly Pro Gly Gly Tyr Gly Asp Asn Leu Gly Gly Gly
                   380                 385                 390
   cag atg tac agc cca cgg gaa atg agg gca aat ggc agc tgg caa gag      1492
   Gln Met Tyr Ser Pro Arg Glu Met Arg Ala Asn Gly Ser Trp Gln Glu
               395                 400                 405
   gct gtg acc ccc tct tca gtg aca tcc cca acg gag gga cca ggg agt      1540
   Ala Val Thr Pro Ser Ser Val Thr Ser Pro Thr Glu Gly Pro Gly Ser
           410                 415                 420
   gtt cac tct gat acc tcc aac tga tcttgcccct cagggtcaca ggggtggggg      1594
   Val His Ser Asp Thr Ser Asn
       425                 430
   ctctcacaag gcgacttgaa gaggacgcag gcttccagag gacaaacccc aatacaggag      1654
   aagcacaaga cagagaaggg ccaatggggt catcccctcc ctaacgagac tctctgtgct      1714
   gggggtgcta attacatggc aggaagaatg gggcctctaa ggggagtgtg gggtctgtct      1774
   ctcccttttt tccatctttt tcctctctcg ctttctttct tacacagaaa catacacata 1834
   ccgagaaacc tatttctcag accccttttt ctcctctgtc tttctctctc cctctcccac 1894
   acctcacaca cacatactcc cacttgcaac tattctgttt ctctcctggg ctcccccact 1954
   ttcccttccc caccccactt gtatgctctg gaatctgtgg agacgccagc cctgcccaat 2014
   cagagatgcc aaaaatgggg acatgacttc tggacagagg acatgggcca cgcccccatg 2074
   catccccacc cccgcccctc cggacggctt acttacctca tacgcagctc atcttaaacc 2134
   aatagaatcg ctcggtggac gagagtgtct gactcagata tctacctcgg agggagtttc 2194
   tgctacttta gggaattatt gactgggctt tggggttgaa cttttttttt tttaaagaaa 2254
   gaaaaagaaa ccctgggatc catctgtttt ttttgttgtt gttgttgttt ttgttgttgt 2314
   tggtggtggt ggtggtggtg gttcttaatt tttaatttag tttggggaag tagcttgttt 2374
   ttttttttat aaatatgttg atttcttgtc tttttttttt tttatttctt actttcccat 2434
   attaggggtg atagccaaag gggttctggt aagagaaagg gggacaaaca gaactggtaa 2494
   agaggccccc ctggctccag gcctgtccat caggaagtaa attttacagg gcaccaagct 2554
   ttgcccccta aaatccctta ggtgttcttt gttcatgcag gcaggtttct gccgcatttg 2614
   atgtggaggc agtgaagggc ttgccctgct ggcctctcat ccccttcttt cccacaaccc 2674
   ttgggcaggg ctggactcag taattttgag gaaattgaag atgccatctt cccctgtgag 2734
   tgacatgtct ttaatttttt aaaaaactac tatttgaaaa ttggaggggg aagaatggga 2794
   agggagttat tgccaaatat gttaaatatg ggttggggtg cttgtatatg tatcttcctc 2854
   aatttcccca taaatgaggt atctttttgt cacaccaaaa tcaaggggta gggagaggga 2914
   ggaggttgca aaaagccaga tgtgggggaa aagtaacatc aacactgtcc catcctcagc 2974
   cctgaactag ctaccatctg atcccctcag acattctcag gattttacaa gactgtcaga 3034
   gtggggaacc cctcccatta aagatccggg caggactggg gacaggttgg aagtgtgatg 3094
   ggtggggggg tgggaggcat gggccggggg cagttctctc ctcacttgta aacttgtgta 3154
   gtttcacaga aaaaaaacaa aatgcagttt taaataaaga aatttctttt ttccctggga 3214
   aaaaaaaaaa aaaaaaa 3231

<210> 4
<211> 430
<212> PRT
<213> Homo sapiens

<400> 4

Met Asp Glu Arg Leu Leu Gly Pro Pro Pro Pro Gly Gly Gly Arg Gly
1               5                   10                  15

Gly Leu Gly Leu Val Ser Gly Glu Pro Gly Gly Pro Gly Glu Pro Pro
        20                  25                  30

Gly Gly Gly Asp Pro Gly Gly Gly Ser Gly Gly Val Pro Gly Gly Arg
    35                  40                  45

Gly Lys Gln Asp Ile Gly Asp Ile Leu Gln Gln Ile Met Thr Ile Thr
    50                  55                  60

Asp Gln Ser Leu Asp Glu Ala Gln Ala Lys Lys His Ala Leu Asn Cys
65                  70                  75                  80

His Arg Met Lys Pro Ala Leu Phe Ser Val Leu Cys Glu Ile Lys Glu
                85                  90                  95

Lys Thr Gly Leu Ser Ile Arg Ser Ser Gln Glu Glu Glu Pro Val Asp
        100                 105                 110

Pro Gln Leu Met Arg Leu Asp Asn Met Leu Leu Ala Glu Gly Val Ala
        115                 120                 125

Gly Pro Glu Lys Gly Gly Gly Ser Ala Ala Ala Ala Ala Ala Ala Ala
    130                 135                 140

Ala Ser Gly Gly Gly Val Ser Pro Asp Asn Ser Ile Glu His Ser Asp
145                 150                 155                 160

Tyr Arg Ser Lys Leu Ala Gln Ile Arg His Ile Tyr His Ser Glu Leu
                165                 170                 175

Glu Lys Tyr Glu Gln Ala Cys Asn Glu Phe Thr Thr His Val Met Asn
        180                 185                 190

Leu Leu Arg Glu Gln Ser Arg Thr Arg Pro Val Ala Pro Lys Glu Met
        195                 200                 205

Glu Arg Met Val Ser Ile Ile His Arg Lys Phe Ser Ala Ile Gln Met
    210                 215                 220

Gln Leu Lys Gln Ser Thr Cys Glu Ala Val Met Ile Leu Arg Ser Arg
225                 230                 235                 240

Phe Leu Asp Ala Arg Arg Lys Arg Arg Asn Phe Ser Lys Gln Ala Thr
                245                 250                 255

Glu Val Leu Asn Glu Tyr Phe Tyr Ser His Leu Ser Asn Pro Tyr Pro
        260                 265                 270

Ser Glu Glu Ala Lys Glu Glu Leu Ala Lys Lys Cys Gly Ile Thr Val
        275                 280                 285

Ser Gln Val Ser Asn Trp Phe Gly Asn Lys Arg Ile Arg Tyr Lys Lys
    290                 295                 300

Asn Ile Gly Lys Phe Gln Glu Glu Ala Asn Ile Tyr Ala Val Lys Thr
305                 310                 315                 320

Ala Val Ser Val Thr Gln Gly Gly His Ser Arg Thr Ser Ser Pro Thr
                325                 330                 335

Pro Pro Ser Ser Ala Gly Ser Gly Gly Ser Phe Asn Leu Ser Gly Ser
            340                 345                 350

Gly Asp Met Phe Leu Gly Met Pro Gly Leu Asn Gly Asp Ser Tyr Ser
        355                 360                 365

Ala Ser Gln Val Glu Ser Leu Arg His Ser Met Gly Pro Gly Gly Tyr
    370                 375                 380

Gly Asp Asn Leu Gly Gly Gly Gln Met Tyr Ser Pro Arg Glu Met Arg
385                 390                 395                 400

Ala Asn Gly Ser Trp Gln Glu Ala Val Thr Pro Ser Ser Val Thr Ser
                405                 410                 415

Pro Thr Glu Gly Pro Gly Ser Val His Ser Asp Thr Ser Asn
            420                 425                 430

<210>  5
<211>  4150
<212>  DNA
<213>  Homo sapiens

<220>
<221>  CDS
<222>  (160)..(714)
<223>  Grem1

<400>  5
actcggtgcg ccttccgcgg accgggcgac ccagtgcacg gccgccgcgt cactctcggt      60

cccgctgacc ccgcgccgag ccccggcggc tctggccgcg gccgcactca gcgccacgcg     120

tcgaaagcgc aggccccgag gacccgccgc actgacagt atg agc cgc aca gcc        174
                                           Met Ser Arg Thr Ala
                                           1               5

tac acg gtg gga gcc ctg ctt ctc ctc ttg ggg acc ctg ctg ccg gct       222
Tyr Thr Val Gly Ala Leu Leu Leu Leu Leu Gly Thr Leu Leu Pro Ala

10                  15                  20

gct gaa ggg aaa aag aaa ggg tcc caa ggt gcc atc ccc ccg cca gac      270
Ala Glu Gly Lys Lys Lys Gly Ser Gln Gly Ala Ile Pro Pro Pro Asp
            25                  30                  35

aag gcc cag cac aat gac tca gag cag act cag tcg ccc cag cag cct      318
Lys Ala Gln His Asn Asp Ser Glu Gln Thr Gln Ser Pro Gln Gln Pro
        40                  45                  50

ggc tcc agg aac cgg ggg cgg ggc caa ggg cgg ggc act gcc atg ccc      366
Gly Ser Arg Asn Arg Gly Arg Gly Gln Gly Arg Gly Thr Ala Met Pro
    55                  60                  65

ggg gag gag gtg ctg gag tcc agc caa gag gcc ctg cat gtg acg gag      414
Gly Glu Glu Val Leu Glu Ser Ser Gln Glu Ala Leu His Val Thr Glu
70                  75                  80                  85

cgc aaa tac ctg aag cga gac tgg tgc aaa acc cag ccg ctt aag cag      462
Arg Lys Tyr Leu Lys Arg Asp Trp Cys Lys Thr Gln Pro Leu Lys Gln
                90                  95                  100

acc atc cac gag gaa ggc tgc aac agt cgc acc atc atc aac cgc ttc      510
Thr Ile His Glu Glu Gly Cys Asn Ser Arg Thr Ile Ile Asn Arg Phe
            105                 110                 115

tgt tac ggc cag tgc aac tct ttc tac atc ccc agg cac atc cgg aag      558
Cys Tyr Gly Gln Cys Asn Ser Phe Tyr Ile Pro Arg His Ile Arg Lys
        120                 125                 130

gag gaa ggt tcc ttt cag tcc tgc tcc ttc tgc aag ccc aag aaa ttc      606
Glu Glu Gly Ser Phe Gln Ser Cys Ser Phe Cys Lys Pro Lys Lys Phe
    135                 140                 145

act acc atg atg gtc aca ctc aac tgc cct gaa cta cag cca cct acc      654
Thr Thr Met Met Val Thr Leu Asn Cys Pro Glu Leu Gln Pro Pro Thr
150                 155                 160                 165

aag aag aag aga gtc aca cgt gtg aag cag tgt cgt tgc ata tcc atc      702
Lys Lys Lys Arg Val Thr Arg Val Lys Gln Cys Arg Cys Ile Ser Ile
                170                 175                 180

gat ttg gat taa gccaaatcca ggtgcaccca gcatgtccta ggaatgcagc      754
Asp Leu Asp

cccaggaagt cccagaccta aaacaaccag attcttactt ggcttaaacc tagaggccag      814

aagaaccccc agctgcctcc tggcaggagc ctgcttgtgc gtagttcgtg tgcatgagtg      874

tggatgggtg cctgtgggtg tttttagaca ccagagaaaa cacagtctct gctagagagc      934

actccctatt ttgtaaacat atctgcttta atggggatgt accagaaacc cacctcaccc      994

cggctcacat ctaaaggggc ggggccgtgg tctggttctg actttgtgtt tttgtgccct      1054

cctggggacc agaatctcct ttcggaatga atgttcatgg aagaggctcc tctgagggca      1114

agagacctgt tttagtgctg cattcgacat ggaaaagtcc ttttaacctg tgcttgcatc      1174

ctcctttcct cctcctcctc acaatccatc tcttcttaag ttgatagtga ctatgtcagt      1234

ctaatctctt gtttgccaag gttcctaaat taattcactt aaccatgatg caaatgtttt      1294

tcattttgtg aagaccctcc agactctggg agaggctggt gtgggcaagg acaagcagga 1354
tagtggagtg agaaagggag ggtggagggt gaggccaaat caggtccagc aaaagtcagt 1414
agggacattg cagaagcttg aaaggccaat accagaacac aggctgatgc ttctgagaaa 1474
gtcttttcct agtatttaac agaacccaag tgaacagagg agaaatgaga ttgccagaaa 1534
gtgattaact ttggccgttg caatctgctc aaacctaaca ccaaactgaa aacataaata 1594
ctgaccactc ctatgttcgg acccaagcaa gttagctaaa ccaaaccaac tcctctgctt 1654
tgtccctcag gtggaaaaga gaggtagttt agaactctct gcataggggt gggaattaat 1714
caaaaacctc agaggctgaa attcctaata cctttccttt atcgtggtta tagtcagctc 1774
atttccattc cactatttcc cataatgctt ctgagagcca ctaacttgat tgataaagat 1834
cctgcctctg ctgagtgtac ctgacagtag tctaagatga gagagtttag ggactactct 1894
gttttagcaa gagatatttt gggggtcttt ttgttttaac tattgtcagg agattgggct 1954
aaagagaaga cgacgagagt aaggaaataa agggaattgc ctctggctag agagtagtta 2014
ggtgttaata cctggtagag atgtaaggga tatgacctcc ctttctttat gtgctcactg 2074
aggatctgag gggaccctgt taggagagca tagcatcatg atgtattagc tgttcatctg 2134
ctactggttg gatggacata actattgtaa ctattcagta tttactggta ggcactgtcc 2194
tctgattaaa cttggcctac tggcaatggc tacttaggat tgatctaagg gccaaagtgc 2254
agggtgggtg aactttattg tactttggat ttggttaacc tgttttcttc aagcctgagg 2314
ttttatatac aaactccctg aatactcttt ttgccttgta tcttctcagc ctcctagcca 2374
agtcctatgt aatatggaaa acaaacactg cagacttgag attcagttgc cgatcaaggc 2434
tctggcattc agagaaccct tgcaactcga gaagctgttt ttatttcgtt tttgttttga 2494
tccagtgctc tcccatctaa caactaaaca ggagccattt caaggcggga gatattttaa 2554
acacccaaaa tgttgggtct gattttcaaa cttttaaact cactactgat gattctcacg 2614
ctaggcgaat ttgtccaaac acatagtgtg tgtgttttgt atacactgta tgaccccacc 2674
ccaaatcttt gtattgtcca cattctccaa caataaagca cagagtggat ttaattaagc 2734
acacaaatgc taaggcagaa ttttgagggt gggagagaag aaaagggaaa gaagctgaaa 2794
atgtaaaacc acaccaggga ggaaaaatga cattcagaac cagcaaacac tgaatttctc 2854
ttgttgtttt aactctgcca caagaatgca atttcgttaa cggagatgac ttaagttggc 2914
agcagtaatc ttcttttagg agcttgtacc acagtcttgc acataagtgc agatttggct 2974
caagtaaaga gaatttcctc aacactaact tcactgggat aatcagcagc gtaactaccc 3034
taaaagcata tcactagcca aagagggaaa tatctgttct tcttactgtg cctatattaa 3094
gactagtaca aatgtggtgt gtcttccaac tttcattgaa aatgccatat ctataccata 3154

ttttattcga gtcactgatg atgtaatgat atattttttc attattatag tagaatattt 3214

ttatggcaag atatttgtgg tcttgatcat acctattaaa ataatgccaa acaccaaata 3274

tgaattttat gatgtacact ttgtgcttgg cattaaaaga aaaaaacaca catcctggaa 3334

gtctgtaagt tgttttttgt tactgtaggt cttcaaagtt aagagtgtaa gtgaaaaatc 3394

tggaggagag gataatttcc actgtgtgga atgtgaatag ttaaatgaaa agttatggtt 3454

atttaatgta attattactt caaatccttt ggtcactgtg atttcaagca tgttttcttt 3514

ttctccttta tatgactttc tctgagttgg gcaaagaaga agctgacaca ccgtatgttg 3574

ttagagtctt ttatctggtc aggggaaaca aaatcttgac ccagctgaac atgtcttcct 3634

gagtcagtgc ctgaatcttt attttttaaa ttgaatgttc cttaaaggtt aacatttcta 3694

aagcaatatt aagaaagact ttaaatgtta ttttggaaga cttacgatgc atgtatacaa 3754

acgaatagca gataatgatg actagttcac acataaagtc cttttaagga gaaaatctaa 3814

aatgaaaagt ggataaacag aacatttata agtgatcagt taatgcctaa gagtgaaagt 3874

agttctattg acattcctca agatatttaa tatcaactgc attatgtatt atgtctgctt 3934

aaatcattta aaaacggcaa agaattatat agactatgag gtaccttgct gtgtaggagg 3994

atgaaagggg agttgatagt ctcataaaac taatttggct tcaagtttca tgaatctgta 4054

actagaattt aattttcacc ccaataatgt tctatatagc ctttgctaaa gagcaactaa 4114

taaattaaac ctattctttc tgtgaaaaaa aaaaaa 4150

<210> 6
<211> 184
<212> PRT
<213> Homo sapiens

<400> 6

Met Ser Arg Thr Ala Tyr Thr Val Gly Ala Leu Leu Leu Leu Leu Gly
1               5                   10                  15

Thr Leu Leu Pro Ala Ala Glu Gly Lys Lys Lys Gly Ser Gln Gly Ala
            20                  25                  30

Ile Pro Pro Pro Asp Lys Ala Gln His Asn Asp Ser Glu Gln Thr Gln
        35                  40                  45

Ser Pro Gln Gln Pro Gly Ser Arg Asn Arg Gly Arg Gly Gln Gly Arg
    50                  55                  60

Gly Thr Ala Met Pro Gly Glu Glu Val Leu Glu Ser Ser Gln Glu Ala
65                  70                  75                  80

Leu His Val Thr Glu Arg Lys Tyr Leu Lys Arg Asp Trp Cys Lys Thr

85                  90                  95

Gln Pro Leu Lys Gln Thr Ile His Glu Glu Gly Cys Asn Ser Arg Thr
            100                 105                 110

Ile Ile Asn Arg Phe Cys Tyr Gly Gln Cys Asn Ser Phe Tyr Ile Pro
        115                 120                 125

Arg His Ile Arg Lys Glu Glu Gly Ser Phe Gln Ser Cys Ser Phe Cys
    130                 135                 140

Lys Pro Lys Lys Phe Thr Thr Met Met Val Thr Leu Asn Cys Pro Glu
145                 150                 155                 160

Leu Gln Pro Pro Thr Lys Lys Lys Arg Val Thr Arg Val Lys Gln Cys
                165                 170                 175

Arg Cys Ile Ser Ile Asp Leu Asp
            180

<210>  7
<211>  652
<212>  DNA
<213>  Homo sapiens

<220>
<221>  misc_feature
<223>  Ly6g6e

<400>  7
agagacaaca tgctgagaga gcagaggaga ccctccaggc aagggcagac tccttgcagg      60

ggcaggctgg gggcccccgc tgcctgctgg gtcaggctgg tgaatctggt catggttccg     120

ccccccagat tcactcccta ggtgtgtttg tttactggtt cctcactgtc ttgctcaaat     180

gctccaactc tacaaatccc gggatctcgg ggtgcagatc acctctccca gattcctgag     240

cctgtgtctg gccatgggca cctccagcat cttcctctgc gtgctgttcc tctgtggggc     300

actgggtctc accatgtccc ctgcccgggg aaggctccgc tgctacatct gtggcttcac     360

caaaccctgc caccctgttc ccaccgagtg tcgggacgat gaagcttgtg gcatcagtat     420

tggcacttca gaccagagtg agatcactga gtgaaaaagc tgcctctcaa gggcccagtg     480

ccctctgcca ggctatgcca cctactggct gcactcctac actctgtggc accactgctg     540

cgagcaggac ctgtgcaaca tagccgcttc cccacagcag ctcaccagcc tcctcgcctc     600

cctgcccctc tttgtggcca gcttcgctgg gagaggacac ctcctccact ag             652

<210>  8
<211>  2533

<212>  DNA
<213>  Homo sapiens

<220>
<221>  CDS
<222>  (115)..(936)
<223>  Olr1

<400>  8
   attcttctat tagataacag tagctattta aatacttctg cagaagctca catattttta      60
   gtttgttgaa gttcgtgact gcttcactct ctcattctta gcttgaattt ggaa atg      117
                                                               Met
                                                               1
   act ttt gat gac cta aag atc cag act gtg aag gac cag cct gat gag      165
   Thr Phe Asp Asp Leu Lys Ile Gln Thr Val Lys Asp Gln Pro Asp Glu
               5                   10                  15
   aag tca aat gga aaa aaa gct aaa ggt ctt cag ttt ctt tac tct cca      213
   Lys Ser Asn Gly Lys Lys Ala Lys Gly Leu Gln Phe Leu Tyr Ser Pro
           20                  25                  30
   tgg tgg tgc ctg gct gct gcg act cta ggg gtc ctt tgc ctg gga tta      261
   Trp Trp Cys Leu Ala Ala Ala Thr Leu Gly Val Leu Cys Leu Gly Leu
       35                  40                  45
   gta gtg acc att atg gtg ctg ggc atg caa tta tcc cag gtg tct gac      309
   Val Val Thr Ile Met Val Leu Gly Met Gln Leu Ser Gln Val Ser Asp
   50                  55                  60                  65
   ctc cta aca caa gag caa gca aac cta act cac cag aaa aag aaa ctg      357
   Leu Leu Thr Gln Glu Gln Ala Asn Leu Thr His Gln Lys Lys Lys Leu
                   70                  75                  80
   gag gga cag atc tca gcc cgg caa caa gca gaa gaa gct tca cag gag      405
   Glu Gly Gln Ile Ser Ala Arg Gln Gln Ala Glu Glu Ala Ser Gln Glu
               85                  90                  95
   tca gaa aac gaa ctc aag gaa atg ata gaa acc ctt gct cgg aag ctg      453
   Ser Glu Asn Glu Leu Lys Glu Met Ile Glu Thr Leu Ala Arg Lys Leu
           100                 105                 110
   aat gag aaa tcc aaa gag caa atg gaa ctt cac cac cag aat ctg aat      501
   Asn Glu Lys Ser Lys Glu Gln Met Glu Leu His His Gln Asn Leu Asn
       115                 120                 125
   ctc caa gaa aca ctg aag aga gta gca aat tgt tca gct cct tgt ccg      549
   Leu Gln Glu Thr Leu Lys Arg Val Ala Asn Cys Ser Ala Pro Cys Pro
   130                 135                 140                 145
   caa gac tgg atc tgg cat gga gaa aac tgt tac cta ttt tcc tcg ggc      597
   Gln Asp Trp Ile Trp His Gly Glu Asn Cys Tyr Leu Phe Ser Ser Gly
                   150                 155                 160
   tca ttt aac tgg gaa aag agc caa gag aag tgc ttg tct ttg gat gcc      645
   Ser Phe Asn Trp Glu Lys Ser Gln Glu Lys Cys Leu Ser Leu Asp Ala
               165                 170                 175
   aag ttg ctg aaa att aat agc aca gct gat ctg gac ttc atc cag caa      693
   Lys Leu Leu Lys Ile Asn Ser Thr Ala Asp Leu Asp Phe Ile Gln Gln
           180                 185                 190
   gca att tcc tat tcc agt ttt cca ttc tgg atg ggg ctg tct cgg agg 741
   Ala Ile Ser Tyr Ser Ser Phe Pro Phe Trp Met Gly Leu Ser Arg Arg
   195 200 205
   aac ccc agc tac cca tgg ctc tgg gag gac ggt tct cct ttg atg ccc 789
   Asn Pro Ser Tyr Pro Trp Leu Trp Glu Asp Gly Ser Pro Leu Met Pro
   210 215 220 225
   cac tta ttt aga gtc cga ggc gct gtc tcc cag aca tac cct tca ggt 837
   His Leu Phe Arg Val Arg Gly Ala Val Ser Gln Thr Tyr Pro Ser Gly
   230 235 240
   acc tgt gca tat ata caa cga gga gct gtt tat gcg gaa aac tgc att 885
   Thr Cys Ala Tyr Ile Gln Arg Gly Ala Val Tyr Ala Glu Asn Cys Ile
   245 250 255
   tta gct gcc ttc agt ata tgt cag aag aag gca aac cta aga gca cag 933
   Leu Ala Ala Phe Ser Ile Cys Gln Lys Lys Ala Asn Leu Arg Ala Gln
   260 265 270
   tga atttgaaggc tctggaagaa aagaaaaaag tctttgagtt ttattctgga 986
   atttaagcta ttctttgtca cttgggtgcc aaacatgaga gcccagaaaa ctgtcattta 1046
   gctggctgca gaactccttt gcagaaactg gggttccagg tgcctggcac ctttatgtca 1106
   acatttttga ttctagctac ctgtattatt tcacctagct tgtcccaagc ttccctgcca 1166
   gcctgaagtc cattttcccc tttttatttt aaaatttgac tcctcttcaa gcttgaaaac 1226
   cctctgaact cagtcttctt tacctcatta tcaccttccc ctcacactcc taaaattgca 1286
   tgaaagacag aacatggaga acttgctcaa gtgcaggcag agagcaaaaa ggggaaatat 1346
   gtctgggaaa aagtgcacgt gaagaaacaa agaaggacag aggccattcc gaaatcaaga 1406
   aactcatgtt cttaacttta aaaaaggtat caatccttgg tttttaaact gtggtccatc 1466
   tccagactct accacttacg gacagacaga cagacagaca cacacacaca cacacacaca 1526
   cattttggga caagtgggga gcccaagaaa gtaattagta agtgagtggt cttttctgta 1586
   agctaatcca caacctgtta ccacttcctg aatcagttat tatttcttca tttttttttc 1646
   taccagagga cagattaata gatttaaccc ttcacaacag ttcttgttag aatcatggga 1706
   tgtgtggccc agaggtaaga atagaatttc tttccctaaa gaacatacct tttgtagatg 1766
   aactcttctc aactctgttt tgctatgcta taattccgaa acatacaaga caaaaaaaat 1826
   gaagacactc aatctagaac aaactaagcc aggtatgcaa atatcgctga atagaaacag 1886
   atggaattag aaatatatct tctattttta ggcttctatt tcctttccac ccactcttca 1946
   caggctattc tactttaaag gaagcctttt tattttgctg cacacaatct agcaggaatc 2006
   tttttttttt tttaagagct gtgtcatcct tatgtaggca agagatgttt gcttttgtta 2066
   aaagctttat tgagatataa ttaacataaa ataaactgaa catatttaaa gtgtactatt 2126
   tgataagttt tcacaccttg tggagaacat gcatactaca attaagagag tgaacatatc 2186
   catcatccct caaagtgtca caatgctcct cctgatgact cctccccaga aaaccaccaa 2246
   tcggctttca ttttgcattt tgtagtttta tgtgaatgga atcatatagt atgtcttttt 2306
   tttttgtctg gcttctttca ctttgcataa ttattttgag attcatatgt ctccatcttg 2366
   atgctcgtat gaattcattc ttttaaatgt tgaatattcc cttgtatgga tataccacaa 2426
   ttcatttacc catttacttg ttgatgacat ttgggttgtt ttagttttgg gatattacaa 2486
   ataaagctgc tgtgaacatt tgtgtacaag aaaaaaaaaa aaaaaaa 2533

<210> 9
<211> 273
<212> PRT
<213> Homo sapiens

<400> 9

Met Thr Phe Asp Asp Leu Lys Ile Gln Thr Val Lys Asp Gln Pro Asp
1               5                   10                  15

Glu Lys Ser Asn Gly Lys Lys Ala Lys Gly Leu Gln Phe Leu Tyr Ser
            20                  25                  30

Pro Trp Trp Cys Leu Ala Ala Ala Thr Leu Gly Val Leu Cys Leu Gly
        35                  40                  45

Leu Val Val Thr Ile Met Val Leu Gly Met Gln Leu Ser Gln Val Ser
    50                  55                  60

Asp Leu Leu Thr Gln Glu Gln Ala Asn Leu Thr His Gln Lys Lys Lys
65                  70                  75                  80

Leu Glu Gly Gln Ile Ser Ala Arg Gln Gln Ala Glu Glu Ala Ser Gln
                85                  90                  95

Glu Ser Glu Asn Glu Leu Lys Glu Met Ile Glu Thr Leu Ala Arg Lys
            100                 105                 110

Leu Asn Glu Lys Ser Lys Glu Gln Met Glu Leu His His Gln Asn Leu
        115                 120                 125

Asn Leu Gln Glu Thr Leu Lys Arg Val Ala Asn Cys Ser Ala Pro Cys
    130                 135                 140

Pro Gln Asp Trp Ile Trp His Gly Glu Asn Cys Tyr Leu Phe Ser Ser
145                 150                 155                 160

Gly Ser Phe Asn Trp Glu Lys Ser Gln Glu Lys Cys Leu Ser Leu Asp
                165                 170                 175

Ala Lys Leu Leu Lys Ile Asn Ser Thr Ala Asp Leu Asp Phe Ile Gln
            180                 185                 190

Gln Ala Ile Ser Tyr Ser Ser Phe Pro Phe Trp Met Gly Leu Ser Arg
        195                 200                 205

Arg Asn Pro Ser Tyr Pro Trp Leu Trp Glu Asp Gly Ser Pro Leu Met
    210                 215                 220

Pro His Leu Phe Arg Val Arg Gly Ala Val Ser Gln Thr Tyr Pro Ser
225                 230                 235                 240

Gly Thr Cys Ala Tyr Ile Gln Arg Gly Ala Val Tyr Ala Glu Asn Cys
                245                 250                 255

Ile Leu Ala Ala Phe Ser Ile Cys Gln Lys Lys Ala Asn Leu Arg Ala
            260                 265                 270

Gln

<210>  10
<211>  1185
<212>  DNA
<213>  Homo sapiens

<220>
<221>  CDS
<222>  (325)..(735)
<223>  Spr1

<400>  10
gtgattacgg agatgccaag tgggtattga ctgctccagg atgtggatgg agggtgtgaa      60

aaccagggtg gggtgacgca ggctctgggt catgataggg agagcaggca gctgggtcct     120

gggctggagg actaaaataa gggacgccac cttcaggggt gacacatcag cccaggcctt     180

cccaacgggt ttgaccagtt ctgttctgat ggtattcctg tgccactggg ctggtccctc     240

ctccactcct cccctataaa gcctcttggg gttcccaggc acccagactc agcccacccc     300

agctttgggg gccagtacat agcc atg atc ctc aac tgg aag ctc ctg ggg        351
                           Met Ile Leu Asn Trp Lys Leu Leu Gly
                           1               5

atc ctg gtc ctt tgc ctg cac acc aga ggc atc tca ggc agc gag ggc       399
Ile Leu Val Leu Cys Leu His Thr Arg Gly Ile Ser Gly Ser Glu Gly
10                  15                  20                  25

cac ccc tct cac cca ccc gca gag gac cga gag gag gca ggc tcc cca       447
His Pro Ser His Pro Pro Ala Glu Asp Arg Glu Glu Ala Gly Ser Pro
                30                  35                  40

aca ttg cct cag ggc ccc cca gtc ccc ggt gac cct tgg cca ggg gca     495
Thr Leu Pro Gln Gly Pro Pro Val Pro Gly Asp Pro Trp Pro Gly Ala
            45                  50                  55

ccc cct ctc ttt gaa gat cct ccg cct acc cgc ccc agt cgt ccc tgg     543
Pro Pro Leu Phe Glu Asp Pro Pro Pro Thr Arg Pro Ser Arg Pro Trp
        60                  65                  70

aga gac ctg cct gaa act gga gtc tgg ctc cct gaa ccg cct aga acg     591
Arg Asp Leu Pro Glu Thr Gly Val Trp Leu Pro Glu Pro Pro Arg Thr
    75                  80                  85

gat cct cct caa cct ccc cgg cct gac gac cct tgg ccg gca gga ccc     639
Asp Pro Pro Gln Pro Pro Arg Pro Asp Asp Pro Trp Pro Ala Gly Pro
90                  95                  100                 105

cag ccc cca gaa aac ccc tgg cct cct gcc cct gag gtg gac aac cga     687
Gln Pro Pro Glu Asn Pro Trp Pro Pro Ala Pro Glu Val Asp Asn Arg
                110                 115                 120

cct cag gag gag cca gac cta gac cca ccc cgg gaa gag tac aga taa     735
Pro Gln Glu Glu Pro Asp Leu Asp Pro Pro Arg Glu Glu Tyr Arg
            125                 130                 135

tggagtcccc tcagccgttc tgttcccagg catctccagg cacccacgcc ctctccaccc     795

tctgattccc cgtgaattct tcccaattta gcctgtctcc ttaaacctct tcctcattcc     855

ctcggtttta ttctgaaccc gtaaggtggt gttctcaata tttcctgtcc cctcctgaga     915

tccatactta gtcctcacat cgcccgtttt ttcctctgac agcctaagcc tactctccta     975

cctcgcctcc aggcctcggc cgcacctacc tcccacccgg tcttcctgcc cgcgcgatcg     1035

ctggggcagg gctatggtac tgtgttccct tctgccacct ggtggccggc ggcaggaact     1095

atcagtagac agctgctgct tccatgaaac ggaaaaataa aaatcatgtt ttcttaaaaa     1155

aaaaaaaaaa aaaaaaaaaa aaaaaaaaaa                                      1185


<210>  11
<211>  136
<212>  PRT
<213>  Homo sapiens

<400>  11

Met Ile Leu Asn Trp Lys Leu Leu Gly Ile Leu Val Leu Cys Leu His
1               5                   10                  15


Thr Arg Gly Ile Ser Gly Ser Glu Gly His Pro Ser His Pro Pro Ala
            20                  25                  30


Glu Asp Arg Glu Glu Ala Gly Ser Pro Thr Leu Pro Gln Gly Pro Pro
        35                  40                  45


Val Pro Gly Asp Pro Trp Pro Gly Ala Pro Pro Leu Phe Glu Asp Pro
    50                  55                  60

Pro Pro Thr Arg Pro Ser Arg Pro Trp Arg Asp Leu Pro Glu Thr Gly
65                  70                  75                  80

Val Trp Leu Pro Glu Pro Pro Arg Thr Asp Pro Pro Gln Pro Pro Arg
                85                  90                  95

Pro Asp Asp Pro Trp Pro Ala Gly Pro Gln Pro Pro Glu Asn Pro Trp
            100                 105                 110

Pro Pro Ala Pro Glu Val Asp Asn Arg Pro Gln Glu Glu Pro Asp Leu
        115                 120                 125

Asp Pro Pro Arg Glu Glu Tyr Arg
    130                 135

<210>  12
<211>  3761
<212>  DNA
<213>  Homo sapiens

<220>
<221>  CDS
<222>  (199)..(1554)
<223>  Msr1

<400>  12
aaatttagat tttgcaaacc tgtgcattga tgagagtgct attgaaacac attaagaaag      60
attttcaacg caggaatgtg tcatttcctt tcttcatgta ccagatgctg aaatactatg     120
agataaagat tttaggtttc aattgtaaag agagagaagt ggataaatca gtgctgcttt     180
ctttaggacg aaagaagt atg gag cag tgg gat cac ttt cac aat caa cag       231
gag gac act gat agc tgc tcc gaa tct gtg aaa ttt gat gct cgc tca       279
atg aca gct ttg ctt cct ccg aat cct aaa aac agc cct tcc ctt caa       327
gag aaa ctg aag tcc ttc aaa gct gca ctg att gcc ctt tac ctc ctc       375
gtg ttt gca gtt ctc atc cct ctc att gga ata gtg gca gct caa ctc       423
ctg aag tgg gaa acg aag aat tgc tca gtt agt tca act aat gca aat       471
gat ata act caa agt ctc acg gga aaa gga aat gac agc gaa gag gaa 519
atg aga ttt caa gaa gtc ttt atg gaa cac atg agc aac atg gag aag 567
aga atc cag cat att tta gac atg gaa gcc aac ctc atg gac aca gag 615
cat ttc caa aat ttc agc atg aca act gat caa aga ttt aat gac att 663
ctt ctg cag cta agt acc ttg ttt tcc tca gtc cag gga cat ggg aat 711
gca ata gat gaa atc tcc aag tcc tta ata agt ttg aat acc aca ttg 759
ctt gat ttg cag ctc aac ata gaa aat ctg aat ggc aaa atc caa gag 807
aat acc ttc aaa caa caa gag gaa atc agt aaa tta gag gag cgt gtt 855
tac aat gta tca gca gaa att atg gct atg aaa gaa gaa caa gtg cat 903
ttg gaa cag gaa ata aaa gga gaa gtg aaa gta ctg aat aac atc act 951
aat gat ctc aga ctg aaa gat tgg gaa cat tct cag acc ttg aga aat 999
atc act tta att caa ggt cct cct gga ccc ccg ggt gaa aaa gga gat 1047
cga ggt ccc act gga gaa agt ggt cca cga gga ttt cca ggt cca ata 1095
ggt cct ccg ggt ctt aaa ggt gat cgg gga gca att ggc ttt cct gga 1143
agt cga gga ctc cca gga tat gcc gga agg cca gga aat tct gga cca 1191
aaa ggc cag aaa ggg gaa aag ggg agt gga aac aca tta act cca ttt 1239
acg aaa gtt cga ctg gtc ggt ggg agc ggc cct cac gag ggg agg gtg     1287
gag ata ctc cac agc ggc cag tgg ggt aca att tgt gac gat cgc tgg     1335
gaa gtg cgc gtt gga cag gtc gtc tgt agg agc ttg gga tac cca ggt     1383
gtt caa gcc gtg cac aag gca gct cac ttt gga caa ggt act ggt cca     1431
ata tgg ctg aat gaa gtg ttt tgt ttt ggg aga gaa tca tct att gaa     1479
gaa tgt aaa att cgg caa tgg ggg aca aga gcc tgt tca cat tct gaa     1527
gat gct gga gtc act tgc act tta taa tgcatcatat tttcattcac     1574
aactatgaaa tcgctgctca aaaatgattt tattaccttg ttcctgtaaa atccatttaa     1634
tcaatattta agagattaag aatattgccc aaataatatt ttagattaca ggattaatat     1694
attgaacacc ttcatgctta ctattttatg tctatattta aatcatttta acttctatag     1754
gtttttaaat ggaattttct aatataatga cttatatgct gaattgaaca ttttgaagtt     1814
tatagcttcc agattacaaa ggccaagggt aatagaaatg cataccagta attggctcca     1874
attcataata tgttcaccag gagattacaa ttttttgctc ttcttgtctt tgtaatctat     1934
ttagttgatt ttaattactt tctgaataac ggaagggatc agaagatatc ttttgtgcct     1994
agattgcaaa atctccaatc cacacatatt gttttaaaat aagaatgtta tccaactatt     2054
aagatatctc aatgtgcaat aacttgtgta ttagatatca atgttaatga tatgtcttgg     2114
ccactatgga ccagggagct tatttttctt gtcatgtact gacaactgtt taattgaatc     2174
atgaagtaaa ttgaaagcag gacatatgag aaaactgacc atcagtatat ttgtccagat     2234
aattggtgga tcaaaaatgc cacttaacag gaagtttagt ttgttatgca ctttaaatgg     2294
aataattagc ttgttacaat tctaggacat ggtgtttaaa atttaaatct gattaatcca     2354
ttttaacaaa caatgcaaac atcttcagtg cagaaggaag agtggtttca actgtttgga     2414
gtcttttatg aagtcagtca acatttacaa ccaaagggcg gggggggggg tggggggtgc     2474
gtctttagtc ctaaagggac aataactctg agcatgcccc aaaaaagtag tttagcaacc     2534
ttttgttggt agtcaaccca tccccagggc catagtgtag agtgtgaaaa gctaccctga     2594
aacccagtaa ttctaccctg aaagtgactg cctgcagaaa gaccagcagt tgatattaaa 2654
gcgcaaatga attcaacctc agccctgaaa ataacagaat tctgaagttt cctatgacta 2714
attcacaaaa aaagtaattg taaactagta ctattatgga attactctac tgttctttct 2774
ttaatagtgg caaatgaaag cataagctta agcatttttt catattctga agtctcacca 2834
cacataataa ccaagtggta gactcacagc cgtccaactt aaaaaggcaa aaccttacct 2894
tggaattgga attactgtaa acagcctact gaaaatgcat ttttatcatg taacattctt 2954
ctacttgttt aacattgctg attttctctg gcagcataat tttgtggtta agagaatgaa 3014
ttctgaatgt acactttctg tctcaaaccc tggctgtaat ttcagctagt taataattct 3074
ttgtgttcag ttccactatc taggtatttt cttcaaaagg taaatacaat ggtttctgaa 3134
agaatcattt gcattatcag cctgtttggg atgtctgaga tcagtgcctc tgggttgtta 3194
atactgtatt gctgtatggt atatgtatgc tgatttacta cttatgcgta agtggtatgc 3254
atgggatgtc tgaaatcagt gcctatgggt tgtcaatagt attaactatt agtgttaact 3314
gttagtatta actattagta ttattaacac taataatagt actattacta ttactatttt 3374
tattttaaaa taaaatttac ctttaaaata ataatagtac tattgctagt actagtacta 3434
ttgctattac tagtactatt actagtacta gtactatgac actgttaata gtactattaa 3494
caacccatag gcacttggga tgtctgagat cagtgcctat gggttgttaa tactatattg 3554
ctgtatggta tatgcatgct gatttaccac ttatgcatag atatatcttt aataagtaat 3614
ctaaaaatcc tttttgtatt tgagagaatc tactaagttc agtccagtca agaaaagaac 3674
ctaatagcac caatacaaat tgaggactta atttactttg gaatgttgaa ttgcatttgt 3734
tccattaaaa aaaacagaaa tttgcga 3761

<210> 13
<211> 451
<212> PRT
<213> Homo sapiens

<400> 13

<210>  14
<211>  1116
<212>  DNA
<213>  Homo sapiens

<220>
<221>  CDS
<222>  (27)..(773)
<223>  Spic

<400>  14
cagaattgtc aatttattaa tgaaat atg acg tgt gtt gaa caa gac aag ctg        53
                             Met Thr Cys Val Glu Gln Asp Lys Leu
                             1               5

ggt caa gca ttt gaa gat gct ttt gag gtt ctg agg caa cat tca act        101
Gly Gln Ala Phe Glu Asp Ala Phe Glu Val Leu Arg Gln His Ser Thr
10                  15                  20                  25

gga gat ctt cag tac tcg cca gat tac aga aat tac ctg gct tta atc        149
Gly Asp Leu Gln Tyr Ser Pro Asp Tyr Arg Asn Tyr Leu Ala Leu Ile
                30                  35                  40

aac cat cgt cct cat gtc aaa gga aat tcc agc tgc tat gga gtg ttg        197

Asn His Arg Pro His Val Lys Gly Asn Ser Ser Cys Tyr Gly Val Leu
            45                  50                  55

cct aca gag gag cct gtc tat aat tgg aga acg gta att aac agt gct      245
Pro Thr Glu Glu Pro Val Tyr Asn Trp Arg Thr Val Ile Asn Ser Ala
        60                  65                  70

gcg gac ttc tat ttt gaa gga aat att cat caa tct ctg cag aac ata      293
Ala Asp Phe Tyr Phe Glu Gly Asn Ile His Gln Ser Leu Gln Asn Ile
    75                  80                  85

act gaa aac cag ctg gta caa ccc act ctt ctc cag caa aag ggg gga      341
Thr Glu Asn Gln Leu Val Gln Pro Thr Leu Leu Gln Gln Lys Gly Gly
90                  95                  100                 105

aaa ggc agg aag aag ctc cga ctg ttt gaa tac ctt cac gaa tcc ctg      389
Lys Gly Arg Lys Lys Leu Arg Leu Phe Glu Tyr Leu His Glu Ser Leu
                110                 115                 120

tat aat ccg gag atg gca tct tgt att cag tgg gta gat aaa acc aaa      437
Tyr Asn Pro Glu Met Ala Ser Cys Ile Gln Trp Val Asp Lys Thr Lys
            125                 130                 135

ggc atc ttt cag ttt gta tca aaa aac aaa gaa aaa ctt gcc gag ctt      485
Gly Ile Phe Gln Phe Val Ser Lys Asn Lys Glu Lys Leu Ala Glu Leu
        140                 145                 150

tgg ggg aaa aga aaa ggc aac agg aag acc atg act tac cag aaa atg      533
Trp Gly Lys Arg Lys Gly Asn Arg Lys Thr Met Thr Tyr Gln Lys Met
    155                 160                 165

gcc agg gca ctc aga aat tac gga aga agt ggg gaa att acc aaa atc      581
Ala Arg Ala Leu Arg Asn Tyr Gly Arg Ser Gly Glu Ile Thr Lys Ile
170                 175                 180                 185

cgg agg aag ctg act tac cag ttc agt gag gcc att ctc caa aga ctc      629
Arg Arg Lys Leu Thr Tyr Gln Phe Ser Glu Ala Ile Leu Gln Arg Leu
                190                 195                 200

tct cca tcc tat ttc ctg ggg aaa gag atc ttc tat tca cag tgt gtt      677
Ser Pro Ser Tyr Phe Leu Gly Lys Glu Ile Phe Tyr Ser Gln Cys Val
            205                 210                 215

caa cct gat caa gaa tat ctc agt tta aat aac tgg aat gca aat tat      725
Gln Pro Asp Gln Glu Tyr Leu Ser Leu Asn Asn Trp Asn Ala Asn Tyr
        220                 225                 230

aat tat aca tat gcc aat tac cat gag cta aat cac cat gat tgc taa      773
Asn Tyr Thr Tyr Ala Asn Tyr His Glu Leu Asn His His Asp Cys
    235                 240                 245

atatactttc atatttcatg gtttactggc atcggaaatc tctacaagtt ttaatgattt      833

ctccctccct ctcttttttt cctcctctga agaaatttag gatttttctc ttaaagcaaa      893

tactaaagag gaaaaaaaat taactttatt gttgctttta tcaaagagta tgtaatctat      953

actaacttgt tgggaaattc tgccaatgaa caactttttt ataataaaaa aaaaaaaaaa     1013

aaaaaaaaaa aaaaaaaaaa aaaaaaaaaa aaaaaaaaaa aaaaaaaaaa aaaaaaaaaa     1073

aaaaaaaaaa aaaaaaaaaa aaaaaaaaaa aaaaaaaaaa aaa                       1116

<210>  15
<211>  248
<212>  PRT
<213>  Homo sapiens

<400>  15

Met Thr Cys Val Glu Gln Asp Lys Leu Gly Gln Ala Phe Glu Asp Ala
1               5                   10                  15

Phe Glu Val Leu Arg Gln His Ser Thr Gly Asp Leu Gln Tyr Ser Pro
            20                  25                  30

Asp Tyr Arg Asn Tyr Leu Ala Leu Ile Asn His Arg Pro His Val Lys
        35                  40                  45

Gly Asn Ser Ser Cys Tyr Gly Val Leu Pro Thr Glu Glu Pro Val Tyr
    50                  55                  60

Asn Trp Arg Thr Val Ile Asn Ser Ala Ala Asp Phe Tyr Phe Glu Gly
65                  70                  75                  80

Asn Ile His Gln Ser Leu Gln Asn Ile Thr Glu Asn Gln Leu Val Gln
                85                  90                  95

Pro Thr Leu Leu Gln Gln Lys Gly Gly Lys Gly Arg Lys Lys Leu Arg
            100                 105                 110

Leu Phe Glu Tyr Leu His Glu Ser Leu Tyr Asn Pro Glu Met Ala Ser
        115                 120                 125

Cys Ile Gln Trp Val Asp Lys Thr Lys Gly Ile Phe Gln Phe Val Ser
    130                 135                 140

Lys Asn Lys Glu Lys Leu Ala Glu Leu Trp Gly Lys Arg Lys Gly Asn
145                 150                 155                 160

Arg Lys Thr Met Thr Tyr Gln Lys Met Ala Arg Ala Leu Arg Asn Tyr
                165                 170                 175

Gly Arg Ser Gly Glu Ile Thr Lys Ile Arg Arg Lys Leu Thr Tyr Gln
            180                 185                 190

Phe Ser Glu Ala Ile Leu Gln Arg Leu Ser Pro Ser Tyr Phe Leu Gly
        195                 200                 205

Lys Glu Ile Phe Tyr Ser Gln Cys Val Gln Pro Asp Gln Glu Tyr Leu
    210                 215                 220

Ser Leu Asn Asn Trp Asn Ala Asn Tyr Asn Tyr Thr Tyr Ala Asn Tyr
225                 230                 235                 240

His Glu Leu Asn His His Asp Cys
                245

<210>  16
<211>  1663
<212>  DNA
<213>  Homo sapiens

<220>
<221>  CDS
<222>  (264)..(1385)
<223>  Nfe2

<400>  16
   gtgcgcctgc ttggggctcc tgtgctcagc tcagcctgag cttccacact cagcgctcag      60
   caatggcccg ggggcggggc gcggtcctcg cagattctca aaggtagccg ggatcctcgt      120
   ccagcagtgt cagctcaggc tcagcctccc cagagacaac accgggagcc tcatctctct      180
   cctcaccctg ctgtgactcc accacaggtt tctagagcca tctgggcttt ccgggaacct      240
   ggaccagact ctggcccagt agg atg tcc ccg tgt cct ccc cag cag agc agg      293
                             Met Ser Pro Cys Pro Pro Gln Gln Ser Arg
                             1               5                   10
   aac agg gtg ata cag ctg tcc act tca gag cta gga gag atg gaa ctg      341
   Asn Arg Val Ile Gln Leu Ser Thr Ser Glu Leu Gly Glu Met Glu Leu
                   15                  20                  25
   act tgg cag gag atc atg tcc atc acc gag ctg cag ggt ctg aat gct      389
   Thr Trp Gln Glu Ile Met Ser Ile Thr Glu Leu Gln Gly Leu Asn Ala
               30                  35                  40
   cca agt gag cca tca ttt gag ccc caa gcc cca gct cca tac ctt gga      437
   Pro Ser Glu Pro Ser Phe Glu Pro Gln Ala Pro Ala Pro Tyr Leu Gly
           45                  50                  55
   cct cca cca ccc aca act tac tgc ccc tgc tca atc cac cca gat tct      485
   Pro Pro Pro Pro Thr Thr Tyr Cys Pro Cys Ser Ile His Pro Asp Ser
       60                  65                  70
   ggc ttc cca ctt cct cca cca cct tat gag ctc cca gca tcc aca tcc      533
   Gly Phe Pro Leu Pro Pro Pro Pro Tyr Glu Leu Pro Ala Ser Thr Ser
   75                  80                  85                  90
   cat gtc cca gat ccc cca tac tcc tat ggc aac atg gcc ata cca gtc      581
   His Val Pro Asp Pro Pro Tyr Ser Tyr Gly Asn Met Ala Ile Pro Val
                   95                  100                 105
   tcc aag cca ctg agc ctc tca ggc ctg ctc agt gag ccg ctc caa gac      629
   Ser Lys Pro Leu Ser Leu Ser Gly Leu Leu Ser Glu Pro Leu Gln Asp
               110                 115                 120
   ccc tta gcc ctc ctg gac att ggg ctg cca gca ggg cca cct aag ccc      677
   Pro Leu Ala Leu Leu Asp Ile Gly Leu Pro Ala Gly Pro Pro Lys Pro
   125                 130                 135
   caa gaa gac cca gaa tcc gac tca gga tta tcc ctc aac tat agc gat      725
   Gln Glu Asp Pro Glu Ser Asp Ser Gly Leu Ser Leu Asn Tyr Ser Asp
       140                 145                 150
   gct gaa tct ctt gag ctg gag ggg aca gag gct ggt cgg cgg cgc agc      773
   Ala Glu Ser Leu Glu Leu Glu Gly Thr Glu Ala Gly Arg Arg Arg Ser
   155                 160                 165                 170
   gaa tat gta gag atg tac cca gtg gag tac ccc tac tca ctc atg ccc      821
   Glu Tyr Val Glu Met Tyr Pro Val Glu Tyr Pro Tyr Ser Leu Met Pro
                   175                 180                 185
   aac tcc ttg gcc cac tcc aac tat acc ttg cca gct gct gag acc ccc      869
   Asn Ser Leu Ala His Ser Asn Tyr Thr Leu Pro Ala Ala Glu Thr Pro
               190                 195                 200
   ttg gcc tta gag ccc tcc tca ggc cct gtg cgg gct aag ccc act gca      917
   Leu Ala Leu Glu Pro Ser Ser Gly Pro Val Arg Ala Lys Pro Thr Ala
           205                 210                 215
   cgg ggg gag gca ggg agt cgg gat gaa cgt cgg gcc ttg gcc atg aag      965
   Arg Gly Glu Ala Gly Ser Arg Asp Glu Arg Arg Ala Leu Ala Met Lys
       220                 225                 230
   att cct ttt cct acg gac aag att gtc aac ttg ccg gta gat gac ttt     1013
   Ile Pro Phe Pro Thr Asp Lys Ile Val Asn Leu Pro Val Asp Asp Phe
   235                 240                 245                 250
   aat gag cta ttg gca agg tac ccg ctg aca gag agc cag cta gcg cta     1061
   Asn Glu Leu Leu Ala Arg Tyr Pro Leu Thr Glu Ser Gln Leu Ala Leu
                   255                 260                 265
   gtc cgg gac atc cga cga cgg ggc aaa aac aag gtg gca gcc cag aac     1109
   Val Arg Asp Ile Arg Arg Arg Gly Lys Asn Lys Val Ala Ala Gln Asn
               270                 275                 280
   tgc cgc aag agg aag ctg gaa acc att gtg cag ctg gag cgg gag ctg     1157
   Cys Arg Lys Arg Lys Leu Glu Thr Ile Val Gln Leu Glu Arg Glu Leu
           285                 290                 295
   gag cgg ctg acc aat gaa cgg gag cgg ctt ctc agg gcc cgc ggg gag     1205
   Glu Arg Leu Thr Asn Glu Arg Glu Arg Leu Leu Arg Ala Arg Gly Glu
       300                 305                 310
   gca gac cgg acc ctg gag gtc atg cgc caa cag ctg aca gag ctg tac     1253
   Ala Asp Arg Thr Leu Glu Val Met Arg Gln Gln Leu Thr Glu Leu Tyr
   315                 320                 325                 330
   cgt gac att ttc cag cac ctt cgg gat gaa tca ggc aac agc tac tct     1301
   Arg Asp Ile Phe Gln His Leu Arg Asp Glu Ser Gly Asn Ser Tyr Ser
                   335                 340                 345
   cct gaa gag tac gcg ctg caa cag gct gcc gat ggg acc atc ttc ctt     1349
   Pro Glu Glu Tyr Ala Leu Gln Gln Ala Ala Asp Gly Thr Ile Phe Leu
               350                 355                 360
   gtg ccc cgg ggg acc aag atg gag gcc aca gac tga gctggcccag          1395
   Val Pro Arg Gly Thr Lys Met Glu Ala Thr Asp
           365                 370
   aggggtggaa ctgctgatgg gatttccttc attcccttct gataaaggta ctccccaacc   1455
   ctgagtccca gaaggagctg agttctctag accagaagag gatgacaatg gcaacaagtg 1515
   tttggaagtt ccaaggtgtg ttcaaagagg cttgccttga gggagggctg gaatctgtct 1575
   tccctgactc ggctcctcag gtctttagcc tccaccttgt ctaagctttg gtctataaag 1635
   tgcgctacag aaaaaaaaaa aaaaaaaa 1663

<210> 17
<211> 373
<212> PRT
<213> Homo sapiens

<400> 17

Met Ser Pro Cys Pro Pro Gln Gln Ser Arg Asn Arg Val Ile Gln Leu
1               5                   10                  15

Ser Thr Ser Glu Leu Gly Glu Met Glu Leu Thr Trp Gln Glu Ile Met
            20                  25                  30

Ser Ile Thr Glu Leu Gln Gly Leu Asn Ala Pro Ser Glu Pro Ser Phe
        35                  40                  45

Glu Pro Gln Ala Pro Ala Pro Tyr Leu Gly Pro Pro Pro Pro Thr Thr
    50                  55                  60

Tyr Cys Pro Cys Ser Ile His Pro Asp Ser Gly Phe Pro Leu Pro Pro
65                  70                  75                  80

Pro Pro Tyr Glu Leu Pro Ala Ser Thr Ser His Val Pro Asp Pro Pro
                85                  90                  95

Tyr Ser Tyr Gly Asn Met Ala Ile Pro Val Ser Lys Pro Leu Ser Leu
            100                 105                 110

Ser Gly Leu Leu Ser Glu Pro Leu Gln Asp Pro Leu Ala Leu Leu Asp
        115                 120                 125

Ile Gly Leu Pro Ala Gly Pro Pro Lys Pro Gln Glu Asp Pro Glu Ser
    130                 135                 140

Asp Ser Gly Leu Ser Leu Asn Tyr Ser Asp Ala Glu Ser Leu Glu Leu
145                 150                 155                 160

Glu Gly Thr Glu Ala Gly Arg Arg Arg Ser Glu Tyr Val Glu Met Tyr
                165                 170                 175

Pro Val Glu Tyr Pro Tyr Ser Leu Met Pro Asn Ser Leu Ala His Ser
            180                 185                 190

Asn Tyr Thr Leu Pro Ala Ala Glu Thr Pro Leu Ala Leu Glu Pro Ser
        195                 200                 205

Ser Gly Pro Val Arg Ala Lys Pro Thr Ala Arg Gly Glu Ala Gly Ser
    210                 215                 220

Arg Asp Glu Arg Arg Ala Leu Ala Met Lys Ile Pro Phe Pro Thr Asp
225                 230                 235                 240

Lys Ile Val Asn Leu Pro Val Asp Asp Phe Asn Glu Leu Leu Ala Arg
                245                 250                 255

Tyr Pro Leu Thr Glu Ser Gln Leu Ala Leu Val Arg Asp Ile Arg Arg
            260                 265                 270

Arg Gly Lys Asn Lys Val Ala Ala Gln Asn Cys Arg Lys Arg Lys Leu
        275                 280                 285

Glu Thr Ile Val Gln Leu Glu Arg Glu Leu Glu Arg Leu Thr Asn Glu
    290                 295                 300

Arg Glu Arg Leu Leu Arg Ala Arg Gly Glu Ala Asp Arg Thr Leu Glu
305                 310                 315                 320

Val Met Arg Gln Gln Leu Thr Glu Leu Tyr Arg Asp Ile Phe Gln His
                325                 330                 335

Leu Arg Asp Glu Ser Gly Asn Ser Tyr Ser Pro Glu Glu Tyr Ala Leu
            340                 345                 350

Gln Gln Ala Ala Asp Gly Thr Ile Phe Leu Val Pro Arg Gly Thr Lys
        355                 360                 365

Met Glu Ala Thr Asp
    370

<210>  18
<211>  1248
<212>  DNA
<213>  Homo sapiens

<220>
<221>  CDS
<222>  (127)..(681)
<223>  Tnfaip8l2

<400>  18
ggccaagcca aagggctctc acactaagtg aagcttctcc attctgtaag ctttccggga       60

acatccaagg caagactggc acccagcaca gcagtgactg accacatacc ccactctcca 120

ggaccc atg gag tcc ttc agc tca aag agc ctg gca ctg caa gca gag 168
Met Glu Ser Phe Ser Ser Lys Ser Leu Ala Leu Gln Ala Glu
1               5                   10

aag aag cta ctg agt aag atg gcg ggt cgc tct gtg gct cat ctc ttc 216
Lys Lys Leu Leu Ser Lys Met Ala Gly Arg Ser Val Ala His Leu Phe
15                  20                  25                  30

ata gat gag aca agc agt gag gtg cta gat gag ctc tac cgt gtg tcc 264
Ile Asp Glu Thr Ser Ser Glu Val Leu Asp Glu Leu Tyr Arg Val Ser
                35                  40                  45

aag gag tac acg cac agc cgg ccc cag gcc cag cgc gtg atc aag gac 312
Lys Glu Tyr Thr His Ser Arg Pro Gln Ala Gln Arg Val Ile Lys Asp
            50                  55                  60

ctg atc aaa gtg gcc atc aag gtg gct gtg ctg cac cgc aat ggc tcc 360
Leu Ile Lys Val Ala Ile Lys Val Ala Val Leu His Arg Asn Gly Ser
        65                  70                  75

ttt ggc ccc agt gag ctg gcc ctg gct acc cgc ttt cgc cag aag ctg 408
Phe Gly Pro Ser Glu Leu Ala Leu Ala Thr Arg Phe Arg Gln Lys Leu
    80                  85                  90

cgg cag ggt gcc atg acg gca ctt agc ttt ggt gag gta gac ttc acc 456
Arg Gln Gly Ala Met Thr Ala Leu Ser Phe Gly Glu Val Asp Phe Thr
95                  100                 105                 110

ttc gag gct gct gtt ctg gct ggc ctg ctg acc gag tgc cgg gat gtg 504
Phe Glu Ala Ala Val Leu Ala Gly Leu Leu Thr Glu Cys Arg Asp Val
                115                 120                 125

ctg cta gag ttg gtg gaa cac cac ctc acg ccc aag tca cat ggc cgc 552
Leu Leu Glu Leu Val Glu His His Leu Thr Pro Lys Ser His Gly Arg
            130                 135                 140

atc cgc cac gtg ttt gat cac ttc tct gac cca ggt ctg ctc acg gcc 600
Ile Arg His Val Phe Asp His Phe Ser Asp Pro Gly Leu Leu Thr Ala
        145                 150                 155

ctc tat ggg cct gac ttc act cag cac ctt ggc aag atc tgt gac gga 648
Leu Tyr Gly Pro Asp Phe Thr Gln His Leu Gly Lys Ile Cys Asp Gly
    160                 165                 170

ctc agg aag ctg cta gac gaa ggg aag ctc tga gagccctgag cctagcacat 701
Leu Arg Lys Leu Leu Asp Glu Gly Lys Leu
175                 180

tccaccttga caaaatggtt gactgagaaa acacagataa tgggcttcct aaccctgctc 761

acctggcact aacacttttc aatcttcagg cttcattcct tcccaagagt gcttttgact 821

ctgagaccag cccaccccca aacagctagt ggagaaggag caatgctgag gggtgaggcc 881

tctctcccac tccagcccca ggacaggaaa cagaactgcc tgaaaaaggt gaagtgaaac 941

ttggatctct atttctccca taagggactt ctgaaacagg gaagccccct cccatgtgaa 1001

ccaaggaaag gaggcacagc ccagagaacc cctttgggga tactaaagac agaagagggg 1061

aaggtggccc ttagagacag agcttggaca gatgccagag gctctgttcc agagtgcagg     1121

aagaaggggc tagggcaggg gagattctca taggggaaat aaaactacta aaatatgaaa     1181

aaaaaaaaaa aaaaaaaaaa aaaaaaaaaa aaaaaaaaaa aaaaaaaaaa aaaaaaaaaa     1241

aaaaaaa                                                               1248

<210>  19
<211>  184
<212>  PRT
<213>  Homo sapiens

<400>  19

Met Glu Ser Phe Ser Ser Lys Ser Leu Ala Leu Gln Ala Glu Lys Lys
1               5                   10                  15

Leu Leu Ser Lys Met Ala Gly Arg Ser Val Ala His Leu Phe Ile Asp
            20                  25                  30

Glu Thr Ser Ser Glu Val Leu Asp Glu Leu Tyr Arg Val Ser Lys Glu
        35                  40                  45

Tyr Thr His Ser Arg Pro Gln Ala Gln Arg Val Ile Lys Asp Leu Ile
    50                  55                  60

Lys Val Ala Ile Lys Val Ala Val Leu His Arg Asn Gly Ser Phe Gly
65                  70                  75                  80

Pro Ser Glu Leu Ala Leu Ala Thr Arg Phe Arg Gln Lys Leu Arg Gln
                85                  90                  95

Gly Ala Met Thr Ala Leu Ser Phe Gly Glu Val Asp Phe Thr Phe Glu
            100                 105                 110

Ala Ala Val Leu Ala Gly Leu Leu Thr Glu Cys Arg Asp Val Leu Leu
        115                 120                 125

Glu Leu Val Glu His His Leu Thr Pro Lys Ser His Gly Arg Ile Arg
    130                 135                 140

His Val Phe Asp His Phe Ser Asp Pro Gly Leu Leu Thr Ala Leu Tyr
145                 150                 155                 160

Gly Pro Asp Phe Thr Gln His Leu Gly Lys Ile Cys Asp Gly Leu Arg
                165                 170                 175

Lys Leu Leu Asp Glu Gly Lys Leu
            180

<210>  20
<211>  1254
<212>  DNA
<213>  Homo sapiens

<220>
<221>  CDS
<222>  (33)..(503)
<223>  Ier3

<400>  20
ctcacttggc cttacactcc gctcggctca cc atg tgt cac tct cgc agc tgc      53
                                    Met Cys His Ser Arg Ser Cys
                                    1               5

cac ccg acc atg acc atc ctg cag gcc ccg acc ccg gcc ccc tcc acc      101
His Pro Thr Met Thr Ile Leu Gln Ala Pro Thr Pro Ala Pro Ser Thr
        10                  15                  20

atc ccg gga ccc cgg cgg ggc tcc ggt cct gag atc ttc acc ttc gac      149
Ile Pro Gly Pro Arg Arg Gly Ser Gly Pro Glu Ile Phe Thr Phe Asp
    25                  30                  35

cct ctc ccg gag ccc gca gcg gcc cct gcc ggg cgc ccc agc gcc tct      197
Pro Leu Pro Glu Pro Ala Ala Ala Pro Ala Gly Arg Pro Ser Ala Ser
40                  45                  50                  55

cgc ggg cac cga aag cgc agc cgc agg gtt ctc tac cct cga gtg gtc      245
Arg Gly His Arg Lys Arg Ser Arg Arg Val Leu Tyr Pro Arg Val Val
                60                  65                  70

cgg cgc cag ctg cca gtc gag gaa ccg aac cca gcc aaa agg ctt ctc      293
Arg Arg Gln Leu Pro Val Glu Glu Pro Asn Pro Ala Lys Arg Leu Leu
            75                  80                  85

ttt ctg ctg ctc acc atc gtc ttc tgc cag atc ctg atg gct gaa gag      341
Phe Leu Leu Leu Thr Ile Val Phe Cys Gln Ile Leu Met Ala Glu Glu
        90                  95                  100

ggt gtg ccg gcg ccc ctg cct cca gag gac gcc cct aac gcc gca tcc      389
Gly Val Pro Ala Pro Leu Pro Pro Glu Asp Ala Pro Asn Ala Ala Ser
    105                 110                 115

ctg gcg ccc acc cct gtg tcc gcc gtc ctc gag ccc ttt aat ctg act      437
Leu Ala Pro Thr Pro Val Ser Ala Val Leu Glu Pro Phe Asn Leu Thr
120                 125                 130                 135

tcg gag ccc tcg gac tac gct ctg gac ctc agc act ttc ctc cag caa      485
Ser Glu Pro Ser Asp Tyr Ala Leu Asp Leu Ser Thr Phe Leu Gln Gln
                140                 145                 150

cac ccg gcc gcc ttc taa ctgtgactcc ccgcactccc caaaaagaat      533
His Pro Ala Ala Phe
            155

ccgaaaaacc acaaagaaac accaggcgta cctggtgcgc gagagcgtat ccccaactgg      593

gacttccgag gcaacttgaa ctcagaacac tacagcggag acgccacccg gtgcttgagg      653

cgggaccgag gcgcacagag accgaggcgc atagagaccg aggcacagcc cagctggggc      713

taggcccggt gggaaggaga gcgtcgttaa tttatttctt attgctccta attaatattt 773

atatgtattt atgtacgtcc tcctaggtga tggagatgtg tacgtaatat ttattttaac 833

ttatgcaagg gtgtgagatg ttccccctgc tgtaaatgca ggtctcttgg tatttattga 893

gctttgtggg actggtggaa gcaggacacc tggaactgcg gcaaagtagg agaagaaatg 953

gggaggactc gggtggggga ggacgtcccg gctgggatga agtctggtgg tgggtcgtaa 1013

gtttaggagg tgactgcatc ctccagcatc tcaactccgt ctgtctactg tgtgagactt 1073

cggcggacca ttaggaatga gatccgtgag atccttccat cttcttgaag tcgcctttag 1133

ggtggctgcg aggtagaggg ttgggggttg gtgggctgtc acggagcgac tgtcgagatc 1193

gcctagtatg ttctgtgaac acaaataaaa ttgatttact gtctgcaaaa aaaaaaaaaa 1253

a 1254

<210> 21
<211> 156
<212> PRT
<213> Homo sapiens

<400> 21

Met Cys His Ser Arg Ser Cys His Pro Thr Met Thr Ile Leu Gln Ala
1               5                   10                  15

Pro Thr Pro Ala Pro Ser Thr Ile Pro Gly Pro Arg Arg Gly Ser Gly
            20                  25                  30

Pro Glu Ile Phe Thr Phe Asp Pro Leu Pro Glu Pro Ala Ala Ala Pro
        35                  40                  45

Ala Gly Arg Pro Ser Ala Ser Arg Gly His Arg Lys Arg Ser Arg Arg
    50                  55                  60

Val Leu Tyr Pro Arg Val Val Arg Arg Gln Leu Pro Val Glu Glu Pro
65                  70                  75                  80

Asn Pro Ala Lys Arg Leu Leu Phe Leu Leu Leu Thr Ile Val Phe Cys
                85                  90                  95

Gln Ile Leu Met Ala Glu Glu Gly Val Pro Ala Pro Leu Pro Pro Glu
            100                 105                 110

Asp Ala Pro Asn Ala Ala Ser Leu Ala Pro Thr Pro Val Ser Ala Val
        115                 120                 125

Leu Glu Pro Phe Asn Leu Thr Ser Glu Pro Ser Asp Tyr Ala Leu Asp
    130                 135                 140

Leu Ser Thr Phe Leu Gln Gln His Pro Ala Ala Phe
145                 150                 155

<210>  22
<211>  4817
<212>  DNA
<213>  Homo sapiens

<220>
<221>  CDS
<222>  (129)..(2546)
<223>  Pik3ap1

<400>  22
gaagcgccag agcggcggcc ggtcccgcgc ggagcccggc gcccctccag cccgagccag      60

gacgccgccg gccccggtcc cggccccggg cacgcagcga gccagggatg tgagcggcgc     120

cccgcggc atg gca gcc tca ggg gtg ccc aga gga tgc gac atc ctc atc      170
         Met Ala Ala Ser Gly Val Pro Arg Gly Cys Asp Ile Leu Ile
         1               5                   10

gtc tac agc ccg gat gcc gag gaa tgg tgc cag tac ctg cag acc ctg      218
Val Tyr Ser Pro Asp Ala Glu Glu Trp Cys Gln Tyr Leu Gln Thr Leu
15                  20                  25                  30

ttc ctg tcc agt cgg cag gtc cgc agc cag aag ata ctg act cac agg      266
Phe Leu Ser Ser Arg Gln Val Arg Ser Gln Lys Ile Leu Thr His Arg
                35                  40                  45

ctg ggc ccc gag gcc tcc ttc tcg gca gag gac cta agc ctt ttc ctc      314
Leu Gly Pro Glu Ala Ser Phe Ser Ala Glu Asp Leu Ser Leu Phe Leu
            50                  55                  60

agc acc cgc tgt gtc gtg gtg ctg ctg tcc gcg gag ctg gtg cag cac      362
Ser Thr Arg Cys Val Val Val Leu Leu Ser Ala Glu Leu Val Gln His
        65                  70                  75

ttc cac aag ccc gcc ttg ctg ccc ctg ctg cag aga gct ttc cat cct      410
Phe His Lys Pro Ala Leu Leu Pro Leu Leu Gln Arg Ala Phe His Pro
    80                  85                  90

ccg cac cgc gtg gtc agg ctg ctc tgc ggc gtg cgg gac agc gag gag      458
Pro His Arg Val Val Arg Leu Leu Cys Gly Val Arg Asp Ser Glu Glu
95                  100                 105                 110

ttc cta gac ttc ttt cca gat tgg gcc cat tgg cag gag ctc acc tgt      506
Phe Leu Asp Phe Phe Pro Asp Trp Ala His Trp Gln Glu Leu Thr Cys
                115                 120                 125

gac gat gag cca gag acc tac gtg gca gct gtg aaa aaa gcc att tcc      554
Asp Asp Glu Pro Glu Thr Tyr Val Ala Ala Val Lys Lys Ala Ile Ser
            130                 135                 140

gaa gat tct ggc tgt gac tca gtc act gac act gag cct gag gac gag      602
Glu Asp Ser Gly Cys Asp Ser Val Thr Asp Thr Glu Pro Glu Asp Glu
        145                 150                 155

aag gtt gtt tcc tac tcg aag cag cag aac ctg ccg acg gtg act tca      650
Lys Val Val Ser Tyr Ser Lys Gln Gln Asn Leu Pro Thr Val Thr Ser

160                 165                 170

cct ggg aac ctg atg gtg gtg cag ccg gac cgc att cgc tgt ggg gca       698
Pro Gly Asn Leu Met Val Val Gln Pro Asp Arg Ile Arg Cys Gly Ala
175                 180                 185                 190

gaa acc act gtc tat gtt att gtg aga tgt aag ctg gat gac agg gtg       746
Glu Thr Thr Val Tyr Val Ile Val Arg Cys Lys Leu Asp Asp Arg Val
                195                 200                 205

gcg aca gaa gca gag ttt tct cct gag gat tct ccc tct gta agg atg       794
Ala Thr Glu Ala Glu Phe Ser Pro Glu Asp Ser Pro Ser Val Arg Met
            210                 215                 220

gaa gcc aag gtg gag aat gag tac acc att tca gtg aag gct ccc aac       842
Glu Ala Lys Val Glu Asn Glu Tyr Thr Ile Ser Val Lys Ala Pro Asn
        225                 230                 235

ctt tca tct ggg aac gtt tct ctg aag ata tat tct gga gac tta gtg       890
Leu Ser Ser Gly Asn Val Ser Leu Lys Ile Tyr Ser Gly Asp Leu Val
    240                 245                 250

gtg tgt gaa acc gtt atc agc tat tat act gac atg gaa gaa att ggg       938
Val Cys Glu Thr Val Ile Ser Tyr Tyr Thr Asp Met Glu Glu Ile Gly
255                 260                 265                 270

aat tta ttg tcc aat gcc gcg aat cct gtg gaa ttc atg tgt cag gcc       986
Asn Leu Leu Ser Asn Ala Ala Asn Pro Val Glu Phe Met Cys Gln Ala
                275                 280                 285

ttt aaa att gtg ccc tac aac aca gag acc ctt gat aaa ctg cta acc      1034
Phe Lys Ile Val Pro Tyr Asn Thr Glu Thr Leu Asp Lys Leu Leu Thr
            290                 295                 300

gaa tcc ctg aag aac aat atc cct gca agc gga ctg cac ctc ttt gga      1082
Glu Ser Leu Lys Asn Asn Ile Pro Ala Ser Gly Leu His Leu Phe Gly
        305                 310                 315

atc aac cag ctg gaa gaa gaa gat atg atg aca aat cag agg gat gaa      1130
Ile Asn Gln Leu Glu Glu Glu Asp Met Met Thr Asn Gln Arg Asp Glu
    320                 325                 330

gag ctg ccc acc ctg ttg cat ttt gct gcg aag tat gga ctg aag aac      1178
Glu Leu Pro Thr Leu Leu His Phe Ala Ala Lys Tyr Gly Leu Lys Asn
335                 340                 345                 350

ctc act gcc ttg ttg ctc acc tgc cca gga gcc ctg cag gcg tac agc      1226
Leu Thr Ala Leu Leu Leu Thr Cys Pro Gly Ala Leu Gln Ala Tyr Ser
                355                 360                 365

gtg gcc aac aag cat ggc cac tac ccc aac acc atc gct gag aaa cac      1274
Val Ala Asn Lys His Gly His Tyr Pro Asn Thr Ile Ala Glu Lys His
            370                 375                 380

ggc ttc agg gac ctg cgg cag ttc atc gac gag tat gtg gaa acg gtg      1322
Gly Phe Arg Asp Leu Arg Gln Phe Ile Asp Glu Tyr Val Glu Thr Val
        385                 390                 395

gac atg ctc aag agt cac att aaa gag gaa ctg atg cac ggg gag gag      1370
Asp Met Leu Lys Ser His Ile Lys Glu Glu Leu Met His Gly Glu Glu
    400                 405                 410

gct gat gct gtg tac gag tcc atg gcc cac ctt tcc aca gac ctg ctt      1418

Ala Asp Ala Val Tyr Glu Ser Met Ala His Leu Ser Thr Asp Leu Leu
415                 420                 425                 430

atg aaa tgc tcg ctc aac ccc ggc tgt gac gag gat ctc tat gag tcc      1466
Met Lys Cys Ser Leu Asn Pro Gly Cys Asp Glu Asp Leu Tyr Glu Ser
                435                 440                 445

atg gct gcc ttt gtc cca gct gcc act gaa gac ctc tat gtt gaa atg      1514
Met Ala Ala Phe Val Pro Ala Ala Thr Glu Asp Leu Tyr Val Glu Met
            450                 455                 460

ctt cag gcc agt aca tct aac cca atc cct gga gat ggt ttc tct cgg      1562
Leu Gln Ala Ser Thr Ser Asn Pro Ile Pro Gly Asp Gly Phe Ser Arg
        465                 470                 475

gcc act aag gac tct atg atc cgc aag ttt tta gaa ggc aac agc atg      1610
Ala Thr Lys Asp Ser Met Ile Arg Lys Phe Leu Glu Gly Asn Ser Met
    480                 485                 490

gga atg acc aat ctg gag aga gat cag tgc cat ctt ggt cag gaa gaa      1658
Gly Met Thr Asn Leu Glu Arg Asp Gln Cys His Leu Gly Gln Glu Glu
495                 500                 505                 510

gat gtt tat cac acg gtg gat gac gat gag gcc ttt tct gtg gac ctg      1706
Asp Val Tyr His Thr Val Asp Asp Asp Glu Ala Phe Ser Val Asp Leu
                515                 520                 525

gcc agc agg ccc cct gtc cca gtg ccc aga cca gag acc act gct cct      1754
Ala Ser Arg Pro Pro Val Pro Val Pro Arg Pro Glu Thr Thr Ala Pro
            530                 535                 540

ggt gct cac cag ctg cct gac aac gaa cca tac att ttt aaa gtt ttt      1802
Gly Ala His Gln Leu Pro Asp Asn Glu Pro Tyr Ile Phe Lys Val Phe
        545                 550                 555

gca gaa aaa agt caa gag cgg cct ggg aat ttc tac gtt tcc tca gag      1850
Ala Glu Lys Ser Gln Glu Arg Pro Gly Asn Phe Tyr Val Ser Ser Glu
    560                 565                 570

agc atc agg aaa ggg ccg ccc gtc aga cca tgg agg gac agg ccc cag      1898
Ser Ile Arg Lys Gly Pro Pro Val Arg Pro Trp Arg Asp Arg Pro Gln
575                 580                 585                 590

tcg agt ata tat gac cct ttt gcg gga atg aaa acg cca ggc cag cgg      1946
Ser Ser Ile Tyr Asp Pro Phe Ala Gly Met Lys Thr Pro Gly Gln Arg
                595                 600                 605

cag ctt atc acc ctc cag gag cag gtg aag ctg ggc att gtc aac gtg      1994
Gln Leu Ile Thr Leu Gln Glu Gln Val Lys Leu Gly Ile Val Asn Val
            610                 615                 620

gat gag gct gtg ctc cac ttc aaa gag tgg cag ctc aac cag aag aaa      2042
Asp Glu Ala Val Leu His Phe Lys Glu Trp Gln Leu Asn Gln Lys Lys
        625                 630                 635

cga tcg gag tcc ttt cgt ttc cag cag gaa aat ctt aaa cgg cta aga      2090
Arg Ser Glu Ser Phe Arg Phe Gln Gln Glu Asn Leu Lys Arg Leu Arg
    640                 645                 650

gac agc atc acc cga aga cag aga gag aag caa aaa tca gga aag cag      2138
Asp Ser Ile Thr Arg Arg Gln Arg Glu Lys Gln Lys Ser Gly Lys Gln
655                 660                 665                 670

aca gac ttg gag atc acg gtc cca att cgg cac tca cag cac ctg cct      2186
Thr Asp Leu Glu Ile Thr Val Pro Ile Arg His Ser Gln His Leu Pro
                675                 680                 685

gca aaa gtg gag ttt gga gtc tat gag agt ggc ccc agg aaa agt gtc      2234
Ala Lys Val Glu Phe Gly Val Tyr Glu Ser Gly Pro Arg Lys Ser Val
            690                 695                 700

att ccc cct agg acg gag ctg aga cga gga gac tgg aaa aca gac agc      2282
Ile Pro Pro Arg Thr Glu Leu Arg Arg Gly Asp Trp Lys Thr Asp Ser
        705                 710                 715

acc tcc agc aca gca agt agc aca agt aac cgc tcc agc acc cgg agc      2330
Thr Ser Ser Thr Ala Ser Ser Thr Ser Asn Arg Ser Ser Thr Arg Ser
    720                 725                 730

ctc ctc agt gtg agc agc ggg atg gaa ggg gac aac gag gat aat gaa      2378
Leu Leu Ser Val Ser Ser Gly Met Glu Gly Asp Asn Glu Asp Asn Glu
735                 740                 745                 750

gtc cct gag gtt acc aga agt cgc agt cca ggc ccc cca caa gtg gat      2426
Val Pro Glu Val Thr Arg Ser Arg Ser Pro Gly Pro Pro Gln Val Asp
                755                 760                 765

ggg aca ccc acc atg tcc ctc gag aga ccc ccc agg gtg cct ccg aga      2474
Gly Thr Pro Thr Met Ser Leu Glu Arg Pro Pro Arg Val Pro Pro Arg
            770                 775                 780

gct gcc tca cag agg cct ccg acc agg gag acc ttc cat cct cct cca      2522
Ala Ala Ser Gln Arg Pro Pro Thr Arg Glu Thr Phe His Pro Pro Pro
        785                 790                 795

cct gtt cca ccc aga gga cgc tga ttccacctcc taaaacctgc ctacttcagg      2576
Pro Val Pro Pro Arg Gly Arg
    800                 805

actttaagac tcacagtctt cagcctgtta atgatgtctt catgttgagt tttatagcat      2636

gactgttgac cttaagatcc attctcattg ctgataatgc tgcagccctg ctggtttggg      2696

cttgcctcga agattttatt aaggcacgaa gaagtgaaaa actaagggct tcattcacca      2756

tcaccaagta tatcgaacca tatacttgtt tgccaaaagg atgaagactt aatcgaaata      2816

cttacctcta atttgccata tcagaagcct aaaaagaatg atcataaatg tacttcacca      2876

gtgattttac tgaaatgcac ttatattagt ctttatgtat ttgctagttc agcctgattt      2936

ctagaagagg ttatagtgtg agacttgtag tattcaagta agataagtga cctaatttta      2996

aaataattct tctacttttc tgtatattca gcagggtatt taagtgctag ggctggtcac      3056

acacaaccaa ctgaaaaaga ctagagggat tagtacaaac tcctcttata cagaaggcaa      3116

atctgaggtt ccacagaagt ctggaaccaa gactattcag ttggttaaat aaagaggtta      3176

gtctagactg ggcctgctca ttctaggtca ccacattttc catctccaaa tagccaggcc      3236

ctctctccct caagaaatgc ccagatgtag aaattcatca gtgcctattg gtcttccaga      3296

attttccatc ttccgtatct cccaggcatg agactaccaa gtttgtttgt ttctttccca      3356

atttgggaat ttatacttca gtatggtttc aacgcagtta tgtttccaga gaacatctag      3416

aagtggctgg aaaccagaag ctggggattc cagggacccc acttagtgct ctatttcctt 3476

tataggtttt atttctggtc atagagagag aaggaccttt gactttttct tcgttgaggc 3536

ttctgaggag gaaaaacaaa cctaaaatag aaatacagtc agcctttcaa atccatgggt 3596

tctgtgtccg tggattcaac caaccttgga tcaaaaatat ttgaaaaaaa atctacaaag 3656

tttcaaaaag caaaacttga atttgctgca tgccaagaag tatgttgaat tcatgtaaat 3716

gaagtgatgt gtaggcattg tattagatat tataagaaat ctagaaatga tttaaagcat 3776

acaggaggat gtgcataggt tatatgcaaa tactatgcta ttttatatat gggacttgag 3836

catttgtgga ttttgatact gggggatcct ggaaccaatc ccccatggat accaaagtac 3896

gactgtagtt atctattttt tacatactta ttattaccac catgctcagt aagtccattt 3956

ttgcatggaa tatggagcct taaaacatgt catgaatttg gagtccctgg cacataaatc 4016

taccttcaaa tcagaggtcc ttaatgatgc ctaaacatac agtaaaatta gaatcagaaa 4076

tacttcttta aaaaatattc aaaatgtgtt tgtttcccat gggattattc tctatcccac 4136

acgaatgtaa aaaaatccac attaatgatc catttaagta tagttttatt gggtcctttt 4196

ctaatgatta aaggttcttt ctcaatttca ttcctcagtc ctgcaagtaa ggactcatac 4256

tgaagagtac tgaaacaagg acttcttgtc agaaacagct tctggaatct tgggttttgt 4316

ttttgttttt tgacaaaata cactattggc catgtccatc acgagagtgt ttgtagtaat 4376

taattacctt gtacaggacc tggcacttag tagcattctt caaatgttcc ctcagtgatc 4436

cttttactct ccttgtcact tatttgggag aaataggggc acgtgagata agaagaagaa 4496

taattttgat gttggtatgc ttgccctgtt acttatagac agtctttgtc ataggcaaac 4556

ttgaatttga tttaaaatag ggctgggaaa aatattcaat aactgtaagc ccccttttgg 4616

atgccaaact tagaattttg tacattctct gatgaacaag catttagatc gtaacatggt 4676

aaagcctatt accagccaat gttgttagca tctttgtatg cacatcactg tttgtgcaat 4736

atatgaatat tttgttgcat tgtattctta tataaaaata agtagaaaac ataaaattga 4796

aattgctgat gagaaaaaaa a 4817

<210> 23
<211> 805
<212> PRT
<213> Homo sapiens

<400> 23

Met Ala Ala Ser Gly Val Pro Arg Gly Cys Asp Ile Leu Ile Val Tyr
1               5                   10                  15

Ser Pro Asp Ala Glu Glu Trp Cys Gln Tyr Leu Gln Thr Leu Phe Leu
            20                  25                  30

Ser Ser Arg Gln Val Arg Ser Gln Lys Ile Leu Thr His Arg Leu Gly
        35                  40                  45

Pro Glu Ala Ser Phe Ser Ala Glu Asp Leu Ser Leu Phe Leu Ser Thr
    50                  55                  60

Arg Cys Val Val Val Leu Leu Ser Ala Glu Leu Val Gln His Phe His
65                  70                  75                  80

Lys Pro Ala Leu Leu Pro Leu Leu Gln Arg Ala Phe His Pro Pro His
                85                  90                  95

Arg Val Val Arg Leu Leu Cys Gly Val Arg Asp Ser Glu Glu Phe Leu
            100                 105                 110

Asp Phe Phe Pro Asp Trp Ala His Trp Gln Glu Leu Thr Cys Asp Asp
        115                 120                 125

Glu Pro Glu Thr Tyr Val Ala Ala Val Lys Lys Ala Ile Ser Glu Asp
    130                 135                 140

Ser Gly Cys Asp Ser Val Thr Asp Thr Glu Pro Glu Asp Glu Lys Val
145                 150                 155                 160

Val Ser Tyr Ser Lys Gln Gln Asn Leu Pro Thr Val Thr Ser Pro Gly
                165                 170                 175

Asn Leu Met Val Val Gln Pro Asp Arg Ile Arg Cys Gly Ala Glu Thr
            180                 185                 190

Thr Val Tyr Val Ile Val Arg Cys Lys Leu Asp Asp Arg Val Ala Thr
        195                 200                 205

Glu Ala Glu Phe Ser Pro Glu Asp Ser Pro Ser Val Arg Met Glu Ala
    210                 215                 220

Lys Val Glu Asn Glu Tyr Thr Ile Ser Val Lys Ala Pro Asn Leu Ser
225                 230                 235                 240

Ser Gly Asn Val Ser Leu Lys Ile Tyr Ser Gly Asp Leu Val Val Cys
                245                 250                 255

Glu Thr Val Ile Ser Tyr Tyr Thr Asp Met Glu Glu Ile Gly Asn Leu
            260                 265                 270

Leu Ser Asn Ala Ala Asn Pro Val Glu Phe Met Cys Gln Ala Phe Lys

275                 280                 285

Ile Val Pro Tyr Asn Thr Glu Thr Leu Asp Lys Leu Leu Thr Glu Ser
    290                 295                 300

Leu Lys Asn Asn Ile Pro Ala Ser Gly Leu His Leu Phe Gly Ile Asn
305                 310                 315                 320

Gln Leu Glu Glu Glu Asp Met Met Thr Asn Gln Arg Asp Glu Glu Leu
                325                 330                 335

Pro Thr Leu Leu His Phe Ala Ala Lys Tyr Gly Leu Lys Asn Leu Thr
            340                 345                 350

Ala Leu Leu Leu Thr Cys Pro Gly Ala Leu Gln Ala Tyr Ser Val Ala
        355                 360                 365

Asn Lys His Gly His Tyr Pro Asn Thr Ile Ala Glu Lys His Gly Phe
    370                 375                 380

Arg Asp Leu Arg Gln Phe Ile Asp Glu Tyr Val Glu Thr Val Asp Met
385                 390                 395                 400

Leu Lys Ser His Ile Lys Glu Glu Leu Met His Gly Glu Glu Ala Asp
                405                 410                 415

Ala Val Tyr Glu Ser Met Ala His Leu Ser Thr Asp Leu Leu Met Lys
            420                 425                 430

Cys Ser Leu Asn Pro Gly Cys Asp Glu Asp Leu Tyr Glu Ser Met Ala
        435                 440                 445

Ala Phe Val Pro Ala Ala Thr Glu Asp Leu Tyr Val Glu Met Leu Gln
    450                 455                 460

Ala Ser Thr Ser Asn Pro Ile Pro Gly Asp Gly Phe Ser Arg Ala Thr
465                 470                 475                 480

Lys Asp Ser Met Ile Arg Lys Phe Leu Glu Gly Asn Ser Met Gly Met
                485                 490                 495

Thr Asn Leu Glu Arg Asp Gln Cys His Leu Gly Gln Glu Glu Asp Val
            500                 505                 510

Tyr His Thr Val Asp Asp Asp Glu Ala Phe Ser Val Asp Leu Ala Ser
        515                 520                 525

Arg Pro Pro Val Pro Val Pro Arg Pro Glu Thr Thr Ala Pro Gly Ala
        530                 535                 540

His Gln Leu Pro Asp Asn Glu Pro Tyr Ile Phe Lys Val Phe Ala Glu
545                 550                 555                 560

Lys Ser Gln Glu Arg Pro Gly Asn Phe Tyr Val Ser Ser Glu Ser Ile
                565                 570                 575

Arg Lys Gly Pro Pro Val Arg Pro Trp Arg Asp Arg Pro Gln Ser Ser
            580                 585                 590

Ile Tyr Asp Pro Phe Ala Gly Met Lys Thr Pro Gly Gln Arg Gln Leu
        595                 600                 605

Ile Thr Leu Gln Glu Gln Val Lys Leu Gly Ile Val Asn Val Asp Glu
    610                 615                 620

Ala Val Leu His Phe Lys Glu Trp Gln Leu Asn Gln Lys Lys Arg Ser
625                 630                 635                 640

Glu Ser Phe Arg Phe Gln Gln Glu Asn Leu Lys Arg Leu Arg Asp Ser
                645                 650                 655

Ile Thr Arg Arg Gln Arg Glu Lys Gln Lys Ser Gly Lys Gln Thr Asp
            660                 665                 670

Leu Glu Ile Thr Val Pro Ile Arg His Ser Gln His Leu Pro Ala Lys
        675                 680                 685

Val Glu Phe Gly Val Tyr Glu Ser Gly Pro Arg Lys Ser Val Ile Pro
    690                 695                 700

Pro Arg Thr Glu Leu Arg Arg Gly Asp Trp Lys Thr Asp Ser Thr Ser
705                 710                 715                 720

Ser Thr Ala Ser Ser Thr Ser Asn Arg Ser Ser Thr Arg Ser Leu Leu
                725                 730                 735

Ser Val Ser Ser Gly Met Glu Gly Asp Asn Glu Asp Asn Glu Val Pro
            740                 745                 750

Glu Val Thr Arg Ser Arg Ser Pro Gly Pro Pro Gln Val Asp Gly Thr
        755                 760                 765

Pro Thr Met Ser Leu Glu Arg Pro Pro Arg Val Pro Pro Arg Ala Ala
    770                 775                 780

Ser Gln Arg Pro Pro Thr Arg Glu Thr Phe His Pro Pro Pro Pro Val
785                 790                 795                 800

Pro Pro Arg Gly Arg
                805

<210>  24
<211>  1870
<212>  DNA
<213>  Homo sapiens

<220>
<221>  CDS
<222>  (451)..(1701)
<223>  Pstpip1

<400>  24
acaaaccttc ctgcgcaggc ctcgggctgc ctgcctgcct gcctgcctgg cccggcccga      60
gctccagcct gcctcttcca ctggccactg cctcccaccc agggctggca tccctgctcc     120
ctgccctggg tcccagactg tgtcctccat caccgcaggg tcggtgaggg gctgggctgg     180
acaccagggc ccgccctccc atcactgagc tccactcctt cctcattttg ctgctgattc      240
tagccccaaa caaaacaggt tgagcttttt cctcccctca gaagctcctc tctggctcgt     300
ggctgccttc tgagtgttgc agacggcgcc ggccgggaag gggggcctgg gccagccctg     360
ccaggactgg gacgctgctg ctggcgcctg gccctccatc aggccagcct gtggcaggag     420
agtgagcttt gccgcggcag acgcctgagg atg atg ccc cag ctg cag ttc aaa      474
gat gcc ttt tgg tgc agg gac ttc aca gcc cac acg ggc tac gag gtg      522
ctg ctg cag cgg ctt ctg gat ggc agg aag atg tgc aaa gac atg gag      570
gag cta ctg agg cag agg gcc cag gcg gag gag cgg tac ggg aag gag      618
ctg gtg cag atc gca cgg aag gca ggt ggc cag acg gag atc aac tcc      666
ctg agg gcc tcc ttt gac tcc ttg aag cag caa atg gag aat gtg ggc      714
agc tca cac atc cag ctg gcc ctg acc ctg cgt gag gag ctg cgg agt      762
ctc gag gag ttt cgt gag agg cag aag gag cag agg aag aag tat gag          810
gcc gtc atg gac cgg gtc cag aag agc aag ctg tcg ctc tac aag aag          858
gcc atg gag tcc aag aag aca tac gag cag aag tgc cgg gac gcg gac          906
gac gcg gag cag gcc ttc gag cgc att agc gcc aac ggc cac cag aag          954
cag gtg gag aag agt cag aac aaa gcc agg cag tgc aag gac tcg gcc         1002
acc gag gca gag cgg gta tac agg cag agc att gcg cag ctg gag aag         1050
gtc cgg gct gag tgg gag cag gag cac cgg acc acc tgt gag gcc ttt         1098
cag ctg caa gag ttt gac cgg ctg acc att ctc cgc aac gcc ctg tgg         1146
gtg cac agc aac cag ctc tcc atg cag tgt gtc aag gat gat gag ctc         1194
tac gag gaa gtg cgg ctg acg ctg gaa ggc tgc agc ata gac gcc gac         1242
atc gac agt ttc atc cag gcc aag agc acg ggc aca gag ccc ccc gct         1290
ccg gtg ccc tac cag aac tat tac gat cgg gag gtc acc ccg ctg acc         1338
agc agc cct ggc ata cag ccg tcc tgc ggc atg ata aag agg ttc tct         1386
gga ctg ctg cac gga agt ccc aag acc act tcg ttg gca gct tct gct         1434
gcg tcc aca gag acc ctg acc ccc acc ccc gag cgg aat gag ggt gtc         1482
tac aca gcc atc gca gtg cag gag ata cag gga aac ccg gcc tca cca         1530
gcc cag gag tac cgg gcg ctc tac gat tat aca gcg cag aac cca gat     1578
gag ctg gac ctg tcc gcg gga gac atc ctg gag gtg atc ctg gaa ggg     1626
gag gat ggc tgg tgg act gtg gag agg aac ggg cag cgt ggc ttc gtc     1674
cct ggt tcc tac ctg gag aag ctt tga ggaagggcca ggagcccctt           1721
cggacctgcc ctgccagtgg agccagcagt gcccccagca ctgtccccac cttgctaggg   1781
cccagaacca agcgtccccc agccccgaga gggagcctgt cgtctcccag ggaataaagg   1841
agtgcgttct gttcaaaaaa aaaaaaaaa                                     1870


<210>  25
<211>  416
<212>  PRT
<213>  Homo sapiens

<400>  25

<210>  26
<211>  60
<212>  DNA
<213>  Artificial

<220>
<223>  Probe A_44_P1004731

<400>  26
ctggcttaca gcttgtttgt tttataactt tacctctctc tgaaaagtct gtaagcaagg      60

<210>  27
<211>  60
<212>  DNA
<213>  Artificial

<220>
<223>  Probe A_42_P592157

<400>  27
aaagctttcg gttttgtttt ttaaactgtt tgcagagtgg agaagatcga tcaggaaggg      60

<210>  28
<211>  60
<212>  DNA
<213>  Artificial

<220>
<223>  Probe A_42_P495820

<400>  28
attatgcagg ctatgacgga actactacct tgctatggat gagggttggg caggatttaa      60

<210>  29
<211>  60
<212>  DNA
<213>  Artificial

<220>
<223>  Probe CUST_1_PI195698246

<400>  29
gtctcaagaa cagagggcta ccttggggag ccataaagag tgtatttaat aaaacgggct      60

<210>  30
<211>  60
<212>  DNA
<213>  Artificial

<220>

<223>  Probe A_44_P377266

<400>  30
aatgaaataa ctaagcatat cttttgagaa ttttattttc ttacatttta aatctgaagg        60


<210>  31
<211>  60
<212>  DNA
<213>  Artificial

<220>
<223>  Probe A_66_P100662

<400>  31
tttgtggtcc ctgttcagtc attatgttgt cccttcgctt ctcttgatca gcagaaagca        60


<210>  32
<211>  60
<212>  DNA
<213>  Artificial

<220>
<223>  Probe A_44_P928825

<400>  32
gaacgtgtgc acaaagtatc agcagaaatc cagtctgtga aagaagaaca agagcatgtg        60


<210>  33
<211>  60
<212>  DNA
<213>  Artificial

<220>
<223>  Probe A_42_P526140

<400>  33
ctcagtgtcc gtgaattggg tatccaagaa catcctgaag ccagaatgtc ttctcagaaa        60


<210>  34
<211>  60
<212>  DNA
<213>  Artificial

<220>
<223>  Probe A_42_P464736

<400>  34
aggctgagtt ctccagacca aaagaccatt tggaagttca aagatgtatt tgaggtttgc        60


<210>  35
<211>  60
<212>  DNA
<213>  Artificial

<220>
<223>  Probe A_43_P20022

<400>  35

agctctgagg ctcctgagct cagcacactg gactttggca aaatgactga ccgggaaacg 60

<210> 36
<211> 60
<212> DNA
<213> Artificial

<220>
<223> Probe A_42_P515405

<400> 36
atttattcta acttatgcag gggtgcgaga tatgccccct tgctgtgaca cagatattta 60

<210> 37
<211> 60
<212> DNA
<213> Artificial

<220>
<223> Probe A_43_P21121

<400> 37
acctggagac ccactgtcac tggtgatggt gtagccctgc tggtttgggt gatccttgaa 60

<210> 38
<211> 60
<212> DNA
<213> Artificial

<220>
<223> Probe A_44_P180717

<400> 38
tggtgtgata aagaggttct ctgggctgct acatggaagt cccaagacca caccttctca 60

<210> 39
<211> 60
<212> DNA
<213> Artificial

<220>
<223> Probe A_42_P602724

<400> 39
gtgactacgg gagtggggtg atgagaagct caaaaccgac ttcctttaat caattaacca 60

<210> 40
<211> 60
<212> DNA
<213> Artificial

<220>
<223> Probe A_44_P332606

<400> 40
cctggtgagt tggggtcttt ttgacccttg tcacggttcc tccaacaaaa acttgcgcag 60

<210>  41
<211>  60
<212>  DNA
<213>  Artificial

<220>
<223>  Probe A_43_P12274

<400>  41
aggcagagga gaaggtgaag gcgtaaaaga agacgccagc actgactatg cctgcatcgt        60


<210>  42
<211>  60
<212>  DNA
<213>  Artificial

<220>
<223>  Probe A_44_P421534

<400>  42
tttctcagaa tgatgctggg caagagatct gccatcttga gaatgattct gatgtatgag        60


<210>  43
<211>  60
<212>  DNA
<213>  Artificial

<220>
<223>  Probe A_44_P248172

<400>  43
ggtttctcct ctatgttgat acagattacc tggattctat atcagaagac gatgagttgt        60


<210>  44
<211>  60
<212>  DNA
<213>  Artificial

<220>
<223>  Probe A_43_P13426

<400>  44
ttctcctttg attgctcttc ctaactggga ccaagttctg ggaaagaagg ctgatgttca        60


<210>  45
<211>  60
<212>  DNA
<213>  Artificial

<220>
<223>  Probe A_43_P11484

<400>  45
atcatattgc tgaaaatctt caaacacaaa ctctggggtg agcattacca ttgaacagtt        60


<210>  46
<211>  60
<212>  DNA

<213>  Artificial

<220>
<223>  Probe A_44_P285534

<400>  46
accccggaaa cgtattccta tttgaagcaa gttgaacgga cagagaaggg aagaagagaa        60


<210>  47
<211>  60
<212>  DNA
<213>  Artificial

<220>
<223>  Probe A_44_P421727

<400>  47
gagatgagta cctccggaaa cagaagacgg agaccatcat ctactcccga gaaaagaacc        60


<210>  48
<211>  20
<212>  DNA
<213>  Artificial

<220>
<223>  Primer RT1-A2 F

<400>  48
tccctccctg ctaccctgag                                                    20


<210>  49
<211>  20
<212>  DNA
<213>  Artificial

<220>
<223>  Primer RT1-A2 R

<400>  49
gccatccaca cttgggtcaa                                                    20


<210>  50
<211>  21
<212>  DNA
<213>  Artificial

<220>
<223>  Primer RT1-DMb F

<400>  50
tcaaatctgc ctcgggtgtt t                                                  21


<210>  51
<211>  20
<212>  DNA
<213>  Artificial

<220>

<223>  Primer RT1-DMb R

<400>  51
gacaaggtgg ggctttcagg                                                    20


<210>  52
<211>  20
<212>  DNA
<213>  Artificial

<220>
<223>  Primer Psmb8 F

<400>  52
cactgctggg cagacatcct                                                    20


<210>  53
<211>  20
<212>  DNA
<213>  Artificial

<220>
<223>  Primer Psmb8 R

<400>  53
gctttgtctc cagcccaggt                                                    20


<210>  54
<211>  20
<212>  DNA
<213>  Artificial

<220>
<223>  Primer Ly6g6e F

<400>  54
cccaggcaaa gggacagaag                                                    20


<210>  55
<211>  20
<212>  DNA
<213>  Artificial

<220>
<223>  Primer Ly6g6e R

<400>  55
tgagaccctc aggcaccaag                                                    20


<210>  56
<211>  20
<212>  DNA
<213>  Artificial

<220>
<223>  Primer Aif1 F

<400>  56

tcccccagcc aagaaagcta                                                    20

<210>  57
<211>  21
<212>  DNA
<213>  Artificial

<220>
<223>  Primer Aif1 R

<400>  57
tcttttccca tgctgctgtc a                                                  21

<210>  58
<211>  19
<212>  DNA
<213>  Artificial

<220>
<223>  Primer Lst1 F

<400>  58
gggcaggagc tccactacg                                                     19

<210>  59
<211>  21
<212>  DNA
<213>  Artificial

<220>
<223>  Primer Lst1 R

<400>  59
cgatgcaggc atagtcagtg c                                                  21

<210>  60
<211>  20
<212>  DNA
<213>  Artificial

<220>
<223>  Primer RT1-CE3 F

<400>  60
tgtcgtcctt ggagccatct                                                    20

<210>  61
<211>  20
<212>  DNA
<213>  Artificial

<220>
<223>  Primer RT1-CE3 R

<400>  61
tcctcacaac aggcaccaga                                                    20

<210>  62
<211>  20
<212>  DNA
<213>  Artificial

<220>
<223>  Primer RT1-CE10 F

<400>  62
acacaggtgg ggaaggagga                                                    20


<210>  63
<211>  22
<212>  DNA
<213>  Artificial

<220>
<223>  Primer RT1-CE10 R

<400>  63
caatctggga gggacacatc ag                                                 22


<210>  64
<211>  23
<212>  DNA
<213>  Artificial

<220>
<223>  Primer RT-BM1(RT1-S3) F

<400>  64
gcagctatgc tcatgttcta ggc                                                23


<210>  65
<211>  20
<212>  DNA
<213>  Artificial

<220>
<223>  Primer RT-BM1(RT1-S3) R

<400>  65
tgccttctga ggccagtcag                                                    20


<210>  66
<211>  22
<212>  DNA
<213>  Artificial

<220>
<223>  Primer Ubd F

<400>  66
tggggtgatg agaagctcaa aa                                                 22


<210>  67
<211>  26
<212>  DNA

<213>  Artificial

<220>
<223>  Primer Ubd R

<400>  67
ccccacctca aatctttatt tcattc                                          26


<210>  68
<211>  21
<212>  DNA
<213>  Artificial

<220>
<223>  Primer Olr1 F

<400>  68
ggaagtcaga agagggcatg g                                               21


<210>  69
<211>  22
<212>  DNA
<213>  Artificial

<220>
<223>  Primer Olr1 R

<400>  69
tcctgggttc aatttccaga gt                                              22


<210>  70
<211>  22
<212>  DNA
<213>  Artificial

<220>
<223>  Primer Ly49si1 F

<400>  70
tggccaatct gaattttcct tg                                              22


<210>  71
<211>  20
<212>  DNA
<213>  Artificial

<220>
<223>  Primer Ly49si1 R

<400>  71
   acatgggaag gggttcatgc                                                  20


<210>  72
<211>  20
<212>  DNA
<213>  Artificial

<220>

<223>  Primer Ly49i9 F

<400>  72
gggacttggc aacctcagga                                                    20


<210>  73
<211>  22
<212>  DNA
<213>  Artificial

<220>
<223>  Primer Ly49i9 R

<400>  73
ttggaacatc tgcacaatgg aa                                                 22


<210>  74
<211>  20
<212>  DNA
<213>  Artificial

<220>
<223>  Primer Cd3z F

<400>  74
agtgcctgct gggatttagc                                                    20


<210>  75
<211>  21
<212>  DNA
<213>  Artificial

<220>
<223>  Primer Cd3z R

<400>  75
catccatggt cacaggcact t                                                  21


<210>  76
<211>  20
<212>  DNA
<213>  Artificial

<220>
<223>  Primer B2m F

<400>  76
gagcaggttg ctccacaggt                                                    20


<210>  77
<211>  24
<212>  DNA
<213>  Artificial

<220>
<223>  Primer B2m R

<400>  77

caagctttga gtgcaagaga ttga 24

## Description

### Field of the invention

The major histocompatibility complex (MHC) is the most important genomic region that contributes to the risk of graft versus host disease (GVHD) after haematopoietic stem cell transplantation. Matching of MHC class I and II genes is essential for the success of transplantation. However, the MHC contains additional genes that also contribute to the risk of developing acute GVHD. The inventors identified rat and human MHC and NKC genes but also non-MHC and non-NKC genes that are regulated during graft versus host reaction (GVHR) in skin explant assays and could therefore serve as biomarkers for GVHD. Several of the respective human genes, including *HLA-DMB, C2, AIF1, SPR1, UBD,* and *OLR1,* are polymorphic. These candidates may therefore contribute to the genetic risk of GVHD in patients.

### Background of the invention

Haematopoietic stem cell transplantation (HSCT) is currently the only potentially curative treatment for many malignant and non-malignant haematological diseases. However, the overall survival rate after transplantation is still only 40 % to 60 % due to severe posttransplant complications, which include graft versus host disease (GVHD), relapse, and infection. Human leukocyte antigen (HLA) matching is essential to reduce the risk of graft rejection and GVHD. However, non-HLA genes also impact on transplant outcome and acute GVHD can be fatal even in patients receiving transplants from HLA-identical matched sibling donors (MSD). The cumulative incidence of grade 2 to 4 GVHD was 35 % in a recent study evaluating 1960 MSD transplants. MSDs are currently available for about one third of the patients and, therefore, alternative donors are needed. HLA-matched unrelated donors (MUD) are more widely accepted than cord blood or mismatched related donors.

The level of HLA matching used for selection of MUDs has changed over time and usually includes now HLA-A, B, C, and DRB1 loci (8/8 match). In some studies matching has been extended to the HLA-DQB1 locus (10/10 match). A large recent study has compared MSD and 8/8 matched MUD transplants in a homogenous cohort of patients with chronic myeloid leukemia and found a 2.44 times higher risk of grade 2 to 4 acute GVHD in 8/8 matched MUD compared to MSD transplants (Arora M, et al. (2009) J Clin Oncol 27: 1644-1652). In another study, the incidence of grade 2 to 4 acute GVHD was still higher in 10/10 matched MUD compared to MSD transplants (Yakoub-Agha I, et al. (2006) J Clin Oncol 24: 5695-5702). The higher risk of GVHD after MUD compared to MSD transplants could be due to a higher degree of similarity in non-HLA genes for siblings, who share 50 % of their genome with the respective recipient. However, also the HLA region itself could contribute to this difference since it harbors, in addition to the classical HLA class I and II genes, more than 200 other genes (Consortium TMS (1999) Nature 401: 921-923), many with immunological functions. In accordance with this hypothesis, mismatching of microsatellite markers in HLA class I, class II, and class III regions was associated with an increased risk of death in 10/10 matched MUD transplants. The HLA complex, as is the whole human genome, is organized into segments of closely linked genetic variants that are inherited as haplotypes on the same DNA strand. HLA haplotypes can be defined by HLA class I and II alleles and they are in strong linkage disequilibrium with defined genetic variants of non-class I/non-class II genes within the haplotype blocks within this region. Interestingly, HLA haplotype mismatching in 10/10 matched MUD transplants was associated with an increased risk of severe acute GVHD (Petersdorf EW, et al. (2007) PLoS Med 4: e8). This finding demonstrates that the HLA complex encodes in addition to HLA-A, B, C, DRB1, and DQB1 further unidentified genes that contribute significantly to the risk of developing acute GVHD. In case of disparity between donor and recipient alleles these genes may function as minor histocompatibility antigens. Alternatively, specific allelic variants may also increase the risk of GVHD, e.g., *TNFA,* a gene located within the class III region of the MHC encoding the pro-inflammatory cytokine tumor necrosis factor alpha (TNF-alpha). Several TNFA polymorphisms have been associated with an increased risk of GVHD and some of them are associated with increased TNF-alpha levels (Dickinson AM, et al. (2007) Expert Rev Mol Med 9: 1-19). The strong linkage disequilibrium within the HLA complex makes it very difficult to identify further non-class I/non-class II HLA genes involved in the pathophysiology of GVHD by genetic association studies alone.

HLA gene expression profiling may be an alternative strategy to identify HLA genes that are involved in the pathophysiology of GVHD. The inventors assumed that at least some non-class I/non-class II HLA genes that contribute to the risk of GVHD change their expression levels during disease progression. However, the genetic variation between clinical samples complicates the situation because allelic variation of gene expression could interfere with expression change in the pathophysiological process.

Accordingly, there is still a need for the identification of genes that contribute significantly to the risk of developing acute GVHD. These genes or gene markers may be used in the assessment of the risk to develop GVHD or GVHR, for the diagnosis of GVHD or GVHR, for monitoring treatment of GVHD or GVHR, and for screening for immunomodulating substances which may be useful in the treatment of GVHD or GVHR.

### Summary of the invention

In a first aspect, the invention relates to a method of predicting the risk of a subject to develop graft versus host reaction (GvHR) or graft versus host disease (GvHD), comprising
(a) determining the expression level of one or more prognostic RNA transcripts, or their corresponding cDNAs, or their expression products, in a sample obtained from said subject, wherein said transcript(s) or expression products is/are the transcript or expression product of one or more genes selected from the group consisting of:
   (i) Msr1, Pik3ap1, Pstpip1, Ctss, Pbx2, Grem1, Ly6g6e, Olr1, Spr1, Spic, Nfe2, Tnfaip8l2, and Ier3; or
   (ii) Msr1, Ctss, Pbx2, Grem1, Ly6g6e, Olr1, Spr1, Spic, and Nfe2; or
   (iii) Pik3ap1, Pstpip1, Tnfaip8l2, and Ier3;
(b) comparing the expression level of said one or more prognostic RNA transcript, or its corresponding cDNA, or its expression product with a corresponding baseline value;
   wherein
   (i) for every unit of increased expression of Olr1, Msr1, Pik3ap1, and/or Pstpip1; or the corresponding cDNA or expression product, said subject is expected to develop GvHR or GvHD; and
   (ii) for every unit of decreased expression of Ctss, Pbx2, Grem1, Ly6g6e, Spr1, Spic, Nfe2, Tnfaip8l2, and/or Ier3; or the corresponding cDNA or expression product, said subject is expected to develop GvHR or GvHD.

In a second aspect, the invention relates to a method of diagnosing graft versus host reaction (GvHR) or graft versus host disease (GvHD) in a subject following transplantation, comprising:
1. determining the expression level of one or more prognostic RNA transcripts, or their corresponding cDNAs, or their expression products, in a sample obtained from said subject, wherein said transcript(s) or expression products is/are the transcript or expression product of one or more genes selected from the group consisting of:
   (i) Msr1, Pik3ap1, Pstpip1, Ctss, Pbx2, Grem1, Ly6g6e, Olr1, Spr1, Spic, Nfe2 Tnfaip8l2, and Ier3; or
   (ii) Msr1, Ctss, Pbx2, Grem1, Ly6g6e, Olr1, Spr1, Spic, and Nfe2; or
   (iii) Pik3ap1, Pstpip1, Tnfaip8l2, and Ier3;
2. comparing the expression level of said one or more prognostic RNA transcript, or its corresponding cDNA, or its expression product with a corresponding baseline value;
   wherein
   (i) every unit of increased expression of Olrl, Msr1, Pik3ap1, and/or Pstpip1, or the corresponding cDNA or expression product, is indicative of GvHR or GvHD; and
   (ii) every unit of decreased expression of Ctss, Pbx2, Grem1, Ly6g6e, Spr1, Spic, Nfe2, Tnfaip8l2, and/or Ier3, or the corresponding cDNA or expression product, is indicative of GvHR or GvHD.

In a third aspect, the invention relates to a method of monitoring the efficacy of treatment of graft versus host reaction (GvHR) or graft versus host disease (GvHD) in a subject following transplantation, comprising:
(a) determining the expression level of one or more prognostic RNA transcripts, or their corresponding cDNAs, or their expression products, in a sample obtained from said subject at a first time point T1, and a later second time point T2, wherein said transcript(s) or expression products is/are the transcript or expression product of one or more genes selected from the group consisting of:
   (i) Msr1, Pik3ap1, Pstpip1, Ctss, Pbx2, Grem1, Ly6g6e, Olr1, Spr1, Spic, Nfe2, Tnfaip8l2, and Ier3; or
   (ii) Msr1, Ctss, Pbx2, Grem1, Ly6g6e, Olr1, Spr1, Spic, and Nfe2; or
   (iii) Pik3ap1, Pstpip1, Tnfaip8l2, and Ier3;
(b) comparing the expression level of said one or more prognostic RNA transcript, or its corresponding cDNA, or its expression product at time point T1 (Δ1) and time point T2 (Δ2) with a corresponding baseline value;
   wherein
   (i) a decline in units of an increased expression of Olr1, Msr1, Pik3ap1, and/or Pstpip1; or the corresponding cDNA or expression product at time point T2 in comparison with the increased expression of said at least one gene at the time point T1 (ΔΔ=Δ1-Δ2), is indicative of effective treatment of GvHR or GvHD; and
   (ii) a decline in units of a decreased expression of Ctss, Pbx2, Grem1, Ly6g6e, Spr1, Spic, Nfe2, Tnfaip8l2, and/or Ier3; or the corresponding cDNA or expression product at time point T2 in comparison with the decreased expression of said at least one gene at the time point T1(ΔΔ=Δ1-Δ2), is indicative of effective treatment of GvHR or GvHD.

In a fourth aspect, the invention relates to a method of screening for a candidate substance for treatment of graft versus host reaction (GvHR) or graft versus host disease (GvHD), comprising:
(a) monitoring the efficacy of treatment by said candidate substance by using the method according to the third aspect in
   (i) a non-human animal model which suffers from GvHR or GvHD and to which the candidate substance has been administered, or
   (ii) in an ex vivo model, including but not limited to cell-based and/or tissue-based GvHR or HvHD assay such as the Skin Explant Assay, wherein said cells and/or tissue have been contacted with said candidate substance; and
(b) selecting a candidate substance which shows effective treatment of GvHR or GvHD.

In a final aspect, the invention pertains to a use of a kit in a method of predicting the risk of developing graft versus host reaction (GvHR) or graft versus host disease (GvHD) according to the first aspect, or in a method of diagnosing GvHR or GvHD according to the second aspect, or in a method of monitoring the efficacy of treatment of GvHR or GvHD according to the third aspect, wherein the kit comprises at least one isolated polynucleotide, wherein each isolated polynucleotide independently comprises
(i) at least 20 contiguous nucleotides of the nucleotide sequence selected from SEQ ID NO: 1, 3, 5, 7, 8, 10, 12, 14, 16, 18, 20, 22, and/or 24; or SEQ ID NO: 26-47, or
(ii) a nucleotide sequence having at least 90% identity to (i), or
(iii) the coding region of a gene comprising a nucleotide sequence according to (i) or (ii), or
(iv) a nucleotide sequence that can specifically hybridize, under conditions of high stringency, to a polynucleotide having a nucleotide sequence according to (i), (ii) or (iii); and
wherein the kit comprises no more than 9000 isolated polynucleotides in total.

### Detailed description of the invention

In an exploratory experiment, the inventors analyzed the expression of 169 genes with human homologues, including the respective MHC and NKC region genes, identified in the rat in human skin explant samples (c.f. example 2, and Table 9). These human skin explants were cultured for 1, 2, or 3 days resulting in GVHR of grades I, II, and III, respectively. Notably, 69 % of all tested human genes were found to be regulated in at least one of these human samples as predicted by the results of the rat expression profiling experiments. 21 %, i. e. 36 of the tested genes, were regulated in all 3 human skin explant samples in accordance with the rat model, but this regulation varied depending on the GVHR grade and the time course of the skin explant assay. Although the inventors only validated these genes firstly on 3 samples, the unexpectedly high concordance rate between the results of rat and human skin explant assays strongly suggests that the rat skin explant assay is an informative model for human GVHR and possibly GVHD.

Interestingly, for some of the genes that were found to be regulated in GVHR and GVHD in the rat, the human homologues are polymorphic and disease associations of gene polymorphisms have been described. These include *HLA-DMB, C2, AIF1, SPR1,* and possibly *UBD.* Therefore, these genes are especially interesting candidates of further non-class I/class II HLA genes that might confer an increased genetic risk of GVHD after HSCT depending on the genotype. In addition, the *OLR1* gene in the NKC is polymorphic and polymorphisms of this gene have been associated with athero-sclerosis, myocardial infarction, and Alzheimer's disease.

Several laboratory tests have been assessed for their ability to predict the risk of GVHD in patients. The skin explant assay has a predictive value of about 80 % when cyclosporine alone is used for GVHD prophylaxis. A gene expression analysis of selected genes may help to further improve the predictive value of the assay. Pre-transplant gene expression profiling of donor peripheral blood mononuclear cells (PBMC) has recently been shown to be a useful tool to predict the risk of GVHD. Post transplant differences in the gene expression profile of PBMC of patients with acute and chronic GVHD compared to non-GVHD samples have been described.

The inventors identified rat and human MHC and NKC genes but also non-MHC and non-NKC genes that are regulated during GVHR in skin explant assays and could therefore serve as biomarkers for GVHD. Several of the respective human genes, including *HLA-DMB, C2, AIF1, SPR1, UBD,* and *OLR1,* are polymorphic. These candidates may therefore contribute to the genetic risk of GVHD in patients.

The inventors observed a statistically significant and strong up or down regulation of 11 MHC, 6 NKC, and 168 genes encoded in other genomic regions, i.e. 4.9 %, 14.0 %, and 2.6 % of the tested genes respectively. The regulation of 7 selected MHC and 3 NKC genes was confirmed by quantitative real-time PCR and in independent skin explant assays. In addition, similar regulations of most of the selected genes were observed in GVHD-affected skin lesions of transplanted rats and in human skin explant assays.

The inventors aimed to identify genes that are regulated during GVHR in the skin explant assay because these genes could be involved in the pathophysiology of GVHR and contribute to the genetic risk of GVHD. Special attention was given to genes encoded within the MHC region for the following reasons: Firstly, evidence has been presented that further risk genes for GVHD in addition to MHC class I and class II genes are present in this region. Secondly, those genes cannot easily be identified by genetic linkage analysis alone due to the strong linkage disequilibrium with MHC class I and class II genes so that expression profiling could be a worthwhile alternative approach. Thirdly, the inventors wanted to focus in this initial study on a fully characterized genomic region of special immunological importance rather than to follow a whole genome expression profiling approach. Importantly, 39 % of the BN rat MHC genes (*RT1ⁿ* haplotype) annotated by Hurt and colleagues (Hurt P, et al. (2004) Genome Res 14: 631-639) were at the time point of array construction not represented in the Agilent database and therefore not represented on the Agilent whole rat genome array. In addition to the MHC region, genes of the NKC region were included because this region encodes *Ly49* genes and their products can function as receptors for the numerous MHC class Ia and Ib gene products encoded in the MHC. A higher percentage of MHC genes and NKC genes than genes in other regions of the genome were found to be regulated in the allogeneic skin explants compared to skin samples co-cultured with syngeneic lymphocytes. Of the 25 MHC genes found to be significantly regulated (p<0.05), 5 are known to be involved in antigen processing and presentation. Besides two of three MHC class Ia genes in the BN strain (*RT1-A1* and *RT1-A2*) that present peptides to cytotoxic T lymphocytes (CTL), the genes *Tap1* and *Psmb8,* encoding a subunit of the antigen transporter and a subunit of the immunoproteasome (also known as LMP7), were found to be up-regulated. *RT1-DMb* encodes a homologue of *HLA-DMB,* a chaperone in the MHC class II presentation pathway. Furthermore, non-classical MHC class Ib genes (*RT1-CE2, RT1-CE3, RT1-CE5, RT1-CE8, RT1-CE10, RT1-CE16, RT1-T24-4, RT-BM1*) were up-regulated during GVHR in the skin explants. The function of the RT1-C/E/M class I genes is not well defined. It is known that they can become targets of CTL and function as ligands for activating or inhibitory NK receptors. RT1-C/E/M incompatibility has been shown to induce skin and pancreas graft rejection and to modulate the fate of MHC class II-mismatched heart grafts. The *RT1-T24-4* gene belongs to a family of genes that was originally identified as pseudogenes in the haplotype *r21.* In the *RT1ⁿ* haplotype all four family members are presumably functional. However, their actual function has not been experimentally demonstrated so far. The *RT-BM1* (*RT1-S3*) gene is assumed to be orthologous to the mouse *H2-T23*gene, which encodes the Qa-1 molecule. This is a functional homologue of HLA-E, which presents leader peptides of MHC class I molecules to the inhibitory NK receptor CD94/NKG2A. Interestingly, its expression can vary substantially depending on the RT1 haplotype. It has to be noticed that no human/rat orthology can be established for the class I genes in the various class I clusters. Therefore, with respect to class I genes, the rat cannot serve as a model for the HLA complex. However, the non-class I genes are clearly orthologous.

In addition to *Tap1, Psmb8,* and *RT1-DMb,* 12 further non-class I MHC genes were found to be regulated in the rat skin explant assays, some of them also involved in the immune response, such as the complement component *C2,* while such a role is strongly assumed for other genes. The allograft inflammatory factor 1 (*Aif1*), was cloned from chronically rejecting rat cardiac allografts and it was also found in transplanted human hearts. Persistent expression of AIF-1 is associated with the development of a cardiac allograft vasculopathy. The expression of AIF-1 is mostly limited to the monocyte/macrophage lineage, and can be augmented by interferon (IFN)-γ. The specific function of the leukocyte specific transcript 1 (*Lst1*) gene is not known, although its strong expression in dendritic cells and functional data suggest an immunomodulatory role. The expression of human *LST1,* specifically of splice variants encoding soluble isoforms, was increased in rheumatoid arthritis-affected blood and synovium and was up-regulated in response to IFN-γ. The immediate early response *3* (*Ier3*) gene is stress-inducible and is involved in the regulation of cell death and oncogenesis. The protein (also known as IEX-1 or IEX-1L) functions in the protection of cells from Fas or TNF-α-induced apoptosis. However, it increases the rate of apoptosis in ultraviolet B irradiated keratinocytes. Distinct domains of the proteins were described to be responsible for pro and anti-apoptotic activities of the protein. The diubiquitin gene (*Ubd*) has been shown to be expressed in rat lymphoblasts, thymus, and testis. In the mouse it is expressed in dendritic cells and B cells, is inducible by IFN-γ, and can cause apoptosis. The protein (also known as FAT10) provides an ubiquitin-independent signal for proteasomal degradation. It has been suggested to participate in antigen processing, but its expression did not affect MHC class I expression or antigen presentation. In view of the reported roles of these genes in the immune response, a direct involvement in GVHD is conceivable.

For the other regulated MHC genes an involvement in immune functions has not been established so far. *Spr1* (or *Psors1c2*) is the psoriasis susceptibility 1 candidate 2 gene and was found to be expressed in the thymus of rats. Its human homologue is expressed in normal and psoriasis skin and has been suggested to confer susceptibility to psoriasis. The function of the gene product is not known so far. *G18* (*Gpsm3*) is an activator of G-protein signaling. *Pbx2* encodes an ubiquitously expressed transcriptional activator. The *Ly6g6e* gene belongs to the lymphocyte antigen 6 (Ly-6) superfamily that encodes proteins attached to the cell surface by a glycosylphospha-tidylinositol (GPI) anchor that is directly involved in signal transduction. Mouse Ly6g6e was found to be highly expressed at the leading edges of cells, on filopodia, which are normally involved in cell adhesion and migration. The mitochondrial ribosomal protein S18B (*Mrsps18b*) gene encodes a 28S subunit protein that belongs to the ribosomal protein S18P family. The functions of the HLA-B associated transcript 5 (*Bat5*) and *Fij13158* (or *RGD1303066*) genes have not been characterized so far. Many of the up-regulated MHC genes are inducible by IFN-γ, a type II cytokine that is primarily secreted by activated T and NK cells. Several studies have demonstrated an increased level of IFN-γ in the early phase of GVHD. Therefore, this cytokine might be highly important for the regulation of the expression of MHC genes during GVHR. The inventors also included the NKC region in the expression profiling which harbors the Ly49 genes that encode NK receptors of the killer cell lectin-like receptor type and some of these have been shown to interact with both MHC class Ia and Ib molecules. In contrast to the MHC region, no reference sequence has been published for the NKC region of the rat. Therefore, 20 genes that were recently assigned to this region in the assembly RGSC v3.4 (Twigger et al. (2008) Nat. Genet. 40: 523-527) were not represented on the array. However, for most of them no function associated with the immune system has been reported. Interestingly, only *Ly49* receptor genes which have an ITIM motif in their cytoplasmic region were up-regulated in the allogeneic skin explant assays. This includes also the *LOC690045* gene which encodes an immunoreceptor similar to *Ly49si1.* It is not clear whether one of these gene products interacts with the MHC class Ib molecules that the inventors found to be up-regulated. Ly49 receptors are normally present mainly on NK cells and the skin explants harbored few leukocytes. However, skin resident lymphocytes can become activated in human skin explant assays. Although few NK cells infiltrating a tissue that normally does not contain these cells might cause a drastic relative change in the presence of *Ly49* transcripts, the possibility should not be dismissed that other cells may express the receptors under pathological conditions. The role of NK cells for GVHR in skin explants needs to be further explored. In general NK cells are assumed to prevent GVHR, improve engraftment and to exert strong graft-versus-leukemia effects without causing GVHD.

In the NKC region the inventors found one non*-Ly49* gene to be regulated. The *Olr1* gene encodes a receptor protein which belongs to the C-type lectin superfamily. The protein (also known as LOX-1) binds, internalizes and degrades oxidized low-density lipoprotein, which induces vascular endothelial cell activation and dysfunction, resulting in pro-inflammatory responses, pro-oxidative conditions and apoptosis. In addition, it acts as a receptor for extracellular heat shock protein 70 on dendritic cells. Binding and internalization of heat shock protein 70/peptide complexes channels peptides into the MHC class I presentation pathway. Thus, the protein is involved in antigen cross-presentation to naive T cells.

In addition to the MHC and NKC region genes, 168 further genes were significantly regulated in allogeneic skin explants. Many of them also have immunological functions and need to be analyzed in more detail in subsequent studies.

The results obtained in the MHC and NKC gene expression profiling experiment were confirmed in most tested cases by qRT-PCR on the skin explant samples. Some genes, e.g. *Aif1* and *Ly49i9,* appeared to be up-regulated even in grade I GVHR. *Olr1,* in contrast, was up-regulated predominantly in grade II and III GVHR in all comparisons. Importantly, several of the MHC and NKC genes that were identified to be regulated in the skin explant assays, including *Aif1, Lst1,* and *Olr1,* were also regulated in the GVHD affected skin of transplanted animals. Thus, the skin explant assay can model GVHD not only histologically but also with respect to gene regulation. However, the up-regulation of the tested *Ly49* genes (*Ly49si1* and *Ly49i9*) that were observed in the skin explant was not clearly confirmed in the GVHD-affected skin of transplanted rats. Skin lesions from transplanted animals are likely to be more heterogeneous with respect to the dynamics of the pathophysiological process than skin explant samples, and this may contribute to the variation in results.

In conclusion, the MHC gene expression profiling approach in the rat skin explant assay identified a number of non-class I/class II genes that might contribute to the MHC-associated risk of GVHD following HSCT. These genes could be directly involved in the pathophysiology of GVHD or serve as molecular markers for GVHD and GVHR. The possibility should not be dismissed, however, that these marker genes could indicate that protective pathways are induced which modulate tissue damage during inflammation. Moreover, their human homologues may be useful for risk assessment, diagnosis, and as potential targets for therapy of GVHD in patients.

Accordingly, in a first aspect, the invention relates to a method of predicting the risk of a subject to develop graft versus host reaction (GvHR) or graft versus host disease (GvHD), comprising
(a) determining the expression level of one or more prognostic RNA transcripts, or their corresponding cDNAs, or their expression products, in a sample obtained from said subject, wherein said transcript(s) or expression products is/are the transcript or expression product of one or more genes selected from the group consisting of:
   (i) Msr1, Pik3ap1, Pstpip1, Ctss, Pbx2, Grem1, Ly6g6e, Olr1, Spr1, Spic, Nfe2, Tnfaip8l2, and Ier3; or
   (ii) Msr1, Ctss, Pbx2, Grem1, Ly6g6e, Olr1, Spr1, Spic, and Nfe2; or
   (iii) Pik3ap1, Pstpip1, Tnfaip8l2, and Ier3;
(b) comparing the expression level of said one or more prognostic RNA transcript, or its corresponding cDNA, or its expression product with a corresponding baseline value;
   wherein
   (i) for every unit of increased expression of Olr1, Msr1, Pik3ap1, and/or Pstpip1; or the corresponding cDNA or expression product, said subject is expected to develop GvHR or GvHD; and
   (ii) for every unit of decreased expression of Ctss, Pbx2, Grem1, Ly6g6e, Spr1, Spic, Nfe2, Tnfaip8l2, and/or Ier3; or the corresponding cDNA or expression product, said subject is expected to develop GvHR or GvHD.

The term "predicting the risk of a subject" is used herein to refer to the prediction of the likelihood of a subject to develop graft versus host reaction (GvHR) or graft versus host disease (GvHD). The method of the invention may be used clinically in order to determine the best treatment modalities and regimen and/or to evaluate whether said patient is likely to respond favourably to a treatment, such as surgical intervention, as for example a transplantation, in particular with regard to dosage and/or drug combinations.

The terms "graft versus host reaction" and "graft versus host disease" may be used synonymously. Usually, 3 criteria must be met in order for GvHD to occur: (1) Administration of an immunocompetent graft, with viable and functional immune cells, (2) the recipient is immunologically disparate - histoincompatible, and (3) the recipient is immunocompromised and therefore cannot destroy or inactivate the transplanted cells. Following transplantation, T cells present in the graft, either as contaminants or intentionally introduced into the host, perceive host tissues as antigenically foreign and attack the tissues of the transplant recipient. GvHD occurs not only when there is a mismatch of a major MHC class I or II antigen but also in the context of disparities between minor histocompatibility antigens. GvHD is a common complication in recipients of bone marrow transplants from, e.g., HLA-identical siblings, who typically differ from each other in many polymorphic proteins encoded by genes unlinked to the MHC.

Clinically, GvHD is divided into acute and chronic forms. Acute and chronic GvHD appear to involve different immune cell subsets, different cytokine profiles, different host targets, and respond differently to treatment. For example, the acute form of GvHD is normally observed within the first 100 days post-transplant, and is a major challenge to transplants owing to associated morbidity and mortality. In contrast thereto, the chronic form of GvHD normally occurs after 100 days. The appearance of moderate to severe cases of chronic GvHD adversely influences long-term survival. In order to determine the expression level of one or more prognostic RNA transcripts, or their corresponding cDNAs, or their expression products of one or more genes, a sample comprising cells from the subject and, thus, the prognostic RNA transcripts or their expression products is first derived from said subject.

The term "sample", as used herein, refers to a sample comprising cells of the subject to be tested, which may be the graft or the host in question, which cells may be homogenized and disrupted in order to release and optionally isolate the prognostic RNA transcripts. Preferably, the sample is a biopsy sample, preferably a biopsy sample of the tissue to be transplanted or of the tissue wherein the transplant is grafted, or a sample of Peripheral Blood Mononuclear Cells (PBMC). A peripheral blood mono-nuclear cell (PBMC) is a blood cell having a round nucleus. In general, these cells are immune cells, such as lymphocytes (e.g., T cells, B cells, and NK cells), monocytes or macrophages. These cells are often extracted from whole blood using ficoll, a hydrophilic polysaccharide that separates layers of blood, with monocytes and lymphocytes forming a buffy coat containing said PBMCs under a layer of plasma. Alternatively, PBMC can be extracted from whole blood using a hypotonic lysis which will preferentially lyse red blood cells. This method results in neutrophils and other polymorpho-nuclear (PMN) cells which are important in innate immune defence being obtained. However any other suitable method may be used in order to isolate PBMC from the subject.

Said RNA transcripts may subsequently be used directly or processed into another form, such as cRNA, cDNA or PCR amplification products, which still represent the expressed genes in said sample of cells, i.e. the transcripts of these genes. RNA can be isolated according to any of a number of methods well known to those of skill in the art. For example, mRNA is isolated using oligo d(T) column chromatography or glass beads. For example, RNA extraction may be performed by using TRIZOL reagent (Invitrogen, Carlsbad, CA, USA), as described in more detail in the examples. Alternatively, a cDNA may be reverse transcribed from said prognostic RNA transcript, RNA transcribed from that cDNA, a DNA amplified from that cDNA, RNA transcribed from the amplified DNA, or the like. Total mRNA can be converted to cDNA and amplified by conventional procedures, for example, by reverse transcription in a *per se* known manner. A cDNA may be amplified by any of a variety of conventional amplification procedures, including PCR. Suitable PCR primers can be selected using any well-known methods. Further examples of primers are given in the Examples section below.

For example, the level of expression of a prognostic RNA transcript or their corresponding cDNA in a sample is determined by hybridizing said RNA transcript or corresponding cDNA to a detectable probe, e.g. by performing a microarray, such as a DNA microarray. Alternatively, the expression level may be determined by using quantitative PCR. Then, the mRNA copy number may be calculated from the amount of hybridization, which generally reflects the level of expression of the polynucleotide in the cells of the sample, normalized to the amount of total RNA (or cDNA) or to the expression level of one or more housekeeping genes.

Methods for detecting hybridization are well known in the art. For example, the prognostic RNA transcript or corresponding cDNA may be labelled with a fluorescent label and levels and patterns of fluorescence indicative of hybridization are measured, e.g. by fluorescence microscopy, preferably confocal fluorescence microscopy. In this detection method, an argon ion laser excites the fluorescent label, emissions are directed to a photomultiplier and the amount of emitted light detected and quantitated. The detected signals are considered to be proportional to the amount of probe/target hybridization complex at each position of the microarray. Further, the fluorescence microscope may be associated with a computer-driven scanner device to generate a quantitative two- dimensional image of hybridization intensity. The scanned image is examined to determine the abundance/expression level of each hybridized target transcript. Alternatively, a fluorescent imaging device, such as a microarray scanner, may be used.

Typically, array fluorescence intensities can be normalized to take into account variations in hybridization intensities when more than one array is used under similar test conditions. This may be achieved by using the intensities derived from internal normalization controls contained on each microarray, e.g. from housekeeping genes. Accordingly, "normalized" refers to the expression level of an RNA transcript relative to the expression level of the total RNA or relative to the expression level of a housekeeping gene. Housekeeping genes are genes that are constitutively transcribed at a relatively constant level across many or all known conditions, since the housekeeping gene's products are typically needed for maintenance of the cell. Examples of housekeeping genes include actin, GAPDH, and ubiquitin.

However, further methods for determining the amount of a polynucleotide are well known in the art and may include any suitable quantitative method. Examples for such further methods are, for example, quantitative PCR, such as real-time PCR, or reverse transcription PCR (RT-PCR), using primers specific for those polynucleotides. Methods for selecting suitable primers for detecting and quantitating the amplified product are known in the art and exemplified in the Examples section below. Alternatively, the expression level may be determined by the expression product(s), i.e. by the polypeptides encoded by said genes. This may be accomplished using immunological methods involving the use of antibodies directed against said polypeptides, e.g. the expression level of the corresponding expression product(s) is determined by ELISA or immunohistochemistry.

In order to perform an ELISA the sample with an unknown amount of expression product is immobilized on a solid support either non-specifically via adsorption to the surface of the solid support or specifically by a so called capture-antibody specific to the expression product. After the antigen is immobilized the detection antibody is added, forming a complex with the antigen. The detection antibody can itself be covalently linked to an enzyme, or can be detected by a secondary antibody linked to an enzyme. Between each step the plate is typically washed with a mild detergent solution to remove any proteins or antibodies that are not specifically bound. Detection occurs by adding an enzymatic substrate to produce a visible signal, which indicates the quantity of expression product in the sample. Immunohistochemistry refers to a method involving localizing the expression product in said cells of the sample using fluorescence labelled antibodies and determining the fluorescence intensity. However, any suitable method may be used for determining the expression level of said expression product(s), such as by way of Western blotting, protein microarray, flow cytometry or surface plasmon resonance.

Thus, in a preferred embodiment, the expression level is determined by DNA microarray analysis or quantitative PCR and subsequent calculation of the mRNA copy number normalized to the amount of total RNA or to the expression level of one or more housekeeping genes. In another preferred embodiment the expression level of the corresponding expression product(s) is determined by ELISA, Western blotting, protein microarray or immunohistochemistry, flow cytometry or surface plasmon resonance.

The term "every unit of increased expression" and the term "every unit of decreased expression" as used herein refers to an expression level of one or more prognostic RNA transcripts, or their corresponding cDNAs, or their expression product(s) that has been found differentially expressed in subjects suffering or prone to suffer from GvHD or GvHR in comparison to healthy subjects. Thus, in case of "every unit of increased expression", the higher the expression level of a gene which is predominantly expressed in the cells of a subject who suffers or is prone to suffer from GvHD or GvHR, the higher is the risk that the subject to be tested is expected to develop GvHD or GvHR. Likewise, in case of "every unit of decreased expression", the lower the expression level of a gene which is predominantly expressed in healthy subjects but not in subjects suffering or prone to suffer from GvHD or GvHR, the higher is the risk that the subject to be tested develops GvHR or GvHD.

The determined expression level may be compared to a corresponding baseline value. As used herein, the term "corresponding baseline value" refers to the level of gene expression in normal cells or PBMCs, e.g. in a sample from a healthy subject or from a "pool" of samples derived from healthy subjects; or from a pool of one or more tissues from healthy subjects. Any of the above types of baseline values may be available in a database compiled from such values. Therefore, in a preferred embodiment, the baseline value may be the expression level of said at least one gene in at least one healthy subject.

An expression level of a gene may be considered as being increased if the log2-fold change is at least 1, such as at least 1.1, or at least 1.2, preferably at least 1.25, such as at least 1.5 or at least 1.75, more preferably at least 2.0, such as at least 2.25 or at least 2.5, and most preferably at least 2.75 or even at least 3.0. Likewise, an expression level of a gene may be considered as being decreased if the log2-fold change is at least -1, such as at least -1.1, or at least -1.2, preferably at least -1.25, such as at least -1.5 or at least -1.75, more preferably at least -2.0, such as at least -2.25 or at least -2.5, and most preferably at least -2.75 or even at least -3.0. Alternatively, the term "increased" amount means herein an amount which is typically at least 120 %, at least 130 %, at least 140 %, at least 150 %, at least 175 %, preferably at least 200 %, at least 225 %, at least 250 %, at least 275 %, more preferably at least 300 %, at least 350 %, or at least 400 %, most preferably at least 500 % of the baseline value.

Likewise, the term "decreased", as meant herein, refers to an amount which is typically less than 90%, less than 85%, less than 80%, less than 75%, more preferably less than 70 %, less than 65 %, less than 60 %, even more preferably less than 50 %, less than 40 %, or less than 30 %, most preferably less than 25 %, less than 20 %, or even less than 10 % of the baseline value.

The term "one or more" as used herein means that either one, two, three, four, five, six, seven, eight, nine, ten, elven, twelve, or all thirteen expression level(s) of said genes is/are determined.

The term "corresponding", as used herein, refers to the baseline value of the same gene as determined in the sample. The genes and their respective reference sequence is given in **Table 10** below as well as in SEQ ID NOs 1-25.

The following combinations of biomarkers are contemplated to be particularly useful:

| Combi nation | Ctss | Pbx2 | Grem1 | Ly6g6e | Olr1 | Spr1 | Msr1 | Spic | Nfe2 | Tnfaip8l2 | Ier3 | Pik3aP1 | Pstpip1 |
|---|---|---|---|---|---|---|---|---|---|---|---|---|---|
| 1 | + | | | | | | | | | | | | |
| 2 | | + | | | | | | | | | | | |
| 3 | | | + | | | | | | | | | | |
| 4 | | | | + | | | | | | | | | |
| 5 | | | | | + | | | | | | | | |
| 6 | | | | | | | | | | | | | |
| 7 | | | | | | + | | | | | | | |
| 8 | | | | | | | + | | | | | | |
| 9 | | | | | | | | + | | | | | |
| 10 | | | | | | | | | + | | | | |
| 11 | | | | | | | | | | + | | | |
| 12 | | | | | | | | | | | + | | |
| 13 | | | | | | | | | | | | + | |
| 14 | | | | | | | | | | | | | + |
| 15 | + | + | | | | | | | | | | | |
| 16 | + | | + | | | | | | | | | | |
| 17 | + | | | + | | | | | | | | | |
| 18 | + | | | | + | | | | | | | | |
| 19 | + | | | | | | | | | | | | |
| 20 | + | | | | | + | | | | | | | |
| 21 | + | | | | | | + | | | | | | |
| 22 | + | | | | | | | + | | | | | |
| 23 | + | | | | | | | | + | | | | |
| 24 | + | | | | | | | | | + | | | |
| 25 | + | | | | | | | | | | + | | |
| 26 | + | | | | | | | | | | | + | |
| 27 | + | | | | | | | | | | | | + |
| 28 | | + | + | | | | | | | | | | |
| 29 | | + | | + | | | | | | | | | |
| 30 | | + | | | + | | | | | | | | |
| 31 | | + | | | | | | | | | | | |
| 32 | | + | | | | + | | | | | | | |
| 33 | | + | | | | | + | | | | | | |
| 34 | | + | | | | | | + | | | | | |
| 35 | | + | | | | | | | + | | | | |
| 36 | | + | | | | | | | | + | | | |
| 37 | | + | | | | | | | | | + | | |
| 38 | | + | | | | | | | | | | + | |
| 39 | | + | | | | | | | | | | | + |
| 40 | | + | | | | | | | | | | | |
| 41 | | + | | | | | | | | | | | |
| 42 | | + | | | | | | | | | | | |
| 43 | | | + | + | | | | | | | | | |
| 44 | | | + | | + | | | | | | | | |
| 45 | | | + | | | | | | | | | | |
| 46 | | | + | | | + | | | | | | | |
| 47 | | | + | | | | + | | | | | | |
| 48 | | | + | | | | | + | | | | | |
| 49 | | | + | | | | | | + | | | | |
| 50 | | | + | | | | | | | + | | | |
| 51 | | | + | | | | | | | | + | | |
| 52 | | | + | | | | | | | | | + | |
| 53 | | | + | | | | | | | | | | + |
| 54 | | | | + | + | | | | | | | | |
| 55 | | | | + | | | | | | | | | |
| 56 | | | | + | | + | | | | | | | |
| 57 | | | | + | | | + | | | | | | |
| 58 | | | | + | | | | + | | | | | |
| 59 | | | | + | | | | | + | | | | |
| 60 | | | | + | | | | | | + | | | |
| 61 | | | | + | | | | | | | + | | |
| 62 | | | | + | | | | | | | | + | |
| 63 | | | | + | | | | | | | | | + |
| 64 | | | | | + | | | | | | | | |
| 65 | | | | | + | + | | | | | | | |
| 66 | | | | | + | | + | | | | | | |
| 67 | | | | | + | | | + | | | | | |
| 68 | | | | | + | | | | + | | | | |
| 69 | | | | | + | | | | | + | | | |
| 70 | | | | | + | | | | | | + | | |
| 71 | | | | | + | | | | | | | + | |
| 72 | | | | | + | | | | | | | | + |
| 73 | | | | | | + | | | | | | | |
| 74 | | | | | | | + | | | | | | |
| 75 | | | | | | | | + | | | | | |
| 76 | | | | | | | | | + | | | | |
| 77 | | | | | | | | | | + | | | |
| 78 | | | | | | | | | | | + | | |
| 79 | | | | | | | | | | | | + | |
| 80 | | | | | | | | | | | | | + |
| 81 | | | | | | + | + | | | | | | |
| 82 | | | | | | + | | + | | | | | |
| 83 | | | | | | + | | | + | | | | |
| 84 | | | | | | + | | | | + | | | |
| 85 | | | | | | + | | | | | + | | |
| 86 | | | | | | + | | | | | | + | |
| 87 | | | | | | + | | | | | | | + |
| 88 | | | | | | | + | + | | | | | |
| 89 | | | | | | | + | | + | | | | |
| 90 | | | | | | | + | | | + | | | |
| 91 | | | | | | | + | | | | + | | |
| 92 | | | | | | | + | | | | | + | |
| 93 | | | | | | | + | | | | | | + |
| 94 | | | | | | | | + | + | | | | |
| 95 | | | | | | | | + | | + | | | |
| 96 | | | | | | | | + | | | + | | |
| 97 | | | | | | | | + | | | | + | |
| 98 | | | | | | | | + | | | | | + |
| 100 | | | | | | | | | + | + | | | |
| 101 | | | | | | | | | + | | + | | |
| 102 | | | | | | | | | + | | | + | |
| 103 | | | | | | | | | + | | | | + |
| 104 | | | | | | | | | | + | + | | |
| 105 | | | | | | | | | | + | | + | |
| 106 | | | | | | | | | | + | | | + |
| 107 | | | | | | | | | | | + | + | |
| 108 | | | | | | | | | | | + | | + |
| 109 | | | | | | | | | | | | + | + |
| 110 | + | + | + | | | | | | | | | | |
| 112 | + | + | | + | | | | | | | | | |
| 113 | + | + | | | + | | | | | | | | |
| 114 | + | + | | | | | | | | | | | |
| 115 | + | + | | | | + | | | | | | | |
| 116 | + | + | | | | | + | | | | | | |
| 117 | + | + | | | | | | + | | | | | |
| 118 | + | + | | | | | | | + | | | | |
| 119 | + | + | | | | | | | | + | | | |
| 120 | + | + | | | | | | | | | + | | |
| 121 | + | + | | | | | | | | | | + | |
| 122 | + | + | | | | | | | | | | | + |
| 123 | | + | + | + | | | | | | | | | |
| 124 | | + | + | | + | | | | | | | | |
| 125 | | + | + | | | | | | | | | | |
| 126 | | + | + | | | + | | | | | | | |
| 127 | | + | + | | | | + | | | | | | |
| 128 | | + | + | | | | | + | | | | | |
| 129 | | + | + | | | | | | + | | | | |
| 130 | | + | + | | | | | | | + | | | |
| 131 | | + | + | | | | | | | | + | | |
| 132 | | + | + | | | | | | | | | + | |
| 133 | | + | + | | | | | | | | | | + |
| 134 | | | + | + | + | | | | | | | | |
| 135 | | | + | + | | | | | | | | | |
| 136 | | | + | + | | + | | | | | | | |
| 137 | | | + | + | | | + | | | | | | |
| 138 | | | + | + | | | | + | | | | | |
| 139 | | | + | + | | | | | + | | | | |
| 140 | | | + | + | | | | | | + | | | |
| 141 | | | + | + | | | | | | | + | | |
| 142 | | | + | + | | | | | | | | + | |
| 143 | | | + | + | | | | | | | | | + |
| 144 | | | | + | + | | | | | | | | |
| 145 | | | | + | + | + | | | | | | | |
| 146 | | | | + | + | | + | | | | | | |
| 147 | | | | + | + | | | + | | | | | |
| 148 | | | | + | + | | | | + | | | | |
| 149 | | | | + | + | | | | | + | | | |
| 150 | | | | + | + | | | | | | + | | |
| 151 | | | | + | + | | | | | | | + | |
| 152 | | | | + | + | | | | | | | | + |
| 153 | | | | | + | + | | | | | | | |
| 154 | | | | | + | | + | | | | | | |
| 155 | | | | | + | | | + | | | | | |
| 156 | | | | | + | | | | + | | | | |
| 157 | | | | | + | | | | | + | | | |
| 158 | | | | | + | | | | | | + | | |
| 159 | | | | | + | | | | | | | + | |
| 160 | | | | | + | | | | | | | | + |
| 161 | | | | | | + | + | | | | | | |
| 162 | | | | | | + | | + | | | | | |
| 163 | | | | | | + | | | + | | | | |
| 164 | | | | | | + | | | | + | | | |
| 165 | | | | | | + | | | | | + | | |
| 166 | | | | | | + | | | | | | + | |
| 167 | | | | | | + | | | | | | | + |
| 168 | | | | | | + | + | + | | | | | |
| 169 | | | | | | + | + | | + | | | | |
| 170 | | | | | | + | + | | | + | | | |
| 171 | | | | | | + | + | | | | + | | |
| 172 | | | | | | + | + | | | | | + | |
| 173 | | | | | | + | + | | | | | | + |
| 174 | | | | | | | + | + | + | | | | |
| 175 | | | | | | | + | + | | + | | | |
| 176 | | | | | | | + | + | | | + | | |
| 177 | | | | | | | + | + | | | | + | |
| 178 | | | | | | | + | + | | | | | + |
| 179 | | | | | | | | + | + | + | | | |
| 180 | | | | | | | | + | + | | + | | |
| 181 | | | | | | | | + | + | | | + | |
| 182 | | | | | | | | + | + | | | | + |
| 183 | | | | | | | | | + | + | + | | |
| 184 | | | | | | | | | + | + | | + | |
| 185 | | | | | | | | | + | + | | | + |
| 186 | | | | | | | | | | + | + | + | |
| 187 | | | | | | | | | | + | + | | + |
| 188 | | | | | | | | | | | + | + | + |
| 189 | + | + | + | + | | | | | | | | | |
| 190 | + | + | + | | + | | | | | | | | |
| 191 | + | + | + | | | | | | | | | | |
| 192 | + | + | + | | | + | | | | | | | |
| 193 | + | + | + | | | | + | | | | | | |
| 194 | + | + | + | | | | | + | | | | | |
| 195 | + | + | + | | | | | | + | | | | |
| 196 | + | + | + | | | | | | | + | | | |
| 197 | + | + | + | | | | | | | | + | | |
| 198 | + | + | + | | | | | | | | | + | |
| 199 | + | + | + | | | | | | | | | | + |
| 200 | | + | + | + | + | | | | | | | | |
| 201 | | + | + | + | | | | | | | | | |
| 202 | | + | + | + | | + | | | | | | | |
| 203 | | + | + | + | | | + | | | | | | |
| 204 | | + | + | + | | | | + | | | | | |
| 205 | | + | + | + | | | | | + | | | | |
| 206 | | + | + | + | | | | | | + | | | |
| 207 | | + | + | + | | | | | | | + | | |
| 208 | | + | + | + | | | | | | | | + | |
| 209 | | + | + | + | | | | | | | | | + |
| 210 | | | + | + | + | | | | | | | | |
| 211 | | | + | + | + | + | | | | | | | |
| 212 | | | + | + | + | | + | | | | | | |
| 213 | | | + | + | + | | | + | | | | | |
| 214 | | | + | + | + | | | | + | | | | |
| 215 | | | + | + | + | | | | | + | | | |
| 216 | | | + | + | + | | | | | | + | | |
| 217 | | | + | + | + | | | | | | | + | |
| 218 | | | + | + | + | | | | | | | | + |
| 219 | | | | + | + | + | | | | | | | |
| 220 | | | | + | + | | + | | | | | | |
| 221 | | | | + | + | | | + | | | | | |
| 222 | | | | + | + | | | | + | | | | |
| 223 | | | | + | + | | | | | + | | | |
| 224 | | | | + | + | | | | | | + | | |
| 225 | | | | + | + | | | | | | | + | |
| 226 | | | | + | + | | | | | | | | + |
| 227 | | | | | + | + | + | | | | | | |
| 228 | | | | | + | + | | + | | | | | |
| 229 | | | | | + | + | | | + | | | | |
| 230 | | | | | + | + | | | | + | | | |
| 231 | | | | | + | + | | | | | + | | |
| 232 | | | | | + | + | | | | | | + | |
| 233 | | | | | + | + | | | | | | | + |
| 234 | | | | | | + | + | + | | | | | |
| 235 | | | | | | + | + | | + | | | | |
| 236 | | | | | | + | + | | | + | | | |
| 237 | | | | | | + | + | | | | + | | |
| 238 | | | | | | + | + | | | | | + | |
| 239 | | | | | | + | + | | | | | | + |
| 240 | | | | | | + | + | + | + | | | | |
| 241 | | | | | | + | + | + | | + | | | |
| 242 | | | | | | + | + | + | | | + | | |
| 243 | | | | | | + | + | + | | | | + | |
| 244 | | | | | | + | + | + | | | | | + |
| 245 | | | | | | | + | + | + | + | | | |
| 246 | | | | | | | + | + | + | | + | | |
| 247 | | | | | | | + | + | + | | | + | |
| 248 | | | | | | | + | + | + | | | | + |
| 249 | | | | | | | | + | + | + | + | | |
| 250 | | | | | | | | + | + | + | | + | |
| 251 | | | | | | | | + | + | + | | | + |
| 252 | | | | | | | | | + | + | + | + | |
| 253 | | | | | | | | | + | + | + | | + |
| 254 | | | | | | | | | | + | + | + | + |
| 255 | + | + | + | + | + | | | | | | | | |
| 256 | + | + | + | + | | | | | | | | | |
| 257 | + | + | + | + | | + | | | | | | | |
| 258 | + | + | + | + | | | + | | | | | | |
| 259 | + | + | + | + | | | | + | | | | | |
| 260 | + | + | + | + | | | | | + | | | | |
| 261 | + | + | + | + | | | | | | + | | | |
| 262 | + | + | + | + | | | | | | | + | | |
| 263 | + | + | + | + | | | | | | | | + | |
| 264 | + | + | + | + | | | | | | | | | + |
| 265 | | + | + | + | + | | | | | | | | |
| 266 | | + | + | + | + | + | | | | | | | |
| 267 | | + | + | + | + | | + | | | | | | |
| 268 | | + | + | + | + | | | + | | | | | |
| 269 | | + | + | + | + | | | | + | | | | |
| 270 | | + | + | + | + | | | | | + | | | |
| 271 | | + | + | + | + | | | | | | + | | |
| 272 | | + | + | + | + | | | | | | | + | |
| 273 | | + | + | + | + | | | | | | | | + |
| 274 | | | + | + | + | + | | | | | | | |
| 275 | | | + | + | + | | + | | | | | | |
| 276 | | | + | + | + | | | + | | | | | |
| 277 | | | + | + | + | | | | + | | | | |
| 278 | | | + | + | + | | | | | + | | | |
| 279 | | | + | + | + | | | | | | + | | |
| 280 | | | + | + | + | | | | | | | + | |
| 281 | | | + | + | + | | | | | | | | + |
| 282 | | | | + | + | + | + | | | | | | |
| 283 | | | | + | + | + | | + | | | | | |
| 284 | | | | + | + | + | | | + | | | | |
| 285 | | | | + | + | + | | | | + | | | |
| 286 | | | | + | + | + | | | | | + | | |
| 287 | | | | + | + | + | | | | | | + | |
| 288 | | | | + | + | + | | | | | | | + |
| 289 | | | | | + | + | + | + | | | | | |
| 290 | | | | | + | + | + | | + | | | | |
| 291 | | | | | + | + | + | | | + | | | |
| 292 | | | | | + | + | + | | | | + | | |
| 293 | | | | | + | + | + | | | | | + | |
| 294 | | | | | + | + | + | | | | | | + |
| 295 | | | | | | + | + | + | + | | | | |
| 296 | | | | | | + | + | + | | + | | | |
| 297 | | | | | | + | + | + | | | + | | |
| 298 | | | | | | + | + | + | | | | + | |
| 299 | | | | | | + | + | + | | | | | + |
| 300 | | | | | | + | + | + | | | | | |
| 301 | | | | | | + | + | + | | | | | |
| 302 | | | | | | + | + | + | | | | | |
| 303 | | | | | | + | + | + | + | + | | | |
| 304 | | | | | | + | + | + | + | | + | | |
| 305 | | | | | | + | + | + | + | | | + | |
| 306 | | | | | | + | + | + | + | | | | + |
| 307 | | | | | | + | + | + | + | | | | |
| 308 | | | | | | + | + | + | + | | | | |
| 309 | | | | | | + | + | + | + | | | | |
| 310 | | | | | | | + | + | + | + | + | | |
| 311 | | | | | | | + | + | + | + | | + | |
| 312 | | | | | | | + | + | + | + | | | + |
| 313 | | | | | | | | + | + | + | + | + | |
| 314 | | | | | | | | + | + | + | + | | + |
| 315 | | | | | | | | | + | + | + | + | + |
| 316 | + | + | + | + | + | | | | | | | | |
| 317 | + | + | + | + | + | + | | | | | | | |
| 318 | + | + | + | + | + | | + | | | | | | |
| 319 | + | + | + | + | + | | | + | | | | | |
| 320 | + | + | + | + | + | | | | + | | | | |
| 321 | + | + | + | + | + | | | | | + | | | |
| 322 | + | + | + | + | + | | | | | | + | | |
| 323 | + | + | + | + | + | | | | | | | + | |
| 324 | + | + | + | + | + | | | | | | | | + |
| 325 | | + | + | + | + | + | | | | | | | |
| 326 | | + | + | + | + | | + | | | | | | |
| 327 | | + | + | + | + | | | + | | | | | |
| 328 | | + | + | + | + | | | | + | | | | |
| 329 | | + | + | + | + | | | | | + | | | |
| 330 | | + | + | + | + | | | | | | + | | |
| 331 | | + | + | + | + | | | | | | | + | |
| 332 | | + | + | + | + | | | | | | | | + |
| 333 | | | + | + | + | + | + | | | | | | |
| 334 | | | + | + | + | + | | + | | | | | |
| 335 | | | + | + | + | + | | | + | | | | |
| 336 | | | + | + | + | + | | | | + | | | |
| 337 | | | + | + | + | + | | | | | + | | |
| 338 | | | + | + | + | + | | | | | | + | |
| 339 | | | + | + | + | + | | | | | | | + |
| 340 | | | | + | + | + | + | + | | | | | |
| 341 | | | | + | + | + | + | | + | | | | |
| 342 | | | | + | + | + | + | | | + | | | |
| 343 | | | | + | + | + | + | | | | + | | |
| 344 | | | | + | + | + | + | | | | | + | |
| 345 | | | | + | + | + | + | | | | | | + |
| 346 | | | | | + | + | + | + | + | | | | |
| 347 | | | | | + | + | + | + | | + | | | |
| 348 | | | | | + | + | + | + | | | + | | |
| 349 | | | | | + | + | + | + | | | | + | |
| 350 | | | | | + | + | + | + | | | | | + |
| 351 | | | | | | + | + | + | + | + | | | |
| 352 | | | | | | + | + | + | + | | + | | |
| 353 | | | | | | + | + | + | + | | | + | |
| 354 | | | | | | + | + | + | + | | | | + |
| 355 | | | | | | + | + | + | + | + | + | | |
| 356 | | | | | | + | + | + | + | + | | + | |
| 357 | | | | | | + | + | + | + | + | | | + |
| 358 | | | | | | | + | + | + | + | + | + | |
| 359 | | | | | | | + | + | + | + | + | | + |
| 360 | | | | | | | | + | + | + | + | + | + |
| 361 | | | | | | | | + | + | + | + | + | |
| 362 | + | + | + | + | + | + | | | | | | | |
| 363 | + | + | + | + | + | | + | | | | | | |
| 364 | + | + | + | + | + | | | + | | | | | |
| 365 | + | + | + | + | + | | | | + | | | | |
| 366 | + | + | + | + | + | | | | | + | | | |
| 367 | + | + | + | + | + | | | | | | + | | |
| 368 | + | + | + | + | + | | | | | | | + | |
| 369 | + | + | + | + | + | | | | | | | | + |
| 370 | | + | + | + | + | + | + | | | | | | |
| 371 | | + | + | + | + | + | | + | | | | | |
| 372 | | + | + | + | + | + | | | + | | | | |
| 373 | | + | + | + | + | + | | | | + | | | |
| 374 | | + | + | + | + | + | | | | | + | | |
| 375 | | + | + | + | + | + | | | | | | + | |
| 376 | | + | + | + | + | + | | | | | | | + |
| 377 | | | + | + | + | + | + | + | | | | | |
| 378 | | | + | + | + | + | + | | + | | | | |
| 379 | | | + | + | + | + | + | | | + | | | |
| 380 | | | + | + | + | + | + | | | | + | | |
| 381 | | | + | + | + | + | + | | | | | + | |
| 382 | | | + | + | + | + | + | | | | | | + |
| 383 | | | | + | + | + | + | + | + | | | | |
| 384 | | | | + | + | + | + | + | | + | | | |
| 385 | | | | + | + | + | + | + | | | + | | |
| 386 | | | | + | + | + | + | + | | | | + | |
| 387 | | | | + | + | + | + | + | | | | | + |
| 388 | | | | | + | + | + | + | + | + | | | |
| 389 | | | | | + | + | + | + | + | | + | | |
| 390 | | | | | + | + | + | + | + | | | + | |
| 391 | | | | | + | + | + | + | + | | | | + |
| 392 | | | | | | + | + | + | + | + | + | | |
| 393 | | | | | | + | + | + | + | + | | + | |
| 394 | | | | | | + | + | + | + | + | | | + |
| 395 | | | | | | + | + | + | + | + | + | + | |
| 396 | | | | | | + | + | + | + | + | + | | + |
| 397 | | | | | | | + | + | + | + | + | + | + |
| 398 | + | + | + | + | + | + | + | | | | | | |
| 399 | + | + | + | + | + | + | | + | | | | | |
| 400 | + | + | + | + | + | + | | | + | | | | |
| 401 | + | + | + | + | + | + | | | | + | | | |
| 402 | + | + | + | + | + | + | | | | | + | | |
| 403 | + | + | + | + | + | + | | | | | | + | |
| 404 | + | + | + | + | + | + | | | | | | | + |
| 405 | | + | + | + | + | + | + | + | | | | | |
| 406 | | + | + | + | + | + | + | | + | | | | |
| 407 | | + | + | + | + | + | + | | | + | | | |
| 408 | | + | + | + | + | + | + | | | | + | | |
| 409 | | + | + | + | + | + | + | | | | | + | |
| 410 | | + | + | + | + | + | + | | | | | | + |
| 411 | | | + | + | + | + | + | + | + | | | | |
| 412 | | | + | + | + | + | + | + | | + | | | |
| 413 | | | + | + | + | + | + | + | | | + | | |
| 414 | | | + | + | + | + | + | + | | | | + | |
| 415 | | | + | + | + | + | + | + | | | | | + |
| 416 | | | | + | + | + | + | + | + | + | | | |
| 417 | | | | + | + | + | + | + | + | | + | | |
| 418 | | | | + | + | + | + | + | + | | | + | |
| 419 | | | | + | + | + | + | + | + | | | | + |
| 420 | | | | | + | + | + | + | + | + | + | | |
| 421 | | | | | + | + | + | + | + | + | | + | |
| 422 | | | | | + | + | + | + | + | + | | | + |
| 423 | | | | | | + | + | + | + | + | + | + | |
| 424 | | | | | | + | + | + | + | + | + | | + |
| 425 | | | | | | + | + | + | + | + | + | + | + |
| 426 | + | + | + | + | + | + | + | + | | | | | |
| 427 | + | + | + | + | + | + | + | | + | | | | |
| 428 | + | + | + | + | + | + | + | | | + | | | |
| 429 | + | + | + | + | + | + | + | | | | + | | |
| 430 | + | + | + | + | + | + | + | | | | | + | |
| 431 | + | + | + | + | + | + | + | | | | | | + |
| 432 | | + | + | + | + | + | + | + | + | | | | |
| 433 | | + | + | + | + | + | + | + | | + | | | |
| 434 | | + | + | + | + | + | + | + | | | + | | |
| 435 | | + | + | + | + | + | + | + | | | | + | |
| 436 | | + | + | + | + | + | + | + | | | | | + |
| 437 | | | + | + | + | + | + | + | + | + | | | |
| 438 | | | + | + | + | + | + | + | + | | + | | |
| 439 | | | + | + | + | + | + | + | + | | | + | |
| 440 | | | + | + | + | + | + | + | + | | | | + |
| 441 | | | | + | + | + | + | + | + | + | + | | |
| 442 | | | | + | + | + | + | + | + | + | | + | |
| 443 | | | | + | + | + | + | + | + | + | | | + |
| 444 | | | | | + | + | + | + | + | + | + | + | |
| 445 | | | | | + | + | + | + | + | + | + | | + |
| 446 | | | | | | + | + | + | + | + | + | + | + |
| 447 | + | + | + | + | + | + | + | + | + | | | | |
| 448 | + | + | + | + | + | + | + | + | | + | | | |
| 449 | + | + | + | + | + | + | + | + | | | + | | |
| 450 | + | + | + | + | + | + | + | + | | | | + | |
| 451 | + | + | + | + | + | + | + | + | | | | | + |
| 452 | | + | + | + | + | + | + | + | + | + | | | |
| 453 | | + | + | + | + | + | + | + | + | | + | | |
| 454 | | + | + | + | + | + | + | + | + | | | + | |
| 455 | | + | + | + | + | + | + | + | + | | | | + |
| 456 | | | + | + | + | + | + | + | + | + | + | | |
| 457 | | | + | + | + | + | + | + | + | + | | + | |
| 458 | | | + | + | + | + | + | + | + | + | | | + |
| 459 | | | | + | + | + | + | + | + | + | + | + | |
| 460 | | | | + | + | + | + | + | + | + | + | | + |
| 461 | | | | | + | + | + | + | + | + | + | + | + |
| 462 | + | + | + | + | + | + | + | + | + | + | | | |
| 463 | + | + | + | + | + | + | + | + | + | | + | | |
| 464 | + | + | + | + | + | + | + | + | + | | | + | |
| 465 | + | + | + | + | + | + | + | + | + | | | | + |
| 466 | | + | + | + | + | + | + | + | + | + | + | | |
| 467 | | + | + | + | + | + | + | + | + | + | | + | |
| 468 | | + | + | + | + | + | + | + | + | + | | | + |
| 469 | | | + | + | + | + | + | + | + | + | + | + | |
| 470 | | | + | + | + | + | + | + | + | + | + | | + |
| 471 | | | | + | + | + | + | + | + | + | + | + | + |
| 472 | + | + | + | + | + | + | + | + | + | + | + | | |
| 473 | + | + | + | + | + | + | + | + | + | + | | + | |
| 474 | + | + | + | + | + | + | + | + | + | + | | | + |
| 475 | | + | + | + | + | + | + | + | + | + | + | + | |
| 476 | | + | + | + | + | + | + | + | + | + | + | | + |
| 477 | | | + | + | + | + | + | + | + | + | + | + | + |
| 478 | + | + | + | + | + | + | + | + | + | + | + | + | |
| 479 | + | + | + | + | + | + | + | + | + | + | + | | + |
| 480 | + | + | + | + | + | + | + | + | + | + | + | + | + |
| 481 | | | | | | | + | | | | | + | + |

In a preferred embodiment, the subject is a mammal, preferably a mouse, rat, guinea pig, cat, dog, sheep, horse, cow, pig, more preferably the subject is a human.

In another preferred embodiment, the method further comprises determining the prognostic transcript of one or more genes selected from the group of genes consisting of Ubd, C2, Lst1, Aif1, C1QTNF7, CEACAM4, MME, IGFBP5, TAP1, CTGF, ANP32A, HCLS1, HTRA1, LGALS7, PTGER2, PTPN7, TGM2, TREM2 and CARD11; or their corresponding cDNAs, or their expression products, wherein
(i) for every unit of increased expression of one or more of Ubd, C2, Aif1, CEACAM4, TAP1, PTGER2, PTPN7, TGM2, TREM2, HCLS1 and/or CARD11; or the corresponding cDNA or expression product, said patient is expected to develop GvHR or GvHD; and
(ii) for every unit of decreased expression of one or more of Lst1, C1QTNF7, MME, CTGF, ANP32A, HTRA1, LGALS7 and/or IGFBP5; or the corresponding cDNAs or expression product(s), said patient is expected to develop GvHR or GvHD.

Accordingly, any combination of genes Ctss, Pbx2, Grem1, Ly6g6e, Olr1, Spr1, Msr1, Spic, Nfe2, Tnfaip8l2, Ier3, Pik3ap1, and Pstpip1 may be combined with any combination of genes Ubd, C2, Lst1, Aif1, C1QTNF7, CEACAM4, MME, IGFBP5, TAP1, CTGF, ANP32A, HCLS1, HTRA1, LGALS7, PTGER2, PTPN7, TGM2, TREM2 and CARD11.

In a second aspect, the invention relates to a method of diagnosing graft versus host reaction (GvHR) or graft versus host disease (GvHD) in a subject following transplantation, comprising:
(a) determining the expression level of one or more prognostic RNA transcripts, or their corresponding cDNAs, or their expression products, in a sample obtained from said subject, wherein said transcript(s) or expression products is/are the transcript or expression product of one or more genes selected from the group consisting of:
   (i) Msr1, Pik3ap1, Pstpip1, Ctss, Pbx2, Grem1, Ly6g6e, Olr1, Spr1, Spic, Nfe2 Tnfaip8l2, and Ier3; or
   (ii) Msr1, Ctss, Pbx2, Grem1, Ly6g6e, Olr1, Spr1, Spic, and Nfe2; or
   (iii) Pik3ap1, Pstpip1, Tnfaip8l2, and Ier3;
(b) comparing the expression level of said one or more prognostic RNA transcript, or its corresponding cDNA, or its expression product with a corresponding baseline value;
   wherein
   (i) every unit of increased expression of Olr1, Msr1, Pik3ap1, and/or Pstpip1, or the corresponding cDNA or expression product, is indicative of GvHR or GvHD; and
   (ii) every unit of decreased expression of Ctss, Pbx2, Grem1, Ly6g6e, Spr1, Spic, Nfe2, Tnfaip8l2, and/or Ier3, or the corresponding cDNA or expression product, is indicative of GvHR or GvHD.

The preferred embodiments of the first aspect are also preferred embodiments of the second aspect, and the same definitions apply.

However, in one particularly preferred embodiment, the baseline value is the expression level of said at least one gene in said subject prior to said transplantation and/or in at least one healthy subject.

In a preferred embodiment of the second aspect, said method further comprises determining the prognostic transcript of one or more genes selected from the group of genes consisting of Ubd, C2, Lst1, Aif1, C1QTNF7, CEACAM4, MME, IGFBP5, TAP1, CTGF, ANP32A, HCLS1, HTRA1, LGALS7, PTGER2, PTPN7, TGM2, TREM2 and CARD11; or their corresponding cDNAs, or their expression products, wherein
(i) every unit of increased expression of Ubd, C2, Aif1, CEACAM4, TAP1, PTGER2, PTPN7, TGM2, TREM2, HCLS1 and/or CARD11; or the corresponding cDNA or expression product, is indicative of GvHR or GvHD; and
(ii) every unit of decreased expression of Lst1, C1QTNF7, MME, CTGF, ANP32A, HTRA1, LGALS7 and/or IGFBP5; or the corresponding cDNA or expression product, is indicative of GvHR or GvHD.

In a third aspect, the invention relates to a method of monitoring the efficacy of treatment of graft versus host reaction (GvHR) or graft versus host disease (GvHD) in a subject following transplantation, comprising:
(a) determining the expression level of one or more prognostic RNA transcripts, or their corresponding cDNAs, or their expression products, in a sample obtained from said subject at a first time point T1, and a later second time point T2, wherein said transcript(s) or expression products is/are the transcript or expression product of one or more genes selected from the group consisting of:
   (i) Msr1, Pik3ap1, Pstpip1, Ctss, Pbx2, Grem1, Ly6g6e, Olr1, Spr1, Spic, Nfe2, Tnfaip8l2, and Ier3; or
   (ii) Msr1, Ctss, Pbx2, Grem1, Ly6g6e, Olr1, Spr1, Spic, and Nfe2; or
   (iii) Pik3ap1, Pstpip1, Tnfaip8l2, and Ier3;
(b) comparing the expression level of said one or more prognostic RNA transcript, or its corresponding cDNA, or its expression product at time point T1 (Δ1) and time point T2 (Δ2) with a corresponding baseline value;
   wherein
   (i) a decline in units of an increased expression of Olr1, Msr1, Pik3ap1, and/or Pstpip1; or the corresponding cDNA or expression product at time point T2 in comparison with the increased expression of said at least one gene at the time point T1 (ΔΔ=Δ1-Δ2), is indicative of effective treatment of GvHR or GvHD; and
   (ii) a decline in units of a decreased expression of Ctss, Pbx2, Grem1, Ly6g6e, Spr1, Spic, Nfe2, Tnfaip8l2, and/or Ier3; or the corresponding cDNA or expression product at time point T2 in comparison with the decreased expression of said at least one gene at the time point Ti(ΔΔ=Δ1-Δ2), is indicative of effective treatment of GvHR or GvHD.

The preferred embodiments of the first and second aspect are also preferred embodiments of the third aspect, and the same definitions apply.

In another preferred embodiment, the method of the third aspect further comprises determining the prognostic transcript of one or more genes selected from the group of genes consisting of Ubd, C2, Lst1, Aif1, C1QTNF7, CEACAM4, MME, IGFBP5, Tap1, Ctgf, ANP32A, HCLS1, HTRA1, LGALS7, PTGER2, PTPN7, TGM2, TREM2 and CARD11; or their corresponding cDNAs, or their expression products, wherein
(i) a decline in units of an increased expression of Ubd, C2, Aif1, CEACAM4, Tap1, PTGER2, PTPN7, TGM2, TREM2, HCLS1 and/or CARD11; or the corresponding cDNA or expression product at time point T2 in comparison with the increased expression of said at least one gene at the time point T1 (ΔΔ=Δ1-Δ2), is indicative of effective treatment of GvHR or GvHD; and
(ii) a decline in units of a decreased expression of Lst1, C1QTNF7, MME, Ctgf, ANP32A, HTRA1, LGALS7 and/or IGFBP5; or the corresponding cDNA or expression product at time point T2 in comparison with the decreased expression of said at least one gene at the time point T1(ΔΔ=Δ1-Δ2), is indicative of effective treatment of GvHR or GvHD.

In a very important fourth aspect, the invention further relates to a method of screening for a candidate substance for treatment of graft versus host reaction (GvHR) or graft versus host disease (GvHD), comprising:
(a) monitoring the efficacy of treatment by said candidate substance by using the method according to the third aspect in
   (i) a non-human animal model which suffers from GvHR or GvHD and to which the candidate substance has been administered, or
   (ii) in an ex vivo model, including but not limited to cell-based and/or tissue-based GvHR or HvHD assay such as the Skin Explant Assay, wherein said cells and/or tissue have been contacted with said candidate substance; and
(b) selecting a candidate substance which shows effective treatment of GvHR or GvHD.

Preferably, the screening method is carried out *in vitro,* i.e. in an ex vivo model, with cultured cells or with tissue, and by applying high throughput procedures. One example of such an ex vivo model is the Skin Explant Assay. This unique, non-artificial, (human) *in vitro* assay technology allows the study of primary and secondary immune responses in the presence of immunomodulatory drugs or allogeneic stem cells, reducing the need for extensive animal testing. Incubation with, for example, human skin, allows skin damage to be assessed by histopathology. The skin is graded for histological damage using criteria similar to that used and observed in the clinical setting. Results correlate with systemic disease and have been shown to predict outcome. The Skin Explant Assay is further exemplified in the Examples section and in the references cited therein.

Candidate substances selected by the screening method according to the invention may be subsequently also tested *in vivo.*

Alternatively, the screening assay may be directly performed in vivo by using a non-human animal model which suffers from GvHR or GvHD. Suitable non-human animal models include rats, mice, guinea pigs, pigs, dogs, and cats. However, it has to be made sure that the scientific gain outweighs any animal suffering, and that the testings are carried out in accordance with national restrictions for animal testings.

A variety of types of putative candidate substances may be tested and identified as suitable. For example, one can utilize known properties of a target protein to devise agents to stimulate or inhibit its production or activity, as desired. That is, one can devise a means to inhibit the action of, or bind, block, remove or otherwise diminish the presence, activity and/or availability of, a protein whose upregulation is associated with GvHD or GvHR; or one can devise a means to stimulate the action of, or to potentiate or enhance the activity of or availability of, a protein whose down-regulation is associated with GvHD or GvHR.

For example, in the case of a cellular receptor, one could expose the receptor to an antagonist, a soluble form of the receptor or a "decoy" ligand binding site of a receptor (to compete for ligand) to inhibit it. Antibodies may be administered to a cell to bind and inactivate (or compete with), or to enhance the activity of, secreted protein products or expressed cell-surface products of genes of interest.

Another approach is to employ antisense oligonucleotides or nucleic acid constructs that inhibit expression of a gene whose down-regulation is desired, in a highly specific manner. Methods to select, test and optimize putative antisense sequences are routine. Nucleic acid constructs may be used to express an antisense molecule of interest, or antisense oligonucleotides as such may be administered to a cell. The oligonucleotides can be DNA or RNA or chimeric mixtures or derivatives or modified versions thereof, single- stranded or double-stranded. The oligonucleotides can be modified at the base moiety, sugar moiety, or phosphate backbone. The oligonucleotide may include other appending groups such as peptides, or agents facilitating transport across the cell membrane, hybridization-triggered cleavage agents, or intercalating agents. Multiple antisense constructs or oligonucleotides specific for different genes can be employed together. The sequences of the down-regulated genes described herein can be used to design the antisense molecules. The antisense sequences may range from about 6 to about 50 nucleotides, and may be as large as 100 or 200 nucleotides, or larger. They may correspond to full-length coding sequences and/or may be genomic sequences that comprise non-coding sequences. Another approach is to use ribozymes that can specifically cleave nucleic acids encoding the overexpressed genes disclosed herein. Such methods are routine in the art and methods of making and using any of a variety of appropriate ribozymes are well known to the skilled worker. A ribozyme having specificity for an mRNA of interest can be designed based upon the nucleotide sequence of, e. g., the corresponding cDNA. Alternatively, the sequence of an overexpressed gene disclosed herein can be used to select a catalytic RNA having a specific ribonuclease activity from a pool of RNA molecules.

Another approach involves double stranded RNAs called small interfering RNAs. A siRNA is a double- stranded RNA molecule comprising self-complementary sense and antisense regions, wherein the antisense region comprises nucleotide sequence that is complementary to nucleotide sequence in a target nucleic acid molecule or a portion thereof, and the sense region has a nucleotide sequence corresponding to the target nucleic acid sequence or a portion thereof. The siRNA can be assembled from two separate oligonucleotides, where one strand is the sense strand and the other is the antisense strand, wherein the antisense and sense strands are self-complementary. The siRNA can be assembled from a single oligonucleotide, where the self-complementary sense and antisense regions of the siRNA are linked by means of a nucleic acid based or non-nucleic acid-based linker. The siRNA may be a polynucleotide having a hairpin secondary structure, i.e. having self-complementary sense and antisense regions. The siRNA may be a circular single-stranded polynucleotide having two or more loop structures and a stem comprising self-complementary sense and antisense regions, wherein the circular polynucleotide can be processed either in vivo or in vitro to generate an active siRNA molecule capable of mediating RNAi. In certain embodiments, the siRNA molecule comprises separate sense and antisense sequences or regions, wherein the sense and antisense regions are covalently linked by nucleotide or non-nucleotide linkers molecules as is known in the art, or are alternately non-covalently linked by ionic interactions, hydrogen bonding, van der Waals interactions, hydrophobic interactions, and/or stacking interactions. RNAi molecules may be used to inhibit gene expression, using conventional procedures. Another approach is to use small molecules, or "compounds", isolated from natural sources or developed synthetically, e.g., by combinatorial chemistry. In general, such molecules are identified from large libraries of natural products or synthetic (or semisynthetic) extracts or chemical libraries according to methods known in the art. Those skilled in the field of drug discovery and development will understand that the precise source of test extracts or compounds is not critical to the methods of the invention. Accordingly, virtually any number of chemical extracts or compounds can be used in the methods described herein. Examples of such extracts or compounds include, but are not limited to, plant-, fungal-, prokaryotic- or animal- based extracts, fermentation broths, and synthetic compounds, as well as modification of existing compounds. Numerous methods are also available for generating random or directed synthesis (e. g., semi-synthesis or total synthesis) of any number of chemical compounds, including, but not limited to, saccharide-, lipid-, peptide-, polypeptide-and nucleic acid-based compounds. Synthetic compound libraries are commercially available, e. g., from Brandon Associates (Merrimack, NH) and Aldrich Chemical (Milwaukee, WI). Alternatively, libraries of natural compounds in the form of bacterial, fungal, plant, and animal extracts are commercially available from a number of sources, e. g., Biotics (Sussex, UK), Xenova (Slough, UK), Harbor Branch Oceangraphics Institute (Ft. Pierce, FL), and PharmaMar, U.S.A. (Cambridge, MA). In addition, natural and synthetically produced libraries are generated, if desired, according to methods known in the art, e.g., by standard extraction and fractionation methods. Furthermore, if desired, any library or compound is readily modified using standard chemical, physical, or biochemical methods.

Methods for introducing candidate substances into cells are conventional. For example, methods of gene transfer may be used, wherein antisense molecules, ribozymes, or siRNAs are introduced into a rectal carcinoma cell of interest, or nucleic acids that encode proteins which modulate (up-regulate or down-regulate) the production or activity of one or more of the genes disclosed herein. Methods of gene transfer are conventional, and include virus-mediated gene transfer, for example, with retroviruses, lentiviruses, and recombinant adenovirus vectors. Adeno-associated virus (AAV) may also be used. Improved efficiency is attained by the use of promoter enhancer elements in the DNA constructs. In addition to virus-mediated gene transfer, physical means well-known in the art can be used for direct gene transfer, including administration of plasmid DNA and particle-bombardment mediated gene transfer. Furthermore, electroporation or calcium phosphate transfection, both well-known means to transfer genes into cell *in vitro,* may also be used. Gene transfer may also be achieved by using "carrier mediated gene transfer". Preferred carriers are targeted liposomes such as immunoliposomes, which can incorporate acylated monoclonal antibodies into the lipid bilayer, or polycations such as asialoglycoprotein/polylysine. Liposomes have been used to encapsulate and deliver a variety of materials to cells, including nucleic acids and viral particles. Preformed liposomes that contain synthetic cationic lipids form stable complexes with polyanionic DNA. Cationic liposomes, liposomes comprising some cationic lipid, that contained a membrane fusion-promoting lipid dioctadecyldimethyl-ammonium-bromide (DDAB) have efficiently transferred heterologous genes into eukaryotic cells and can mediate high level cellular expression of transgenes, or mRNA, by delivering them into a variety of cultured cell lines. In still a final aspect, the invention describes the use of a kit in a method of predicting the risk of developing graft versus host reaction (GvHR) or graft versus host disease (GvHD) according to the first aspect, or in a method of diagnosing GvHR or GvHD according to the second aspect, or in a method of monitoring the efficacy of treatment of GvHR or GvHD according to the third aspect, wherein the kit comprises at least one isolated polynucleotide, wherein each isolated polynucleotide independently comprises
(i) at least 20 contiguous nucleotides of the nucleotide sequence selected from SEQ ID NO: 1, 3, 5, 7, 8, 10, 12, 14, 16, 18, 20, 22, and/or 24; or SEQ ID NO: 26-47, or
(ii) a nucleotide sequence having at least 90% identity to (i), or
(iii) the coding region of a gene comprising a nucleotide sequence according to (i) or (ii), or
(iv) a nucleotide sequence that can specifically hybridize, under conditions of high stringency, to a polynucleotide having a nucleotide sequence according to (i), (ii) or (iii); and
wherein the kit comprises no more than 9000 isolated polynucleotides in total.

The isolated polynucleotide may have at least 90% identity to a polynucleotide comprising a nucleotide sequence selected from the group of nucleotide sequences consisting of at least 20 contiguous nucleotides of the nucleotide sequence selected from SEQ ID NO: 1, 3, 5, 7, 8, 10, 12, 14, 16, 18, 20, 22, and/or 24; or SEQ ID NO: 26-47; more preferably to the CDS encoded therein. Preferably, said isolated polynucleotide, has a nucleotide sequence having at least 92 %, at least 94 %, at least 96 %, at least 98 %, or 99 % nucleotide sequence identity to a polynucleotide comprising a nucleotide sequence selected from the group of nucleotide sequences consisting of at least 20 contiguous nucleotides of the nucleotide sequence selected from SEQ ID NO: 1, 3, 5, 7, 8, 10, 12, 14, 16, 18, 20, 22, and/or 24; or SEQ ID NO: 26-47; more preferably to the CDS encoded therein.

Generally, a nucleotide sequence has "at least x % identity" with another nucleotide sequence or any of the sequences given above if, when the sequence identity between those to aligned sequences is at least x %. Such an alignment can be performed using for example publicly available computer homology programs such as the "BLAST" program provided at the NCBI homepage at http://www.ncbi.nlm.nih.gov/blast/blast.cgi, using the default settings provided therein. Further methods of calculating sequence identity percentages of sets of nucleic acid sequences are known in the art.

Preferably, the isolated polynucleotides comprise at least 25, preferably at least 30, more preferably at least 35, even more preferably at least 40, most preferably 50, in particular 60 contiguous nucleotides.

In another preferred embodiment, the isolated polynucleotides are arranged in an array, in particular wherein the kit comprises no more than 8000, preferably no more than 7000, more preferably no more than 6000, even more preferably no more than 5000 or even no more than 4000, most preferably no more than 3000 or even no more than 2000, in particular no more than 1000 or even no more than 500 or no more than 100 isolated polynucleotides in total.

The isolated polynucleotides of the kit may be used as probes in a hybridization method, however, in a more preferred embodiment, the isolated polynucleotides are arranged in an array. The term "array", as used herein, means an ordered arrangement of addressable, accessible, spatially discrete or identifiable, molecules disposed on a surface. Moreover, the array may be a microarray (sometimes referred to as a DNA "chip"). Microarrays allow for massively parallel gene expression analysis. Furthermore, the hybridization signal from each of the array elements is individually distinguishable. Arrays can comprise any number of sites that comprise probes, from about 5 to, in the case of a microarray, tens to hundreds of thousands or more. Microfluidic devises are also contemplated.

Any suitable, compatible surfaces can be used in conjunction with this array. The surface (usually a solid, preferably a suitable rigid or semi-rigid support) may be any organic or inorganic material or a combination thereof, including, merely by way of example, plastics such as polypropylene or polystyrene; ceramic; silicon; (fused) silica, quartz or glass, which can have the thickness of, for example, a glass microscope slide or a glass cover slip; paper, such as filter paper; diazotized cellulose; nitrocellulose filters; nylon membrane; or polyacrylamide gel pad. Substrates that are transparent to light are useful when the method of performing an assay involves optical detection. Suitable surfaces include membranes, filters, chips, slides, wafers, fibers, magnetic or nonmagnetic beads, gels, tubing, plates, polymers, microparticles, capillaries, or the like. The surface can have a variety of surface forms, such as wells, trenches, pins, channels and pores, to which the isolated polynucleotides are bound. It can, for example, be a flat surface such as a square, rectangle, or circle; a curved surface; or a three dimensional surface such as a bead, particle, strand, precipitate, tube, sphere, etc.

Methods of making DNA arrays, including microarrays are conventional. For example, the probes may be synthesized directly on the surface; or preformed molecules, such as oligonucleotides or cDNAs, may be introduced onto (e. g., bound to, or otherwise immobilized on) the surface. Among suitable fabrication methods are photolithography, pipetting, drop-touch, piezoelectric printing (ink-jet), or the like.

Furthermore, the probes do not have to be directly bound to the substrate, but rather can be bound to the substrate through a linker group. The linker groups are typically about 6 to 50 atoms long to provide exposure to the attached nucleic acid probe. Preferred linker groups include ethylene glycol oligomers, diamines, diacids and the like. Reactive groups on the substrate surface react with one of the terminal portions of the linker to bind the linker to the substrate. The other terminal portion of the linker is then functionalized for binding the nucleic acid probe.

The kit may optionally further comprise, isolated polynucleotides that act as internal controls. The controls may be positive controls or negative controls, examples of which will be evident to the skilled worker. The determined amounts obtained by use of the kit should reflect accurately the amounts of control target polynucleotide added to the sample.

The kit may further comprise means for carrying out a method of the invention, means for reading hybridization results and instructions for performing a method, such as a diagnostic method. Hybridization results may be units of fluorescence. Other optional elements of the kit may include suitable buffers, media components, or the like; a computer or computer-readable medium for storing and/or evaluating the assay results; containers; or packaging materials. Reagents for performing suitable controls may also be included. The reagents of the kit may be in containers in which the reagents are stable, e.g., in lyophilized form or stabilized liquids. The reagents may also be in single use form, e.g., in single reaction form for diagnostic use. The following examples are meant to further illustrate, but not limit, the invention. The examples comprise technical features, and it will be appreciated that the invention relates also to combinations of the technical features presented in this exemplifying section.

### Brief description of the figures

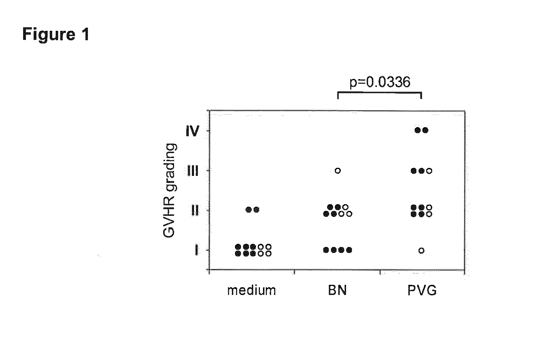

**Figure 1****. Induction of a GVHR in BN rat skin explants exposed to PVG lymphocytes.** A summary of the histological GVHR grading of BN skin samples cultured in medium alone, together with syngeneic BN lymphocytes, and together with pre-stimulated allogeneic PVG lymphocytes (n=12 in each group) is given. The samples represented by closed circles were used for both gene expression profiling and qRT-PCR experiments, whereas the other samples were only used for gene expression profiling. The pair-wise comparison (U test) indicated a significant difference between skin explant cultures with BN and PVG lymphocytes.

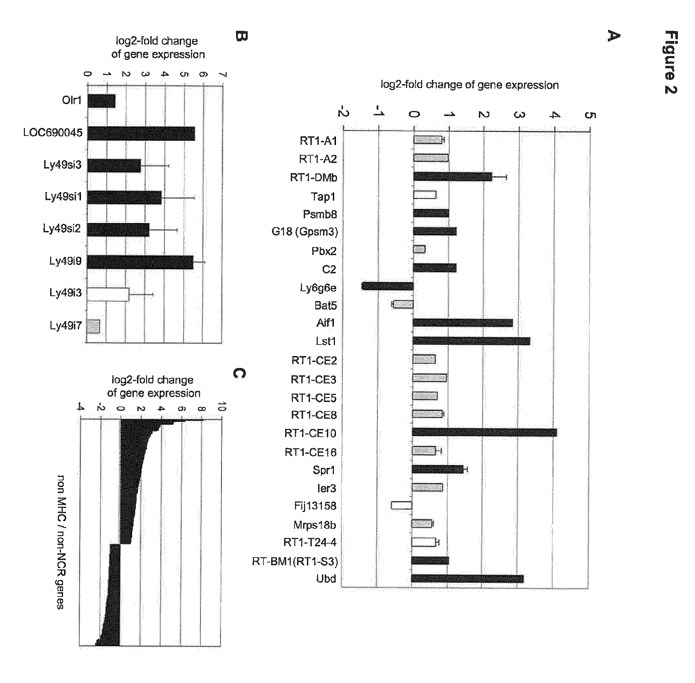

**Figure 2****. Expression profiling of BN skin explant samples exposed to allogeneic (PVG) lymphocytes in comparison to those exposed to syngeneic (BN) lymphocytes. (*A*)** The log2-fold changes in gene expression of significantly regulated MHC genes (p<0.05) are shown. **(*B*)** The log2-fold changes in gene expression of significantly regulated NKC genes (p<0.05) are shown. **(*C*)** The log2-fold changes in gene expression of 168 significantly (p<0.05) and strongly (log2-fold change ≥1 or ≤-1) regulated non-MHC and non-NKC genes indicate the range of observed alterations in gene expression levels among the 6342 tested genes. In panels *A* and *B*, black bars indicate a strong change (log2-fold change ≥1 or ≤-1), dotted bars alterations below this amplitude, and white bars expression changes that were not detected at a significant level with all, but at least with 50 % of the probes present on the array for that gene. When more than one probe indicated a significant change of gene expression the means and standard deviations of the log2-fold changes are shown (see **Tab. 5, 6,** and **7** for further details).

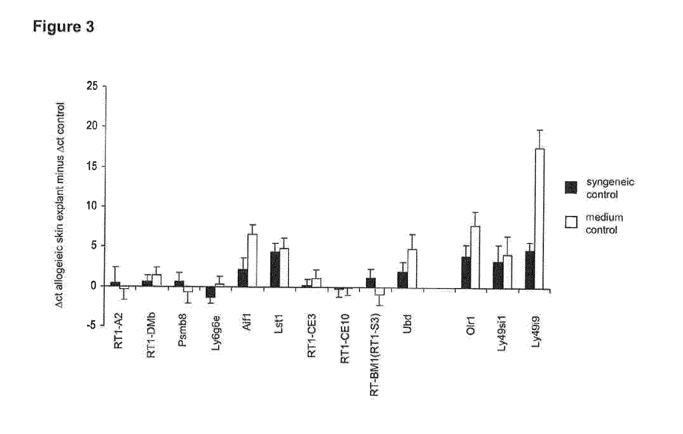

**Figure 3****. Verification of the regulation in gene expression observed in the microarray experiment by qRT-PCR.** A subgroup of 8 samples used for the microarray experiment (see **Fig. 1****)** was analyzed by qRT-PCR for the expression of 10 MHC and 3 NKC genes. The ΔΔct value was calculated, i.e. the Act (*Gapdh-* gene of interest) of the allogeneic skin explant samples minus Act (*Gapdh-* gene of interest) of the corresponding control sample. The control sample was either a parallel skin explant exposed to syngeneic lymphocytes as in the microarray experiment (syngeneic control, black bars) or a parallel skin explant sample cultured in medium only (medium control, white bars). The means of the ΔΔct values plus SEM are shown. A positive value indicates an up-regulation of gene expression in the allogeneic samples.

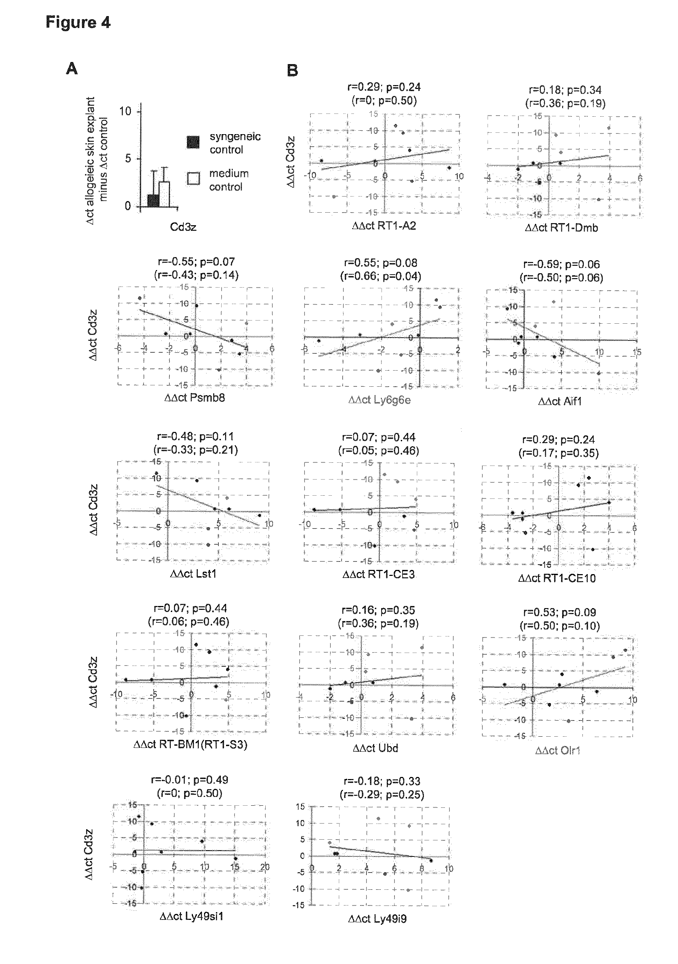

**Figure 4****. Analysis of T cell infiltration in skin explants. (*A*)** Analysis of *Cd3z* gene expression in the same samples as shown in **Fig. 3****. (*B*)** Correlation of *Cd3z* and other gene expression levels (ΔΔct values for allogeneic skin explants minus syngeneic controls) in these samples. Pearson's correlation coefficients (r) and the p-values for the corresponding tests are given above the diagrams. In brackets Spearman's correlation coefficients (r) and the p-values for the corresponding tests are shown.

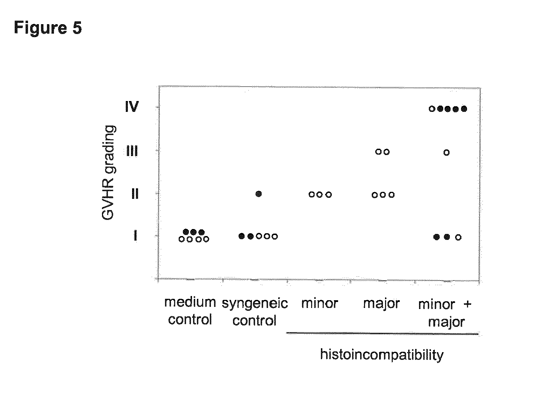

**Figure 5****. Induction of a GVHR in a second series of BN (filled circles) and LEW.1N (open circles) rat skin explants.** Skin explants were co-cultured with pre-stimulated allogeneic lymphocytes from rats with a minor (BN lymphocytes and LEW.1N skin), major (LEW.1A (*RT1^{a}*) or LEW.1AV1 (*RT1^{av1}*) lymphocytes and LEW.1N skin), or a minor and major histoincompatibility (PVG lymphocytes (*RT1^{c}*) and BN skin or LOU/C (*RT1^{u}*) lymphocytes and LEW.1N skin). A summary of the histological GVHR grading of skin samples cultured in medium alone, together with syngeneic BN or LEW.1N lymphocytes, and together with allogeneic lymphocytes is given.

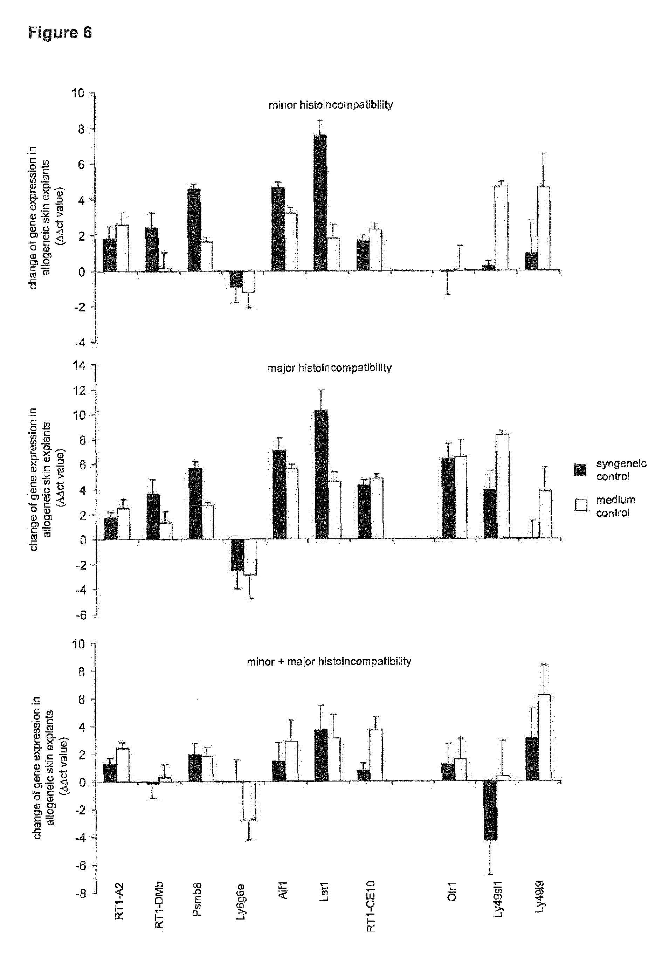

**Figure 6****. Verification of gene regulations observed in the microarray experiment by qRT-PCR in an independent set of 17 skin explant assays.** Three samples were derived from skin explant assays with minor (upper panel), 5 with major (middle panel), and 9 with minor and major histoincompatibility (lower panel). The GVHR grading for these samples is shown in **Fig. 5****.** The expression of 7 MHC and 3 NKC was analyzed by qRT-PCR. The ΔΔct value, i.e. Act (*Gapdh-* gene of interest) of the allogeneic skin explant samples minus mean of Act (*Gapdh-* gene of interest) of the corresponding control samples (BN or LEW.1N, respectively), was calculated. The control samples were either skin explant samples exposed to syngeneic lymphocytes (syngeneic control) or skin explant samples cultured in medium only without added lymphocytes (medium control) and their GVHR grading is also shown in **Fig. 5****.** The means of the ΔΔct values plus SEM are shown. A positive value indicates an up-regulation of gene expression in the allogeneic samples.

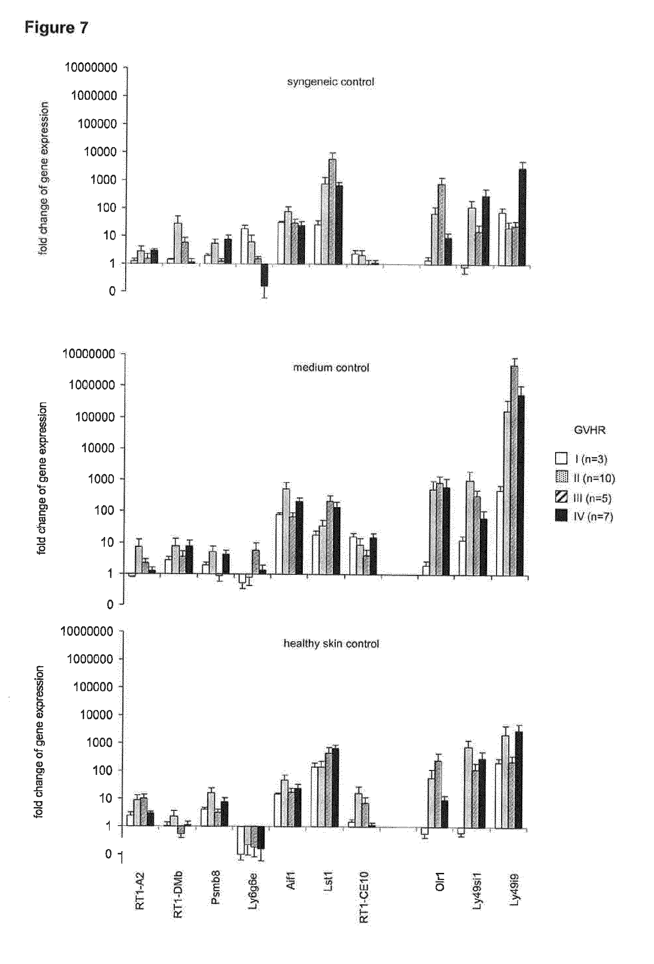

**Figure 7****. Analysis of MHC and NKC gene regulation in skin explants exposed to pre-stimulated allogeneic lymphocytes depending on GVHR grading (from left to right: grade I (white), grade II (light grey/pointed), grade III (dark grey/striped), grade IV (black)).** The expression of 7 MHC and 3 NKC was analyzed by qRT-PCR. The relative changes of gene expression levels were calculated using a mathematical model for relative quantification of real-time PCR data which also takes into account variations of the amplification efficiencies of different primer pairs (Pfaffl MW (2001) Nucleic Acids Res 29: e45). The means plus SEM are shown. A value >1 indicates an up-regulation of gene expression in the allogeneic samples. The control samples were either skin explant samples exposed to syngeneic lymphocytes (syngeneic control, upper panel), skin explant samples cultured in medium only (medium control, mean panel), or freshly frozen healthy skin samples (healthy skin control, lower panel).

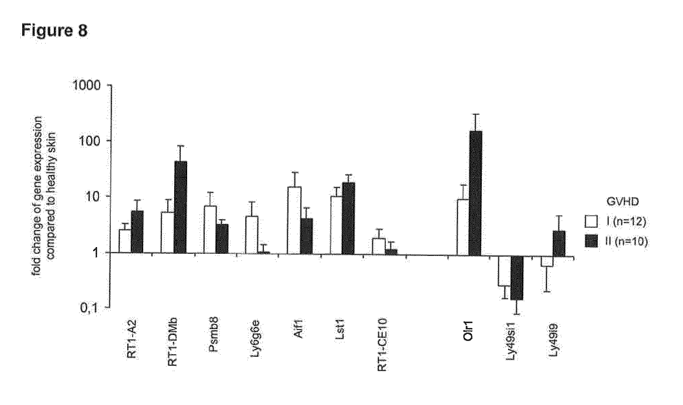

**Figure 8****. Analysis of MHC and NKC gene regulation in GVHD skin lesions from transplanted animals.** BN (*RT1ⁿ*) rats were transplanted with bone marrow of PVG (*RT1^{c}*) rats. Rats that developed acute GVHD were scarified and skin lesions with signs of GVHD were obtained for RNA preparation and histology. The expression of 7 MHC and 3 NKC was analyzed by qRT-PCR using the *B2m* gene as reference. The relative changes of gene expression levels were calculated (Pfaffl MW (2001) Nucleic Acids Res 29: e45). The means plus SEM are shown for skin lesion with grade I and grade II GVHD. A value >1 indicates an up-regulation of gene expression in the allogeneic samples. The control samples were freshly frozen skin samples from healthy BN rats (n=7).

### Examples

### Example 1: Expression profiling of GVHR in rat skin explants

The inventors decided to analyze a rat model of GVHD making use of genetically well-defined inbred stains. Importantly, the non-class I/non-class II genes of human (HLA) and rat (RT1) MHCs are highly conserved. However, the size and organization of MHC class I encoding regions are considerably variable and the rat possesses a significant number of MHC class Ib genes for which no human homologues exist. At least some of these genes have already been proven to encode ligands for inhibitory or activating natural killer (NK) receptors (Naper C, et al. (1999) Eur J Immunol 29: 2046-2053 ; Naper C, et al. (2005) J Immunol 174: 2702-2711). In the rat, in contrast to human, NK receptors of the Ly49 killer cell lectin-like receptor type predominate over killer cell Ig-like receptor genes. Therefore, the inventors also included the natural killer complex (NKC) in the expression profiling which harbors the Ly49 genes and additional natural cytotoxicity receptor genes.

To reduce the complexity of the experimental approach, the inventors used an in-vitro-model of the graft versus host reaction (GVHR) - the skin explant assay. This assay has been shown to be a sensitive predictor of GVHD in patients (Sviland L, et al. (2001) Hum Immunol 62: 1277-1281). It was also used to study the pathophysiology of GVHR (Dickinson AM, et al. (2002) Nat Med 8: 410-414). Recently, the inventors developed a rat skin explant assay (Novota P, et al. (2008) Transplantation 85: 1809-1816). This standardized in-vitro-model allows for studying gene expression during GVHR in a setting that is not influenced by undefined genetic differences between tissue samples which is unavoidable in human studies. Presently, the inventors used this model to analyze the MHC and NKC gene expression profiles of GVHR.

For the rat skin explant assays, rats of the inbred strains LEW.1N (*RT1ⁿ*), LEW.1A (*RT1^{a}*), LEW.1AV1 (*RT1^{avl}*), LOU/C (*RT1^{u}*)*,* and BUF (*RT1^{b}*) were bred in the central animal facility of the Medical Faculty of the University of Göttingen. Rats of the strains PVG/OlaHsd (*RT2^{c}*) and BN/RijHsd (*RT1ⁿ*) were purchased from Harlan Winkelmann (Borchen, Germany). Animals between 10 and 20 weeks of age were used for the experiments. For transplantation experiments, PVG rats of the RT7.2 allotype (allelic variant *RT7^{b}*)*,* originally obtained from Harlan OLAC, UK), were bred at the animal facility of the University of Oslo and BN rats were purchased from Harlan.

Rat skin explant assays were performed as previously described in detail (Novota P, Sviland L, Zinöcker S, Stocki P, Balavarca Y, et al. (2008) Correlation of Hsp70-1 and Hsp70-2 gene expression with the degree of graft-versus-host reaction in a rat skin explant model. Transplantation 85: 1809-1816). Briefly, mononuclear cells were obtained from rat spleens. Responder and irradiated (25 Gy) stimulator splenocytes were co-cultured in a MLR and the proliferation of responder lymphocytes was tested by [methyl-³H]-thymidine (Amersham, Braunschweig, Germany) incorporation. The stimulation index was calculated as described (Novota P, et al. (2008), supra). After 7 days 10⁶ responder lymphocytes were added to freshly obtained skin samples from the stimulator strain that were cultured in 200 µl NaHCO₃-buffered Dulbecco's modified Eagle's medium (DMEM; Biochrom) supplemented with 3 % normal rat serum, 2 mM L-glutamine, 1 mM sodium pyruvate, and antibiotics in round-bottomed microtitre plates (Sarstedt, Nümbrecht, Germany). The skin samples were excised from the paws of rats after washing with 70 % ethanol. The subcutaneous fat tissue was removed and the samples were trimmed to a size of approximately 1.5 × 1.5 mm. Skin samples cultured in medium only and samples co-cultured with lymphocytes from a "syngeneic MLR" were used as controls. After 3 days, the skin explants were washed with *N-*2-hydroxyethylpiperazine-*N*-2-ethanesulfonic acid (HEPES)-buffered DMEM and snap frozen in liquid nitrogen and stored at -80°C for RNA preparation. Parallel samples were fixed in 10 % neutral-buffered formalin, sectioned, and stained with hematoxylin and eosin (H&E). The histological evaluation of the skin explants was performed blind by an expert histopathologist (L.S.) based on the grading system described by Lerner (Lerner KG, et al. (1974) Transplant Proc 6: 367-371).

To obtain skin explant samples for an expression profiling experiment, the inventors used BN rats (*RT1ⁿ*) as recipients and PVG rats (*RT1^{c}*) as donors. This combination is mismatched for minor and major histocompatibility antigens, which gives rise to GVHR grades I to IV (Novota P, et al. (2008), supra). PVG splenocytes were stimulated for 7 days in a mixed lymphocyte reaction (MLR) with irradiated BN splenocytes. Syngeneic co-cultures (BN plus irradiated BN splenocytes) were performed as control experiments. The stimulation index indicated a specific proliferation of PVG lymphocytes in response to irradiated BN lymphocytes in contrast to syngeneic cultures of BN lymphocytes (p<0.0001, U test; n=12 responder animals per strain, data not shown). After 7 days the PVG and BN lymphocytes were harvested, added to fresh BN skin samples from 12 individual animals and cultured for 3 further days. For further controls, additional BN skin samples from the same animals were cultured in medium only. On day 3 the skin samples were harvested and snap frozen for RNA preparation. Parallel samples were fixed and assayed for histological evidence of GVHR **(****Fig. 1****).** Co-culture of BN skin explants with pre-stimulated allogeneic PVG lymphocytes resulted in higher grade GVHR than co-culture with BN lymphocytes (p=0.0336; U test). As in a previous experimental series (Novota P, et al. (2008) Transplantation 85: 1809-1816), the syngeneic lymphocyte co-culture more frequently resulted in GVHR-like pathology of grade II or higher than culture of the skin explants in medium only.

RNA was prepared from the 24 BN skin explants exposed either to syngeneic (BN; n=12) or to allogeneic (PVG, n=12) lymphocytes and used for MHC gene expression profiling.

RNA extraction was carried out using TRIZOL reagent (Invitrogen, Carlsbad, CA, USA) according to the manufacturer's recommendations. Afterwards, the RNA samples were treated with RQ1 RNase free DNase (Promega, Madison, WI, USA) for 20 min at 37 °C in order to remove genomic DNA contaminations. The RNA was then purified as described previously (Novota P, et al. (2008) Transplantation 85: 1809-1816). Quantity and quality of extracted RNA were controlled by capillary electrophoresis

### Microarray experiment

For the expression profiling, a custom-designed oligo DNA microarray (Agilent) was designed. For this purpose the annotated sequence of the MHC of the BN strain was used (Hurt P, et al. (2004) Genome Res 14: 631-639). The 15K microarray covered 224 MHC genes by 649 oligonucleotide probes and 43 NKC genes by 101 probes. For 88 of these genes, i.e. 39.3 %, the inventors had to design custom probes. A list of the MHC genes in the chromosomal order with all results obtained in the expression profiling experiment is given in the **Table 5a.**

These probes were spotted in triplicates. Further probes representing 6342 genes were added mainly to allow for data normalization. A two-color 12 × 2 paired swap design (Landgrebe J, et al. (2004) In Silico Biol 4: 461-470) using 24 arrays was applied, comparing RNA samples from 12 independent allogeneic and 12 independent syngeneic skin explant assays. Aliquots of total RNA (200 ng) were used as starting material. The "Low RNA Input linear Amplification Kit Plus, two color" (Agilent, 5188-5340) and the "RNA Spike-In Kit" (Agilent, 5188-5279) were used for cDNA synthesis and *in-vitro* transcription according to the manufacturer's recommendations. Quantity and dye incorporation rates of the amplified cRNAs were determined using the NanoDrop ND-1000 UV-VIS Spectrophotometer version 3.2.1 (NanoDrop Technologies, Wilmington, DE, USA). Afterwards, 300 ng aliquots of Cy3 and Cy5-labeled cRNAs from syngeneic and allogeneic skin explant assays, respectively, were mixed and hybridized to the microarrays. The hybridization was performed for 17 hours at 10 rpm and 65 °C. After washing, Cy3 and Cy5 intensities were detected by two-color scanning using a DNA microarray scanner (Agilent, G2505B) at 5 micron resolution. Scanned image files were visually inspected for artifacts. The generated raw data were extracted using the Feature Extraction 9.1 software (Agilent).

The normalization of the raw microarray data was done with a non-linear loess regression (Yang YH, et al. (2002) Nucleic Acids Res 30: e15). Differentially expressed genes were identified by an analysis of variance (ANOVA) mixed effects model (Landgrebe J, et al. (2004) In Silico Biol 4: 461-470) using SAS PROC MIXED. The resulting p-values were adjusted with the Benjamini-Hochberg method to control the false discovery rate (Benjamini Y, Hochberg Y (1995) J Roy Statist Soc Ser B 57: 289-300). The microarray data were generated conforming to the MIAME guidelines and have been deposited in NCBI's Gene Expression Omnibus (accessible through GEO series accession number GSE17928). For a general analysis of the gene expression data the PANTHER (Protein ANalysis THrough Evolutionary Relationships) system (Thomas PD, et al. (2003) Genome Res 13: 2129-2141) was used, which classifies genes by their functions (www.pantherdb.org/tools/genexAnalysis.jsp). The microarray data were mapped to PANTHER molecular function and biological process categories, as well as to biological pathways (Thomas PD, et al. (2006) Nucleic Acids Res 34: W645-650).

For 42 of the 224 MHC genes, a probe on the array indicated a significant regulation (p<0.05) in the allogeneic skin explant assays (n=12) compared to the syngeneic controls (n=12) **(Tab. 5b).** Eleven of these MHC genes showed on average at least a 2-fold up-regulation (log2-fold change ≥1) or 50 % reduction (log2-fold change ≤-1) of mRNA levels **(****Fig. 2*****A*, Tab. 5c).** This amplitude of change is conventionally considered to be biologically relevant. Of these genes one was down-regulated (*Ly6g6e*) while 10 were up-regulated **(****Fig. 2*****A*).** Fourteen further MHC genes were regulated significantly (p<0.05) but with smaller amplitude **(Tab. 5c).** The regulation of 17 MHC genes appeared to be more doubtful because less than 50 % of the probes for that gene indicated a significant regulation. Thus, the inventors considered 25 MHC genes to be significantly regulated in the expression profiling experiment **(****Fig. 2*****A*).** These included the classical class Ia genes *RT1-A1* and *RT1-A2,* 8 non-classical class Ib genes (*RT1-CE2, RT1-CE3, RT1-CE5, RT1-CE8, RT1-CE10, RT1-CE16, RT1-T24-4, RT-BM1*) and 3 genes involved in antigen presentation (*RT1-DMb, Tap1, Psmb8*). Furthermore, 43 genes of the NKC region, as a second important immune gene cluster, were represented on the array including all *Ly49* genes in this region **(Tab. 6a).** For 8 of the 43 NKC genes represented on the array, a probe indicated a significant regulation (p<0.05) in the allogeneic skin explant assays compared to the syngeneic controls **(Tab. 6b, 6c).** In addition to the *Olr1* gene, 6 *Ly49* genes appeared to be up-regulated in the allogeneic skin explant assays **(****Fig. 2*****B*).** Not all probes for the *Ly49i3* gene indicated a significant up-regulation. However, all significant results for this gene indicated a strong regulation (log2-fold change >2). A statistically significant (p<0.05) but only moderate up-regulation (log2-fold change <1) was detected for the *Ly49i7* gene.

Probes for 6342 additional genes from all chromosomes were included mainly to allow for data normalization. For 168 of the non-MHC/non-NKC genes, a probe on the array indicated a significant (p<0.05) and strong (log2-fold change ≥1 or ≤-1) regulation in the allogeneic skin explant assays compared to the syngeneic controls **(****Fig. 3*****C*, Tab. 7).** The 20 genes showing the strongest change in expression levels are shown in **Table 1.**

**Table 1. The 20 most strongly regulated non-MHC/non-NKC genes in allogeneic skin explants compared to syngeneic controls as revealed by the microarray experiment**

| **gene** | **log2-fold change** | **adjusted p-value** | **gene description** |
|---|---|---|---|
| LOC685020 | 8.18 | 0.0100 | paired immunoglobin-like type 2 receptor alpha |
| Ptpns1l3 | 6.36 | 0.0100 | protein tyrosine phosphatase, non-receptor type substrate 1-like 3 |
| Fcgr3a | 5.24 | 0.0100 | Fc fragment of IgG, low affinity IIIa, receptor |
| Nat8 | 5.14 | 0.0100 | Rattus norvegicus endogenous retrovirus mRNA, partial sequence [AY212271] |
| Ccl9 | 4.16 | 0.0100 | chemokine (C-C motif) ligand 9 |
| XM_226926 | 3.92 | 0.0149 | Rattus norvegicus similar to protein tyrosine phosphatase, non-receptor type substrate; brain immunological-like with tyrosine-based motifs (LOC310212) |
| Hck | 3.87 | 0.0100 | hemopoietic cell kinase |
| Trem2 | 3.78 | 0.0100 | triggering receptor expressed on myeloid cells 2 |
| Ccl6 | 3.71 | 0.0100 | Rattus norvegicus chemokine (C-C motif) ligand 6 |
| Cd36 | 3.57 | 0.0100 | CD36 antigen |
| Igf1 | 3.23 | 0.0100 | insulin-like growth factor 1 |
| Ctss | 3.15 | 0.0100 | cathepsin S |
| Gzmc | 3.11 | 0.0373 | granzyme C |
| LOC100048479 | 2.97 | 0.0373 | one cut domain, family member 1 |
| Plscr1 | 2.83 | 0.0100 | phospholipid scramblase 1 |
| Nfe2 | 2.74 | 0.0149 | nuclear factor, erythroid derived 2 |
| Prg4 | 2.74 | 0.0149 | proteoglycan 4 |
| Spic | 2.68 | 0.0278 | Spi-C transcription factor |
| Fcgr2b | 2.62 | 0.0100 | Fc receptor, IgG, low affinity IIb |
| LOC498277 | 2.61 | 0.0100 | similar to Low affinity immunoglobulin gamma Fc region receptor III precursor |

All 20 genes were up-regulated and they included several genes with functions clearly associated with the immune response such as genes encoding chemokines (*Ccl9, Ccl6*), Fc receptors (*Fcgr3a, Fcgr2b*), the proteases cathepsin S (*Ctss*) and granzyme C (*Gzmc*), and the inflammatory triggering receptor on myeloid cells 2 (*Trem2*).

The percentage of significantly (p<0.05) and strongly (log2-fold change ≥1 or ≤-1) up- or down-regulated genes was higher in the NKC region (14.0 %) compared to MHC region (4.9 %) and the genes encoded in other regions of the genome (2.6 %). This difference was even more pronounced for up-regulated genes. 14.0 % of the NKC, but only 4.5 % of the MHC and 1.5 % of the other genes were up-regulated **(Tab. 2).**

**Table 2. Proportion of regulated genes as indicated by the gene expression profiling experiment**

| **region** | **analyzed genes** | **regulated¹** | **up-regulated** | **down-regulated** |
|---|---|---|---|---|
| **MHC** | 224 | 11 (4.9 %) | 10 (4.5 %) | 1 (0.4 %) |
| **NKC** | 43 | 6 (14.0 %) | 6 (14.0%) | 0 (0 %) |
| **others** | 6342 | 168 (2.6 %) | 93 (1.5 %) | 75 (1.2 %) |

| | | | | |
|---|---|---|---|---|
| ¹ Only those genes that were both significantly (p<0.05) and strongly (log2-fold change ≥1 or ≤-1) regulated were taken into account for this comparison. | | | | |

For a general analysis of the gene expression data the PANTHER system (Thomas PD, et al. (2003) Genome Res 13: 2129-2141) was used. With this tool the inventors found a significant up-regulation of genes taking part in "immunity and defence" (p<0.0001, binominal test). More specifically, genes involved in "T cell-mediated immunity" (p<0.0001), "NK cell-mediated immunity" (p<0.0001), "cytokine and chemokine-mediated signaling" (p=0.0032), and "B cell and antibody-mediated immunity" (p=0.0235) were up-regulated. Genes involved in "complement-mediated immunity" (p=0.0336) and "cell adhesion" (p=0.0003) were down-regulated (data not shown).

### Validation of rat candidate genes by quantitative real-time PCR

To determine the reliability of the microarray results, the inventors analyzed the expression of 13 selected genes from the MHC and NKC regions by qRT-PCR experiments in 8 of the sample pairs that had been used for the microarrays (see **Fig. 1****).** Specific primers for 10 MHC and 3 NKC genes were designed **(Tab. 8).** To generate external standard curves and to calculate the amplification efficiency of each primer pair, a pool of 20 random cDNAs was amplified in serial 10-fold dilutions (Pfaffl MW (2001) Nucleic Acids Res 29: e45). The amplification reactions were carried out as described previously (Novota P, et al. (2008) Transplantation 85: 1809-1816) using an ABI 7500 Real-Time PCR System. The data were analyzed with the ABI 7500 SDS software (Applied Biosystems). As internal control, mRNA expression of housekeeping genes *Gapdh* (Rn_Gapd_1_SG QuantiTect Primer Assay QT00199633, Qiagen, Hilden, Germany) or *B2m* were monitored. To normalize variations in the RNA concentration in different samples, the ct values obtained in real-time PCR for the genes were corrected by the ct-value obtained for the housekeeping gene in the same sample (Act = ct housekeeping gene - ct gene of interest). For direct comparison with microarray data, the relative changes of mRNA expression were calculated using the ΔΔct method (ΔΔct = Act sample of interest - Δct control sample) (Livak KJ, Schmittgen TD (2001) Methods 25: 402-408). For additional analyses, the relative changes of gene expression levels were calculated using a mathematical model for relative quantification of real-time PCR data which takes into account variations of the amplification efficiencies of different primer pairs (Pfaffl MW (2001), supra).

For 12 genes the regulation that was observed in the microarray experiment was confirmed by qRT-PCR as indicated by a regulation into the same direction when the allogeneic and syngeneic skin explant assays were compared using the ΔΔ cycle threshold (ct) method for relative quantification of gene expression **(****Fig. 3****).** Only one gene, *RT1-CE10,* was found to be strongly up-regulated in allogeneic skin explants in the microarray experiment but slightly down-regulated in qRT-PCR. In the qRT-PCR experiments, the inventors also included parallel skin explants that were cultured in medium only. Eight genes (*RT1-DMb, Aif1, Lst1, RT1-CE3, Ubd, Olr1, Ly49si1,* and *Ly49i9*) showed an up-regulation in the allogeneic skin explant assay also in this comparison **(****Fig. 3****).** Six of these genes (*Aif1, Lst1, Ubd, Olr1, Ly49si1,* and *Ly49i9*) were clearly found to be up-regulated in both comparisons.

The up-regulation of genes in skin explants could be due to the change of gene expression in cells of the skin or due to infiltration of donor lymphocytes. Non-infiltrating or non-attaching donor lymphocytes were washed off before freezing of the skin explants and therefore would not contribute significantly to the results. Infiltrating lymphocytes were rarely seen in skin explants by histological analysis (data not shown). To further determine T cell infiltration at the RNA level, the inventors analyzed the expression of the CD3 zeta chain in qRT-PCR. *Cd3z* expression was found to be up-regulated in comparison to syngeneic controls and medium controls **(****Fig. 4*****A*).** The expression of most tested genes showed no correlation with *Cd3z* mRNA levels **(****Fig. 4*****B*)**. Only two of the genes analyzed in qRT-PCR (*Ly6g6e* and *Olr1*) showed a moderately positive correlation (r>0.50) with the *Cd3z* expression level **(****Fig. 4*****B*)**. Importantly, *Ly6g6e* was down- and not up-regulated in allogeneic skin explants. The expression levels of three up-regulated genes (*Psmb8, Aif1,* and *Lst1*) were even negatively associated with *Cd3z* expression **(****Fig. 4*****B*)**. Thus, of the tested genes only the increase of *Olr1* expression may be formally explained by infiltrating T cells. However, *Olr1* has not been described to be expressed in T cells. Therefore, infiltration of skin explants with T cells is unlikely to explain the observed gene expression changes.

Next the inventors determined the expression of 10 selected genes in an independent set of skin explant assays. Skin explants derived from BN (*RT1ⁿ*) and LEW.1N (*RT1ⁿ*) rats were co-cultured with pre-stimulated allogeneic lymphocytes from rats with minor (BN lymphocytes and LEW.1N skin), major (LEW.1A (*RT1^{a}*) or LEW.1AV1 (*RT1^{av1}*) lymphocytes and LEW.1N skin), or minor and major histoincompatibility (PVG lymphocytes (*RT1^{c}*) and BN skin or LOU/C (*RT1^{u}*) lymphocytes and LEW.1N skin). Skin samples cultured with syngeneic lymphocytes (BN or LEW.1N) or cultured in medium only served as controls. The GVHR grading obtained in these experiments is shown in **Figure 5****.** The general regulation of the selected genes during GVHR was reproduced in this second experimental set when compared to skin explants exposed to syngeneic lymphocytes and also to samples cultured in medium only **(****Fig. 6****).** *Aif1* and *Lst1* were the most consistently up-regulated genes in skin explants with minor, major, and minor plus major histoincompatibility. The samples with minor plus major histoincompatibility showed the highest variation in gene regulation **(****Fig. 6****).** However, these samples were also most heterogeneous in the GVHR grading **(****Fig. 5****).** Therefore, the inventors analyzed the gene regulation dependent from the GVHR grading in samples from both experimental sets.

### Regulation of selected MHC and NKC genes during GVHR

The expression of 7 MHC and 3 NKC genes was evaluated in the skin explant samples showing grade I, II, III or IV GVHR **(****Fig. 7****).** To provide an even more accurate comparison of the different genes in this evaluation of the data, the relative changes of gene expression levels were calculated using a mathematical model for relative quantification of real-time PCR data which takes into account variations in the amplification efficiencies of different primer pairs (Pfaffl MW (2001) Nucleic Acids Res 29: e45). When compared to skin explants exposed to syngeneic lymphocytes or to medium controls, the genes *Aif1, Lst1, Olr1,* and *Ly49i9* were consistently up-regulated. *Ly6g6e* was down-regulated in some but not all comparisons. The expression of *Aif1, Lst1* and *Ly49i9* was found to be increased in all GVHR grades. The extremely high up-regulation of *Ly49i9* encoding an NK receptor in comparison to medium controls might be explained by complete absence of NK cells in normal skin biopsies and infiltration of few NK cells during GVHR. When the gene expression was compared to freshly frozen healthy skin, the principal findings were confirmed. Interestingly, *Olr1* was up-regulated mainly in grade II and III GVHR samples when compared to syngeneic control skin explants and healthy skin. Thus, this gene could be a marker of intermediate grade GVHR.

### Regulation of selected MHC and NKC genes during GVHD

Next, the inventors wanted to know whether the genes found to be differentially expressed in GVHR in skin explant assays were also regulated *in vivo* in GVHD. For this purpose the inventors analyzed skin samples from BN rats that were transplanted with bone marrow from PVG rats and developed acute GVHD.

Transplantation experiments were approved by the Experimental Animal Board under the Ministry of Agriculture of Norway (ID 09.1514, 09.1515 and VIT 09.1512). Male PVG (*RT7^{b}*) rats served as bone marrow and lymph node donors. Mononuclear bone marrow cells were purified by density gradient centrifugation in Nycoprep 1.077A (Medinor ASA, Norway). The cells were depleted of T cells by magnetic separation using anti-CD5 (Ox19) and anti-αβ T cell receptor (R73) antibodies conjugated to pan-mouse IgG coated Dynabeads (Dynal Biotech ASA, Norway). This procedure reduced the CD3⁺ T cell content in the bone marrow from 3 % to less than 0.3 %. Male BN rats were used as recipients. They were irradiated (9 Gy) and subsequently received an i.v. injection of 30×10⁶ PVG.7b T cell-depleted bone marrow cells. 14 days post transplantation, 1.5×10⁶ lymph node cells were injected i.v. to evoke GVHD. The rats were regularly monitored for GVHD symptoms. Rats suffering from irreversible GVHD were sacrificed and skin samples were processed for RNA preparation and histology in parallel.

The analyzed skin samples showed in histology a grade I or grade II GVHD. The results of qRT-PCR for 7 MHC genes and 3 NKC genes are shown in **Figure 8****.** The strongest up-regulation in GVHD-affected skin was observed for *RT1-DMb, Aif1, Lst1,* and *Olr1.* Thus, most genes that were found to be regulated in GVHR in skin explants were also regulated in GVHD-affected skin. However, the *Ly49si1* gene that was up-regulated consistently in allogeneic skin explants showing GVHR of grade II and above appeared to be down-regulated in GVHD. Compared to the skin explant samples, also the *Ly49i9* gene was only moderately up-regulated in grade II GVHD samples from transplanted rats.

### Example 2: Regulation of selected MHC and NKC genes during GVHR in human skin explant assays

Finally, the inventors explored the regulation of the identified genes during GVHR in human skin explant assays.

Validation of the rat candidate genes with human homologues was done by qRT-PCR on clinical samples of GvhD skin and normal skin samples. This was done by relative quantification using custom designed Taqman low density array (TLDA) cards (Applied Biosystems), each card contained 4 replicates of 95 unique genes and a control gene, *18S.* The qRT-PCR reactions were set up using Taqman x2 gene expression mastermix (Applied Biosystems), 50 ng RNA equivalent of cDNA and the total volume adjusted to 200 µl with nuclease free water (Quiagen). The TLDA cards were run on a 7900 qRT-PCR system (Applied Biosystems) using the TLDA block and analysed using the RQ manager 1.2 software (Applied Biosystems). To normalize variations in the RNA concentration and quality in different samples, the ct values obtained in real-time PCR for the genes were corrected by the ct-value obtained for the housekeeping gene in the same sample (Act = ct housekeeping gene - ct gene of interest) then the relative changes in RNA expression were calculated using the ΔΔct method (ΔΔct = Act sample of interest - Δct control sample) using the average Act values of 5 normal skins as the control sample for each of the 9 GVHD skins.

At 1, 2 and 3 days of co-culture with alloreactive lymphocytes skin samples of one donor were taken and analyzed in comparison to parallel samples cultured in medium only. At day 1 a GVHR of grade I was observed that increased to grade II at day 2 and grade III at day 3. The inventors determined the expression of 15 MHC and 1 NKC gene by qRT-PCR **(Tab. 3).**

**Table 3. Regulation of MHC and NCR candidate genes in human skin explants**

| | **regulation in rat skin explant assays (expression profiling)** | **regulation in human skin explant assay** | | | **concordance rate** |
|---|---|---|---|---|---|
| | | **day 1 (GVHR I)** | **day 2 (GVHR II)** | **day 3 (GVHR III)** | |
| **MHC region** | | | | | |
| HLA-DMB | ↑**¹** | - | ↑ | - | 1/3 |
| TAP1 | **(**↑**)** | ↑ | ↑ | ↑ | 3/3 |
| PSMB8 | ↑ | ↑ | ↑ | ↑ | 3/3 |
| G18 (GPSM3) | ↑ | n.d. | n.d. | n.d. | |
| PBX2 | **(**↑**)** | ↓ | n.d. | ↑ | 1/3 |
| C2 | ↑ | ↑ | ↑ | ↓ | 2/3 |
| LY6G6E | ↓ | n.d. | ↑ | n.d. | 0/3 |
| BAT5 | ↓ | - | - | - | 0/3 |
| AIF1 | ↑ | ↓ | ↑ | ↓ | 1/3 |
| LST1 | ↑ | - | ↑ | n.d. | 1/3 |
| SPR1 (PSORS1C2) | ↑ | - | - | ↑ | 1/3 |
| IER3 | ↑ | ↓ | ↑ | - | 1/3 |
| FLI13158 | **(**↓**)** | ↓ | ↓ | - | 2/3 |
| MRPS18B | **(**↑**)** | ↓ | ↓ | ↓ | 0/3 |
| UBD | ↑ | ↑ | ↑ | ↑ | 3/3 |

| **NCR region** | | | | | |
|---|---|---|---|---|---|
| OLR1 | ↑ | ↑ | ↑ | n.d. | 2/3 |

| | | | | | |
|---|---|---|---|---|---|
| ¹ Explanation of symbols: ↑ up-regulated mRNA expression level (log2-fold change ≥ 1) ↓ down-regulated mRNA level (log2-fold change ≤ -1) - unchanged mRNA expression level (log2-fold change > -1 and < 1) (↑) significant (p < 0.05) but moderate up-regulation (log2-fold change < 1) of mRNA expression level in the rat expression profiling experiment (↓) significant (p < 0.05) but moderate down-regulation (log2-fold change > -1) of mRNA expression level n.d. no mRNA detected | | | | | |

Of these 16 genes 12 (75 %) were regulated at least in one skin explant sample in the way predicted by the results of the rat expression profiling experiments **(Tab. 4).** Three genes *TAP1, PSMB8,* and *UBD* were up-regulated in all 3 human skin explant samples. The genes *C2, FLI13158,* and *OLR1* were regulated in 2 of the 3 samples as predicted by the rat experiments. In addition, the inventors determined the expression of 153 non-MHC/non-NCR genes that were identified to be regulated in rat skin explant assays. Also of these genes 105 (69 %) were regulated in at least one of the human skin explant samples in accordance with the results obtained in the rat model **(Tab. 4).** These results suggest that the *in vitro* rat model of the skin explant assay gives evidences of gene expression changes that are very likely to occur also in human skin explant assays during GVHR.

**Table 4. Proportion of concordantly regulated in MHC, NKC, and genes encoded in other regions in human skin explant assays in comparison to rat skin explant assays**

| **region** | **analyzed human genes** | **mRNA not detected** | **concordantly regulated in human skin explant assays in comparison to rat skin explant assays** | | | **not concordantly regulated** |
|---|---|---|---|---|---|---|
| | | | 3/3 | 2/3 | 1/3 | 0/3 |
| **MHC** | 15 | 1 (7 %) | 3 (20 %) | 2 (13 %) | 6 (40 %) | 3 (20 %) |
| **NKC** | 1 | 0 (0 %) | 0 (0 %) | 1 (100 %) | 0 (0%) | 0 (0 %) |
| **others** | 153 | 18 (12 %) | 33 (22 %) | 31 (20 %) | 41 (27 %) | 30 (20%) |

In a follow-up study, 24 genes have been identified in additional validation tests. The results are shown in **Table 9.** The probes used and the reference sequences are shown in **Table 10.** The additional validation tests confirmed the significant regulation of gene expression, i.e. up-regulation or down-regulation, preferably down-regulation for Ctss, Pbx2, Spr1, Spic, Nfe2, Tnfaip8l2, Ier3, and Lst1.

### Statistical analyses not related to microarray experiments

Paired comparisons between experimental groups were performed using the nonparametric Mann-Whitney U test. Pearson's and Spearman's correlation coefficients were calculated to determine the correlation between mRNA expression levels of two genes. The statistical analyses were performed using WinSTAT® software.

### Example 3: mRNA expression profiling in in human clinical GVHD biopsies

Further studies were undertaken to evaluate the expression markers also under clinical conditions. Therefore, new tests were performed using skin explant assay as well as mRNA expression profiling studies directly on clinical GVHD biopsies to validate the results from the previous skin explant studies. The clinical GVHD biopsies were taken from hematopoietic stem cell transplantation (HSCT) patients. These data are summarized in **Table 11.**

### Experimental Skin Explants Assays using Autologous HSCT patients and Normal Controls

Peripheral blood mononuclear cells (PBMC) and skin samples were obtained from autologous HSCT patients following informed consent and approval from the North Tyneside Research Ethics Committee. Buffy coat from HLA mismatched normal blood donations were obtained from Newcastle National Blood Service with consent. Skin explant assays were performed as previously described [5,6], 1x10⁷ responder PBMC from healthy volunteers was cultured with an equal number of irradiated PBMC from autologous HSCT patients, in 10 ml complete medium (RPMI 1640 supplemented with antibiotics, 2 mM L-glutamine and 10% heat inactivated human AB serum) in a 25 cm² flask. Standard 4mm punch skin biopsy specimens were obtained pre-transplant from the auto HSCT patients and divided into 12 equal sized pieces. After 7 days of culture, the MLR primed lymphocytes were washed and resuspended in complete medium supplemented with 20% heat inactivated autologous (patient) serum and co-cultured in duplicate with patient skin at a cell concentration of 1x10⁶ cells/well in a volume of 200 µl/well in 96- well round-bottomed microtitre plates. In addition each skin sample was also cultured in duplicate in culture medium alone as a negative or medium only control. A time course experiment was set up to enable RNA expression analysis to be assessed early, (day 1) and late, (days 2 and 3) to monitor the interaction of sensitised T cells with recipient skin. Parallel control skins were incubated in medium only on days 1, 2 and 3 and used as the comparators. The skin samples were removed from the time series, duplicate control and MLR skin explant on days one, two or three, one sample was fixed in 10% buffered formalin, sectioned and stained with H&E and duplicate sample placed in RNAlater (Ambion) and stored at -80°C prior to RNA extraction.

The histopathological evaluation of the skin explants for graft versus host reaction (GVHR) was performed independently by at least two assessors. Grade I histopathological damage in skin biopsies was regarded as background and was normally observed in the medium control. All biopsies presenting histopathological damage of grade II or above were regarded as GVHR positive.

### Clinical Biopsies

Standard 4mm punch biopsies or scrape biopsies were obtained from 10 patients at various time points post transplant at onset of acute GvHD together with normal skin skin controls (n=10). RNA was extracted from these biopsies as described below.

### RNA extraction and cDNA production

RNA was extracted from the skin samples stored in RNA later using the Ambion mir-Vana miRNA Isolation Kit according to the manufacturer's recommendations and quantified using the NanoDrop ND-1000 spectrophotometer (Thermo Scientific). cDNA was generated by random hexamer priming, briefly equal quantities of RNA and 2 x strength cDNA mix containing random hexamer primers (Pharmacia), dNTPs (Roche), reverse transcriptase (MMLVRT - Invitrogen) and an RNase inhibitor (Rnasin - Promega) were incubated at 37°C for 2 hours with a further incubation at 65°C for 10 minutes to denature the reverse transcriptase.

### Validation of candidate genes by quantitative real-time PCR

Validation of the candidate genes in the human skin explant assay and clinical biopsies was done by qRT-PCR. For this relative quantification with three custom designed Taqman low density array (TLDA) cards (Applied Biosystems) were used each card contained 4 replicates of 94 unique genes and two control genes, 18S and GAPDH, giving a total of 282 genes. The qRT-PCR reactions were set up using Taqman x2 gene expression mastermix (Applied Biosystems), 50 ng RNA equivalent of cDNA and the total volume adjusted to 200 µl with nuclease free water (Quiagen). The reaction mix was loaded onto the TLDA cards and the cards were run on a 7900 qRT-PCR system (Applied Biosystems) and analysed using the RQ manager 1.2 software (Applied Biosystems). The relative changes in RNA expression were calculated using the ΔΔct method, that is, ΔΔct = Act sample of interest - Δct control sample, where the Act is the ct of the control gene - the ct of the gene of interest.

Genes which showed a consistent change in expression between the medium only control skin and the MLR skin or in the clinical aGVHD skin compared to normal skin were investigated further using additional normal (n=10) and clinical aGVHD (n=10) skin samples. Real time PCR was carried out using individual TaqMan assays (Applied Biosystems) for the genes of interest and the control gene GAPDH (Applied Biosystems). The reactions were set up in triplicate using Taqman x2 gene expression mastermix, 10 to 20ng RNA equivalent of cDNA and the manufacturer's recommended concentration of primer/probe mix. The reactions were run on a 7900 qRT-PCR system (Applied Biosystems) and analysed using the SDS 2.3 software, normalisation of expression was performed using GAPDH gene, expression results and ACT values were calculated as above.

### Statistical Analysis

Comparisons between the experimental groups were carried out using the non-parametric Mann-Whitney U test using Graphpad prism 5 software (Graphpad Software inc.).

### Table 5. Expression profiling results of MHC genes

In **Table 5a,** results for all 224 MHC genes are shown in their chromosomal order (Hurt P, et al. (2004) Genome Res 14: 631-639). The expression profiling results of BN skin explant samples exposed to pre-stimulated allogeneic (PVG) lymphocytes in comparison to those exposed to syngeneic (BN) lymphocytes are given. The log2-fold changes and the fold changes in gene expression are shown for every oligonucleotide probe used. The adjusted p-values are indicated. Significant change is defined by p<0.05 and strong change is defined by log2-fold change ≥1 or ≤-1; i.e. fold change ≥2 or ≤0.5. In addition, the identification numbers of the probes on the arrays are given (probe ID) together with the information whether these probes were taken from the Agilent database or custom designed. **Table 5b** contains the same information for all MHC genes for which at least one probe indicated a significant alteration of gene expression. In **Table 5c,** the data for those genes are summarized that are considered to be regulated significantly because either at least a single probe indicated a significant (p<0.05) and strong (log2-fold change ≥1 or ≤-1) regulation or at least 50 % of the gene probes indicated a significant (p<0.05) regulation of gene expression.

**Table 5a**

| **Gene order** | **Gene Symbol** | **log2-Fold Change** | **Fold Change** | **adj.P-value** | **Gene Description** | **Probe ID** | **Probe Design** |
|---|---|---|---|---|---|---|---|
| 1 | 3930402F13Rik (Zbtb9) | 0,08 | 1,06 | 0,7687 | zinc finger and BTB domain containing 9 | A_43_P10072 | Agilent |
| 1 | 3930402F13Rik (Zbtb9) | 0,04 | 1,03 | 0,8180 | zinc finger and BTB domain containing 9 | A_43_P20769 | Agilent |
| 2 | Syngap1 | -0,17 | 0,89 | 0,1557 | synaptic Ras GTPase activating protein 1 homolog (rat) | A_44_P470444 | Agilent |
| 3 | Cuta | 0,21 | 1,16 | 0,4430 | cutA divalent cation tolerance homolog (E. coli) | A_42_P765298 | Agilent |
| 4 | Phf1 | 0,21 | 1,16 | 0,2688 | PHD finger protein 1 | A_44_P1057137 | Agilent |
| 5 | Kifc1 | -0,23 | 0,85 | 0,4351 | kinesin family member C1 | A_44_P1042372 | Agilent |
| 6 | AA926063 | -0,06 | 0,96 | 0,6022 | gene corresponding to rat EST acc. no. AA926063 | A_44_P128110 | Agilent |
| 7 | Daxx | 0,07 | 1,05 | 0,5495 | Fas death domain-associated protein | A_42_P622574 | Agilent |
| 8 | Znf297 | -0,21 | 0,86 | 0,1375 | zinc finger protein 297 | A_43_P18449 | Agilent |
| 8 | Znf297 | -0,12 | 0,92 | 0,8050 | zinc finger protein 297 | A_42_P486012 | Agilent |
| 8 | Znf297 | -0,06 | 0,96 | 0,7324 | zinc finger protein 297 | A_43_P20215 | Agilent |
| 8 | Znf297 | 0,12 | 1,09 | 0,4409 | zinc finger protein 297 | A_43_P20683 | Agilent |
| 9 | Tapbp | 0,31 | 1,24 | 0,3259 | TAP binding protein | A_42_P698972 | Agilent |
| 10 | Rab2l | -0,15 | 0,90 | 0,3580 | RAB2, member RAS oncogene family-like | A_44_P465986 | Agilent |
| 11 | Ke2 | -0,07 | 0,95 | 0,8616 | H2-K region expressed gene 2 | A_44_P498712 | Agilent |
| 12 | Bing4 (Wdr46) | -0,13 | 0,91 | 0,6702 | WD repeat domain 46 | A_44_P158675 | Agilent |
| 13 | B3galt4 | 0,01 | 1,01 | 0,9910 | UDP-Gal:betaGlcNAc beta 1,3-galactosyltransferase, polypeptide 4 | A_42_P692926 | Agilent |
| 14 | Rps18 | -0,32 | 0,80 | 0,1017 | ribosomal protein S18 | A_42_P582859 | Agilent |
| 15 | Sacm2l (Vps52) | 0,00 | 1,00 | 0,9771 | similar to vacuolar protein sorting 52 | A_43_P12732 | Agilent |
| 16 | RT1-A1 | 0,70 | 1,62 | 0,0149 | RT1 class I | CUST_1_P1202535318 | custom |
| 16 | RT1-A1 | 0,75 | 1,68 | 0,0100 | RT1 class I | CUST_2_PI202535318 | custom |
| 16 | RT1-A1 | 0,80 | 1,74 | 0,0149 | RT1 class I | CUST_3_PI202535318 | custom |
| 16 | RT1-A1 | 0,86 | 1,82 | 0,0100 | RT1 class I | CUST_4_PI202535318 | custom |
| 16 | RT1-A1 | 0,91 | 1,88 | 0,0100 | RT1 class I | CUST_5_PI202535318 | custom |
| 17 | RT1-A2 | 0,98 | 1,97 | 0,0100 | RT1 class I | A_44_P296155 | Agilent |
| 18 | RT1-A3 | 0,28 | 1,21 | 0,4444 | RT1 class I | A_44_P501234 | Agilent |
| 19 | Ring1 | -0,14 | 0,91 | 0,5739 | ring finger protein 1 | A_44_P100117 | Agilent |
| 20 | Hsd17b8 | 0,06 | 1,04 | 0,8435 | hydroxysteroid (17-beta) dehydrogenase 8 | A_43_P15081 | Agilent |
| 21 | Ke4 | -0,03 | 0,98 | 0,8962 | RT1 class I, locus Ke4 | CUST_1_PI195698117 | custom |
| 21 | Ke4 | -0,04 | 0,97 | 0,8617 | RT1 class I, locus Ke4 | CUST_2_PI195698117 | custom |
| 21 | Ke4 | -0,02 | 0,99 | 0,9361 | RT1 class I, locus Ke4 | CUST_3_PI195698117 | custom |
| 21 | Ke4 | 0,01 | 1,01 | 0,9700 | RT1 class I, locus Ke4 | CUST_4_PI195698117 | custom |
| 21 | Ke4 | -0,05 | 0,97 | 0,7835 | RT1 class I, locus Ke4 | CUST_5_PI195698117 | custom |
| 22 | Rxrb | -0,14 | 0,91 | 0,5922 | retinoid X receptor beta | A_52_P519689 | Agilent |
| 22 | Rxrb | -0,06 | 0,96 | 0,8238 | retinoid X receptor beta | CUST_11_PI207500742 | custom |
| 22 | Rxrb | -0,03 | 0,98 | 0,8934 | retinoid X receptor beta | CUST_12_PI207500742 | custom |
| 22 | Rxrb | -0,08 | 0,95 | 0,6808 | retinoid X receptor beta | CUST_13_PI207500742 | custom |
| 22 | Rxrb | -0,03 | 0,98 | 0,9004 | retinoid X receptor beta | CUST_14_PI207500742 | custom |
| 22 | Rxrb | 0,01 | 1,01 | 0,9575 | retinoid X receptor beta | CUST_15_PI207500742 | custom |
| 23 | Col11a2 | -0,45 | 0,73 | 0,0533 | procollagen, type XI, alpha 2 | A_44_P527024 | Agilent |
| 24 | RT1-Hb | 0,04 | 1,03 | 0,7868 | RT1 class II, H beta | A_44_P250763 | Agilent |
| 25 | RT1-Ha | 0,07 | 1,05 | 0,6681 | RT1 class II, H alpha | CUST_1_PI195698201 | custom |
| 25 | RT1-Ha | 0,02 | 1,01 | 0,8589 | RT1 class II, H alpha | CUST_2_PI195698201 | custom |
| 25 | RT1-Ha | -0,11 | 0,93 | 0,5819 | RT1 class II, H alpha | CUST_3_PI195698201 | custom |
| 25 | RT1-Ha | 0,00 | 1,00 | 0,9980 | RT1 class II, H alpha | CUST_4_PI195698201 | custom |
| 25 | RT1-Ha | -0,08 | 0,95 | 0,5019 | RT1 class II, H alpha | CUST_5_PI195698201 | custom |
| 26 | RT1-DOa | -0,06 | 0,96 | 0,7324 | RT1 class II, locus DOa | A_44_P344228 | Agilent |
| 27 | Brd2 | 0,46 | 1,38 | 0,0825 | bromodomain containing 2 | A_42_P558503 | Agilent |
| 28 | RT1-DMa | 0,87 | 1,83 | 0,0681 | histocompatibility 2, class II, locus DMa | A_42_P473314 | Agilent |
| 29 | RT1-DMb | 2,59 | 6,02 | 0,0100 | major histocompatibility complex, class II, DM beta | CUST_1_PI195698203 | custom |
| 29 | RT1-DMb | 2,77 | 6,82 | 0,0100 | major histocompatibility complex, class II, DM beta | CUST_2_PI195698203 | custom |
| 29 | RT1-DMb | 1,93 | 3,81 | 0,0149 | major histocompatibility complex, class II, DM beta | CUST_3_PI195698203 | custom |
| 29 | RT1-DMb | 1,87 | 3,66 | 0,0149 | major histocompatibility complex, class II, DM beta | CUST_4_PI195698203 | custom |
| 29 | RT1-DMb | 1,94 | 3,84 | 0,0100 | major histocompatibility complex, class II, DM beta | CUST_5_PI195698203 | custom |
| 30 | Psmb9 | 0,50 | 1,41 | 0,1412 | proteasome (prosome, macropain) subunit, beta type 9 (large multifunctional peptidase 2) | A_42_P759756 | Agilent |
| 31 | Tap1 | 0,53 | 1,44 | 0,1159 | transporter 1, ATP-binding cassette, sub-family B (MDR/TAP) | A_43_P15763 | Agilent |
| 31 | Tap1 | 0,63 | 1,55 | 0,0390 | transporter 1, ATP-binding cassette, sub-family B (MDR/TAP) | A_44_P451916 | Agilent |
| 32 | Psmb8 | 1,00 | 2,00 | 0,0336 | proteasome (prosome, macropain) subunit, beta type 8 (large multifunctional peptidase 7) | A_42_P761035 | Agilent |
| 33 | Tap2 | 0,44 | 1,36 | 0,1639 | transporter 2, ATP-binding cassette, sub-family B (MDR/TAP) | A_42_P797381 | Agilent |
| 34 | RT1-DOb | 0,39 | 1,31 | 0,0573 | RT1 class II, locus DOb | A_44_P294965 | Agilent |
| 35 | RT1-Bb | -0,29 | 0,82 | 0,6142 | RT1 class II, locus Bb | A_44_P552452 | Agilent |
| 36 | RT1-Ba | -0,14 | 0,91 | 0,8110 | RT1 class II, locus Ba | A_44_P128248 | Agilent |
| 36 | RT1-Ba | 0,18 | 1,13 | 0,2934 | RT1 class II, locus Ba | A_44_P194167 | Agilent |
| 36 | RT1-Ba | -0,09 | 0,94 | 0,8443 | RT1 class II, locus Ba | A_43_P14429 | Agilent |
| 37 | RT1-Db1 | 0,36 | 1,28 | 0,5390 | RT1 class II, D beta 1 | A_44_P130513 | Agilent |
| 38 | RT1-Db2 | 0,62 | 1,54 | 0,2275 | RT1 class II, D beta 2 | CUST_1_P1201011278 | custom |
| 38 | RT1-Db2 | 0,65 | 1,57 | 0,2267 | RT1 class II, D beta 2 | CUST_2_PI201011278 | custom |
| 38 | RT1-Db2 | 0,67 | 1,59 | 0,1732 | RT1 class II, D beta 2 | CUST_3_PI201011278 | custom |
| 38 | RT1-Db2 | 0,86 | 1,82 | 0,0856 | RT1 class II, D beta 2 | CUST_4_PI201011278 | custom |
| 38 | RT1-Db2 | 1,00 | 2,00 | 0,0755 | RT1 class II, D beta 2 | CUST_5_PI201011278 | custom |
| 39 | RT1-Da | 0,22 | 1,16 | 0,6541 | RT1 class II, D alpha | A_44_P991532 | Agilent |
| 40 | Btnl2 | -0,09 | 0,94 | 0,4427 | butyrophilin-like 2 (MHC class II associated) | A_23_P376686 | Agilent |
| 41 | Btnl3 | -0,10 | 0,93 | 0,2949 | butyrophilin-like 3 | A_42_P788302 | Agilent |
| 42 | Tesb | 0,18 | 1,13 | 0,1346 | testis specific basic protein | CUST_4_PI1956982050 | custom |
| 42 | Tesb | 0,14 | 1,10 | 0,5685 | testis specific basic protein | CUST_5_PI1956982050 | custom |
| 43 | Btnl4 | 0,75 | 1,68 | 0,1649 | butyrophilin-like 4 | CUST_44_PI240872834 0 | custom |
| 43 | Btnl4 | 0,71 | 1,64 | 0,1853 | butyrophilin-like 4 | CUST_45_PI240872834 0 | custom |
| 44 | Btnl5 | 0,20 | 1,15 | 0,4751 | butyrophilin-like 5 | CUST_7_PI207500742 | custom |
| 45 | Btnl6 | -0,08 | 0,95 | 0,3599 | butyrophilin-like 6 | CUST_1_PI201011255 | custom |
| 45 | Btnl6 | -0,29 | 0,82 | 0,0847 | butyrophilin-like 6 | CUST_2_PI201011255 | custom |
| 45 | Btnl6 | -0,01 | 0,99 | 0,9153 | butyrophilin-like 6 | CUST_3_PI201011255 | custom |
| 45 | Btnl6 | 0,00 | 1,00 | 0,9864 | butyrophilin-like 6 | CUST_4_PI201011255 | custom |
| 45 | Btnl6 | 0,06 | 1,04 | 0,5623 | butyrophilin-like 6 | CUST_5_PI201011255 | custom |
| 46 | Btnl7 | -0,12 | 0,92 | 0,2797 | butyrophilin-like 7 | A_44_P212575 | Agilent |
| 46 | Btnl7 | 0,08 | 1,06 | 0,7469 | butyrophilin-like 7 | CUST_1_PI201011264 | custom |
| 46 | Btnl7 | -0,04 | 0,97 | 0,7249 | butyrophilin-like 7 | CUST_2_PI201011264 | custom |
| 46 | Btnl7 | -0,07 | 0,95 | 0,5583 | butyrophilin-like 7 | CUST_3_PI201011264 | custom |
| 46 | Btnl7 | -0,08 | 0,95 | 0,5922 | butyrophilin-like 7 | CUST_4_PI201011264 | custom |
| 46 | Btnl7 | -0,28 | 0,82 | 0,0879 | butyrophilin-like 7 | CUST_5_PI201011264 | custom |
| 47 | Btnl8 | 0,09 | 1,06 | 0,4680 | butyrophilin-like 8 | A_44_P379412 | Agilent |
| 47 | Btnl8 | 0,26 | 1,20 | 0,2238 | butyrophilin-like 8 | CUST_6_PI207500742 | custom |
| 47 | Btnl8 | -0,16 | 0,90 | 0,2688 | butyrophilin-like 8 | CUST_8_PI207500742 | custom |
| 47 | Btnl8 | -0,03 | 0,98 | 0,8236 | butyrophilin-like 8 | CUST_9_PI207500742 | custom |
| 47 | Btnl8 | 0,12 | 1,09 | 0,3144 | butyrophilin-like 8 | CUST_10_PI207500742 | custom |
| 48 | Btnl9 | -0,03 | 0,98 | 0,8134 | butyrophilin-like 9 | A_32_P187951 | Agilent |
| 48 | Btnl9 | -0,11 | 0,93 | 0,3818 | butyrophilin-like 9 | A_23_P81280 | Agilent |
| 49 | C4-2 | -0,97 | 0,51 | 0,1521 | complement component 4, gene 2 | A_42_P494900 | Agilent |
| 50 | Notch4 | -0,90 | 0,54 | 0,1159 | Notch homolog 4 | A_42_P734094 | Agilent |
| 51 | G18 (Gpsm3) | 1,23 | 2,35 | 0,0315 | G18 protein | A_42_P569708 | Agilent |
| 52 | Pbx2 | 0,33 | 1,26 | 0,0466 | pre-B-cell leukemia transcription factor 2 | A_42_P592157 | Agilent |
| 53 | Ager | 0,10 | 1,07 | 0,4914 | advanced glycosylation end product-specific receptor | A_43_P15393 | Agilent |
| 54 | Rnf5 | 0,57 | 1,48 | 0,0315 | ring finger protein 5 | A_51_P204582 | Agilent |
| 54 | Rnf5 | 0,26 | 1,20 | 0,0674 | ring finger protein 5 | CUST_1_PI207500742 | custom |
| 54 | Rnf5 | 0,21 | 1,16 | 0,1445 | ring finger protein 5 | CUST_2_PI207500742 | custom |
| 54 | Rnf5 | 0,17 | 1,13 | 0,2905 | ring finger protein 5 | CUST_3_PI207500742 | custom |
| 54 | Rnf5 | 0,22 | 1,16 | 0,1626 | ring finger protein 5 | CUST_4_PI207500742 | custom |
| 54 | Rnf5 | 0,19 | 1,14 | 0,1707 | ring finger protein 5 | CUST_5_PI207500742 | custom |
| 55 | Agpat1 | 0,14 | 1,10 | 0,6400 | 1-acylglycerol-3-phosphate O-acyltransferase 1 | A_44_P419004 | Agilent |
| 56 | Ng3 | -0,18 | 0,88 | 0,3016 | NG3 protein | CUST_51_PI209196805 | custom |
| 56 | Ng3 | 0,07 | 1,05 | 0,6808 | NG3 protein | CUST_52_PI209196805 | custom |
| 56 | Ng3 | -0,12 | 0,92 | 0,3181 | NG3 protein | CUST_53_PI209196805 | custom |
| 56 | Ng3 | -0,29 | 0,82 | 0,2314 | NG3 protein | CUST_54_PI209196805 | custom |
| 56 | Ng3 | -0,07 | 0,95 | 0,8401 | NG3 protein | CUST_55_PI209196805 | custom |
| 57 | Ppt2 | -0,12 | 0,92 | 0,6876 | palmitoyl-protein thioesterase 2 | A_44_P343303 | Agilent |
| 58 | Ng5 | 0,18 | 1,13 | 0,2523 | NG5 protein | CUST_1_PI195698205 | custom |
| 58 | Ng5 | 0,06 | 1,04 | 0,6520 | NG5 protein | CUST_2_PI195698205 | custom |
| 58 | Ng5 | 0,22 | 1,16 | 0,1750 | NG5 protein | CUST_3_PI195698205 | custom |
| 58 | Ng5 | 0,18 | 1,13 | 0,1494 | NG5 protein | CUST_4_PI195698205 | custom |
| 58 | Ng5 | 0,13 | 1,09 | 0,4775 | NG5 protein | CUST_5_PI195698205 | custom |
| 59 | Fkbpl | -0,05 | 0,97 | 0,8864 | FK506 binding protein-like | A_44_P1048901 | Agilent |
| 60 | Crebl1 | 0,36 | 1,28 | 0,5778 | cAMP responsive element binding protein-like 1 | A_44_P292503 | Agilent |
| 61 | Tnx | 0,04 | 1,03 | 0,6633 | tenascin-X | CUST_2_PI2010111961 | custom |
| 61 | Tnx | 0,03 | 1,02 | 0,8015 | tenascin-X | CUST_3_PI2010111961 | custom |
| 62 | Cyp21a1 | -0,09 | 0,94 | 0,3540 | cytochrome P450, family 21, subfamily a, polypeptide 1 | A_44_P381937 | Agilent |
| 63 | C4-1 | -0,76 | 0,59 | 0,1375 | complement component 4, gene 1 | A_43_P21634 | Agilent |
| 64 | Stk19 | 0,28 | 1,21 | 0,2657 | serine/threonine kinase 19 | A_44_P491782 | Agilent |
| 65 | Dom3z | 0,27 | 1,21 | 0,3198 | DOM-3 homolog Z | A_44_P158709 | Agilent |
| 66 | Skiv2l | -0,24 | 0,85 | 0,3408 | superkiller viralicidic activity 2-like | A_44_P292558 | Agilent |
| 67 | Rdbp | -0,47 | 0,72 | 0,1367 | RD RNA-binding protein | A_44_P266879 | Agilent |
| 68 | Bf (CfB) | -0,71 | 0,61 | 0,3004 | complement factor B | A_44_P419064 | Agilent |
| 69 | C2 | 1,22 | 2,33 | 0,0325 | complement component 2 | A_44_P332606 | Agilent |
| 70 | Ng35 | -0,04 | 0,97 | 0,7095 | Ng35 protein | A_43_P17778 | Agilent |
| 71 | Bat8 (Ehmt2) | -0,23 | 0,85 | 0,5125 | euchromatic histone lysine N-methyltransferase 2 | A_44_P1057272 | Agilent |
| 72 | Ng22 (Slc44a4) | -0,20 | 0,87 | 0,3134 | solute carrier family 44, member 4 | A_43_P18443 | Agilent |
| 72 | Ng22 (Slc44a4) | -0,81 | 0,57 | 0,0598 | solute carrier family 44, member 4 | A_44_P1037285 | Agilent |
| 73 | Neu1 | 0,22 | 1,16 | 0,3690 | neuraminidase 1 | A_43_P12574 | Agilent |
| 74 | Hspa1b | 0,06 | 1,04 | 0,5939 | heat shock 70kD protein 1B (mapped) | A_44_P532958 | Agilent |
| 75 | Hspa1a | 0,07 | 1,05 | 0,8419 | heat shock 70kD protein 1A | A_44_P1042876 | Agilent |
| 76 | Hspa1l | -0,20 | 0,87 | 0,2682 | heat shock 70kD protein 1-like (mapped) | A_42_P541025 | Agilent |
| 77 | Lsm2 | 0,08 | 1,06 | 0,8227 | LSM2 homolog, U6 small nuclear RNA associated [S. cerevisiae] | A_51_P314931 | Agilent |
| 77 | Lsm2 | 0,03 | 1,02 | 0,9332 | LSM2 homolog, U6 small nuclear RNA associated [S. cerevisiae] | CUST_6_PI209196805 | custom |
| 77 | Lsm2 | 0,12 | 1,09 | 0,7330 | LSM2 homolog, U6 small nuclear RNA associated [S. cerevisiae] | CUST_7_PI209196805 | custom |
| 77 | Lsm2 | -0,04 | 0,97 | 0,8943 | LSM2 homolog, U6 small nuclear RNA associated [S. cerevisiae] | CUST_8_PI209196805 | custom |
| 77 | Lsm2 | 0,13 | 1,09 | 0,6868 | LSM2 homolog, U6 small nuclear RNA associated [S. cerevisiae] | CUST_9_PI209196805 | custom |
| 77 | Lsm2 | 0,06 | 1,04 | 0,8429 | LSM2 homolog, U6 small nuclear RNA associated [S. cerevisiae] | CUST_10_PI209196805 | custom |
| 78 | G7e | -0,35 | 0,78 | 0,8265 | G7e pseudogen | CUST_1_PI2010111701 | custom |
| 78 | G7e | -0,21 | 0,86 | 0,6235 | G7e pseudogen | CUST_2_PI2010111701 | custom |
| 79 | Vars2 | 0,08 | 1,06 | 0,7163 | valyl-tRNA synthetase | A_42_P646976 | Agilent |
| 80 | G7c | -0,14 | 0,91 | 0,2006 | G7c protein | A_44_P325599 | Agilent |
| 80 | G7c | -0,03 | 0,98 | 0,9121 | G7c protein | CUST_26_PI209196805 | custom |
| 80 | G7c | -0,10 | 0,93 | 0,6167 | G7c protein | CUST_27_PI209196805 | custom |
| 80 | G7c | -0,05 | 0,97 | 0,8127 | G7c protein | CUST_28_PI209196805 | custom |
| 80 | G7c | -0,10 | 0,93 | 0,3349 | G7c protein | CUST_29_PI209196805 | custom |
| 80 | G7c | -0,07 | 0,95 | 0,6486 | G7c protein | CUST_30_PI209196805 | custom |
| 81 | Ng23 | 0,00 | 1,00 | 0,9979 | Ng23 protein | A_51_P233727 | Agilent |
| 82 | Msh5 | 0,01 | 1,01 | 0,9515 | mutS homolog 5 (E. coli) | A_43_P23342 | Agilent |
| 83 | Clic1 | 0,05 | 1,04 | 0,7886 | chloride intracellular channel 1 | A_44_P1028007 | Agilent |
| 84 | Ddah2 | 0,11 | 1,08 | 0,6564 | dimethylarginine dimethylaminohydrolase 2 | CUST_1_PI195698222 | custom |
| 84 | Ddah2 | 0,17 | 1,13 | 0,3684 | dimethylarginine dimethylaminohydrolase 2 | CUST_2_PI195698222 | custom |
| 84 | Ddah2 | 0,15 | 1,11 | 0,4977 | dimethylarginine dimethylaminohydrolase 2 | CUST_3_PI195698222 | custom |
| 84 | Ddah2 | 0,13 | 1,09 | 0,5078 | dimethylarginine dimethylaminohydrolase 2 | CUST_4_PI195698222 | custom |
| 84 | Ddah2 | 0,12 | 1,09 | 0,5019 | dimethylarginine dimethylaminohydrolase 2 | CUST_5_PI195698222 | custom |
| 85 | G6b | -0,01 | 0,99 | 0,8795 | G6b protein | A_44_P334847 | Agilent |
| 86 | Ly6g6c | 0,12 | 1,09 | 0,7567 | lymphocyte antigen 6 complex, locus G6C | CUST_1_PI195698232 | custom |
| 86 | Ly6g6c | 0,11 | 1,08 | 0,7656 | lymphocyte antigen 6 complex, locus G6C | CUST_2_PI195698232 | custom |
| 86 | Ly6g6c | 0,12 | 1,09 | 0,7537 | lymphocyte antigen 6 complex, locus G6C | CUST_3_PI195698232 | custom |
| 86 | Ly6g6c | 0,37 | 1,29 | 0,2006 | lymphocyte antigen 6 complex, locus G6C | CUST_4_PI195698232 | custom |
| 86 | Ly6g6c | 0,38 | 1,30 | 0,1845 | lymphocyte antigen 6 complex, locus G6C | CUST_5_PI195698232 | custom |
| 87 | Ly6g6d | 0,30 | 1,23 | 0,4948 | lymphocyte antigen 6 complex, locus G6D | CUST_1_PI195698244 | custom |
| 87 | Ly6g6d | 0,28 | 1,21 | 0,4856 | lymphocyte antigen 6 complex, locus G6D | CUST_2_PI195698244 | custom |
| 87 | Ly6g6d | 0,19 | 1,14 | 0,6310 | lymphocyte antigen 6 complex, locus G6D | CUST_3_PI195698244 | custom |
| 87 | Ly6g6d | 0,47 | 1,39 | 0,3675 | lymphocyte antigen 6 complex, locus G6D | CUST_4_PI195698244 | custom |
| 87 | Ly6g6d | 0,36 | 1,28 | 0,5300 | lymphocyte antigen 6 complex, locus G6D | CUST_5_PI195698244 | custom |
| 88 | Ly6g6e | -1,38 | 0,38 | 0,0416 | lymphocyte antigen 6 complex, locus G6E | CUST_1_PI195698246 | custom |
| 88 | Ly6g6e | -1,42 | 0,37 | 0,0523 | lymphocyte antigen 6 complex, locus G6E | CUST_2_PI_195698246 | custom |
| 88 | Ly6g6e | -1,39 | 0,38 | 0,0623 | lymphocyte antigen 6 complex, locus G6E | CUST_3_PI195698246 | custom |
| 88 | Ly6g6e | -1,44 | 0,37 | 0,0416 | lymphocyte antigen 6 complex, locus G6E | CUST_4_PI195698246 | custom |
| 88 | Ly6g6e | -1,46 | 0,36 | 0,0433 | lymphocyte antigen 6 complex, locus G6E | CUST_5_PI195698246 | custom |
| 89 | G6f (Ly6g6f) | -0,15 | 0,90 | 0,2839 | lymphocyte antigen 6 complex, locus G6F | CUST_1_PI195701417 | custom |
| 89 | G6f (Ly6g6f) | 0,22 | 1,16 | 0,0965 | lymphocyte antigen 6 complex, locus G6F | CUST_2_PI195701417 | custom |
| 89 | G6f (Ly6g6f) | -0,02 | 0,99 | 0,8965 | lymphocyte antigen 6 complex, locus G6F | CUST_3_PI195701417 | custom |
| 89 | G6f (Ly6g6f) | 0,05 | 1,04 | 0,7887 | lymphocyte antigen 6 complex, locus G6F | CUST_4_PI195701417 | custom |
| 89 | G6f (Ly6g6f) | 0,41 | 1,33 | 0,0716 | lymphocyte antigen 6 complex, locus G6F | CUST_5_PI195701417 | custom |
| 90 | Bat5 | -0,60 | 0,66 | 0,0100 | HLA-B associated transcript 5 | CUST_1_PI195830595 | custom |
| 90 | Bat5 | -0,48 | 0,72 | 0,0100 | HLA-B associated transcript 5 | CUST_2_PI195830595 | custom |
| 90 | Bat5 | -0,54 | 0,69 | 0,0180 | HLA-B associated transcript 5 | CUST_3_PI195830595 | custom |
| 90 | Bat5 | -0,53 | 0,69 | 0,0229 | HLA-B associated transcript 5 | CUST_4_PI195830595 | custom |
| 90 | Bat5 | -0,58 | 0,67 | 0,0100 | HLA-B associated transcript 5 | CUST_5_PI195830595 | custom |
| 91 | Ly6g5c | -0,18 | 0,88 | 0,4183 | lymphocyte antigen 6 complex, locus G5C | A_44_P355842 | Agilent |
| 92 | Ly6g5b | 0,01 | 1,01 | 0,9526 | lymphocyte antigen 6 complex, locus G5B | A_44_P111744 | Agilent |
| 93 | Csnk2b | -0,40 | 0,76 | 0,4907 | casein kinase 2, beta subunit | A_44_P453337 | Agilent |
| 94 | Bat4 | -0,06 | 0,96 | 0,7985 | Bat4 gene | CUST_1_PI195941286 | custom |
| 94 | Bat4 | 0,02 | 1,01 | 0,9500 | Bat4 gene | CUST_2_PI195941286 | custom |
| 94 | Bat4 | 0,00 | 1,00 | 0,9979 | Bat4 gene | CUST_3_PI195941286 | custom |
| 94 | Bat4 | 0,02 | 1,01 | 0,9284 | Bat4 gene | CUST_4_PI195941286 | custom |
| 94 | Bat4 | 0,04 | 1,03 | 0,8698 | Bat4 gene | CUST_5_PI195941286 | custom |
| 95 | G4 | -0,12 | 0,92 | 0,6277 | G4 protein | A_44_P327945 | Agilent |
| 96 | Apom | -0,31 | 0,81 | 0,1188 | apolipoprotein M | A_43_P15453 | Agilent |
| 97 | Bat3 | -0,04 | 0,97 | 0,8843 | HLA-B-associated transcript 3 | A_42_P506345 | Agilent |
| 98 | Bat2 | -0,08 | 0,95 | 0,6799 | HLA-B associated transcript 2 | CUST_1_PI195941289 | custom |
| 98 | Bat2 | -0,02 | 0,99 | 0,9413 | HLA-B associated transcript 2 | CUST_2_PI195941289 | custom |
| 98 | Bat2 | -0,07 | 0,95 | 0,7889 | HLA-B associated transcript 2 | CUST_3_PI195941289 | custom |
| 98 | Bat2 | -0,11 | 0,93 | 0,5007 | HLA-B associated transcript 2 | CUST_5_PI195941289 | custom |
| 98 | Bat2 | 0,05 | 1,04 | 0,63 | HLA-B associated transcript 2 | CUST_4_PI195941289 | custom |
| 99 | E230034005Rik | -0,06 | 0,96 | 0,4994 | E230034005Rik gene | A_44_P255078 | Agilent |
| 100 | Aif1 | 2,83 | 7,11 | 0,0100 | allograft inflammatory factor 1 | A_44_P421534 | Agilent |
| 101 | Ncr3 | -0,20 | 0,87 | 0,4300 | natural cytotoxicity triggering receptor 3 | A_43_P22986 | Agilent |
| 102 | Lst1 | 3,32 | 9,99 | 0,0100 | leucocyte specific transcript 1 | A_43_P12274 | Agilent |
| 103 | Ltb | 1,15 | 2,22 | 0,0693 | lymphotoxin B | A_42_P550914 | Agilent |
| 104 | Tnf | 0,32 | 1,25 | 0,0924 | tumor necrosis factor | A_43_P11513 | Agilent |
| 105 | Lta | 1,10 | 2,14 | 0,0523 | lymphotoxin A | A_43_P15592 | Agilent |
| 106 | Nfkbil1 | -0,01 | 0,99 | 0,9859 | nuclear factor of kappa light polypeptide gene enhancer in B-cells inhibitor-like 1 | CUST_1_PI195941300 | custom |
| 106 | Nfkbil1 | 0,10 | 1,07 | 0,8117 | nuclear factor of kappa light polypeptide gene enhancer in B-cells inhibitor-like 1 | CUST_2_PI195941300 | custom |
| 106 | Nfkbil1 | 0,10 | 1,07 | 0,8007 | nuclear factor of kappa light polypeptide gene enhancer in B-cells inhibitor-like 1 | CUST_3_PI195941300 | custom |
| 106 | Nfkbil1 | 0,03 | 1,02 | 0,9472 | nuclear factor of kappa light polypeptide gene enhancer in B-cells inhibitor-like 1 | CUST_4_PI195941300 | custom |
| 106 | Nfkbil1 | 0,17 | 1,13 | 0,6007 | nuclear factor of kappa light polypeptide gene enhancer in B-cells inhibitor-like 1 | CUST_5_PI195941300 | custom |
| 107 | Atp6v1g2 | -0,15 | 0,90 | 0,2622 | ATPase, H+ transporting, V1 subunit G isoform 2 | A_44_P484719 | Agilent |
| 108 | Bat1a | -0,56 | 0,68 | 0,0769 | HLA-B-associated transcript 1A | A_42_P784188 | Agilent |
| 109 | RT1-CE1 | 0,45 | 1,37 | 0,0668 | RT1 class I, CE1 | A_44_P513029 | Agilent |
| 110 | RT1-CE2 | 0,64 | 1,56 | 0,0278 | RT1 class I, CE2 | A_44_P107372 | Agilent |
| 111 | RT1-CE3 | 0,96 | 1,95 | 0,0100 | RT1 class I, CE3 | A_44_P274061 | Agilent |
| 112 | RT1-CE4 | 0,43 | 1,35 | 0,1222 | RT1 class I, CE4 | A_44_P440514 | Agilent |
| 113 | RT1-CE5 | 0,70 | 1,62 | 0,0395 | RT1 class I, CE5 | A_44_P172850 | Agilent |
| 114 | RT1-CE6 | 0,18 | 1,13 | 0,6413 | RT1-CE6 gene | A_44_P547954 | Agilent |
| 115 | RT1-CE7 | 0,45 | 1,37 | 0,1503 | RT1 class I, CE7 | A_42_P511265 | Agilent |
| 116 | RT1-CE8 | 0,90 | 1,87 | 0,0278 | RT1 class I, CE8 | CUST_1_PI201011245 | custom |
| 116 | RT1-CE8 | 0,91 | 1,88 | 0,0100 | RT1 class I, CE8 | CUST_2_PI201011245 | custom |
| 116 | RT1-CE8 | 0,78 | 1,72 | 0,0229 | RT1 class I, CE8 | CUST_3_PI201011245 | custom |
| 116 | RT1-CE8 | 0,84 | 1,79 | 0,0100 | RT1 class I, CE8 | CUST_4_PI201011245 | custom |
| 116 | RT1-CE8 | 0,79 | 1,73 | 0,0149 | RT1 class I, CE8 | CUST_5_PI201011245 | custom |
| 117 | RT1-CE9 | 0,80 | 1,74 | 0,0315 | RT1 class I, CE9 | CUST_1_P1201011241 | custom |
| 117 | RT1-CE9 | 0,35 | 1,27 | 0,1745 | RT1 class I, CE9 | CUST_2_PI201011241 | custom |
| 117 | RT1-CE9 | 0,74 | 1,67 | 0,0539 | RT1 class I, CE9 | CUST_3_PI201011241 | custom |
| 117 | RT1-CE9 | 0,24 | 1,18 | 0,3698 | RT1 class I, CE9 | CUST_4_PI201011241 | custom |
| 117 | RT1-CE9 | 0,81 | 1,75 | 0,0373 | RT1 class I, CE9 | CUST_5_PI201011241 | custom |
| 118 | RT1-CE10 | 4,09 | 17,03 | 0,0100 | RT1 class I, CE10 | A_44_P389019 | Agilent |
| 119 | RT1-CE11 | 0,28 | 1,21 | 0,2867 | RT1 class I, CE11 | CUST_1_PI195941302 | custom |
| 119 | RT1-CE11 | 0,65 | 1,57 | 0,0315 | RT1 class I, CE11 | CUST_2_PI195941302 | custom |
| 119 | RT1-CE11 | 0,22 | 1,16 | 0,2638 | RT1 class I, CE11 | CUST_3_PI195941302 | custom |
| 119 | RT1-CE11 | 0,16 | 1,12 | 0,3957 | RT1 class I, CE11 | CUST_4_PI195941302 | custom |
| 119 | RT1-CE11 | 0,38 | 1,30 | 0,0980 | RT1 class I, CE11 | CUST_5_PI195941302 | custom |
| 120 | RT1-CE12 | 0,43 | 1,35 | 0,1710 | RT1 class I, CE12 | CUST_1_PI195941305 | custom |
| 120 | RT1-CE12 | -0,10 | 0,93 | 0,4503 | RT1 class I, CE12 | CUST_2_PI195941305 | custom |
| 120 | RT1-CE12 | 0,34 | 1,27 | 0,1043 | RT1 class I, CE12 | CUST_3_PI195941305 | custom |
| 120 | RT1-CE12 | 0,04 | 1,03 | 0,8574 | RT1 class I, CE12 | CUST_4_PI195941305 | custom |
| 120 | RT1-CE12 | 0,56 | 1,47 | 0,0310 | RT1 class I, CE12 | CUST_5_PI195941305 | custom |
| 121 | RT1-CE13 | -0,42 | 0,75 | 0,1923 | RT1 class I, CE13 | CUST_1_PI197795816 | custom |
| 121 | RT1-CE13 | -0,46 | 0,73 | 0,2116 | RT1 class I, CE13 | CUST_2_PI197795816 | custom |
| 121 | RT1-CE13 | 0,37 | 1,29 | 0,1077 | RT1 class I, CE13 | CUST_3_PI197795816 | custom |
| 121 | RT1-CE13 | 0,38 | 1,30 | 0,1263 | RT1 class I, CE13 | CUST_4_PI197795816 | custom |
| 121 | RT1-CE13 | 0,40 | 1,32 | 0,0752 | RT1 class I, CE13 | CUST_5_PI197795816 | custom |
| 122 | RT1-CE14 | 0,39 | 1,31 | 0,1076 | RT1 class I, CE14 | CUST_1_PI195941310 | custom |
| 122 | RT1-CE14 | 0,35 | 1,27 | 0,1471 | RT1 class I, CE14 | CUST_2_PI195941310 | custom |
| 122 | RT1-CE14 | 0,30 | 1,23 | 0,1626 | RT1 class I, CE14 | CUST_3_PI195941310 | custom |
| 122 | RT1-CE14 | 0,25 | 1,19 | 0,2529 | RT1 class I, CE14 | CUST_4_PI195941310 | custom |
| 122 | RT1-CE14 | 0,25 | 1,19 | 0,2735 | RT1 class I, CE14 | CUST_5_PI195941310 | custom |
| 123 | RT1-CE15 | 0,28 | 1,21 | 0,2085 | RT1 class I, CE15 | CUST_1_PI195941312 | custom |
| 123 | RT1-CE15 | 0,26 | 1,20 | 0,2210 | RT1 class I, CE15 | CUST_2_PI195941312 | custom |
| 123 | RT1-CE15 | 0,30 | 1,23 | 0,1395 | RT1 class I, CE15 | CUST_3_PI195941312 | custom |
| 123 | RT1-CE15 | 0,29 | 1,22 | 0,1795 | RT1 class I, CE15 | CUST_4_PI195941312 | custom |
| 123 | RT1-CE15 | 0,35 | 1,27 | 0,1157 | RT1 class I, CE15 | CUST_5_PI195941312 | custom |
| 124 | RT1-CE16 | 0,54 | 1,45 | 0,0325 | RT1 class I, CE16 (RT1 class Ib, locus Cl) | A_44_P867246 | Agilent |
| 124 | RT1-CE16 | 0,78 | 1,72 | 0,0206 | RT1 class 1, CE16 (RT1 class Ib, locus Cl) | A_44_P554925 | Agilent |
| 125 | Pou5f1 | 0,02 | 1,01 | 0,8552 | POU domain, class 5, transcription factor 1 | CUST_1_PI195941317 | custom |
| 125 | Pou5f1 | -0,12 | 0,92 | 0,5977 | POU domain, class 5, transcription factor 1 | CUST_2_PI195941317 | custom |
| 125 | Pou5f1 | 0,07 | 1,05 | 0,7099 | POU domain, class 5, transcription factor 1 | CUST_3_PI195941317 | custom |
| 125 | Pou5f1 | 0,15 | 1,11 | 0,2432 | POU domain, class 5, transcription factor 1 | CUST_4_PI195941317 | custom |
| 125 | Pou5f1 | -0,07 | 0,95 | 0,5946 | POU domain, class 5, transcription factor 1 | CUST_5_PI195941317 | custom |
| 126 | Tcf19 | -0,19 | 0,88 | 0,5212 | transcription factor 19 | A_42_P591665 | Agilent |
| 127 | Hcr | -0,19 | 0,88 | 0,1202 | HCR (a-helix coiled-coil rod homolog) | A_52_P669964 | Agilent |
| 127 | Hcr | -0,26 | 0,84 | 0,2118 | HCR (a-helix coiled-coil rod homolog) | CUST_11_PI209196805 | custom |
| 127 | Hcr | -0,26 | 0,84 | 0,2030 | HCR (a-helix coiled-coil rod homolog) | CUST_12_PI209196805 | custom |
| 127 | Hcr | -0,25 | 0,84 | 0,2461 | HCR (a-helix coiled-coil rod homolog) | CUST_13_PI209196805 | custom |
| 127 | Hcr | -0,16 | 0,90 | 0,3650 | HCR (a-helix coiled-coil rod homolog) | CUST_14_PI209196805 | custom |
| 127 | Hcr | -0,15 | 0,90 | 0,5193 | HCR (a-helix coiled-coil rod homolog) | CUST_15_PI209196805 | custom |
| 128 | Spr1 | 1,26 | 2,39 | 0,0206 | psoriasis susceptibility 1 candidate 2 (human) | A_66_P100662 | Agilent |
| 128 | Spr1 | 1,39 | 2,62 | 0,0180 | psoriasis susceptibility 1 candidate 2 (human) | A_51_P212958 | Agilent |
| 128 | Spr1 | 1,36 | 2,57 | 0,0206 | psoriasis susceptibility 1 candidate 2 (human) | A_51_P212956 | Agilent |
| 128 | Spr1 | 1,50 | 2,83 | 0,0100 | psoriasis susceptibility 1 candidate 2 (human) | CUST_56_PI209196805 | custom |
| 128 | Spr1 | 1,52 | 2,87 | 0,0100 | psoriasis susceptibility 1 candidate 2 (human) | CUST_57_PI209196805 | custom |
| 128 | Spr1 | 1,51 | 2,85 | 0,0100 | psoriasis susceptibility 1 candidate 2 (human) | CUST_58_PI209196805 | custom |
| 128 | Spr1 | 1,50 | 2,83 | 0,0100 | psoriasis susceptibility 1 candidate 2 (human) | CUST_59_PI209196805 | custom |
| 128 | Spr1 | 1,58 | 2,99 | 0,0100 | psoriasis susceptibility 1 candidate 2 (human) | CUST_60_PI209196805 | custom |
| 129 | Cdsn | 0,37 | 1,29 | 0,2732 | corneodesmosin | CUST_1_PI201011238 | custom |
| 129 | Cdsn | 0,84 | 1,79 | 0,0100 | corneodesmosin | CUST_2_PI201011238 | custom |
| 129 | Cdsn | 0,38 | 1,30 | 0,2184 | corneodesmosin | CUST_3_PI201011238 | custom |
| 129 | Cdsn | 0,32 | 1,25 | 0,3754 | corneodesmosin | CUST_4_PI201011238 | custom |
| 129 | Cdsn | 0,40 | 1,32 | 0,1769 | corneodesmosin | CUST_5_PI201011238 | custom |
| 130 | Stg | 0,13 | 1,09 | 0,4327 | Stg protein | A_44_P161038 | Agilent |
| 130 | Stg | 0,06 | 1,04 | 0,8258 | Stg protein | A_43_P12304 | Agilent |
| 131 | CB741658 | -0,09 | 0,94 | 0,3912 | CB741658 gene | CUST_1_PI197795805 | custom |
| 131 | CB741658 | 0,05 | 1,04 | 0,5990 | CB741658 gene | CUST_2_PI197795805 | custom |
| 131 | CB741658 | -0,05 | 0,97 | 0,6299 | CB741658 gene | CUST_3_PI197795805 | custom |
| 131 | CB741658 | -0,03 | 0,98 | 0,7498 | CB741658 gene | CUST_4_PI197795805 | custom |
| 131 | CB741658 | -0,05 | 0,97 | 0,5704 | CB741658 gene | CUST_5_PI197795805 | custom |
| 132 | Dpcr1 | -0,11 | 0,93 | 0,3540 | diffuse panbronchiolitis critical region 1 (human) | A_66_PI12041 | Agilent |
| 132 | Dpcr1 | -0,17 | 0,89 | 0,0877 | diffuse panbronchiolitis critical region 1 (human) | CUST_36_PI209196805 | custom |
| 132 | Dpcr1 | -0,10 | 0,93 | 0,4426 | diffuse panbronchiolitis critical region 1 (human) | CUST_37_PI209196805 | custom |
| 132 | Dpcr1 | -0,11 | 0,93 | 0,2585 | diffuse panbronchiolitis critical region 1 (human) | CUST_38_PI209196805 | custom |
| 132 | Dpcr1 | -0,14 | 0,91 | 0,2435 | diffuse panbronchiolitis critical region 1 (human) | CUST_39_PI209196805 | custom |
| 132 | Dpcr1 | -0,04 | 0,97 | 0,7474 | diffuse panbronchiolitis critical region 1 (human) | CUST_40_PI209196805 | custom |
| 133 | E030032D13Rik | -0,24 | 0,85 | 0,0701 | E030032D13Rik gene | A_44_P341977 | Agilent |
| 134 | Kiaa1885 | -0,08 | 0,95 | 0,5867 | KIAA1885 protein | A_44_P1007561 | Agilent |
| 135 | Gtf2h4 | -0,09 | 0,94 | 0,7537 | general transcription factor II H, polypeptide 4 | CUST_1_PI197795807 | custom |
| 135 | Gtf2h4 | -0,03 | 0,98 | 0,9410 | general transcription factor II H, polypeptide 4 | CUST_2_PI197795807 | custom |
| 135 | Gtf2h4 | -0,12 | 0,92 | 0,7090 | general transcription factor II H, polypeptide 4 | CUST_3_PI197795807 | custom |
| 135 | Gtf2h4 | -0,02 | 0,99 | 0,9552 | general transcription factor II H, polypeptide 4 | CUST_4_PI197795807 | custom |
| 135 | Gtf2h4 | -0,06 | 0,96 | 0,8501 | general transcription factor II H, polypeptide 4 | CUST_5_PI197795807 | custom |
| 136 | Ddr1 | -0,18 | 0,88 | 0,1499 | discoidin domain receptor family, member 1 | A_44_P515494 | Agilent |
| 137 | CB707485l | -0,01 | 0,99 | 0,9561 | gene corresponding to rat EST CB707485 | CUST_1_PI201011227 | custom |
| 137 | CB707485l | 0,08 | 1,06 | 0,5379 | gene corresponding to rat EST CB707485 | CUST_2_PI201011227 | custom |
| 137 | CB707485l | 0,04 | 1,03 | 0,7953 | gene corresponding to rat EST CB707485 | CUST_3_PI201011227 | custom |
| 137 | CB707485l | 0,07 | 1,05 | 0,5259 | gene corresponding to rat EST CB707485 | CUST_4_PI201011227 | custom |
| 137 | CB707485l | -0,08 | 0,95 | 0,7190 | gene corresponding to rat EST CB707485 | CUST_5_PI201011227 | custom |
| 138 | Ier3 | 0,87 | 1,83 | 0,0229 | immediate early response 3 | A_42_P515405 | Agilent |
| 139 | Flot1 | 0,04 | 1,03 | 0,8901 | flotillin 1 | A_44_PI023498 | Agilent |
| 140 | Tubb5 | 0,16 | 1,12 | 0,2875 | tubulin, beta 5 | A_44_P825566 | Agilent |
| 141 | Kiaa0170 (Mdc1) | 0,02 | 1,01 | 0,9108 | mediator of DNA damage checkpoint 1 | A_42_P627572 | Agilent |
| 142 | Nrm | -0,06 | 0,96 | 0,8031 | nurim (nuclear envelope membrane protein) | CUST_1_PI197795809 | custom |
| 142 | Nrm | -0,11 | 0,93 | 0,6622 | nurim (nuclear envelope membrane protein) | CUST_2_PI197795809 | custom |
| 142 | Nrm | -0,20 | 0,87 | 0,3384 | nurim (nuclear envelope membrane protein) | CUST_3_PI197795809 | custom |
| 142 | Nrm | -0,05 | 0,97 | 0,8551 | nurim (nuclear envelope membrane protein) | CUST_4_PI197795809 | custom |
| 142 | Nrm | 0,03 | 1,02 | 0,8504 | nurim (nuclear envelope membrane protein) | CUST_5_PI197795809 | custom |
| 143 | Kiaa1949 | 0,42 | 1,34 | 0,0481 | KIAA1949 protein | CUST_1_PI201011218 | custom |
| 143 | Kiaa1949 | 0,49 | 1,40 | 0,0457 | KIAA1949 protein | CUST_2_PI201011218 | custom |
| 143 | Kiaa1949 | 0,33 | 1,26 | 0,1378 | KIAA1949 protein | CUST_3_PI201011218 | custom |
| 143 | Kiaa1949 | 0,39 | 1,31 | 0,0993 | KIAA1949 protein | CUST_4_PI201011218 | custom |
| 143 | Kiaa1949 | 0,34 | 1,27 | 0,1184 | KIAA1949 protein | CUST_5_PI201011218 | custom |
| 144 | Ddx16 | 0,04 | 1,03 | 0,8954 | DEAH (Asp-Glu-Ala-His) box polypeptide 16 | A_44_P379461 | Agilent |
| 144 | Ddx16 | -0,27 | 0,83 | 0,0797 | DEAH (Asp-Glu-Ala-His) box polypeptide 16 | A_43_P20689 | Agilent |
| 145 | Mgc15854 (RGD1302996) | 0,12 | 1,09 | 0,5094 | hypothetical protein MGC15854 | A_42_P508754 | Agilent |
| 145 | Mgc15854 (RGD1302996) | 0,05 | 1,04 | 0,8290 | hypothetical protein MGC15854 | A_44_P1002280 | Agilent |
| 146 | Flj13158 (RGD1303066) | -0,25 | 0,84 | 0,0832 | hypothetical protein FU13158 | A_44_P278509 | Agilent |
| 146 | Flj13158 (RGD1303066) | -0,57 | 0,67 | 0,0378 | hypothetical protein FU13158 | A_44_P654250 | Agilent |
| 147 | Mrps18b | 0,52 | 1,43 | 0,0474 | mitochondrial ribosomal protein S18B | CUST_1_PI197795811 | custom |
| 147 | Mrps18b | 0,49 | 1,40 | 0,0378 | mitochondrial ribosomal protein S18B | CUST_2_PI197795811 | custom |
| 147 | Mrps18b | 0,57 | 1,48 | 0,0267 | mitochondrial ribosomal protein S18B | CUST_3_PI197795811 | custom |
| 147 | Mrps18b | 0,59 | 1,51 | 0,0365 | mitochondrial ribosomal protein S18B | CUST_4_PI197795811 | custom |
| 147 | Mrps18b | 0,62 | 1,54 | 0,0254 | mitochondrial ribosomal protein S18B | CUST_5_PI197795811 | custom |
| 148 | Ppp1r10 | 0,49 | 1,40 | 0,1582 | protein phosphatase 1, regulatory subunit 10 | A_42_P497323 | Agilent |
| 149 | Abcf1 | 0,46 | 1,38 | 0,0832 | ATP-binding cassette, sub-family F (GCN20), member 1 | CUST_46_PI209196805 | custom |
| 149 | Abcf1 | 0,44 | 1,36 | 0,1863 | ATP-binding cassette, sub-family F (GCN20), member 1 | CUST_47_PI209196805 | custom |
| 149 | Abcf1 | 0,34 | 1,27 | 0,2797 | ATP-binding cassette, sub-family F (GCN20), member 1 | CUST_48_PI209196805 | custom |
| 149 | Abcf1 | 0,30 | 1,23 | 0,3188 | ATP-binding cassette, sub-family F (GCN20), member 1 | CUST_49_PI209196805 | custom |
| 149 | Abcf1 | 0,34 | 1,27 | 0,2180 | ATP-binding cassette, sub-family F (GCN20), member 1 | CUST_50_PI209196805 | custom |
| 150 | Cat56 (Prr3) | -0,01 | 0,99 | 0,9791 | proline-rich polypeptide 3 | A_44_P299349 | Agilent |
| 151 | Gnl1 | 0,05 | 1,04 | 0,8944 | guanine nucleotide binding protein, related sequence 1 | A_65_P05751 | Agilent |
| 151 | Gnl1 | -0,04 | 0,97 | 0,6698 | guanine nucleotide binding protein, related sequence 1 | A_66_P118660 | Agilent |
| 151 | Gnl1 | 0,02 | 1,01 | 0,9496 | guanine nucleotide binding protein, related sequence 1 | A_51_P102809 | Agilent |
| 151 | Gnl1 | -0,15 | 0,90 | 0,5093 | guanine nucleotide binding protein, related sequence 1 | A_51_P102814 | Agilent |
| 151 | Gnl1 | 0,07 | 1,05 | 0,8093 | guanine nucleotide binding protein, related sequence 1 | A_52_P491766 | Agilent |
| 151 | Gnl1 | -0,24 | 0,85 | 0,2708 | guanine nucleotide binding protein, related sequence 1 | CUST_41_PI209196805 | custom |
| 151 | Gnl1 | -0,17 | 0,89 | 0,4205 | guanine nucleotide binding protein, related sequence 1 | CUST_42_PI209196805 | custom |
| 151 | Gnl1 | 0,03 | 1,02 | 0,9311 | guanine nucleotide binding protein, related sequence 1 | CUST_43_PI209196805 | custom |
| 151 | Gnl1 | 0,02 | 1,01 | 0,9448 | guanine nucleotide binding protein, related sequence 1 | CUST_44_PI209196805 | custom |
| 151 | Gnl1 | 0,03 | 1,02 | 0,8853 | guanine nucleotide binding protein, related sequence 1 | CUST_45_PI209196805 | custom |
| 152 | RT1-T24-1 | 0,25 | 1,19 | 0,2040 | RT1 class I, T24, gene 1 | A_44_P187530 | Agilent |
| 153 | RT1-T24-2 | -0,01 | 0,99 | 0,9531 | RT1 class I, T24, gene 2 | A_44_P215023 | Agilent |
| 154 | RT1-T24-3 | 0,31 | 1,24 | 0,1540 | RT1 class I, T24, gene 3 | CUST_1_PI201011214 | custom |
| 154 | RT1-T24-3 | 0,42 | 1,34 | 0,0336 | RT1 class I, T24, gene 3 | CUST_2_PI201011214 | custom |
| 154 | RT1-T24-3 | 0,27 | 1,21 | 0,1454 | RT1 class I, T24, gene 3 | CUST_3_PI201011214 | custom |
| 154 | RT1-T24-3 | 0,31 | 1,24 | 0,0847 | RT1 class I, T24, gene 3 | CUST_4_PI201011214 | custom |
| 154 | RT1-T24-3 | 0,08 | 1,06 | 0,6030 | RT1 class I, T24, gene 3 | CUST_5_PI201011214 | custom |
| 155 | RT1-T24-4 | 0,57 | 1,48 | 0,0345 | RT1 class I, T24, gene 4 | CUST_1_PI197795813 | custom |
| 155 | RT1-T24-4 | 0,76 | 1,69 | 0,0206 | RT1 class I, T24, gene 4 | CUST_2_PI197795813 | custom |
| 155 | RT1-T24-4 | 0,72 | 1,65 | 0,0206 | RT1 class I, T24, gene 4 | CUST_3_PI197795813 | custom |
| 155 | RT1-T24-4 | 0,39 | 1,31 | 0,0611 | RT1 class I, T24, gene 4 | CUST_4_PI197795813 | custom |
| 155 | RT1-T24-4 | 0,51 | 1,42 | 0,0939 | RT1 class I, T24, gene 4 | CUST_5_PI197795813 | custom |
| 156 | RT-BM1 (RT1-S3) | 1,06 | 2,08 | 0,0416 | RT1 class I, RT-BM1 | A_44_P454420 | Agilent |
| 157 | RT1-N3 | 0,20 | 1,15 | 0,3890 | RT1 class I, N3 | A_42_P521707 | Agilent |
| 158 | RT1-O1 | -0,03 | 0,98 | 0,8512 | RT1 class I, O1 | CUST_1_PI197795863 | custom |
| 158 | RT1-O1 | -0,05 | 0,97 | 0,6261 | RT1 class I, O1 | CUST_2_PI197795863 | custom |
| 158 | RT1-O1 | 0,08 | 1,06 | 0,3128 | RT1 class I, O1 | CUST_3_PI197795863 | custom |
| 158 | RT1-O1 | 0,01 | 1,01 | 0,8904 | RT1 class I, O1 | CUST_4_PI197795863 | custom |
| 158 | RT1-O1 | -0,15 | 0,90 | 0,3468 | RT1 class I, O1 | CUST_5_PI197795863 | custom |
| 159 | RT1-S2 | -0,31 | 0,81 | 0,2437 | RT1 class I, S2 | CUST_1_PI2010111700 | custom |
| 159 | RT1-S2 | -0,25 | 0,84 | 0,2765 | RT1 class I, S2 | CUST_5_PI2010111700 | custom |
| 160 | RT1-N2 | 0,06 | 1,04 | 0,7225 | RT1 class I, N2 | CUST_1_PI197795818 | custom |
| 160 | RT1-N2 | -0,02 | 0,99 | 0,9142 | RT1 class I, N2 | CUST_2_PI197795818 | custom |
| 160 | RT1-N2 | 0,09 | 1,06 | 0,6061 | RT1 class I, N2 | CUST_3_PI197795818 | custom |
| 160 | RT1-N2 | 0,01 | 1,01 | 0,9575 | RT1 class I, N2 | CUST_4_PI197795818 | custom |
| 160 | RT1-N2 | 0,01 | 1,01 | 0,9481 | RT1 class I, N2 | CUST_5_PI197795818 | custom |
| 160 | RT1-N2 | 0,02 | 1,01 | 0,9122 | RT1 class I, N2 | A_44_P379367 | Agilent |
| 161 | RT1-O2 | -0,38 | 0,77 | 0,2262 | RT1 class I, 02 | CUST_1_PI201011211 | custom |
| 161 | RT1-O2 | 0,57 | 1,48 | 0,0345 | RT1 class I, 02 | CUST_2_PI201011211 | custom |
| 161 | RT1-O2 | -0,09 | 0,94 | 0,6330 | RT1 class I, O2 | CUST_3_PI201011211 | custom |
| 161 | RT1-O2 | 0,55 | 1,46 | 0,0424 | RT1 class I, O2 | CUST_4_PI201011211 | custom |
| 161 | RT1-O2 | 0,22 | 1,16 | 0,3389 | RT1 class I, 02 | CUST_5_PI201011211 | custom |
| 162 | RT1-O3 | -0,30 | 0,81 | 0,2438 | RT1 class I, O3 | CUST_1_PI201011202 | custom |
| 162 | RT1-O3 | -0,13 | 0,91 | 0,5468 | RT1 class I, O3 | CUST_2_PI201011202 | custom |
| 162 | RT1-O3 | 0,50 | 1,41 | 0,0546 | RT1 class I, 03 | CUST_3_PI201011202 | custom |
| 162 | RT1-O3 | 0,50 | 1,41 | 0,0457 | RT1 class I, 03 | CUST_4_PI201011202 | custom |
| 162 | RT1-O3 | 0,23 | 1,17 | 0,2975 | RT1 class I, O3 | CUST_5_PI201011202 | custom |
| 163 | RT1-V1 | 0,10 | 1,07 | 0,5153 | RT1 class I, V1 | CUST_1_PI201011196 | custom |
| 163 | RT1-V1 | 0,05 | 1,04 | 0,6614 | RT1 class I, V1 | CUST_2_PI201011196 | custom |
| 163 | RT1-V1 | 0,03 | 1,02 | 0,8018 | RT1 class I, V1 | CUST_3_PI201011196 | custom |
| 163 | RT1-V1 | 0,03 | 1,02 | 0,7265 | RT1 class I, V1 | CUST_4_PI201011196 | custom |
| 163 | RT1-V1 | 0,11 | 1,08 | 0,3219 | RT1 class I, V1 | CUST_5_PI201011196 | custom |
| 164 | RT1-T18 | 0,12 | 1,09 | 0,5019 | histocompatibility 2, T region locus 18 | A_44_P358361 | Agilent |
| 164 | RT1-T18 | 0,67 | 1,59 | 0,0828 | histocompatibility 2, T region locus 18 | A_44_P358358 | Agilent |
| 165 | RT1-P1 | 0,42 | 1,34 | 0,1795 | RT1 class I, P1 | CUST_1_PI201011193 | custom |
| 165 | RT1-P1 | 0,43 | 1,35 | 0,1897 | RT1 class I, P1 | CUST_2_PI201011193 | custom |
| 165 | RT1-P1 | 0,33 | 1,26 | 0,3012 | RT1 class I, P1 | CUST_3_PI201011193 | custom |
| 165 | RT1-P1 | 0,38 | 1,30 | 0,2049 | RT1 class I, P1 | CUST_4_PI201011193 | custom |
| 165 | RT1-P1 | 0,31 | 1,24 | 0,2951 | RT1 class I, P1 | CUST_5_PI201011193 | custom |
| 166 | RT1-V2 | 0,02 | 1,01 | 0,8517 | RT1 class I, V2 | CUST_1_PI201011189 | custom |
| 166 | RT1-V2 | 0,07 | 1,05 | 0,3934 | RT1 class I, V2 | CUST_2_PI201011189 | custom |
| 166 | RT1-V2 | 0,01 | 1,01 | 0,9606 | RT1 class I, V2 | CUST_3_PI201011189 | custom |
| 166 | RT1-V2 | -0,01 | 0,99 | 0,9455 | RT1 class I, V2 | CUST_4_PI201011189 | custom |
| 166 | RT1-V2 | 0,06 | 1,04 | 0,6161 | RT1 class I, V2 | CUST_5_PI201011189 | custom |
| 167 | RT1-P2 | 0,14 | 1,10 | 0,2561 | RT1 class I, P2 | CUST_1_PI201011184 | custom |
| 167 | RT1-P2 | -0,01 | 0,99 | 0,9599 | RT1 class I, P2 | CUST_2_PI201011184 | custom |
| 167 | RT1-P2 | -0,03 | 0,98 | 0,7705 | RT1 class I, P2 | CUST_3_PI201011184 | custom |
| 167 | RT1-P2 | 0,01 | 1,01 | 0,9284 | RT1 class I, P2 | CUST_4_PI201011184 | custom |
| 167 | RT1-P2 | -0,03 | 0,98 | 0,8477 | RT1 class I, P2 | CUST_5_PI201011184 | custom |
| 168 | Flj22638 (Rpp21) | 0,10 | 1,07 | 0,6826 | ribonuclease P 21 subunit | A_44_P1017763 | Agilent |
| 168 | Flj22638 (Rpp21) | -0,02 | 0,99 | 0,8997 | ribonuclease P 21 subunit | A_44_P1017757 | Agilent |
| 169 | Trim39 | -0,32 | 0,80 | 0,1210 | tripartite motif-containing 39 | A_44_P245427 | Agilent |
| 170 | RT1-M10-1 | 0,02 | 1,01 | 0,8675 | RT1 class I, M10, gene 1 | CUST_1_PI201011161 | custom |
| 170 | RT1-M10-1 | -0,19 | 0,88 | 0,1707 | RT1 class I, M10, gene 1 | CUST_2_PI201011161 | custom |
| 170 | RT1-M10-1 | 0,03 | 1,02 | 0,7954 | RT1 class I, M10, gene 1 | CUST_3_PI201011161 | custom |
| 170 | RT1-M10-1 | -0,01 | 0,99 | 0,9161 | RT1 class I, M10, gene 1 | CUST_4_PI201011161 | custom |
| 170 | RT1-M10-1 | -0,04 | 0,97 | 0,6779 | RT1 class I, M10, gene 1 | CUST_5_PI201011161 | custom |
| 171 | RT1-M10-2 | -0,09 | 0,94 | 0,2987 | RT1 class I, M10, gene 2 | CUST_1_PI201011180 | custom |
| 171 | RT1-M10-2 | -0,06 | 0,96 | 0,6569 | RT1 class I, M10, gene 2 | CUST_2_PI201011180 | custom |
| 171 | RT1-M10-2 | -0,01 | 0,99 | 0,9375 | RT1 class I, M10, gene 2 | CUST_3_PI201011180 | custom |
| 171 | RT1-M10-2 | 0,03 | 1,02 | 0,7545 | RT1 class I, M10, gene 2 | CUST_4_PI201011180 | custom |
| 171 | RT1-M10-2 | -0,02 | 0,99 | 0,8053 | RT1 class I, M10, gene 2 | CUST_5_PI201011180 | custom |
| 172 | RT1-M1-1 | -0,01 | 0,99 | 0,9358 | RT1 class I, M1, gene 1 | CUST_1_PI201011178 | custom |
| 172 | RT1-M1-1 | -0,11 | 0,93 | 0,4445 | RT1 class I, M1, gene 1 | CUST_2_PI201011178 | custom |
| 172 | RT1-M1-1 | 0,54 | 1,45 | 0,0278 | RT1 class I, M1, gene 1 | CUST_3_PI201011178 | custom |
| 172 | RT1-M1-1 | -0,17 | 0,89 | 0,1632 | RT1 class I, M1, gene 1 | CUST_4_PI201011178 | custom |
| 172 | RT1-M1-1 | -0,05 | 0,97 | 0,7839 | RT1 class I, M1, gene 1 | CUST_5_PI201011178 | custom |
| 173 | RT1-M1-2 | -0,11 | 0,93 | 0,3479 | RT1 class I, M1, gene 2 | CUST_1_PI197795822 | custom |
| 173 | RT1-M1-2 | -0,22 | 0,86 | 0,1078 | RT1 class I, M1, gene 2 | CUST_2_PI197795822 | custom |
| 173 | RT1-M1-2 | -0,03 | 0,98 | 0,7000 | RT1 class I, M1, gene 2 | CUST_3_PI197795822 | custom |
| 173 | RT1-M1-2 | -0,02 | 0,99 | 0,8325 | RT1 class I, M1, gene 2 | CUST_4_PI197795822 | custom |
| 173 | RT1-M1-2 | 0,00 | 1,00 | 0,9910 | RT1 class I, M1, gene 2 | CUST_5_PI197795822 | custom |
| 174 | RT1-M1-3 | -0,10 | 0,93 | 0,2338 | RT1 class I, M1, gene 3 | CUST_1_PI201011175 | custom |
| 174 | RT1-M1-3 | -0,02 | 0,99 | 0,9164 | RT1 class I, M1, gene 3 | CUST_2_PI201011175 | custom |
| 174 | RT1-M1-3 | -0,01 | 0,99 | 0,9246 | RT1 class I, M1, gene 3 | CUST_3_PI201011175 | custom |
| 174 | RT1-M1-3 | 0,03 | 1,02 | 0,7901 | RT1 class I, M1, gene 3 | CUST_4_PI201011175 | custom |
| 174 | RT1-M1-3 | -0,09 | 0,94 | 0,2805 | RT1 class I, M1, gene 3 | CUST_5_PI201011175 | custom |
| 175 | RT1-M1-4 | -0,23 | 0,85 | 0,1261 | RT1 class I, M1, gene 4 | A_44_P213221 | Agilent |
| 176 | RT1-M1-5 | 0,04 | 1,03 | 0,7001 | RT1 class I, M1, gene 5 | A_44_P506413 | Agilent |
| 177 | RT1-M7 | -0,08 | 0,95 | 0,3109 | RT1 class I, M7 | CUST_1_PI201011173 | custom |
| 177 | RT1-M7 | -0,30 | 0,81 | 0,0433 | RT1 class I, M7 | CUST_2_PI201011173 | custom |
| 177 | RT1-M7 | 0,04 | 1,03 | 0,5727 | RT1 class I, M7 | CUST_3_PI201011173 | custom |
| 177 | RT1-M7 | -0,05 | 0,97 | 0,7154 | RT1 class I, M7 | CUST_4_PI201011173 | custom |
| 177 | RT1-M7 | -0,32 | 0,80 | 0,1162 | RT1 class I, M7 | CUST_5_PI201011173 | custom |
| 178 | RT1-M8 | -0,23 | 0,85 | 0,0654 | RT1 class I, M8 | CUST_1_PI201011170 | custom |
| 178 | RT1-M8 | -0,04 | 0,97 | 0,6168 | RT1 class I, M8 | CUST_2_PI201011170 | custom |
| 178 | RT1-M8 | -0,31 | 0,81 | 0,1655 | RT1 class I, M8 | CUST_3_PI201011170 | custom |
| 178 | RT1-M8 | -0,22 | 0,86 | 0,2766 | RT1 class I, M8 | CUST_5_PI201011170 | custom |
| 178 | RT1-M8 | 0,01 | 1,01 | 0,9933 | RT1 class I, M8 | CUST_4_PI201011170 | custom |
| 179 | RT1-M10-3 | -0,02 | 0,99 | 0,9071 | RT1 class I, M10, gene 3 | CUST_1_PI201011167 | custom |
| 179 | RT1-M10-3 | -0,27 | 0,83 | 0,0424 | RT1 class I, M10, gene 3 | CUST_2_PI201011167 | custom |
| 179 | RT1-M10-3 | -0,06 | 0,96 | 0,6730 | RT1 class I, M10, gene 3 | CUST_3_PI201011167 | custom |
| 179 | RT1-M10-3 | -0,04 | 0,97 | 0,6161 | RT1 class I, M10, gene 3 | CUST_4_PI201011167 | custom |
| 179 | RT1-M10-3 | -0,06 | 0,96 | 0,5878 | RT1 class I, M10, gene 3 | CUST_5_PI201011167 | custom |
| 180 | RT1-M10-4 | 0,08 | 1,06 | 0,4351 | RT1 class I, M10, gene 4 | CUST_1_PI197795820 | custom |
| 180 | RT1-M10-4 | 0,09 | 1,06 | 0,4057 | RT1 class I, M10, gene 4 | CUST_2_PI197795820 | custom |
| 180 | RT1-M10-4 | 0,26 | 1,20 | 0,3213 | RT1 class I, M10, gene 4 | CUST_3_PI197795820 | custom |
| 180 | RT1-M10-4 | -0,62 | 0,65 | 0,0539 | RT1 class I, M10, gene 4 | CUST_4_PI197795820 | custom |
| 180 | RT1-M10-4 | 0,07 | 1,05 | 0,6195 | RT1 class I, M10, gene 4 | CUST_5_PI197795820 | custom |
| 181 | Trim26 | -0,04 | 0,97 | 0,8676 | tripartite motif-containing 26 | CUST_1_PI197795824 | custom |
| 181 | Trim26 | -0,04 | 0,97 | 0,8113 | tripartite motif-containing 26 | CUST_2_PI197795824 | custom |
| 181 | Trim26 | -0,20 | 0,87 | 0,1379 | tripartite motif-containing 26 | CUST_3_PI197795824 | custom |
| 181 | Trim26 | 0,07 | 1,05 | 0,6779 | tripartite motif-containing 26 | CUST_4_PI197795824 | custom |
| 181 | Trim26 | -0,01 | 0,99 | 0,9756 | tripartite motif-containing 26 | CUST_5_PI197795824 | custom |
| 182 | Trim15 | -0,99 | 0,50 | 0,0539 | tripartite motif-containing 15 | CUST_1_PI201011159 | custom |
| 182 | Trim15 | -0,96 | 0,51 | 0,0722 | tripartite motif-containing 15 | CUST_2_PI201011159 | custom |
| 182 | Trim15 | -0,90 | 0,54 | 0,0858 | tripartite motif-containing 15 | CUST_3_PI201011159 | custom |
| 182 | Trim15 | -0,83 | 0,56 | 0,0654 | tripartite motif-containing 15 | CUST_4_PI201011159 | custom |
| 182 | Trim15 | -1,02 | 0,49 | 0,0603 | tripartite motif-containing 15 | CUST_5_PI201011159 | custom |
| 183 | Trim10 | -0,26 | 0,84 | 0,2418 | tripartite motif protein 10 | CUST_1_PI197795826 | custom |
| 183 | Trim10 | -0,18 | 0,88 | 0,5016 | tripartite motif protein 10 | CUST_2_PI197795826 | custom |
| 183 | Trim10 | -0,15 | 0,90 | 0,5471 | tripartite motif protein 10 | CUST_3_PI197795826 | custom |
| 183 | Trim10 | -0,26 | 0,84 | 0,2463 | tripartite motif protein 10 | CUST_4_PI197795826 | custom |
| 183 | Trim10 | -0,20 | 0,87 | 0,2290 | tripartite motif protein 10 | CUST_5_PI197795826 | custom |
| 184 | Trim40 | -0,22 | 0,86 | 0,0923 | tripartite motif-containing 40 | CUST_1_PI209196805 | custom |
| 184 | Trim40 | 0,00 | 1,00 | 0,9664 | tripartite motif-containing 40 | CUST_2_PI209196805 | custom |
| 184 | Trim40 | 0,08 | 1,06 | 0,5878 | tripartite motif-containing 40 | CUST_3_PI209196805 | custom |
| 184 | Trim40 | -0,06 | 0,96 | 0,6191 | tripartite motif-containing 40 | CUST_4_PI209196805 | custom |
| 184 | Trim40 | -0,08 | 0,95 | 0,4748 | tripartite motif-containing 40 | CUST_5_PI209196805 | custom |
| 185 | Trim31 | -0,04 | 0,97 | 0,8529 | tripartite motif-containing 31 | A_51_P490840 | Agilent |
| 185 | Trim31 | -0,04 | 0,97 | 0,7567 | tripartite motif-containing 31 | CUST_21_PI209196805 | custom |
| 185 | Trim31 | 0,00 | 1,00 | 0,9980 | tripartite motif-containing 31 | CUST_22_PI209196805 | custom |
| 185 | Trim31 | -0,06 | 0,96 | 0,5133 | tripartite motif-containing 31 | CUST_23_PI209196805 | custom |
| 185 | Trim31 | -0,16 | 0,90 | 0,1958 | tripartite motif-containing 31 | CUST_24_PI209196805 | custom |
| 185 | Trim31 | -0,04 | 0,97 | 0,7351 | tripartite motif-containing 31 | CUST_25_PI209196805 | custom |
| 186 | 1700031A10Rik | -0,14 | 0,91 | 0,4950 | gene corresponding to Riken clone 1700031A10 | A_52_P515192 | Agilent |
| 186 | 1700031A10Rik | -0,10 | 0,93 | 0,2703 | gene corresponding to Riken clone 1700031A10 | CUST_31_PI209196805 | custom |
| 186 | 1700031A10Rik | -0,13 | 0,91 | 0,3373 | gene corresponding to Riken clone 1700031A10 | CUST_32_PI209196805 | custom |
| 186 | 1700031A10Rik | -0,08 | 0,95 | 0,5598 | gene corresponding to Riken clone 1700031A10 | CUST_33_PI209196805 | custom |
| 186 | 1700031A10Rik | -0,02 | 0,99 | 0,9034 | gene corresponding to Riken clone 1700031A10 | CUST_34_PI209196805 | custom |
| 186 | 1700031A10Rik | -0,05 | 0,97 | 0,7178 | gene corresponding to Riken clone 1700031A10 | CUST_35_PI209196805 | custom |
| 187 | Rnf39 | 0,05 | 1,04 | 0,6605 | Ring finger protein Lirf | CUST_1_PI195698208 | custom |
| 187 | Rnf39 | 0,06 | 1,04 | 0,5793 | Ring finger protein Lirf | CUST_2_PI195698208 | custom |
| 187 | Rnf39 | -0,02 | 0,99 | 0,8552 | Ring finger protein Lirf | CUST_3_PI195698208 | custom |
| 187 | Rnf39 | -0,28 | 0,82 | 0,0579 | Ring finger protein Lirf | CUST_4_PI195698208 | custom |
| 187 | Rnf39 | -0,06 | 0,96 | 0,6264 | Ring finger protein Lirf | CUST_5_PI195698208 | custom |
| 188 | Ppp1r11 | 0,14 | 1,10 | 0,5417 | protein phosphatase 1, regulatory (inhibitor) subunit 11 | CUST_1_PI197795829 | custom |
| 188 | Ppp1r11 | 0,14 | 1,10 | 0,4917 | protein phosphatase 1, regulatory (inhibitor) subunit 11 | CUST_2_PI197795829 | custom |
| 188 | Ppp1r11 | 0,10 | 1,07 | 0,6213 | protein phosphatase 1, regulatory (inhibitor) subunit 11 | CUST_3_PI197795829 | custom |
| 188 | Ppp1r11 | 0,13 | 1,09 | 0,4615 | protein phosphatase 1, regulatory (inhibitor) subunit 11 | CUST_4_PI197795829 | custom |
| 188 | Ppp1r11 | 0,09 | 1,06 | 0,6711 | protein phosphatase 1, regulatory (inhibitor) subunit 11 | CUST_5_PI197795829 | custom |
| 189 | Znrd1 | 0,22 | 1,16 | 0,3879 | zinc ribbon domain containing, 1 | A_44_P404931 | Agilent |
| 190 | Tctex4 | -0,20 | 0,87 | 0,2497 | t-complex testis-expressed 4, rat homologue | CUST_1_PI201011154 | custom |
| 190 | Tctex4 | -0,10 | 0,93 | 0,6520 | t-complex testis-expressed 4, rat homologue | CUST_2_PI201011154 | custom |
| 190 | Tctex4 | -0,02 | 0,99 | 0,9728 | t-complex testis-expressed 4, rat homologue | CUST_3_PI201011154 | custom |
| 190 | Tctex4 | -0,14 | 0,91 | 0,7705 | t-complex testis-expressed 4, rat homologue | CUST_4_PI201011154 | custom |
| 190 | Tctex4 | -0,18 | 0,88 | 0,6959 | t-complex testis-expressed 4, rat homologue | CUST_5_PI201011154 | custom |
| 191 | RT1-M6-2 | 0,29 | 1,22 | 0,2232 | RT1 class I, M6, gene 2 | A_44_P309052 | Agilent |
| 192 | RT1-M6-1 | 0,25 | 1,19 | 0,1939 | RT1 class I, M6, gene 1 | CUST_1_PI197795831 | custom |
| 192 | RT1-M6-1 | 0,14 | 1,10 | 0,2419 | RT1 class I, M6, gene 1 | CUST_2_PI197795831 | custom |
| 192 | RT1-M6-1 | 0,09 | 1,06 | 0,5742 | RT1 class I, M6, gene 1 | CUST_3_PI197795831 | custom |
| 192 | RT1-M6-1 | 0,15 | 1,11 | 0,2707 | RT1 class I, M6, gene 1 | CUST_4_PI197795831 | custom |
| 192 | RT1-M6-1 | 0,13 | 1,09 | 0,5124 | RT1 class I, M6, gene 1 | CUST_5_PI197795831 | custom |
| 193 | RT1-M4 | -0,05 | 0,97 | 0,6379 | RT1 class I, M4 | A_44_P260445 | Agilent |
| 193 | RT1-M4 | -0,03 | 0,98 | 0,8888 | RT1 class I, M4 | CUST_1_PI201011151 | custom |
| 193 | RT1-M4 | 0,01 | 1,01 | 0,9694 | RT1 class I, M4 | CUST_2_PI201011151 | custom |
| 193 | RT1-M4 | 0,25 | 1,19 | 0,2536 | RT1 class I, M4 | CUST_3_PI201011151 | custom |
| 193 | RT1-M4 | -0,01 | 0,99 | 0,9413 | RT1 class I, M4 | CUST_4_PI201011151 | custom |
| 193 | RT1-M4 | -0,11 | 0,93 | 0,6425 | RT1 class I, M4 | CUST_5_PI201011151 | custom |
| 194 | RT1-M5 | -0,13 | 0,91 | 0,2545 | RT1 class Ib, locus M5 | CUST_1_PI197795834 | custom |
| 194 | RT1-M5 | -0,02 | 0,99 | 0,9122 | RT1 class Ib, locus M5 | CUST_2_PI197795834 | custom |
| 194 | RT1-M5 | -0,05 | 0,97 | 0,6483 | RT1 class Ib, locus M5 | CUST_3_PI197795834 | custom |
| 194 | RT1-M5 | 0,03 | 1,02 | 0,8395 | RT1 class Ib, locus M5 | CUST_4_PI197795834 | custom |
| 194 | RT1-M5 | -0,05 | 0,97 | 0,6199 | RT1 class Ib, locus M5 | CUST_5_PI197795834 | custom |
| 195 | Zfp57 | 0,13 | 1,09 | 0,6841 | zinc finger protein 57 | CUST_1_PI197795840 | custom |
| 195 | Zfp57 | -0,43 | 0,74 | 0,0681 | zinc finger protein 57 | CUST_2_PI197795840 | custom |
| 195 | Zfp57 | -0,40 | 0,76 | 0,0611 | zinc finger protein 57 | CUST_3_PI197795840 | custom |
| 195 | Zfp57 | -0,34 | 0,79 | 0,0401 | zinc finger protein 57 | CUST_4_PI197795840 | custom |
| 195 | Zfp57 | -0,29 | 0,82 | 0,0940 | zinc finger protein 57 | CUST_5_PI197795840 | custom |
| 196 | Mog | -0,26 | 0,84 | 0,1591 | myelin oligodendrocyte glycoprotein | A_43_PI2283 | Agilent |
| 197 | Gabbr1 | -0,17 | 0,89 | 0,4183 | gamma-aminobutyric acid (GABA) B receptor 1 | A_43_PI2481 | Agilent |
| 198 | 9430032L10Rik | 0,05 | 1,04 | 0,5965 | gene corresponding to Riken clone 9430032L10 | CUST_1_PI201011147 | custom |
| 198 | 9430032L10Rik | 0,02 | 1,01 | 0,8261 | gene corresponding to Riken clone 9430032L10 | CUST_2_PI201011147 | custom |
| 198 | 9430032L10Rik | 0,02 | 1,01 | 0,8425 | gene corresponding to Riken clone 9430032L10 | CUST_3_PI201011147 | custom |
| 198 | 9430032L10Rik | 0,04 | 1,03 | 0,8307 | gene corresponding to Riken clone 9430032L10 | CUST_4_PI201011147 | custom |
| 198 | 9430032L10Rik | -0,03 | 0,98 | 0,8685 | gene corresponding to Riken clone 9430032L10 | CUST_5_PI201011147 | custom |
| 199 | Or1 | -0,08 | 0,95 | 0,5471 | olfactory receptor 1750 (predicted) | A_52_P410245 | Agilent |
| 199 | Or1 | -0,02 | 0,99 | 0,8675 | olfactory receptor 1750 (predicted) | CUST_16_PI209196805 | custom |
| 199 | Or1 | -0,11 | 0,93 | 0,4597 | olfactory receptor 1750 (predicted) | CUST_17_PI209196805 | custom |
| 199 | Or1 | -0,02 | 0,99 | 0,9034 | olfactory receptor 1750 (predicted) | CUST_18_PI209196805 | custom |
| 199 | Or1 | -0,04 | 0,97 | 0,8090 | olfactory receptor 1750 (predicted) | CUST_19_PI209196805 | custom |
| 199 | Or1 | -0,05 | 0,97 | 0,7090 | olfactory receptor 1750 (predicted) | CUST_20_PI209196805 | custom |
| 200 | Or2 | -0,07 | 0,95 | 0,6299 | olfactory receptor 1749 (predicted) | CUST_1_PI197795848 | custom |
| 200 | Or2 | -0,08 | 0,95 | 0,3091 | olfactory receptor 1749 (predicted) | CUST_2_PI197795848 | custom |
| 200 | Or2 | 0,07 | 1,05 | 0,5007 | olfactory receptor 1749 (predicted) | CUST_3_PI197795848 | custom |
| 200 | Or2 | -0,05 | 0,97 | 0,6808 | olfactory receptor 1749 (predicted) | CUST_4_PI197795848 | custom |
| 200 | Or2 | -0,03 | 0,98 | 0,8117 | olfactory receptor 1749 (predicted) | CUST_5_PI197795848 | custom |
| 201 | Or3 | -0,11 | 0,93 | 0,4566 | olfactory receptor 1748 (predicted) | CUST_1_PI197795850 | custom |
| 201 | Or3 | -0,13 | 0,91 | 0,2329 | olfactory receptor 1748 (predicted) | CUST_2_PI197795850 | custom |
| 201 | Or3 | -0,17 | 0,89 | 0,1773 | olfactory receptor 1748 (predicted) | CUST_3_PI197795850 | custom |
| 201 | Or3 | -0,06 | 0,96 | 0,6310 | olfactory receptor 1748 (predicted) | CUST_4_PI197795850 | custom |
| 201 | Or3 | -0,27 | 0,83 | 0,1077 | olfactory receptor 1748 (predicted) | CUST_5_PI197795850 | custom |
| 202 | Or4 | -0,20 | 0,87 | 0,2322 | olfactory receptor 1747 (predicted) | CUST_1_PI201011143 | custom |
| 202 | Or4 | -0,01 | 0,99 | 0,9720 | olfactory receptor 1747 (predicted) | CUST_2_PI201011143 | custom |
| 202 | Or4 | -0,21 | 0,86 | 0,1923 | olfactory receptor 1747 (predicted) | CUST_3_PI201011143 | custom |
| 202 | Or4 | -0,18 | 0,88 | 0,2355 | olfactory receptor 1747 (predicted) | CUST_4_PI201011143 | custom |
| 202 | Or4 | -0,10 | 0,93 | 0,5972 | olfactory receptor 1747 (predicted) | CUST_5_PI201011143 | custom |
| 203 | Or5 | 0,10 | 1,07 | 0,4571 | olfactory receptor 1746 (predicted) | CUST_1_PI197795852 | custom |
| 203 | Or5 | 0,04 | 1,03 | 0,7809 | olfactory receptor 1746 (predicted) | CUST_2_PI197795852 | custom |
| 203 | Or5 | -0,03 | 0,98 | 0,8583 | olfactory receptor 1746 (predicted) | CUST_3_PI197795852 | custom |
| 203 | Or5 | 0,13 | 1,09 | 0,3836 | olfactory receptor 1746 (predicted) | CUST_4_PI197795852 | custom |
| 203 | Or5 | -0,02 | 0,99 | 0,9090 | olfactory receptor 1746 (predicted) | CUST_5_PI197795852 | custom |
| 204 | Ubd | 3,19 | 9,13 | 0,0345 | ubiquitin D | A_42_P602724 | Agilent |
| 205 | Or6 | 0,03 | 1,02 | 0,8855 | olfactory receptor 1745 (predicted) | CUST_1_PI201011139 | custom |
| 205 | Or6 | 0,14 | 1,10 | 0,2994 | olfactory receptor 1745 (predicted) | CUST_2_PI201011139 | custom |
| 205 | Or6 | -0,06 | 0,96 | 0,6676 | olfactory receptor 1745 (predicted) | CUST_3_PI201011139 | custom |
| 205 | Or6 | -0,09 | 0,94 | 0,8444 | olfactory receptor 1745 (predicted) | CUST_4_PI201011139 | custom |
| 205 | Or6 | -0,09 | 0,94 | 0,7413 | olfactory receptor 1745 (predicted) | CUST_5_PI201011139 | custom |
| 206 | Or7 | 0,14 | 1,10 | 0,2389 | olfactory receptor 1744 (predicted) | CUST_1_PI197795854 | custom |
| 206 | Or7 | 0,00 | 1,00 | 0,9829 | olfactory receptor 1744 (predicted) | CUST_2_PI197795854 | custom |
| 206 | Or7 | -0,09 | 0,94 | 0,6165 | olfactory receptor 1744 (predicted) | CUST_3_PI197795854 | custom |
| 206 | Or7 | 0,12 | 1,09 | 0,5423 | olfactory receptor 1744 (predicted) | CUST_4_PI197795854 | custom |
| 206 | Or7 | -0,07 | 0,95 | 0,4992 | olfactory receptor 1744 (predicted) | CUST_5_PI197795854 | custom |
| 207 | Or8 | 0,01 | 1,01 | 0,9701 | olfactory receptor 1743 (predicted) | CUST_1_PI197795856 | custom |
| 207 | Or8 | -0,10 | 0,93 | 0,5207 | olfactory receptor 1743 (predicted) | CUST_2_PI197795856 | custom |
| 207 | Or8 | -0,14 | 0,91 | 0,2325 | olfactory receptor 1743 (predicted) | CUST_3_PI197795856 | custom |
| 207 | Or8 | -0,10 | 0,93 | 0,4321 | olfactory receptor 1743 (predicted) | CUST_4_PI197795856 | custom |
| 207 | Or8 | -0,31 | 0,81 | 0,0310 | olfactory receptor 1743 (predicted) | CUST_5_PI197795856 | custom |
| 208 | Or9 | -0,02 | 0,99 | 0,8743 | olfactory receptor 1742 (predicted) | A_44_P365332 | Agilent |
| 208 | Or9 | 0,03 | 1,02 | 0,8131 | olfactory receptor 1742 (predicted) | CUST_1_PI197795876 | custom |
| 208 | Or9 | -0,19 | 0,88 | 0,1014 | olfactory receptor 1742 (predicted) | CUST_2_PI197795876 | custom |
| 208 | Or9 | -0,26 | 0,84 | 0,0623 | olfactory receptor 1742 (predicted) | CUST_3_PI197795876 | custom |
| 208 | Or9 | -0,04 | 0,97 | 0,6038 | olfactory receptor 1742 (predicted) | CUST_4_PI197795876 | custom |
| 208 | Or9 | -0,02 | 0,99 | 0,9014 | olfactory receptor 1742 (predicted) | CUST_5_PI197795876 | custom |
| 209 | RT1-M3-2 | -0,07 | 0,95 | 0,8384 | RT1 class Ib, locus M3 | CUST_1_PI201011135 | custom |
| 209 | RT1-M3-2 | -0,03 | 0,98 | 0,9327 | RT1 class Ib, locus M3 | CUST_2_PI201011135 | custom |
| 209 | RT1-M3-2 | -0,08 | 0,95 | 0,8425 | RT1 class Ib, locus M3 | CUST_3_PI201011135 | custom |
| 209 | RT1-M3-2 | -0,13 | 0,91 | 0,6541 | RT1 class Ib, locus M3 | CUST_4_PI201011135 | custom |
| 209 | RT1-M3-2 | -0,10 | 0,93 | 0,7667 | RT1 class Ib, locus M3 | CUST_5_PI201011135 | custom |
| 210 | Or10 | 0,07 | 1,05 | 0,6326 | olfactory receptor 1740 (predicted) | CUST_1_PI201011133 | custom |
| 210 | Or10 | -0,10 | 0,93 | 0,2049 | olfactory receptor 1740 (predicted) | CUST_2_PI201011133 | custom |
| 210 | Or10 | -0,09 | 0,94 | 0,5788 | olfactory receptor 1740 (predicted) | CUST_3_PI201011133 | custom |
| 210 | Or10 | -0,08 | 0,95 | 0,5345 | olfactory receptor 1740 (predicted) | CUST_4_PI201011133 | custom |
| 210 | Or10 | -0,10 | 0,93 | 0,2687 | olfactory receptor 1740 (predicted) | CUST_5_PI201011133 | custom |
| 211 | RT1-M3-1 | 0,24 | 1,18 | 0,4938 | RT1 class Ib, locus M3 | CUST_1_PI197795861 | custom |
| 211 | RT1-M3-1 | 0,27 | 1,21 | 0,5424 | RT1 class Ib, locus M3 | CUST_2_PI197795861 | custom |
| 211 | RT1-M3-1 | 0,25 | 1,19 | 0,5596 | RT1 class Ib, locus M3 | CUST_3_PI197795861 | custom |
| 211 | RT1-M3-1 | 0,03 | 1,02 | 0,9375 | RT1 class Ib, locus M3 | CUST_4_PI197795861 | custom |
| 211 | RTI-M3-1 | 0,14 | 1,10 | 0,7567 | RT1 class Ib, locus M3 | CUST_5_PI197795861 | custom |
| 212 | Or11 | 0,03 | 1,02 | 0,7761 | olfactory receptor 1739 (predicted) | A_44_P433163 | Agilent |
| 213 | Or12 | -0,07 | 0,95 | 0,6171 | olfactory receptor 1738 (predicted) | CUST_1_PI197795865 | custom |
| 213 | Or12 | -0,21 | 0,86 | 0,1188 | olfactory receptor 1738 (predicted) | CUST_2_PI197795865 | custom |
| 213 | Or12 | -0,19 | 0,88 | 0,1498 | olfactory receptor 1738 (predicted) | CUST_3_PI197795865 | custom |
| 213 | Or12 | 0,02 | 1,01 | 0,9088 | olfactory receptor 1738 (predicted) | CUST_4_PI197795865 | custom |
| 213 | Or12 | 0,06 | 1,04 | 0,6232 | olfactory receptor 1738 (predicted) | CUST_5_PI197795865 | custom |
| 214 | Or13 | -0,01 | 0,99 | 0,9278 | olfactory receptor 1737 (predicted) | CUST_1_PI197795867 | custom |
| 214 | Or13 | -0,38 | 0,77 | 0,0345 | olfactory receptor 1737 (predicted) | CUST_2_PI197795867 | custom |
| 214 | Or13 | -0,07 | 0,95 | 0,6831 | olfactory receptor 1737 (predicted) | CUST_3_PI197795867 | custom |
| 214 | Or13 | 0,06 | 1,04 | 0,6537 | olfactory receptor 1737 (predicted) | CUST_4_PI197795867 | custom |
| 214 | Or13 | 0,02 | 1,01 | 0,8695 | olfactory receptor 1737 (predicted) | CUST_5_PI197795867 | custom |
| 215 | Or14 | 0,04 | 1,03 | 0,6686 | olfactory receptor 1736 (predicted) | CUST_1_PI197795870 | custom |
| 215 | Or14 | 0,00 | 1,00 | 0,9849 | olfactory receptor 1736 (predicted) | CUST_2_PI197795870 | custom |
| 215 | Or14 | 0,01 | 1,01 | 0,9194 | olfactory receptor 1736 (predicted) | CUST_3_PI197795870 | custom |
| 215 | Or14 | -0,26 | 0,84 | 0,2740 | olfactory receptor 1736 (predicted) | CUST_4_PI197795870 | custom |
| 215 | Or14 | -0,14 | 0,91 | 0,1027 | olfactory receptor 1736 (predicted) | CUST_5_PI197795870 | custom |
| 216 | Or15 | -0,07 | 0,95 | 0,3931 | olfactory receptor 1735 (predicted) | CUST_1_PI197795872 | custom |
| 216 | Or15 | -0,14 | 0,91 | 0,2867 | olfactory receptor 1735 (predicted) | CUST_2_PI197795872 | custom |
| 216 | Or15 | 0,00 | 1,00 | 0,9952 | olfactory receptor 1735 (predicted) | CUST_3_PI197795872 | custom |
| 216 | Or15 | -0,08 | 0,95 | 0,6808 | olfactory receptor 1735 (predicted) | CUST_4_PI197795872 | custom |
| 216 | Or15 | -0,07 | 0,95 | 0,5993 | olfactory receptor 1735 (predicted) | CUST_5_PI197795872 | custom |
| 217 | Or27 | -0,04 | 0,97 | 0,8286 | olfactory receptor 1716 (predicted) | CUST_1_PI201011130 | custom |
| 217 | Or27 | -0,09 | 0,94 | 0,4929 | olfactory receptor 1716 (predicted) | CUST_2_PI201011130 | custom |
| 217 | Or27 | -0,01 | 0,99 | 0,9401 | olfactory receptor 1716 (predicted) | CUST_3_PI201011130 | custom |
| 217 | Or27 | -0,04 | 0,97 | 0,6989 | olfactory receptor 1716 (predicted) | CUST_4_PI201011130 | custom |
| 217 | Or27 | -0,07 | 0,95 | 0,6330 | olfactory receptor 1716 (predicted) | CUST_5_PI201011130 | custom |
| 218 | Or26 | -0,07 | 0,95 | 0,4471 | olfactory receptor 1718 (predicted) | A_44_P505752 | Agilent |
| 219 | Or28 | -0,05 | 0,97 | 0,5892 | olfactory receptor 1714 (predicted) | CUST_1_PI197795859 | custom |
| 219 | Or28 | -0,24 | 0,85 | 0,0490 | olfactory receptor 1714 (predicted) | CUST_2_PI197795859 | custom |
| 219 | Or28 | -0,01 | 0,99 | 0,9454 | olfactory receptor 1714 (predicted) | CUST_3_PI197795859 | custom |
| 219 | Or28 | -0,02 | 0,99 | 0,8444 | olfactory receptor 1714 (predicted) | CUST_4_PI197795859 | custom |
| 219 | Or28 | -0,03 | 0,98 | 0,8464 | olfactory receptor 1714 (predicted) | CUST_5_PI197795859 | custom |
| 220 | RT1-M3-3 | -0,12 | 0,92 | 0,3297 | RT1 class Ib, locus M3 | CUST_1_PI201011128 | custom |
| 220 | RT1-M3-3 | -0,06 | 0,96 | 0,6580 | RT1 class Ib, locus M3 | CUST_2_PI201011128 | custom |
| 220 | RT1-M3-3 | -0,08 | 0,95 | 0,3186 | RT1 class Ib, locus M3 | CUST_3_PI201011128 | custom |
| 220 | RT1-M3-3 | -0,12 | 0,92 | 0,3465 | RT1 class Ib, locus M3 | CUST_4_PI201011128 | custom |
| 220 | RT1-M3-3 | -0,18 | 0,88 | 0,3305 | RT1 class Ib, locus M3 | CUST_5_PI201011128 | custom |
| 222 | Or29 | -0,02 | 0,99 | 0,8250 | olfactory receptor 29 | A_44_P411999 | Agilent |
| 223 | RT1-M2 | 0,04 | 1,03 | 0,6219 | RT1 class Ib, locus M2 | A_44_P154023 | Agilent |
| 224 | Or30 | 0,03 | 1,02 | 0,7708 | olfactory receptor 1730 (predicted) | CUST_1_PI197795878 | custom |
| 224 | Or30 | -0,06 | 0,96 | 0,5708 | olfactory receptor 1730 (predicted) | CUST_2_PI197795878 | custom |
| 224 | Or30 | 0,00 | 1,00 | 0,9771 | olfactory receptor 1730 (predicted) | CUST_3_PI197795878 | custom |
| 224 | Or30 | -0,36 | 0,78 | 0,0940 | olfactory receptor 1730 (predicted) | CUST_4_PI197795878 | custom |
| 224 | Or30 | 0,05 | 1,04 | 0,7708 | olfactory receptor 1730 (predicted) | CUST_5_PI197795878 | custom |

**Table 5b**

| **Gene order** | **Gene Symbol** | **log2-Fold Change** | **Fold Change** | **adj.P-value** | **Gene Description** | **Probe ID** | **Probe Design** |
|---|---|---|---|---|---|---|---|
| 16 | RT1-A1 | 0,70 | 1,62 | 0,0149 | RT1 class I | CUST_1_PI202535318 | custom |
| 16 | RT1-A1 | 0,75 | 1,68 | 0,0100 | RT1 class I | CUST_2_PI202535318 | custom |
| 16 | RT1-A1 | 0,80 | 1,74 | 0,0149 | RT1 class I | CUST_3_PI202535318 | custom |
| 16 | RT1-A1 | 0,86 | 1,82 | 0,0100 | RT1 class I | CUST_4_PI202535318 | custom |
| 16 | RT1-A1 | 0,91 | 1,88 | 0,0100 | RT1 class I | CUST_5_PI202535318 | custom |
| 17 | RT1-A2 | 0,98 | 1,97 | 0,0100 | RT1 class I | A_44_P296155 | Agilent |
| 29 | RT1-DMb | 2,59 | 6,02 | 0,0100 | major histocompatibility complex, class II, DM beta | CUST_1_PI195698203 | custom |
| 29 | RT1-DMb | 2,77 | 6,82 | 0,0100 | major histocompatibility complex, class II, DM beta | CUST_2_PI195698203 | custom |
| 29 | RT1-DMb | 1,93 | 3,81 | 0,0149 | major histocompatibility complex, class II, DM beta | CUST_3_PI195698203 | custom |
| 29 | RT1-DMb | 1,87 | 3,66 | 0,0149 | major histocompatibility complex, class II, DM beta | CUST_4_PI195698203 | custom |
| 29 | RT1-DMb | 1,94 | 3,84 | 0,0100 | major histocompatibility complex, class II, DM beta | CUST_5_PI195698203 | custom |
| 31 | Tap1 | 0,53 | 1,44 | 0,1159 | transporter 1, ATP-binding cassette, sub-family B (MDR/TAP) | A_43_P15763 | Agilent |
| 31 | Tap1 | 0,63 | 1,55 | 0,0390 | transporter 1, ATP-binding cassette, sub-family B (MDR/TAP) | A_44_P451916 | Agilent |
| 32 | Psmb8 | 1,00 | 2,00 | 0,0336 | proteasome (prosome, macropain) subunit, beta type 8 (large multifunctional peptidase 7) | A_42_P761035 | Agilent |
| 51 | G18 (Gpsm3) | 1,23 | 2,35 | 0,0315 | G18 protein | A_42_P569708 | Agilent |
| 52 | Pbx2 | 0,33 | 1,26 | 0,0466 | pre-B-cell leukemia transcription factor 2 | A_42_P592157 | Agilent |
| 54 | Rnf5 | 0,57 | 1,48 | 0,0315 | ring finger protein 5 | A_51_P204582 | Agilent |
| 54 | Rnf5 | 0,26 | 1,20 | 0,0674 | ring finger protein 5 | CUST_1_PI207500742 | custom |
| 54 | Rnf5 | 0,21 | 1,16 | 0,1445 | ring finger protein 5 | CUST_2_PI207500742 | custom |
| 54 | Rnf5 | 0,17 | 1,13 | 0,2905 | ring finger protein 5 | CUST_3_PI207500742 | custom |
| 54 | Rnf5 | 0,22 | 1,16 | 0,1626 | ring finger protein 5 | CUST_4_PI207500742 | custom |
| 54 | Rnf5 | 0,19 | 1,14 | 0,1707 | ring finger protein 5 | CUST_5_PI207500742 | custom |
| 69 | C2 | 1,22 | 2,33 | 0,0325 | complement component 2 | A_44_P332606 | Agilent |
| 88 | Ly6g6e | -1,38 | 0,38 | 0,0416 | lymphocyte antigen 6 complex, locus G6E | CUST_1_PI195698246 | custom |
| 88 | Ly6g6e | -1,42 | 0,37 | 0,0523 | lymphocyte antigen 6 complex, locus G6E | CUST_2_PI195698246 | custom |
| 88 | Ly6g6e | -1,39 | 0,38 | 0,0623 | lymphocyte antigen 6 complex, locus G6E | CUST_3_PI195698246 | custom |
| 88 | Ly6g6e | -1,44 | 0,37 | 0,0416 | lymphocyte antigen 6 complex, locus G6E | CUST_4_PI195698246 | custom |
| 88 | Ly6g6e | -1,46 | 0,36 | 0,0433 | lymphocyte antigen 6 complex, locus G6E | CUST_5_PI195698246 | custom |
| 90 | Bat5 | -0,60 | 0,66 | 0,0100 | HLA-B associated transcript 5 | CUST_1_PI195830595 | custom |
| 90 | Bat5 | -0,48 | 0,72 | 0,0100 | HLA-B associated transcript 5 | CUST_2_PI195830595 | custom |
| 90 | Bat5 | -0,54 | 0,69 | 0,0180 | HLA-B associated transcript 5 | CUST_3_PI195830595 | custom |
| 90 | Bat5 | -0,53 | 0,69 | 0,0229 | HLA-B associated transcript 5 | CUST_4_PI195830595 | custom |
| 90 | Bat5 | -0,58 | 0,67 | 0,0100 | HLA-B associated transcript 5 | CUST_5_PI195830595 | custom |
| 100 | Aif1 | 2,83 | 7,11 | 0,0100 | allograft inflammatory factor 1 | A_44_P421534 | Agilent |
| 102 | Lst1 | 3,32 | 9,99 | 0,0100 | leucocyte specific transcript 1 | A_43_P12274 | Agilent |
| 110 | RT1-CE2 | 0,64 | 1,56 | 0,0278 | RT1 class I, CE2 | A_44_P107372 | Agilent |
| 111 | RT1-CE3 | 0,96 | 1,95 | 0,0100 | RT1 class I, CE3 | A_44_P274061 | Agilent |
| 113 | RT1-CE5 | 0,70 | 1,62 | 0,0395 | RT1 class I, CE5 | A_44_P172850 | Agilent |
| 116 | RT1-CE8 | 0,90 | 1,87 | 0,0278 | RT1 class I, CE8 | CUST_1_PI201011245 | custom |
| 116 | RT1-CE8 | 0,91 | 1,88 | 0,0100 | RT1 class I, CE8 | CUST_2_PI201011245 | custom |
| 116 | RT1-CE8 | 0,78 | 1,72 | 0,0229 | RT1 class I, CE8 | CUST_3_PI201011245 | custom |
| 116 | RT1-CE8 | 0,84 | 1,79 | 0,0100 | RT1 class I, CE8 | CUST_4_PI201011245 | custom |
| 116 | RT1-CE8 | 0,79 | 1,73 | 0,0149 | RT1 class I, CE8 | CUST_5_PI201011245 | custom |
| 117 | RT1-CE9 | 0,80 | 1,74 | 0,0315 | RT1 class I, CE9 | CUST_1_PI201011241 | custom |
| 117 | RT1-CE9 | 0,35 | 1,27 | 0,1745 | RT1 class I, CE9 | CUST_2_PI201011241 | custom |
| 117 | RT1-CE9 | 0,74 | 1,67 | 0,0539 | RT1 class I, CE9 | CUST_3_PI201011241 | custom |
| 117 | RT1-CE9 | 0,24 | 1,18 | 0,3698 | RT1 class I, CE9 | CUST_4_PI201011241 | custom |
| 117 | RT1-CE9 | 0,81 | 1,75 | 0,0373 | RT1 class I, CE9 | CUST_5_PI201011241 | custom |
| 118 | RT1-CE10 | 4,09 | 17,03 | 0,0100 | RT1 class I, CE10 | A_44_P389019 | Agilent |
| 119 | RT1-CE11 | 0,28 | 1,21 | 0,2867 | RT1 class I, CE11 | CUST_1_PI195941302 | custom |
| 119 | RT1-CE11 | 0,65 | 1,57 | 0,0315 | RT1 class I, CE11 | CUST_2_PI195941302 | custom |
| 119 | RT1-CE11 | 0,22 | 1,16 | 0,2638 | RT1 class I, CE11 | CUST_3_PI195941302 | custom |
| 119 | RT1-CE11 | 0,16 | 1,12 | 0,3957 | RT1 class I, CE11 | CUST_4_PI195941302 | custom |
| 119 | RT1-CE11 | 0,38 | 1,30 | 0,0980 | RT1 class I, CE11 | CUST_5_PI195941302 | custom |
| 120 | RT1-CE12 | 0,43 | 1,35 | 0,1710 | RT1 class I, CE12 | CUST_1_PI195941305 | custom |
| 120 | RT1-CE12 | -0,10 | 0,93 | 0,4503 | RT1 class I, CE12 | CUST_2_PI195941305 | custom |
| 120 | RT1-CE12 | 0,34 | 1,27 | 0,1043 | RT1 class I, CE12 | CUST_3_PI195941305 | custom |
| 120 | RT1-CE12 | 0,04 | 1,03 | 0,8574 | RT1 class I, CE12 | CUST_4_PI195941305 | custom |
| 120 | RT1-CE12 | 0,56 | 1,47 | 0,0310 | RT1 class I, CE12 | CUST_5_PI195941305 | custom |
| 124 | RT1-CE16 | 0,54 | 1,45 | 0,0325 | RT1 class I, CE16 (RT1 class Ib, locus Cl) | A_44_P867246 | Agilent |
| 124 | RT1-CE16 | 0,78 | 1,72 | 0,0206 | RT1 class I, CE16 (RT1 class Ib, locus Cl) | A_44_P554925 | Agilent |
| 128 | Spr1 | 1,26 | 2,39 | 0,0206 | psoriasis susceptibility 1 candidate 2 (human) | A_66_P100662 | Agilent |
| 128 | Spr1 | 1,39 | 2,62 | 0,0180 | psoriasis susceptibility 1 candidate 2 (human) | A_51_P212958 | Agilent |
| 128 | Spr1 | 1,36 | 2,57 | 0,0206 | psoriasis susceptibility 1 candidate 2 (human) | A_51_P212956 | Agilent |
| 128 | Spr1 | 1,50 | 2,83 | 0,0100 | psoriasis susceptibility 1 candidate 2 (human) | CUST_56_PI209196805 | custom |
| 128 | Spr1 | 1,52 | 2,87 | 0,0100 | psoriasis susceptibility 1 candidate 2 (human) | CUST_57_PI209196805 | custom |
| 128 | Spr1 | 1,51 | 2,85 | 0,0100 | psoriasis susceptibility 1 candidate 2 (human) | CUST_58_PI209196805 | custom |
| 128 | Spr1 | 1,50 | 2,83 | 0,0100 | psoriasis susceptibility 1 candidate 2 (human) | CUST_59_PI209196805 | custom |
| 128 | Spr1 | 1,58 | 2,99 | 0,0100 | psoriasis susceptibility 1 candidate 2 (human) | CUST_60_PI209196805 | custom |
| 129 | Cdsn | 0,37 | 1,29 | 0,2732 | corneodesmosin | CUST_1_PI201011238 | custom |
| 129 | Cdsn | 0,84 | 1,79 | 0,0100 | corneodesmosin | CUST_2_PI201011238 | custom |
| 129 | Cdsn | 0,38 | 1,30 | 0,2184 | corneodesmosin | CUST_3_PI201011238 | custom |
| 129 | Cdsn | 0,32 | 1,25 | 0,3754 | corneodesmosin | CUST_4_PI201011238 | custom |
| 129 | Cdsn | 0,40 | 1,32 | 0,1769 | corneodesmosin | CUST_5_PI201011238 | custom |
| 138 | Ier3 | 0,87 | 1,83 | 0,0229 | immediate early response 3 | A_42_P515405 | Agilent |
| 143 | Kiaa1949 | 0,42 | 1,34 | 0,0481 | KIAA1949 protein | CUST_1_PI201011218 | custom |
| 143 | Kiaa1949 | 0,49 | 1,40 | 0,0457 | KIAA1949 protein | CUST_2_PI201011218 | custom |
| 143 | Kiaa1949 | 0,33 | 1,26 | 0,1378 | KIAA1949 protein | CUST_3_PI201011218 | custom |
| 143 | Kiaa1949 | 0,39 | 1,31 | 0,0993 | KIAA1949 protein | CUST_4_PI201011218 | custom |
| 143 | Kiaa1949 | 0,34 | 1,27 | 0,1184 | KIAA1949 protein | CUST_5_PI201011218 | custom |
| 146 | Flj13158 (RGD1303066) | -0,25 | 0,84 | 0,0832 | hypothetical protein FU13158 | A_44_P278509 | Agilent |
| 146 | Flj13158 (RGD1303066) | -0,57 | 0,67 | 0,0378 | hypothetical protein FLJ13158 | A_44_P654250 | Agilent |
| 147 | Mrps18b | 0,52 | 1,43 | 0,0474 | mitochondrial ribosomal protein S18B | CUST_1_PI197795811 | custom |
| 147 | Mrps18b | 0,49 | 1,40 | 0,0378 | mitochondrial ribosomal protein S18B | CUST_2_PI197795811 | custom |
| 147 | Mrps18b | 0,57 | 1,48 | 0,0267 | mitochondrial ribosomal protein S18B | CUST_3_PI197795811 | custom |
| 147 | Mrps18b | 0,59 | 1,51 | 0,0365 | mitochondrial ribosomal protein S18B | CUST_4_PI197795811 | custom |
| 147 | Mrps18b | 0,62 | 1,54 | 0,0254 | mitochondrial ribosomal protein S18B | CUST_5_PI197795811 | custom |
| 154 | RT1-T24-3 | 0,31 | 1,24 | 0,1540 | RT1 class I, T24, gene 3 | CUST_1_PI201011214 | custom |
| 154 | RT1-T24-3 | 0,42 | 1,34 | 0,0336 | RT1 class I, T24, gene 3 | CUST_2_PI201011214 | custom |
| 154 | RT1-T24-3 | 0,27 | 1,21 | 0,1454 | RT1 class I, T24, gene 3 | CUST_3_PI201011214 | custom |
| 154 | RT1-T24-3 | 0,31 | 1,24 | 0,0847 | RT1 class I, T24, gene 3 | CUST_4_PI201011214 | custom |
| 154 | RT1-T24-3 | 0,08 | 1,06 | 0,6030 | RT1 class I, T24, gene 3 | CUST_5_PI201011214 | custom |
| 155 | RT1-T24-4 | 0,57 | 1,48 | 0,0345 | RT1 class I, T24, gene 4 | CUST_1_PI197795813 | custom |
| 155 | RT1-T24-4 | 0,76 | 1,69 | 0,0206 | RT1 class I, T24, gene 4 | CUST_2_PI197795813 | custom |
| 155 | RT1-T24-4 | 0,72 | 1,65 | 0,0206 | RT1 class I, T24, gene 4 | CUST_3_PI197795813 | custom |
| 155 | RT1-T24-4 | 0,39 | 1,31 | 0,0611 | RT1 class I, T24, gene 4 | CUST_4_PI197795813 | custom |
| 155 | RT1-T24-4 | 0,51 | 1,42 | 0,0939 | RT1 class I, T24, gene 4 | CUST_5_PI197795813 | custom |
| 156 | RT-BM1 (RT1- S3) | 1,06 | 2,08 | 0,0416 | RT1 class I, RT-BM1 | A_44_P454420 | Agilent |
| 161 | RT1-O2 | -0,38 | 0,77 | 0,2262 | RT1 class I, O2 | CUST_1_PI201011211 | custom |
| 161 | RT1-O2 | 0,57 | 1,48 | 0,0345 | RT1 class I, O2 | CUST_2_PI201011211 | custom |
| 161 | RT1-O2 | -0,09 | 0,94 | 0,6330 | RT1 class I, O2 | CUST_3_PI201011211 | custom |
| 161 | RT1-O2 | 0,55 | 1,46 | 0,0424 | RT1 class I, O2 | CUST_4_PI201011211 | custom |
| 161 | RT1-O2 | 0,22 | 1,16 | 0,3389 | RT1 class I, O2 | CUST_5_PI201011211 | custom |
| 162 | RT1-O3 | -0,30 | 0,81 | 0,2438 | RT1 class I, O3 | CUST_1_PI201011202 | custom |
| 162 | RT1-O3 | -0,13 | 0,91 | 0,5468 | RT1 class I, O3 | CUST_2_PI201011202 | custom |
| 162 | RTI-O3 | 0,50 | 1,41 | 0,0546 | RT1 class I, O3 | CUST_3_PI201011202 | custom |
| 162 | RT1-O3 | 0,50 | 1,41 | 0,0457 | RT1 class I, O3 | CUST_4_PI201011202 | custom |
| 162 | RT1-O3 | 0,23 | 1,17 | 0,2975 | RT1 class I, O3 | CUST_5_PI201011202 | custom |
| 172 | RT1-M1-1 | -0,01 | 0,99 | 0,9358 | RT1 class I, M1, gene 1 | CUST_1_PI201011178 | custom |
| 172 | RT1-M1-1 | -0,11 | 0,93 | 0,4445 | RT1 class I, M1, gene 1 | CUST_2_PI201011178 | custom |
| 172 | RT1-M1-1 | 0,54 | 1,45 | 0,0278 | RT1 class I, M1, gene 1 | CUST_3_PI201011178 | custom |
| 172 | RT1-M1-1 | -0,17 | 0,89 | 0,1632 | RT1 class I, M1, gene 1 | CUST_4_PI201011178 | custom |
| 172 | RT1-M1-1 | -0,05 | 0,97 | 0,7839 | RT1 class I, M1, gene 1 | CUST_5_PI201011178 | custom |
| 177 | RT1-M7 | -0,08 | 0,95 | 0,3109 | RT1 class I, M7 | CUST_1_PI201011173 | custom |
| 177 | RT1-M7 | -0,30 | 0,81 | 0,0433 | RT1 class I, M7 | CUST_2_PI201011173 | custom |
| 177 | RT1-M7 | 0,04 | 1,03 | 0,5727 | RT1 class I, M7 | CUST_3_PI201011173 | custom |
| 177 | RT1-M7 | -0,05 | 0,97 | 0,7154 | RT1 class I, M7 | CUST_4_PI201011173 | custom |
| 177 | RT1-M7 | -0,32 | 0,80 | 0,1162 | RT1 class I, M7 | CUST_5_PI201011173 | custom |
| 179 | RT1-M10-3 | -0,02 | 0,99 | 0,9071 | RT1 class I, M10, gene 3 | CUST_1_PI201011167 | custom |
| 179 | RT1-M10-3 | -0,27 | 0,83 | 0,0424 | RT1 class I, M10, gene 3 | CUST_2_PI201011167 | custom |
| 179 | RT1-M10-3 | -0,06 | -0,96 | 0,6730 | RT1 class I, M10, gene 3 | CUST_3_PI201011167 | custom |
| 179 | RT1-M10-3 | -0,04 | 0,97 | 0,6161 | RT1 class I, M10, gene 3 | CUST_4_PI201011167 | custom |
| 179 | RT1-M10-3 | -0,06 | 0,96 | 0,5878 | RT1 class I, M10, gene 3 | CUST_5_PI201011167 | custom |
| 195 | Zfp57 | 0,13 | 1,09 | 0,6841 | zinc finger protein 57 | CUST_1_PI197795840 | custom |
| 195 | Zfp57 | -0,43 | 0,74 | 0,0681 | zinc finger protein 57 | CUST_2_PI197795840 | custom |
| 195 | Zfp57 | -0,40 | 0,76 | 0,0611 | zinc finger protein 57 | CUST_3_PI197795840 | custom |
| 195 | Zfp57 | -0,34 | 0,79 | 0,0401 | zinc finger protein 57 | CUST_4_PI197795840 | custom |
| 195 | Zfp57 | -0,29 | 0,82 | 0,0940 | zinc finger protein 57 | CUST_5_PI197795840 | custom |
| 204 | Ubd | 3,19 | 9,13 | 0,0345 | ubiquitin D | A_42_P602724 | Agilent |
| 207 | Or8 | 0,01 | 1,01 | 0,9701 | olfactory receptor 1743 (predicted) | CUST_1_PI197795856 | custom |
| 207 | Or8 | -0,10 | 0,93 | 0,5207 | olfactory receptor 1743 (predicted) | CUST_2_PI197795856 | custom |
| 207 | Or8 | -0,14 | 0,91 | 0,2325 | olfactory receptor 1743 (predicted) | CUST_3_PI197795856 | custom |
| 207 | Or8 | -0,10 | 0,93 | 0,4321 | olfactory receptor 1743 (predicted) | CUST_4_PI197795856 | custom |
| 207 | Or8 | -0,31 | 0,81 | 0,0310 | olfactory receptor 1743 (predicted) | CUST_5_PI197795856 | custom |
| 214 | Or13 | -0,01 | 0,99 | 0,9278 | olfactory receptor 1737 (predicted) | CUST_1_PI197795867 | custom |
| 214 | Or13 | -0,38 | 0,77 | 0,0345 | olfactory receptor 1737 (predicted) | CUST_2_PI197795867 | custom |
| 214 | Or13 | -0,07 | 0,95 | 0,6831 | olfactory receptor 1737 (predicted) | CUST_3_PI197795867 | custom |
| 214 | Or13 | 0,06 | 1,04 | 0,6537 | olfactory receptor 1737 (predicted) | CUST_4_PI197795867 | custom |
| 214 | Or13 | 0,02 | 1,01 | 0,8695 | olfactory receptor 1737 (predicted) | CUST_5_PI197795867 | custom |
| 219 | Or28 | -0,05 | 0,97 | 0,5892 | olfactory receptor 1714 (predicted) | CUST_1_PI197795859 | custom |
| 219 | Or28 | -0,24 | 0,85 | 0,0490 | olfactory receptor 1714 (predicted) | CUST_2_PI197795859 | custom |
| 219 | Or28 | -0,01 | 0,99 | 0,9454 | olfactory receptor 1714 (predicted) | CUST_3_PI197795859 | custom |
| 219 | Or28 | -0,02 | 0,99 | 0,8444 | olfactory receptor 1714 (predicted) | CUST_4_PI197795859 | custom |
| 219 | Or28 | -0,03 | 0,98 | 0,8464 | olfactory receptor 1714 (predicted) | CUST_5_PI197795859 | custom |

**Table 5c**

| **Gene order** | **Gene Symbol** | **log2-Fold Change** | **Fold Change** | **adj.P-value** | **Gene Description** | **Probe ID** | **Probe Design** |
|---|---|---|---|---|---|---|---|
| 16 | RT1-A1 | 0,70 | 1,62 | 0,0149 | RT1 class I | CUST_1_PI202535318 | custom |
| 16 | RT1-A1 | 0,75 | 1,68 | 0,0100 | RT1 class I | CUST_2_PI202535318 | custom |
| 16 | RT1-A1 | 0,80 | 1,74 | 0,0149 | RT1 class I | CUST_3_PI202535318 | custom |
| 16 | RT1-A1 | 0,86 | 1,82 | 0,0100 | RT1 class I | CUST_4_PI202535318 | custom |
| 16 | RT1-A1 | 0,91 | 1,88 | 0,0100 | RT1 class I | CUST_5_PI202535318 | custom |
| 17 | RT1-A2 | 0,98 | 1,97 | 0,0100 | RT1 class I | A_44_P296155 | Agilent |
| 29 | RT1-DMb | 2,59 | 6,02 | 0,0100 | major histocompatibility complex, class II, DM beta | CUST_1_PI195698203 | custom |
| 29 | RT1-DMb | 2,77 | 6,82 | 0,0100 | major histocompatibility complex, class II, DM beta | CUST_2_PI195698203 | custom |
| 29 | RT1-DMb | 1,93 | 3,81 | 0,0149 | major histocompatibility complex, class II, DM beta | CUST_3_PI195698203 | custom |
| 29 | RT1-DMb | 1,87 | 3,66 | 0,0149 | major histocompatibility complex, class II, DM beta | CUST_4_PI195698203 | custom |
| 29 | RT1-DMb | 1,94 | 3,84 | 0,0100 | major histocompatibility complex, class II, DM beta | CUST_5_PI195698203 | custom |
| 31 | Tap1 | 0,63 | 1,55 | 0,0390 | transporter 1, ATP-binding cassette, sub-family B (MDR/TAP) | A_44_P451916 | Agilent |
| 32 | Psmb8 | 1,00 | 2,00 | 0,0336 | proteasome (prosome, macropain) subunit, beta type 8 (large multifunctional peptidase 7) | A_42_P761035 | Agilent |
| 51 | G18 (Gpsm3) | 1,23 | 2,35 | 0,0315 | G18 protein | A_42_P569708 | Agilent |
| 52 | Pbx2 | 0,33 | 1,26 | 0,0466 | pre-B-cell leukemia transcription factor 2 | A_42_P592157 | Agilent |
| 69 | C2 | 1,22 | 2,33 | 0,0325 | complement component 2 | A_44_P332606 | Agilent |
| 88 | Ly6g6e | -1,38 | 0,38 | 0,0416 | lymphocyte antigen 6 complex, locus G6E | CUST_1_PI195698246 | custom |
| 88 | Ly6g6e | -1,44 | 0,37 | 0,0416 | lymphocyte antigen 6 complex, locus G6E | CUST_4_PI195698246 | custom |
| 88 | Ly6g6e | -1,46 | 0,36 | 0,0433 | lymphocyte antigen 6 complex, locus G6E | CUST_5_PI195698246 | custom |
| 90 | Bat5 | -0,60 | 0,66 | 0,0100 | HLA-B associated transcript 5 | CUST_1_PI195830595 | custom |
| 90 | Bat5 | -0,48 | 0,72 | 0,0100 | HLA-B associated transcript 5 | CUST_2_PI195830595 | custom |
| 90 | Bat5 | -0,54 | 0,69 | 0,0180 | HLA-B associated transcript 5 | CUST_3_PI195830595 | custom |
| 90 | Bat5 | -0,53 | 0,69 | 0,0229 | HLA-B associated transcript 5 | CUST_4_PI195830595 | custom |
| 90 | Bat5 | -0,58 | 0,67 | 0,0100 | HLA-B associated transcript 5 | CUST_5_PI195830595 | custom |
| 100 | Aif1 | 2,83 | 7,11 | 0,0100 | allograft inflammatory factor 1 | A_44_P421534 | Agilent |
| 102 | Lst1 | 3,32 | 9,99 | 0,0100 | leucocyte specific transcript 1 | A_43_P12274 | Agilent |
| 110 | RT1-CE2 | 0,64 | 1,56 | 0,0278 | RT1 class I, CE2 | A_44_P107372 | Agilent |
| 111 | RT1-CE3 | 0,96 | 1,95 | 0,0100 | RT1 class I, CE3 | A_44_P274061 | Agilent |
| 113 | RT1-CE5 | 0,70 | 1,62 | 0,0395 | RT1 class I, CE5 | A_44_P172850 | Agilent |
| 116 | RT1-CE8 | 0,90 | 1,87 | 0,0278 | RT1 class I, CE8 | CUST_1_PI201011245 | custom |
| 116 | RT1-CE8 | 0,91 | 1,88 | 0,0100 | RT1 class I, CE8 | CUST_2_PI201011245 | custom |
| 116 | RT1-CE8 | 0,78 | 1,72 | 0,0229 | RT1 class I, CE8 | CUST_3_PI201011245 | custom |
| 116 | RT1-CE8 | 0,84 | 1,79 | 0,0100 | RT1 class I, CE8 | CUST_4_PI201011245 | custom |
| 116 | RT1-CE8 | 0,79 | 1,73 | 0,0149 | RT1 class I, CE8 | CUST_5_PI201011245 | custom |
| 118 | RT1-CE10 | 4,09 | 17,03 | 0,0100 | RT1 class I, CE10 | A_44_P389019 | Agilent |
| 124 | RT1-CE16 | 0,54 | 1,45 | 0,0325 | RT1 class I, CE16 (RT1 class Ib, locus Cl) | A_44_P867246 | Agilent |
| 124 | RT1-CE16 | 0,78 | 1,72 | 0,0206 | RT1 class I, CE16 (RT1 class Ib, locus Cl) | A_44_P554925 | Agilent |
| 128 | Spr1 | 1,26 | 2,39 | 0,0206 | psoriasis susceptibility 1 candidate 2 (human) | A_66_P100662 | Agilent |
| 128 | Spr1 | 1,39 | 2,62 | 0,0180 | psoriasis susceptibility 1 candidate 2 (human) | A_51_P212958 | Agilent |
| 128 | Spr1 | 1,36 | 2,57 | 0,0206 | psoriasis susceptibility 1 candidate 2 (human) | A_51_P212956 | Agilent |
| 128 | Spr1 | 1,50 | 2,83 | 0,0100 | psoriasis susceptibility 1 candidate 2 (human) | CUST_56_PI209196805 | custom |
| 128 | Spr1 | 1,52 | 2,87 | 0,0100 | psoriasis susceptibility 1 candidate 2 (human) | CUST_57_PI209196805 | custom |
| 128 | Spr1 | 1,51 | 2,85 | 0,0100 | psoriasis susceptibility 1 candidate 2 (human) | CUST_58_PI209196805 | custom |
| 128 | Spr1 | 1,50 | 2,83 | 0,0100 | psoriasis susceptibility 1 candidate 2 (human) | CUST_59_PI209196805 | custom |
| 128 | Spr1 | 1,58 | 2,99 | 0,0100 | psoriasis susceptibility 1 candidate 2 (human) | CUST_60_PI209196805 | custom |
| 138 | Ier3 | 0,87 | 1,83 | 0,0229 | immediate early response 3 | A_42_P515405 | Agilent |
| 146 | Flj13158 (RGD1303066) | -0,57 | 0,67 | 0,0378 | hypothetical protein FU13158 | A_44_P654250 | Agilent |
| 147 | Mrps18b | 0,52 | 1,43 | 0,0474 | mitochondrial ribosomal protein S18B | CUST_1_PI197795811 | custom |
| 147 | Mrps18b | 0,49 | 1,40 | 0,0378 | mitochondrial ribosomal protein S18B | CUST_2_PI197795811 | custom |
| 147 | Mrps18b | 0,57 | 1,48 | 0,0267 | mitochondrial ribosomal protein S18B | CUST_3_PI197795811 | custom |
| 147 | Mrps18b | 0,59 | 1,51 | 0,0365 | mitochondrial ribosomal protein S18B | CUST_4_PI197795811 | custom |
| 147 | Mrps18b | 0,62 | 1,54 | 0,0254 | mitochondrial ribosomal protein S18B | CUST_5_PI197795811 | custom |
| 155 | RT1-T24-4 | 0,57 | 1,48 | 0,0345 | RT1 class I, T24, gene 4 | CUST_1_PI197795813 | custom |
| 155 | RT1-T24-4 | 0,76 | 1,69 | 0,0206 | RT1 class I, T24, gene 4 | CUST_2_PI197795813 | custom |
| 155 | RT1-T24-4 | 0,72 | 1,65 | 0,0206 | RT1 class I, T24, gene 4 | CUST_3_PI197795813 | custom |
| 156 | RT-BM1 (RT1-S3) | 1,06 | 2,08 | 0,0416 | RT1 class I, RT-BM1 | A_44_P454420 | Agilent |
| 204 | Ubd | 3,19 | 9,13 | 0,0345 | ubiquitin D | A_42_P602724 | Agilent |

### Table 6. Expression profiling results of NKC genes

In **Table 6a,** results for all 43 NKC genes investigated are indicated in their chromosomal order (*Klrg; Pzp* to *Csda*). The expression profiling results of BN skin explant samples exposed to pre-stimulated allogeneic (PVG) lymphocytes in comparison to those exposed to syngeneic (BN) lymphocytes are given. The log2-fold changes and the fold changes in gene expression are shown for every oligonucleotide probe used. The adjusted p-values are indicated. Significant change is defined by p<0.05 and strong change is defined by log2-fold change ≥1 or ≤-1; i.e. fold change ≥2 or ≤0.5. In addition, the identification numbers of the probes on the arrays are given (probe ID) together with the information whether these probes were taken from the Agilent database or custom designed. **Table 6b** contains the information for all NKC genes for which at least one probe indicted a significant alteration of gene expression. In **Table 6c,** the data for those genes are summarized that are considered to be regulated significantly because either at least a single probe indicated a significant (p<0.05) and strong (log2-fold change ≥1 or ≤-1) regulation or at least 50 % of the probes indicated a significant (p<0.05) regulation of gene expression.

**Table 6a**

| **Gene order** | **Gene Symbol** | **log2-Fold Change** | **Fold Change** | **adj.P-value** | **Gene Description** | **Probe ID** | **Probe Design** |
|---|---|---|---|---|---|---|---|
| 1 | Klrg1 | -0,05 | 0,97 | 0,7388 | Rattus norvegicus killer cell lectin-like receptor subfamily G, member 1 (Klrg1) | A_43_P15509 | Agilent |
| 2 | Pzp | 0,20 | 1,15 | 0,1307 | pregnancy zone protein | A_44_P214900 | Agilent |
| 3 | RGD1565709 | nt | | | similar to ovostatin-2 | | |
| 4 | Klrb1a | 0,27 | 1,21 | 0,1808 | killer cell lectin-like receptor subfamily B member 1a | A_42_P598304 | Agilent |
| 5 | Klrb1b | -0,08 | 0,95 | 0,7253 | killer cell lectin-like receptor subfamily B member 1B | A_44_P210547 | Agilent |
| 6 | LOC500331 | -0,10 | 0,93 | 0,5986 | Rattus norvegicus similar to osteoclast inhibitory lectin (LOC500331) | A_44_P311870 | Agilent |
| 7 | RGD1562831 | -0,10 | 0,93 | 0,5986 | similar to osteoclast inhibitory lectin | A_44_P311870 | Agilent |
| 8 | LOC689757 (Clec2d3) | nt | | | similar to osteoclast inhibitory lectin | | |
| 9 | LOC689770 (Clr4, Clec2d4) | nt | | | similar to osteoclast inhibitory lectin | | |
| 10 | Clec2d (Ocil) | -0,33 | 0,80 | 0,3029 | C-type lectin domain family 2, member D (osteoclast inhibitory lectin) | A_44_P137003 | Agilent |
| 11 | Cle2dl1 | nt | | | C-type lectin domain family 2 member d-like 1 | | |
| 12 | LOC689800 | -0,02 | 0,99 | 0,9178 | similar to osteoclast inhibitory lectin | A_44_P391750 | Agilent |
| 13 | Klrblf | nt | | | killer cell lectin-like receptor subfamily B member 1F | | |
| 14 | Clec2h | nt | | | C-type lectin domain family 2, member h | | |
| 15 | Clec2e | nt | | | C-type lectin domain family 2, member E | | |
| 16 | RGD1563148 (Clrb, Clec2d11) | -0,02 | 0,99 | 0,9178 | similar to osteoclast inhibitory lectin | A_44_P391750 | Agilent |
| 17 | Cd69 | 0,69 | 1,61 | 0,1845 | CD69 antigen | A_43_P16166 | Agilent |
| 18 | RGD1564770 | nt | | | similar to CD69 antigen (p60, early T-cell activation antigen) | | |
| 19 | Clec12b | nt | | | C-type lectin domain family 12, member B | | |
| 20 | Clec1b | 0,93 | 1,91 | 0,0500 | C-type lectin domain family 1, member b | A_44_P869774 | Agilent |
| 21 | Clec9a | nt | | | C-type lectin domain family 9, member a | | |
| 22 | Clecla | nt | | | C-type lectin domain family 1, member a | | |
| 23 | Clec7a | nt | | | C-type lectin domain family 7, member a | | |
| 24 | Olr1 | 1,41 | 2,66 | 0,0390 | oxidized low density lipoprotein (lectin-like) receptor 1 | A_44_P377266 | Agilent |
| 25 | LOC689963 | nt | | | hypothetical protein LOC689963 | | |
| 26 | Gabarapl1 | 0,26 | 1,20 | 0,2049 | gamma-aminobutyric acid (GABA(A)) receptor-associated protein-like 1 | CUST_6_PI209816013 | custom |
| 26 | Gabarapl1 | 0,21 | 1,16 | 0,3468 | gamma-aminobutyric acid (GABA(A)) receptor-associated protein-like 1 | CUST_7_PI209816013 | custom |
| 26 | Gabarapl1 | 0,18 | 1,13 | 0,4143 | gamma-aminobutyric acid (GABA(A)) receptor-associated protein-like 1 | CUST_8_PI209816013 | custom |
| 26 | Gabarapl1 | 0,12 | 1,09 | 0,5487 | gamma-aminobutyric acid (GABA(A)) receptor-associated protein-like 1 | CUST_9_PI209816013 | custom |
| 26 | Gabarapl1 | 0,19 | 1,14 | 0,2042 | gamma-aminobutyric acid (GABA(A)) receptor-associated protein-like 1 | CUST_10_PI209816013 | custom |
| 27 | Klre1 | -0,17 | 0,89 | 0,2053 | killer cell lectin-like receptor family E member 1 | A_44_P536089 | Agilent |
| 27 | Klre1 | -0,12 | 0,92 | 0,6467 | killer cell lectin-like receptor family E member 1 | A_43_P16744 | Agilent |
| 28 | Klrd1 | 0,21 | 1,16 | 0,3290 | killer cell lectin-like receptor, subfamily D, member 1, CD94 | A_43_P11543 | Agilent |
| 29 | Klrk1 | -0,02 | 0,99 | 0,9332 | killer cell lectin-like receptor subfamily K, member 1, NKG2D | A_43_P13194 | Agilent |
| 30 | Klrc3 | -0,13 | 0,91 | 0,6020 | killer cell lectin-like receptor subfamily C member 3 | A_44_P255149 | Agilent |
| 31 | Klrc2 | 0,00 | 1,00 | 0,9796 | killer cell lectin-like receptor subfamily C, member 2 | A_43_P11997 | Agilent |
| 32 | Klrc1 | -0,13 | 0,91 | 0,6020 | killer cell lectin-like receptor subfamily C member 1, NKG2A | A_44_P2551491 | Agilent |
| 33 | Klri1 | -0,18 | 0,88 | 0,3321 | killer cell lectin-like receptor family I member 1 | CUST_46_PI209816013 | custom |
| 33 | Klri1 | -0,14 | 0,91 | 0,4180 | killer cell lectin-like receptor family I member 1 | CUST_47_PI209816013 | custom |
| 33 | Klri1 | 0,16 | 1,12 | 0,5780 | killer cell lectin-like receptor family I member 1 | CUST_48_PI209816013 | custom |
| 33 | Klri1 | -0,11 | 0,93 | 0,5043 | killer cell lectin-like receptor family I member 1 | CUST_49_PI209816013 | custom |
| 33 | Klri1 | -0,16 | 0,90 | 0,3331 | killer cell lectin-like receptor family I member 1 | CUST_50_PI209816013 | custom |
| 34 | Klri2 | -0,12 | 0,92 | 0,2434 | killer cell lectin-like receptor family I member 2 | A_44_P590906 | Agilent |
| 35 | Klrh1 | -0,08 | 0,95 | 0,6380 | killer cell lectin-like receptor subfamily H, member 1 | A_43_P13373 | Agilent |
| 36 | LOC690020 | nt | | | similar to killer cell lectin-like receptor, subfamily A, member 17 | | |
| 37 | LOC690045 | 5,56 | 47,18 | 0,0100 | similar to immunoreceptor Ly49si1 | A_43_P10690 | Agilent |
| 38 | Ly49si3 | 3,67 | 12,73 | 0,0180 | immunoreceptor Ly49si3 | CUST_21_PI209816013 | custom |
| 38 | Ly49si3 | 4,82 | 28,25 | 0,0100 | immunoreceptor Ly49si3 | CUST_22_PI209816013 | custom |
| 38 | Ly49si3 | 2,23 | 4,69 | 0,0310 | immunoreceptor Ly49si3 | CUST_23_PI209816013 | custom |
| 38 | Ly49si3 | 1,22 | 2,33 | 0,0411 | immunoreceptor Ly49si3 | CUST_24_PI209816013 | custom |
| 38 | Ly49si3 | 1,79 | 3,46 | 0,0365 | immunoreceptor Ly49si3 | CUST_25_PI209816013 | custom |
| 39 | RGD1561306 | nt | | | similar to immunoreceptor Ly49si3 | | |
| 40 | Ly49si1 | 1,82 | 3,53 | 0,0517 | immunoreceptor Ly49si1 | CUST_56_PI209816013 | custom |
| 40 | Ly49si1 | 2,71 | 6,54 | 0,0325 | immunoreceptor Ly49si1 | CUST_57_PI209816013 | custom |
| 40 | Ly49si1 | 2,18 | 4,53 | 0,0362 | immunoreceptor Ly49si1 | CUST_58_PI209816013 | custom |
| 40 | Ly49si1 | 5,79 | 55,33 | 0,0100 | immunoreceptor Ly49si1 | CUST_59_PI209816013 | custom |
| 40 | Ly49si1 | 4,67 | 25,46 | 0,0100 | immunoreceptor Ly49si1 | CUST_60_PI209816013 | custom |
| 41 | RGD1563110 | nt | | | similar to immunoreceptor Ly49si3 | | |
| 42 | Ly49si2 | 3,64 | 12,47 | 0,0180 | immunoreceptor Ly49si2 | CUST_36_PI209816013 | custom |
| 42 | Ly49si2 | 4,60 | 24,25 | 0,0100 | immunoreceptor Ly49si2 | CUST_37_PI209816013 | custom |
| 42 | Ly49si2 | 4,44 | 21,71 | 0,0100 | immunoreceptor Ly49si2 | CUST_38_PI209816013 | custom |
| 42 | Ly49si2 | 1,76 | 3,39 | 0,0310 | immunoreceptor Ly49si2 | CUST_39_PI209816013 | custom |
| 42 | Ly49si2 | 1,67 | 3,18 | 0,0373 | immunoreceptor Ly49si2 | CUST_40_PI209816013 | custom |
| 43 | LOC690097 | nt | | | similar to immunoreceptor Ly49si3 | | |
| 44 | LOC502907 | nt | | | similar to immunoreceptor Ly49si1 | | |
| 45 | Ly49i9 | 5,27 | 38,59 | 0,0100 | Ly49 inhibitory receptor 9 | CUST_66_PI209816013 | custom |
| 45 | Ly49i9 | 5,13 | 35,02 | 0,0100 | Ly49 inhibitory receptor 9 | CUST_67_PI209816013 | custom |
| 45 | Ly49i9 | 5,15 | 35,51 | 0,0100 | Ly49 inhibitory receptor 9 | CUST_68_PI209816013 | custom |
| 45 | Ly49i9 | 5,29 | 39,12 | 0,0100 | Ly49 inhibitory receptor 9 | CUST_69_PI209816013 | custom |
| 45 | Ly49i9 | 6,60 | 97,01 | 0,0100 | Ly49 inhibitory receptor 9 | CUST_70_PI209816013 | custom |
| 46 | Ly49s5 | 0,01 | 1,01 | 0,9723 | Ly49 stimulatory receptor 5 | CUST_41_PI209816013 | custom |
| 46 | Ly49s5 | -0,07 | 0,95 | 0,5774 | Ly49 stimulatory receptor 5 | CUST_42_PI209816013 | custom |
| 46 | Ly49s5 | -0,11 | 0,93 | 0,3468 | Ly49 stimulatory receptor 5 | CUST_43_PI209816013 | custom |
| 46 | Ly49s5 | -0,03 | 0,98 | 0,8268 | Ly49 stimulatory receptor 5 | CUST_44_PI209816013 | custom |
| 46 | Ly49s5 | -0,08 | 0,95 | 0,5682 | Ly49 stimulatory receptor 5 | CUST_45_PI209816013 | custom |
| 47 | Ly49i5 | -0,06 | 0,96 | 0,8065 | Ly49 inhibitory receptor 5 | CUST_76_PI209816013 | custom |
| 47 | Ly49i5 | 0,07 | 1,05 | 0,5957 | Ly49 inhibitory receptor 5 | CUST_77_PI209816013 | custom |
| 47 | Ly49i5 | -0,01 | 0,99 | 0,9703 | Ly49 inhibitory receptor 5 | CUST_78_PI209816013 | custom |
| 47 | Ly49i5 | 0,00 | 1,00 | 0,9905 | Ly49 inhibitory receptor 5 | CUST_79_PI209816013 | custom |
| 47 | Ly49i5 | 0,05 | 1,04 | 0,6808 | Ly49 inhibitory receptor 5 | CUST_80_PI209816013 | custom |
| 48 | Klra22 | -0,04 | 0,97 | 0,7497 | killer cell lectin-like receptor subfamily A, member 22 | A_44_P266817 | Agilent |
| 49 | Ly49s6 | 0,19 | 1,14 | 0,1938 | Ly49 stimulatory receptor 6 | CUST_26_PI209816013 | custom |
| 49 | Ly49s6 | -0,01 | 0,99 | 0,9561 | Ly49 stimulatory receptor 6 | CUST_27_PI209816013 | custom |
| 49 | Ly49s6 | 0,02 | 1,01 | 0,9047 | Ly49 stimulatory receptor 6 | CUST_28_PI209816013 | custom |
| 49 | Ly49s6 | -0,18 | 0,88 | 0,2611 | Ly49 stimulatory receptor 6 | CUST_29_PI209816013 | custom |
| 49 | Ly49s6 | -0,14 | 0,91 | 0,3529 | Ly49 stimulatory receptor 6 | CUST_30_PI209816013 | custom |
| 50 | Ly49s4 | 0,15 | 1,11 | 0,2267 | Ly49 stimulatory receptor 4 | CUST_61_PI209816013 | custom |
| 50 | Ly49s4 | 0,09 | 1,06 | 0,2799 | Ly49 stimulatory receptor 4 | CUST_62_PI209816013 | custom |
| 50 | Ly49s4 | 0,06 | 1,04 | 0,3468 | Ly49 stimulatory receptor 4 | CUST_63_PI209816013 | custom |
| 50 | Ly49s4 | -0,03 | 0,98 | 0,7923 | Ly49 stimulatory receptor 4 | CUST_64_PI209816013 | custom |
| 50 | Ly49s4 | -0,07 | 0,95 | 0,5814 | Ly49 stimulatory receptor 4 | CUST_65_PI209816013 | custom |
| 51 | Ly49s3 | 0,09 | 1,06 | 0,4744 | Ly-49 stimulatory receptor 3 | A_44_P111662 | Agilent |
| 52 | Ly49i4 | -0,01 | 0,99 | 0,9090 | Ly49 inhibitory receptor 4 | A_44_P250375 | Agilent |
| 53 | Ly49i3 | -0,14 | 0,91 | 0,3217 | Ly49 inhibitory receptor 3 | CUST_81_PI209816013 | custom |
| 53 | Ly49i3 | 0,14 | 1,10 | 0,1803 | Ly49 inhibitory receptor 3 | CUST_82_PI209816013 | custom |
| 53 | Ly49i3 | 1,30 | 2,46 | 0,0325 | Ly49 inhibitory receptor 3 | CUST_84_PI209816013 | custom |
| 53 | Ly49i3 | 3,06 | 8,34 | 0,0180 | Ly49 inhibitory receptor 3 | CUST_85_PI209816013 | custom |
| 53 | Ly49i3 | 0,01 | 1,01 | 0,9333 | Ly49 inhibitory receptor 3 | CUST_83_PI209816013 | custom |
| 54 | Ly49i2 | 0,03 | 1,02 | 0,8446 | Ly49 inhibitory receptor 2 | A_44_P360539 | Agilent |
| 55 | Ly49i6 | 0,05 | 1,04 | 0,7829 | Ly49 inhibitory receptor 6 | CUST_71_PI209816013 | custom |
| 55 | Ly49i6 | 0,18 | 1,13 | 0,1258 | Ly49 inhibitory receptor 6 | CUST_72_PI209816013 | custom |
| 55 | Ly49i6 | 0,14 | 1,10 | 0,4065 | Ly49 inhibitory receptor 6 | CUST_73_PI209816013 | custom |
| 55 | Ly49i6 | -0,07 | 0,95 | 0,6385 | Ly49 inhibitory receptor 6 | CUST_74_PI209816013 | custom |
| 55 | Ly49i6 | 0,05 | 1,04 | 0,7708 | Ly49 inhibitory receptor 6 | CUST_75_PI209816013 | custom |
| 56 | Ly49s8 | 0,01 | 1,01 | 0,9448 | Ly49 stimulatory receptor 8 | CUST_11_PI209816013 | custom |
| 56 | Ly49s8 | 0,69 | 1,61 | 0,0755 | Ly49 stimulatory receptor 8 | CUST_12_PI209816013 | custom |
| 56 | Ly49s8 | 1,12 | 2,17 | 0,1471 | Ly49 stimulatory receptor 8 | CUST_13_PI209816013 | custom |
| 56 | Ly49s8 | 0,55 | 1,46 | 0,0733 | Ly49 stimulatory receptor 8 | CUST_14_PI209816013 | custom |
| 56 | Ly49s8 | 0,66 | 1,58 | 0,1253 | Ly49 stimulatory receptor 8 | CUST_15_PI209816013 | custom |
| 57 | Ly49s7 | 0,12 | 1,09 | 0,3177 | Ly49 stimulatory receptor 7 | A_44_P118897 | Agilent |
| 58 | Klra5 | 1,27 | 2,41 | 0,0984 | killer cell lectin-like receptor, subfamily A, member 5 | CUST_1_PI209816013 | custom |
| 58 | Klra5 | 0,46 | 1,38 | 0,2146 | killer cell lectin-like receptor, subfamily A, member 5 | CUST_2_PI209816013 | custom |
| 58 | Klra5 | 1,00 | 2,00 | 0,0940 | killer cell lectin-like receptor, subfamily A, member 5 | CUST_3_PI209816013 | custom |
| 58 | Klra5 | 0,99 | 1,99 | 0,0844 | killer cell lectin-like receptor, subfamily A, member 5 | CUST_4_PI209816013 | custom |
| 58 | Klra5 | 0,90 | 1,87 | 0,0845 | killer cell lectin-like receptor, subfamily A, member 5 | CUST_5_PI209816013 | custom |
| 59 | Ly49i7 | 0,67 | 1,59 | 0,0395 | immunoreceptor Ly49i7 | A_44_P821875 | Agilent |
| 60 | Ly49i8 | 0,07 | 1,05 | 0,5972 | immunoreceptor Ly49i8 | A_44_P652293 | Agilent |
| 61 | LOC690303 | nt | | | similar to mago-nashi homolog | | |
| 62 | Styk1 | nt | | | serine/threonine/tyrosine kinase 1 | | |
| 63 | Csda | -0,50 | 0,71 | 0,09 | cold shock domain protein A | A_42_P631493 | Agilent |

**Table 6b**

| **Gene order** | **Gene Symbol** | **log2-Fold Change** | **Fold Change** | **adj.P-value** | **Gene Description** | **Probe ID** | **Probe Design** |
|---|---|---|---|---|---|---|---|
| 24 | Olr1 | 1,41 | 2,66 | 0,0390 | oxidized low density lipoprotein (lectin-like) receptor 1 | A_44_P377266 | Agilent |
| 37 | LOC690045 | 5,56 | 47,18 | 0,0100 | similar to immunoreceptor Ly49si1 | A_43_P10690 | Agilent |
| 38 | Ly49si3 | 3,67 | 12,73 | 0,0180 | immunoreceptor Ly49si3 | CUST_21_PI209816013 | custom |
| 38 | Ly49si3 | 4,82 | 28,25 | 0,0100 | immunoreceptor Ly49si3 | CUST_22_PI209816013 | custom |
| 38 | Ly49si3 | 2,23 | 4,69 | 0,0310 | immunoreceptor Ly49si3 | CUST_23_PI209816013 | custom |
| 38 | Ly49si3 | 1,22 | 2,33 | 0,0411 | immunoreceptor Ly49si3 | CUST_24_PI209816013 | custom |
| 38 | Ly49si3 | 1,79 | 3,46 | 0,0365 | immunoreceptor Ly49si3 | CUST_25_PI209816013 | custom |
| 40 | Ly49si1 | 1,82 | 3,53 | 0,0517 | immunoreceptor Ly49si1 | CUST_56_PI209816013 | custom |
| 40 | Ly49si1 | 2,71 | 6,54 | 0,0325 | immunoreceptor Ly49si1 | CUST_57_PI209816013 | custom |
| 40 | Ly49si1 | 2,18 | 4,53 | 0,0362 | immunoreceptor Ly49si1 | CUST_58_PI209816013 | custom |
| 40 | Ly49si1 | 5,79 | 55,33 | 0,0100 | immunoreceptor Ly49si1 | CUST_59_PI209816013 | custom |
| 40 | Ly49si1 | 4,67 | 25,46 | 0,0100 | immunoreceptor Ly49si1 | CUST_60_PI209816013 | custom |
| 42 | Ly49si2 | 3,64 | 12,47 | 0,0180 | immunoreceptor Ly49si2 | CUST_36_PI209816013 | custom |
| 42 | Ly49si2 | 4,60 | 24,25 | 0,0100 | immunoreceptor Ly49si2 | CUST_37_PI209816013 | custom |
| 42 | Ly49si2 | 4,44 | 21,71 | 0,0100 | immunoreceptor Ly49si2 | CUST_38_PI209816013 | custom |
| 42 | Ly49si2 | 1,76 | 3,39 | 0,0310 | immunoreceptor Ly49si2 | CUST_39_PI209816013 | custom |
| 42 | Ly49si2 | 1,67 | 3,18 | 0,0373 | immunoreceptor Ly49si2 | CUST_40_PI209816013 | custom |
| 45 | Ly49i9 | 5,27 | 38,59 | 0,0100 | Ly49 inhibitory receptor 9 | CUST_66_PI209816013 | custom |
| 45 | Ly49i9 | 5,13 | 35,02 | 0,0100 | Ly49 inhibitory receptor 9 | CUST_67_PI209816013 | custom |
| 45 | Ly49i9 | 5,15 | 35,51 | 0,0100 | Ly49 inhibitory receptor 9 | CUST_68_PI209816013 | custom |
| 45 | Ly49i9 | 5,29 | 39,12 | 0,0100 | Ly49 inhibitory receptor 9 | CUST_69_PI209816013 | custom |
| 45 | Ly49i9 | 6,60 | 97,01 | 0,0100 | Ly49 inhibitory receptor 9 | CUST_70_PI209816013 | custom |
| 53 | Ly49i3 | -0,14 | 0,91 | 0,3217 | Ly49 inhibitory receptor 3 | CUST_81_PI209816013 | custom |
| 53 | Ly49i3 | 0,14 | 1,10 | 0,1803 | Ly49 inhibitory receptor 3 | CUST_82_PI209816013 | custom |
| 53 | Ly49i3 | 1,30 | 2,46 | 0,0325 | Ly49 inhibitory receptor 3 | CUST_84_PI209816013 | custom |
| 53 | Ly49i3 | 3,06 | 8,34 | 0,0180 | Ly49 inhibitory receptor 3 | CUST_85_PI209816013 | custom |
| 53 | Ly49i3 | 0,01 | 1,01 | 0,9333 | Ly49 inhibitory receptor 3 | CUST_83_PI209816013 | custom |
| 59 | Ly49i7 | 0,67 | 1,59 | 0,0395 | immunoreceptor Ly49i7 | A_44_P821875 | Agilent |

**Table 6c**

| **Gene order** | **Gene Symbol** | **log2-Fold Change** | **Fold Change** | **adj.P-value** | **Gene Description** | **Probe ID** | **Probe Design** |
|---|---|---|---|---|---|---|---|
| 24 | Olr1 | 1,41 | 2,66 | 0,0390 | oxidized low density lipoprotein (lectin-like) receptor 1 | A_44_P377266 | Agilent |
| 37 | LOC690045 | 5,56 | 47,18 | 0,0100 | similar to immunoreceptor Ly49si1 | A_43_P10690 | Agilent |
| 38 | Ly49si3 | 3,67 | 12,73 | 0,0180 | immunoreceptor Ly49si3 | CUST_21_PI209816013 | custom |
| 38 | Ly49si3 | 4,82 | 28,25 | 0,0100 | immunoreceptor Ly49si3 | CUST_22_PI209816013 | custom |
| 38 | Ly49si3 | 2,23 | 4,69 | 0,0310 | immunoreceptor Ly49si3 | CUST_23_PI209816013 | custom |
| 38 | Ly49si3 | 1,22 | 2,33 | 0,0411 | immunoreceptor Ly49si3 | CUST_24_PI209816013 | custom |
| 38 | Ly49si3 | 1,79 | 3,46 | 0,0365 | immunoreceptor Ly49si3 | CUST_25_PI209816013 | custom |
| 40 | Ly49si1 | 2,71 | 6,54 | 0,0325 | immunoreceptor Ly49si1 | CUST_57_PI209816013 | custom |
| 40 | Ly49si1 | 2,18 | 4,53 | 0,0362 | immunoreceptor Ly49si1 | CUST_58_PI209816013 | custom |
| 40 | Ly49si1 | 5,79 | 55,33 | 0,0100 | immunoreceptor Ly49si1 | CUST_59_PI209816013 | custom |
| 40 | Ly49si1 | 4,67 | 25,46 | 0,0100 | immunoreceptor Ly49si1 | CUST_60_PI209816013 | custom |
| 42 | Ly49si2 | 3,64 | 12,47 | 0,0180 | immunoreceptor Ly49si2 | CUST_36_PI209816013 | custom |
| 42 | Ly49si2 | 4,60 | 24,25 | 0,0100 | immunoreceptor Ly49si2 | CUST_37_PI209816013 | custom |
| 42 | Ly49si2 | 4,44 | 21,71 | 0,0100 | immunoreceptor Ly49si2 | CUST_38_PI209816013 | custom |
| 42 | Ly49si2 | 1,76 | 3,39 | 0,0310 | immunoreceptor Ly49si2 | CUST_39_PI209816013 | custom |
| 42 | Ly49si2 | 1,67 | 3,18 | 0,0373 | immunoreceptor Ly49si2 | CUST_40_PI209816013 | custom |
| 45 | Ly49i9 | 5,27 | 38,59 | 0,0100 | Ly49 inhibitory receptor 9 | CUST_66_PI209816013 | custom |
| 45 | Ly49i9 | 5,13 | 35,02 | 0,0100 | Ly49 inhibitory receptor 9 | CUST_67_PI209816013 | custom |
| 45 | Ly49i9 | 5,15 | 35,51 | 0,0100 | Ly49 inhibitory receptor 9 | CUST_68_PI209816013 | custom |
| 45 | Ly49i9 | 5,29 | 39,12 | 0,0100 | Ly49 inhibitory receptor 9 | CUST_69_PI209816013 | custom |
| 45 | Ly49i9 | 6,60 | 97,01 | 0,0100 | Ly49 inhibitory receptor 9 | CUST_70_PI209816013 | custom |
| 53 | Ly49i3 | 1,30 | 2,46 | 0,0325 | Ly49 inhibitory receptor 3 | CUST_84_PI209816013 | custom |
| 53 | Ly49i3 | 3,06 | 8,34 | 0,0180 | Ly49 inhibitory receptor 3 | CUST_85_PI209816013 | custom |
| 59 | Ly49i7 | 0,67 | 1,59 | 0,0395 | immunoreceptor Ly49i7 | A_44_P821875 | Agilent |

### Table 7. Regulated non-MHC non-NKC genes

The expression profiling results of non-MHC non-NKC genes are given for those genes that were both significantly (p<0.05) and strongly (log2-fold change ≥1 or ≤-1; i.e. fold change ≥2 or ≤0.5) regulated. The log2-fold changes and the fold changes in gene expression are shown. The adjusted p-values are indicated. For 20 of these genes at least two different probes were present on the array. In 8 cases (indicated by gene symbols in bold) the second probe indicated the same strong and significant regulation and in 7 further cases (indicated by gene symbols in italics) the second probe indicated a regulation with borderline amplitude or significance. In 5 cases (indicated by gene symbols in blue font) the results of the two probes for a gene did not confirm each other. Furthermore, the identification numbers of the probes on the arrays are given (probe ID) together with the information whether these probes were taken from the Agilent database or custom designed.

| **Genes** | **Gene Symbol** | **log2-Fold Change** | **Fold Change** | **adj.P-value** | **Gene Description** | **Probe ID** |
|---|---|---|---|---|---|---|
| 106 | NCAM1 | -2,42 | 0,19 | 0,0180 | neural cell adhesion molecule 1 | A_43_P12573 |
| 118 | Pdzrn3 | -2,41 | 0,19 | 0,0100 | PDZ domain containing RING finger 3 | A_42_P481087 |
| 142 | Serpine1 | -2,26 | 0,21 | 0,0149 | serine (or cysteine) peptidase inhibitor, clade E, member 1 | A_42_P758220 |
| 110 | Nfe2l3 | -2,24 | 0,21 | 0,0254 | nuclear factor, erythroid derived 2, like 3 | A_44_P393978 |
| 46 | Drd5 | -2,19 | 0,22 | 0,0336 | dopamine receptor 5 | A_43_P15525 |
| 86 | Lmcd 1 | -1,96 | 0,26 | 0,0206 | LIM and cysteine-rich domains 1 | A_42_P749591 |
| 146 | SNAP25 | -1,92 | 0,26 | 0,0416 | synaptosomal-associated protein 25 | A_43_P12469 |
| 85 | Lgals7 | -1,87 | 0,27 | 0,0149 | lectin, galactose binding, soluble 7 | A_43_P12249 |
| 98 | Lox | -1,80 | 0,29 | 0,0229 | Rattus norvegicus lysyl oxidase (Lox), mRNA [NM_017061] | A_42_P585695 |
| 59 | Grem1 | -1,78 | 0,29 | 0,0315 | gremlin 1 | A_42_P495820 |
| 29 | Cfi | -1,77 | 0,29 | 0,0362 | complement factor I | A_42_P693316 |
| 30 | Chl1 | -1,77 | 0,29 | 0,0100 | cell adhesion molecule with homology to L1CAM | A_44_P1029697 |
| 124 | Postn | -1,73 | 0,30 | 0,0310 | periostin, osteoblast specific factor | A_44_P525235 |
| 132 | Ptprd | -1,68 | 0,31 | 0,0416 | protein tyrosine phosphatase, receptor type, D | A_43_P10925 |
| 115 | Pcdh21 | -1,65 | 0,32 | 0,0325 | protocadherin 21 | A_42_P596050 |
| 137 | Rarres2 | -1,65 | 0,32 | 0,0100 | Rattus norvegicus retinoic acid receptor responder (tazarotene induced) 2 (Rarres2), mRNA [NM_001013427] | A_42_P628853 |
| 19 | Ccl27 | -1,61 | 0,33 | 0,0373 | chemokine (C-C motif) ligand 27 | A_42_P683840 |
| 33 | COL12A1 | -1,57 | 0,34 | 0,0149 | collagen, type XII, alpha 1 | A_43_P15760 |
| 102 | Mme | -1,57 | 0,34 | 0,0278 | membrane metallo endopeptidase | A_43_P11484 |
| 42 | Cxcl12 | -1,56 | 0,34 | 0,0365 | chemokine (C-X-C motif) ligand 12 | A_43_P12144 |
| 152 | Tgfbi | -1,54 | 0,34 | 0,0100 | transforming growth factor, beta induced | A_44_P620106 |
| 9 | Apoe | -1,52 | 0,35 | 0,0206 | apolipoprotein E | A_44_P171440 |
| 15 | C1s | -1,47 | 0,36 | 0,0149 | complement component 1, s subcomponent | A_43_P15364 |
| 34 | Col1a2 | -1,47 | 0,36 | 0,0149 | collagen, type I, alpha 2 | A_43_P12783 |
| 7 | Anp32a | -1,45 | 0,37 | 0,0278 | acidic (leucine-rich) nuclear phosphoprotein 32 family, member A | A_43_P11613 |
| 36 | Col8a1 | -1,40 | 0,38 | 0,0229 | collagen, type VIII, alpha 1 | A_44_P140684 |
| 40 | Cthrc1 | -1,40 | 0,38 | 0,0490 | collagen triple helix repeat containinq 1 | A_44_P144591 |
| 60 | Grin2c | -1,39 | 0,38 | 0,0373 | Rattus norvegicus glutamate receptor, ionotropic, NMDA2C (Grin2c), mRNA [NM_012575] | A_42_P738337 |
| 94 | *LOC684607* | -1,39 | 0,38 | 0,0345 | similar to nuclear receptor binding protein | A_44_P191287 |
| 73 | Igfbp5 | -1,36 | 0,39 | 0,0433 | Rattus norvegicus cDNA clone IMAGE:7110383 [BC087030] | A_44_P264240 |
| 119 | Perp | -1,36 | 0,39 | 0,0267 | PERP, TP53 apoptosis effector | A_42_P768883 |
| 127 | Prom2 | -1,35 | 0,39 | 0,0416 | prominin 2 | A_42_P530761 |
| 31 | Chn1 | -1,32 | 0,40 | 0,0100 | chimerin (chimaerin) 1 | A_43_P15576 |
| 87 | LOC10004492 7 | -1,30 | 0,41 | 0,0449 | similar to TNF-stimulated gene 6 protein | A_43_P16110 |
| 35 | Col5a3 | -1,28 | 0,41 | 0,0395 | collagen, type V, alpha 3 | A_44_P197290 |
| 165 | Wisp1 | -1,27 | 0,41 | 0,0100 | WNT1 inducible signaling pathway protein 1 | A_42_P816427 |
| 37 | Cpe | -1,25 | 0,42 | 0,0100 | carboxypeptidase E | A_42_P708169 |
| 42 | Cxcl12 | -1,24 | 0,42 | 0,0365 | chemokine (C-X-C motif) ligand 12 | A_44_P337351 |
| 108 | Nell2 | -1,23 | 0,43 | 0,0365 | Rattus norvegicus nel-like 2 homolog (chicken) (Nell2), mRNA [NM_031070] | A_43_P12500 |
| 151 | *Tcfap2b* | -1,21 | 0,43 | 0,0100 | transcription factor AP-2 beta | A_42_P463781 |
| 3 | Adcy2 | -1,20 | 0,44 | 0,0449 | adenylate cyclase 2 | A_43_P15311 |
| 32 | Clu | -1,20 | 0,44 | 0,0310 | clusterin | A_44_P311126 |
| 55 | Fst | -1,20 | 0,44 | 0,0298 | follistatin | A_44_P108588 |
| 56 | Fzd1 | -1,20 | 0,44 | 0,0149 | frizzled homolog 1 (Drosophila) | A_44_P170527 |
| 1 | Abcc1 | -1,18 | 0,44 | 0,0310 | ATP-binding cassette, sub-family C (CFTR/MRP), member 1 | A_44_P252417 |
| 81 | Itpr3 | -1,18 | 0,44 | 0,0100 | inositol 1,4,5-triphosphate receptor 3 | A_42_P572461 |
| 126 | Prkcdbp | -1,18 | 0,44 | 0,0180 | protein kinase C, delta binding protein | A_42_P736812 |
| 150 | Tacstd2 | -1,18 | 0,44 | 0,0206 | tumor-associated calcium signal transducer 2 | A_42_P468712 |
| 10 | Asam | -1,15 | 0,45 | 0,0298 | adipocyte-specific adhesion molecule | A_44_P292495 |
| 44 | Dclk1 | -1,15 | 0,45 | 0,0267 | doublecortin-like kinase 1 | A_42_P787216 |
| 135 | Ptprz1 | -1,15 | 0,45 | 0,0100 | protein tyrosine phosphatase, receptor type Z, polypeptide 1 | A_42_P475885 |
| 47 | EGR1 | -1,14 | 0,45 | 0,0149 | early growth response 1 | A_42_P623792 |
| 58 | Gpr98 | -1,14 | 0,45 | 0,0206 | G protein-coupled receptor 98 | A_42_P478080 |
| 84 | Lgals1 | -1,13 | 0,46 | 0,0229 | lectin, galactose binding, soluble 1 | A_42_P759159 |
| 111 | *Nfib* | -1,13 | 0,46 | 0,0533 | nuclear factor I/B | A_42_P752916 |
| 4 | Adcy8 | -1,12 | 0,46 | 0,0149 | adenylate cyclase 8 | A_42_P466362 |
| 27 | Cdh5_predict ed | -1,12 | 0,46 | 0,0254 | PREDICTED: Rattus norvegicus cadherin 5 (predicted) (Cdh5_predicted), mRNA [XM_226213] | A_44_P121658 |
| 44 | Dclk1 | -1,12 | 0,46 | 0,0278 | doublecortin-like kinase 1 | A_44_P172645 |
| 154 | *Thbs4* | -1,12 | 0,46 | 0,0466 | thrombospondin 4 | A_44_P337311 |
| 69 | Htra1 | -1,11 | 0,46 | 0,0100 | HtrA serine peptidase 1 | A_43_P12648 |
| 133 | Ptprf | -1,10 | 0,47 | 0,0345 | protein tyrosine phosphatase, receptor type, F | A_43_P11993 |
| 104 | Mtss1_predict ed | -1,09 | 0,47 | 0,0325 | PREDICTED: Rattus norvegicus metastasis suppressor 1 (predicted) (Mtss1_predicted), mRNA [XM_001064860] | A_44_P554679 |
| 144 | Serpinf1 | -1,09 | 0,47 | 0,0206 | serine (or cysteine) peptidase inhibitor, clade F, member 1 | A_42_P709525 |
| 14 | C1qtnf7 | -1,08 | 0,47 | 0,0310 | C1q and tumor necrosis factor related protein 7 | A_44_P248172 |
| 51 | Fam89a | -1,08 | 0,47 | 0,0481 | family with sequence similarity 89, member A | A_42_P619403 |
| 143 | Serpine2 | -1,08 | 0,47 | 0,0481 | serine (or cysteine) peptidase inhibitor, clade E, member 2 | A_43_P15697 |
| 61 | Gsn | -1,06 | 0,48 | 0,0267 | gelsolin | A_44_P1014163 |
| 101 | Med13l | -1,06 | 0,48 | 0,0229 | mediator complex subunit 13-like | A_44_P169863 |
| 113 | Ntrk2 | -1,06 | 0,48 | 0,0100 | neurotrophic tyrosine kinase, receptor, type 2 | A_42_P538400 |
| 117 | Pdgfrb | -1,05 | 0,48 | 0,0206 | platelet derived growth factor receptor, beta polypeptide | A_43_P15740 |
| 149 | Sulf1 | -1,05 | 0,48 | 0,0325 | sulfatase 1 | A_43_P13252 |
| 39 | Ctqf | -1,04 | 0,49 | 0,0466 | connective tissue growth factor | A_42_P484738 |
| 50 | Ercc5 | -1,04 | 0,49 | 0,0315 | excision repair cross-complementing rodent repair deficiency, complementation group 5 | A_44_P1019654 |
| 100 | Ltbp1 | -1,04 | 0,49 | 0,0180 | latent transforming growth factor beta binding protein 1 | A_43_P14871 |
| 114 | Papss2 | -1,04 | 0,49 | 0,0395 | 3'-phosphoadenosine 5'-phosphosulfate synthase 2 | A_42_P513050 |
| 111 | *Nfib* | -1,03 | 0,49 | 0,0457 | nuclear factor I/B | A_43_P15686 |
| 68 | Hrh3 | -1,02 | 0,49 | 0,0395 | histamine receptor H3 | A_43_P15338 |
| 23 | Ccnd1 | -1,01 | 0,50 | 0,0100 | cyclin D1 | A_44_P189299 |
| 94 | *LOC684607* | -0,88 | 0,54 | 0,0634 | similar to nuclear receptor binding protein | A_44_P250983 |
| 154 | *Thbs4* | -0,87 | 0,55 | 0,0648 | thrombospondin 4 | A_43_P15768 |
| 151 | *Tcfap2b* | -0,85 | 0,55 | 0,0345 | transcription factor AP-2 beta | A_43_P18397 |
| 113 | Ntrk2 | -0,37 | 0,77 | 0,2827 | neurotrophic tyrosine kinase, receptor, type 2 | A_42_P631184 |
| 101 | Med13l | -0,32 | 0,80 | 0,1024 | mediator complex subunit 13-like | A_44_P473186 |
| 166 | Wnt7a | -0,21 | 0,86 | 0,2198 | wingless-related MMTV integration site 7A | A_44_P623953 |
| 114 | Papss2 | 0,02 | 1,01 | 0,9620 | 3'-phosphoadenosine 5'-phosphosulfate synthase 2 | A_44_P119160 |
| 66 | Hmha1 | 0,22 | 1,16 | 0,1958 | histocompatibility (minor) HA-1 | A_43_P20339 |
| 168 | Zfp36 | 0,54 | 1,45 | 0,2006 | zinc finger protein 36 | A_44_P435596 |
| 18 | Ccl1 | 0,60 | 1,52 | 0,0791 | chemokine (C-C motif) ligand 1 | CUST_51_PI24087 2834 |
| 76 | *Il1rn* | 0,73 | 1,66 | 0,0100 | interleukin 1 receptor antagonist | A_43_P15503 |
| 163 | Tyrobp | 0,91 | 1,88 | 0,0229 | Tyro protein tyrosine kinase binding protein | A_44_P526676 |
| 22 | Ccl9 | 1,00 | 2,00 | 0,0254 | chemokine (C-C motif) ligand 9 | A_43_P22206 |
| 153 | Tgm2 | 1,00 | 2,00 | 0,0254 | transglutaminase 2, C polypeptide | A_44_P1007347 |
| 121 | Plaur | 1,02 | 2,03 | 0,0229 | plasminogen activator, urokinase receptor | A_44_P468141 |
| 128 | Pstpip1 | 1,07 | 2,10 | 0,0325 | proline-serine-threonine phosphatase-interacting protein 1 | A_44_P180717 |
| 64 | Hcls1 | 1,09 | 2,13 | 0,0325 | hematopoietic cell specific Lyn substrate 1 | A_43_P21322 |
| 160 | Treml1 | 1,10 | 2,14 | 0,0373 | triggering receptor expressed on myeloid cells-like 1 | A_44_P798023 |
| 18 | Ccl1 | 1,12 | 2,17 | 0,0298 | chemokine (C-C motif) ligand 1 | CUST_52_PI24087 2834 |
| 77 | *Il2rb* | 1,13 | 2,19 | 0,0815 | interleukin 2 receptor, beta chain | A_44_P265709 |
| 26 | Cd8b1 | 1,16 | 2,23 | 0,0229 | CD8b molecule | A_42_P480723 |
| 79 | Itgax | 1,16 | 2,23 | 0,0457 | Rattus norvegicus integrin alpha X (Itgax), mRNA [NM_031691] | A_42_P700646 |
| 20 | *Ccl3* | 1,17 | 2,25 | 0,0762 | chemokine (C-C motif) ligand 3 | A_42_P714311 |
| 112 | Nfkbia | 1,18 | 2,27 | 0,0180 | nuclear factor of kappa light polypeptide gene enhancer in B-cells inhibitor, alpha | A_42_P544487 |
| 122 | Plk3 | 1,18 | 2,27 | 0,0315 | polo-like kinase 3 (Drosophila) | A_44_P135224 |
| 147 | Snx10 | 1,18 | 2,27 | 0,0345 | sorting nexin 10 | A_43_P16967 |
| 49 | Epha2 | 1,20 | 2,30 | 0,0278 | Eph receptor A2 | A_42_P569711 |
| 145 | Slfn2 | 1,20 | 2,30 | 0,0206 | schlafen 2 | A_44_P469113 |
| 120 | Pik3ap1 | 1,21 | 2,31 | 0,0278 | phosphoinositide-3-kinase adaptor protein 1 | A_43_P21121 |
| 18 | Ccl1 | 1,23 | 2,35 | 0,0378 | chemokine (C-C motif) ligand 1 | CUST_53_PI24087 2834 |
| 166 | Wnt7a | 1,24 | 2,36 | 0,0336 | wingless-related MMTV integration site 7A | A_44_P135238 |
| 78 | Itgam | 1,25 | 2,38 | 0,0424 | integrin alpha M | A_43_P15993 |
| 11 | AW141130 | 1,27 | 2,41 | 0,0254 | EST291162 Normalized rat brain, Bento Soares Rattus sp. cDNA clone RGIBD16 5' end similar to interleukin-3 receptor B subunit, mRNA sequence [AW141130] | A_44_P635423 |
| 161 | Trib3 | 1,27 | 2,41 | 0,0278 | tribbles homolog 3 (Drosophila) | A_42_P543774 |
| 5 | Adipor2 | 1,31 | 2,48 | 0,0254 | adiponectin receptor 2 | A_44_P1013376 |
| 5 | Adipor2 | 1,31 | 2,48 | 0,0100 | adiponectin receptor 2 | A_42_P523357 |
| 82 | L37967 | 1,32 | 2,50 | 0,0345 | RATTCRAL Rattus norvegicus T-cell receptor alpha-chain mRNA [L37967] | A_43_P16248 |
| 134 | Ptprj | 1,37 | 2,58 | 0,0378 | protein tyrosine phosphatase, receptor type, J | A_43_P15275 |
| 18 | Ccl1 | 1,38 | 2,60 | 0,0325 | chemokine (C-C motif) ligand 1 | CUST_54_PI24087 2834 |
| 2 | Adcy10 | 1,40 | 2,64 | 0,0325 | adenylate cyclase 10 | A_42_P460021 |
| 38 | Csf2 | 1,40 | 2,64 | 0,0401 | colony stimulating factor 2 (granulocyte-macrophage) | A_43_P16294 |
| 71 | Ifitm1 | 1,43 | 2,69 | 0,0180 | interferon induced transmembrane protein 1 | A_42_P676304 |
| 138 | RGD1561143 | 1,45 | 2,73 | 0,0310 | similar to cell surface receptor FDFACT | A_44_P182601 |
| 92 | LOC681069 | 1,46 | 2,75 | 0,0401 | similar to paired immunoglobinlike type 2 receptor beta | A_44_P330565 |
| 168 | Zfp36 | 1,47 | 2,77 | 0,0378 | zinc finger protein 36 | A_42_P648055 |
| 48 | Emb | 1,49 | 2,81 | 0,0315 | embigin | A_44_P304220 |
| 99 | Lpxn | 1,49 | 2,81 | 0,0278 | leupaxin | A_43_P23014 |
| 66 | Hmha1 | 1,51 | 2,85 | 0,0365 | histocompatibility (minor) HA-1 | A_44_P992516 |
| 141 | Rhoh | 1,52 | 2,87 | 0,0378 | ras homolog gene family, member H | A_43_P23152 |
| 18 | Ccl1 | 1,53 | 2,89 | 0,0390 | chemokine (C-C motif) ligand 1 | CUST_55_PI24087 2834 |
| 76 | *Il1rn* | 1,56 | 2,95 | 0,0100 | interleukin 1 receptor antagonist | A_44_P462661 |
| 6 | AF216218 | 1,57 | 2,97 | 0,0254 | AF216218 Rattus norvegicus orphanin FQ receptor gene (OFQR), complete cds, alternatively spliced [AF216218] | A_44_P442838 |
| 67 | Hmox1 | 1,57 | 2,97 | 0,0100 | heme oxygenase (decycling) 1 | A_42_P652275 |
| 157 | Tnfsf13 | 1,57 | 2,97 | 0,0278 | tumor necrosis factor (ligand) superfamily, member 13 | A_42_P773636 |
| 93 | LOC683463 | 1,58 | 2,99 | 0,0325 | similar to paired-Ig-like receptor B | A_42_P841620 |
| 70 | Ifi47 | 1,65 | 3,14 | 0,0310 | interferon gamma inducible protein 47 | A_44_P174992 |
| 25 | Cd83 | 1,68 | 3,20 | 0,0481 | CD83 antigen | A_42_P767128 |
| 65 | Hk3 | 1,70 | 3,25 | 0,0206 | hexokinase 3 | A_44_P114207 |
| 45 | Dok3 | 1,72 | 3,29 | 0,0325 | docking protein 3 | A_42_P468452 |
| 28 | Ceacam10 | 1,74 | 3,34 | 0,0254 | CEA-related cell adhesion molecule 10 | A_43_P13426 |
| 83 | Lcp2 | 1,78 | 3,43 | 0,0310 | lymphocyte cytosolic protein 2 | A_42_P671389 |
| 116 | Pcsk1 | 1,80 | 3,48 | 0,0395 | proprotein convertase subtilisin/kexin type 1 | A_42_P570848 |
| 16 | C5ar1 | 1,83 | 3,56 | 0,0298 | complement component 5a receptor 1 | A_42_P572521 |
| 129 | Ptger2 | 1,85 | 3,61 | 0,0278 | prostaglandin E receptor 2, subtype EP2 | A_43_P12508 |
| 77 | *Il2rb* | 1,86 | 3,63 | 0,0365 | interleukin 2 receptor, beta chain | A_42_P555801 |
| 53 | Fcqr3 | 1,89 | 3,71 | 0,0100 | Fc receptor, IgG, low affinity III | A_44_P168405 |
| 164 | Vav1 | 1,89 | 3,71 | 0,0229 | vav 1 oncogene | A_42_P572413 |
| 17 | Card11 | 1,93 | 3,81 | 0,0206 | caspase recruitment domain family, member 11 | A_44_P421727 |
| 158 | Trem1 | 1,94 | 3,84 | 0,0206 | triggering receptor expressed on myeloid cells 1 | A_44_P354415 |
| 155 | Tlr2 | 1,95 | 3,86 | 0,0315 | toll-like receptor 2 | A_43_P19763 |
| 136 | Rarres1 | 2,00 | 4,00 | 0,0345 | retinoic acid receptor responder (tazarotene induced) 1 | A_42_P528691 |
| 89 | LOC498277 | 2,05 | 4,14 | 0,0100 | similar to Low affinity immunoglobulin gamma Fc region receptor III precursor (IgG Fc receptor III) (Fc-gamma RIII) (FcRIII) | A_44_P482476 |
| 96 | LOC685157 | 2,05 | 4,14 | 0,0180 | similar to paired immunoglobin-like type 2 receptor beta | A_44_P745407 |
| 12 | Batf | 2,06 | 4,17 | 0,0310 | basic leucine zipper transcription factor, ATF-like | A_42_P624111 |
| 13 | Bcl2a1d | 2,08 | 4,23 | 0,0254 | B-cell leukemia/lymphoma 2 related protein A1d | A_43_P13182 |
| 20 | *Ccl3* | 2,11 | 4,32 | 0,0278 | chemokine (C-C motif) ligand 3 | A_43_P11666 |
| 163 | Tyrobp | 2,14 | 4,41 | 0,0100 | Tyro protein tyrosine kinase bindinq protein | A_42_P807697 |
| 8 | Apob48r | 2,15 | 4,44 | 0,0373 | apolipoprotein B48 receptor | A_44_P194387 |
| 57 | Gpnmb | 2,19 | 4,56 | 0,0149 | glycoprotein (transmembrane) nmb | A_42_P517381 |
| 43 | Cxcl2 | 2,21 | 4,63 | 0,0206 | chemokine (C-X-C motif) ligand 2 | A_43_P12885 |
| 103 | Msr1 | 2,22 | 4,66 | 0,0373 | macrophage scavenger receptor 1 | A_44_P928825 |
| 130 | Ptpn7 | 2,26 | 4,79 | 0,0206 | protein tyrosine phosphatase, non-receptor type 7 | A_42_P653257 |
| 91 | LOC680910 | 2,31 | 4,96 | 0,0180 | similar to paired immunoglobin-like type 2 receptor beta | A_44_P187246 |
| 162 | Trpv2 | 2,32 | 4,99 | 0,0100 | transient receptor potential cation channel, subfamily V, member 2 | A_42_P816020 |
| 156 | Tnfaip8l2 | 2,33 | 5,03 | 0,0100 | tumor necrosis factor, alpha-induced protein 8-like 2 | A_43_P20022 |
| 74 | Igsf6 | 2,34 | 5,06 | 0,0395 | immunoglobulin superfamily, member 6 | A_42_P588738 |
| 140 | Rgs1 | 2,36 | 5,13 | 0,0373 | regulator of G-protein signaling 1 | A_43_P16318 |
| 107 | Ncf1 | 2,40 | 5,28 | 0,0100 | neutrophil cytosolic factor 1 | A_44_P298049 |
| 75 | Il1b | 2,43 | 5,39 | 0,0395 | interleukin 1 beta | A_43_P14911 |
| 139 | RGD1561778 | 2,55 | 5,86 | 0,0206 | similar to dendritic cell-derived immunoglobulin(Ig)-like receptor 1, DIgR1 - mouse | A_44_P176053 |
| 80 | Itgb2 | 2,56 | 5,90 | 0,0100 | integrin beta 2 | A_42_P591344 |
| 91 | LOC680910 | 2,59 | 6,02 | 0,0229 | similar to paired immunoglobin-like type 2 receptor beta | A_44_P463899 |
| 72 | Igf1 | 2,61 | 6,11 | 0,0100 | insulin-like growth factor 1 | A_44_P126021 |
| 90 | LOC498277 | 2,61 | 6,11 | 0,0100 | similar to Low affinity immunoglobulin gamma Fc region receptor III precursor (IgG Fc receptor III) (Fc-gamma RIII) (FcRIII) | A_43_P12955 |
| 52 | Fcqr2b | 2,62 | 6,15 | 0,0100 | Fc receptor, IgG, low affinity IIb | A_42_P735417 |
| 148 | Spic | 2,68 | 6,41 | 0,0278 | Spi-C transcription factor (Spi-1/PU.1 related) | A_42_P526140 |
| 109 | Nfe2 | 2,74 | 6,68 | 0,0149 | nuclear factor, erythroid derived 2 | A_42_P464736 |
| 125 | Prg4 | 2,74 | 6,68 | 0,0149 | proteoglycan 4 (megakaryocyte stimulating factor, articular superficial zone protein) | A_43_P14460 |
| 123 | Plscr1 | 2,83 | 7,11 | 0,0100 | phospholipid scramblase 1 | A_44_P1025102 |
| 88 | LOC10004847 9 | 2,97 | 7,84 | 0,0373 | one cut domain, family member 1 | A_42_P701060 |
| 62 | Gzmc | 3,11 | 8,63 | 0,0373 | granzyme C | A_42_P774527 |
| 41 | Ctss | 3,15 | 8,88 | 0,0100 | cathepsin S | A_44_P1004731 |
| 72 | Igf1 | 3,23 | 9,38 | 0,0100 | insulin-like growth factor 1 | A_44_P366723 |
| 24 | Cd36 | 3,57 | 11,88 | 0,0100 | CD36 antigen | A_43_P12588 |
| 21 | Ccl6 | 3,71 | 13,09 | 0,0100 | Rattus norvegicus chemokine (C-C motif) ligand 6 (Ccl6), mRNA [NM_001004202] | A_43_P16707 |
| 159 | Trem2 | 3,78 | 13,74 | 0,0100 | triggering receptor expressed on myeloid cells 2 | A_42_P512838 |
| 63 | Hck | 3,87 | 14,62 | 0,0100 | hemopoietic cell kinase | A_43_P11749 |
| 167 | XM_226926 | 3,92 | 15,14 | 0,0149 | Rattus norvegicus similar to protein tyrosine phosphatase, non-receptor type substrate; brain immunological-like with tyrosine-based motifs (LOC310212), mRNA [XM-226926] | A_44_P375194 |
| 22 | Ccl9 | 4,16 | 17,88 | 0,0100 | chemokine (C-C motif) ligand 9 | A_42_P560084 |
| 105 | Nat8 | 5,14 | 35,26 | 0,0100 | Rattus norvegicus endogenous retrovirus mRNA, partial sequence [AY212271] | A_44_P594411 |
| 54 | Fcgr3a | 5,24 | 37,79 | 0,0100 | Fc fragment of IgG, low affinity IIIa, receptor | A_42_P798429 |
| 131 | Ptpns1l3 | 6,36 | 82,14 | 0,0100 | protein tyrosine phosphatase, non-receptor type substrate 1-like 3 | A_44_P248248 |
| 95 | LOC685020 | 8,18 | 290,02 | 0,0100 | paired immunoglobin-like type 2 receptor alpha | A_44_P715240 |

**Table 8. Primer sequences used for mRNA expression analysis**

| | **Primer sequence 5'-3'¹** | **Amplicon (bp)** | **Proximity to poly-A (bp)** | **Efficiency coefficient (*E*)²** |
|---|---|---|---|---|
| *RT1-A2* | F: TCCCTCCCTGCTACCCTGAG (SEQ ID NO: 48) | 103 | 105 | 1.93 |
| | R: GCCATCCACACTTGGGTCAA (SEQ ID NO: 49) | | | |
| *RT1-DMb* | F: TCAAATCTGCCTCGGGTGTTT (SEQ ID NO: 50) | 80 | 53 | 1.87 |
| | R: GACAAGGTGGGGCTTTCAGG (SEQ ID NO: 51) | | | |
| *Psmb8* | F: CACTGCTGGGCAGACATCCT (SEQ ID NO: 52) | 109 | 91 | 1.92 |
| | R: GCTTTGTCTCCAGCCCAGGT (SEQ ID NO: 53) | | | |
| *Ly6g6e* | F: CCCAGGCAAAGGGACAGAAG (SEQ ID NO: 54) | 87 | 151 | 1.97 |
| | R:TGAGACCCTCAGGCACCAAG (SEQ ID NO: 55) | | | |
| *Aif1* | F: TCCCCCAGCCAAGAAAGCTA (SEQ ID NO: 56) | 99 | 51 | 1.86 |
| | R: TCTTTTCCCATGCTGCTGTCA (SEQ ID NO: 57) | | | |
| *Lst1* | F: GGGCAGGAGCTCCACTACG (SEQ ID NO: 58) | 118 | 20 | 1.89 |
| | R: CGATGCAGGCATAGTCAGTGC (SEQ ID NO: 59) | | | |
| *RT1-CE3* | F: TGTCGTCCTTGGAGCCATCT (SEQ ID NO: 60) R:TCCTCACAACAGGCACCAGA (SEQ ID NO: 61) | 62 | 106 | 1.91 |
| *RT1-CE10* | F: ACACAGGTGGGGAAGGAGGA (SEQ ID NO: 62) | 82 | 10 | 1.94 |
| | R : CAATCTGGGAGGGACACATCAG (SEQ ID NO: 63) | | | |
| *RT-BM1 (RT1-53)* | F: GCAGCTATGCTCATGTTCTAGGC (SEQ ID NO: 64) | 62 | 7 | 1.89 |
| | R:TGCCTTCTGAGGCCAGTCAG (SEQ ID NO: 65) | | | |
| *Ubd* | F: TGGGGTGATGAGAAGCTCAAAA (SEQ ID NO: 66) | 105 | 7 | 1.92 |
| | R: CCCCACCTCAAATCTTTATTTCATTC (SEQ ID NO: 67) | | | |
| *Olr1* | F: GGAAGTCAGAAGAGGGCATGG (SEQ ID NO: 68) | 89 | 271 | 1.90 |
| | R:TCCTGGGTTCAATTTCCAGAGT (SEQ ID NO: 69) | | | |
| *Ly49si1* | F: TGGCCAATCTGAATTTTCCTTG (SEQ ID NO: 70) | 115 | 36 | 1.84 |
| | R:ACATGGGAAGGGGTTCATGC (SEQ ID NO: 71) | | | |
| *Ly49i9* | F: GGGACTTGGCAACCTCAGGA (SEQ ID NO: 72) | 110 | 179 | 1.88 |
| | R:TTGGAACATCTGCACAATGGAA (SEQ ID NO: 73) | | | |
| *Cd3z* | F: AGTGCCTGCTGGGATTTAGC (SEQ ID NO: 74) | 118 | 50 | 1.93 |
| | R: CATCCATGGTCACAGGCACTT (SEQ ID NO: 75) | | | |
| *B2m* | F: GAGCAGGTTGCTCCACAGGT (SEQ ID NO: 76) | 128 | 246 | 1.94 |
| | R:CAAGCTTTGAGTGCAAGAGATTGA (SEQ ID NO: 77) | | | |

| | | | | |
|---|---|---|---|---|
| ¹ F: forward primer, R: reverse primer ² The real-time PCR efficiency coefficient *(E)* of one cycle in the exponential phase was calculated according to the equation: *E*= 10^{[-1/slope of standard curve]} | | | | |

**Table 9**

| **Gene** | **Gene description** | **Tested organism** | **Log2-fold change (rat microarray)** | **Tested organism** | **Concordance rate first 3 human skin explants** | **Concordance rate in further human skin explants** | **Log2-fold change (human data)** |
|---|---|---|---|---|---|---|---|
| Ctss | cathepsin S | Rattus novegicus | 3,15 | Homo sapiens | 3/3 | 7/9 | -1,25 |
| Pbx2 | Pre-B-cell leukemia homeobox 2 | Rattus novegicus | 0,33 | Homo sapiens | 1/3 | 7/9 | -1,5 |
| Grem1 | Gremlin -1 inhibitor in the TGF beta signaling pathway | Rattus novegicus | -1,78 | Homo sapiens | 2/3 | 6/9 | -3 |
| Ly6g6e | lymphocyte antigen 6 complex, locus G6E | Rattus novegicus | -1,43 | Homo sapiens | 0/3 | 6/9 | -2,25 |
| Spr1 | psoriasis susceptibility 1 candidate 2 (human) | Rattus novegicus | 1,45 | Homo sapiens | 1/3 | 5/5 | -1,25 |
| Msr1 | macrophage scavenger protein | Rattus novegicus | 2,22 | Homo sapiens | 1/3 | 4/9 | 1,5 |
| Spic | Spi-C transcription factor | Rattus novegicus | 2,68 | Homo sapiens | 0/3 | 4/9 | -2 |
| Nfe2 | nuclear factor, erythroid derived 2 | Rattus novegicus | 2,74 | Homo sapiens | 0/3 | 3/9 | -1,5 |
| Tnfaip8l2 | tumor necrosis factor, alpha-induced protein 8-like 2 | Rattus novegicus | 2,33 | Homo sapiens | 3/3 | 3/9 | -1,5 |
| Ier3 | Immediate early response 3 | Rattus novegicus | 0,87 | Homo sapiens | 1/3 | 2/9 | -1,5 |
| Pik3ap1 | phosphoinositide-3-kinase adaptor protein 1 | Rattus novegicus | 1,21 | Homo sapiens | 3/3 | 1/9 | 1 |
| Pstpip1 | proline-serine-threonine phospha-tase-interacting protein 1 | Rattus novegicus | 1,07 | Homo sapiens | 3/3 | 1/9 | 2 |
| Ubd | ubiquitin D | Rattus novegicus | 3,19 | Homo sapiens | 3/3 | 4/9 | 1,25 |
| C2 | complement component 2 | Rattus novegicus | 1,22 | Homo sapiens | 2/3 | 1/9 | 1 |
| Lst1 | leukocyte specific transcript 1 | Rattus novegicus | 3,32 | Homo sapiens | 1/3 | 5/9 | -1,25 |
| Aif1 | allograft inflammatory factor 1 | Rattus novegicus | 2,83 | Homo sapiens | 1/3 | 3/9 | 1,25 |
| C1QTNF7 | C1q and TNF related protein 7 | Rattus novegicus | -1,08 | Homo sapiens | 0/3 | 8/9 | -2 |
| MME | Membrane metallo-endopeptidase expressed by B and T cells upon induction of apotosis | Rattus novegicus | -1,75 | Homo sapiens | 3/3 | 6/9 | -2 |
| IGFBP5 | Insulin-like growth factor-binding protein 5 | Rattus novegicus | -1,36 | Homo sapiens | 1/3 | 6/9 | -2 |
| CARD11 | apoptosis and scaffolding | Rattus novegicus | 1,93 | Homo sapiens | 3/3 | 6/9 | -2 |

**Table 10**

| **Gene** | **RefSeq (human)** | **Probe ID Applied Biosystems TLDA card (human)** | **Probe ID Agilent microarray chip (rat)** | **RefSeq (rat)** | **Entrez Ge-neID (rat)** | **Sequence (Agilent microarray chip, rat)** |
|---|---|---|---|---|---|---|
| Ctss | N_004079.3 | CTSS-Hs00175403_m1 | A_44_P1004731 | NM_017320 | ID:50654 | CTGGCTTACAGCTTGTTTGTTTTATAACTTTAC- CTCTCTCTGAAAAGTCTGTAAGCAAGG (SEQ ID NO: 26) |
| Pbx2 | NM_002586.4 | PBX2-Hs00855025_s1 | A_42_P592157 | NM_001002828 | ID:406164 | AAAGCTTTCGGTTTTGTTTTTTAAACTGTTTGCAGAGTGGAGAA- GATCGATCAGGAAGGG (SEQ ID NO: 27) |
| Grem1 | NM_013372.5 | GREM1-Hs00171951_m1 | A_42_P495820 | N_019282 | ID:50566 | ATTATGCAGGCTATGACGGAACTACTACCTTGC- TATGGATGAGGGTTGGGCAGGATTTAA (SEQ ID NO: 28) |
| Ly6g6e | NR_003673 | LY6G6E-Hs00225567_m1 | CUST_1_PI195698 246 | NM_027366 | ID:406866 | GTCTCAAGAACAGAGGGCTACCTTGGGGAGCCATAAAGAGTG- TATTTAATAAAACGGGCT (SEQ ID NO: 29) |
| Spr1 | NM_014069.2 | PSORS1C2-Hs00204152_m1 | A_66_P100662 | NM_020576 | ID:57390 | TTTGTGGTCCCTGTTCAGTCATTATGTTGTCCCTTCGCTTCTCTT- GATCAGCAGAAAGCA (SEQ ID NO: 31) |
| Msr1 | NM_138715.2 | MSR1-Hs00234007_m1 | A_44_P928825 | XM_573919 | ID:498638 | GAACGTGTGCACAAAGTATCAGCAGAAATCCAGTCTGTGAAA- GAAGAACAAGAGCATGTG (SEQ ID NO: 32) |
| Spic | NM_152323.1 | SPIC-Hs00951473_g1 | A_42_P526140 | NM_011461 | ID:20728 | CTCAGTGTCCGTGAATTGGGTATCCAAGAACATCCTGAA- GCCAGAATGTCTTCTCAGAAA (SEQ ID NO: 33) |
| Nfe2 | N_001136023 | NFE2-Hs00232351_m1 | A_42_P464736 | NM_001012224 | ID:366998 | AGGCTGAGTTCTCCAGACCAAAAGACCATTTGGAAGTTCAAA- GATGTATTTGAGGTTTGC (SEQ ID NO: 34) |
| Tnfaip8l2 | NM_024575.3 | TNFAIP8L2-Hs00226190_m1 | A_43_P20022 | N_027206 | ID:310663 | AGCTCTGAGGCTCCTGAGCTCAGCACACTGGACTTTGG- CAAAATGACTGACCGGGAAACG (SEQ ID NO: 35) |
| Ier3 | NM_003897.3 | IER3-Hs00174674_m1 | A_42_P515405 | NM_212505 | ID:15937 | ATTTATTCTAACTTATGCAGGGGTGCGAGATATGCCCCCTT- GCTGTGACACAGATATTTA (SEQ ID NO: 36) |
| Pik3ap1 | NM_152309.2 | PIK3AP1-Hs00381030_m1 | A_43_P21121 | N_001106368 | ID:294048 | ACCTGGAGACCCACTGTCACTGGTGATGGTG- TAGCCCTGCTGGTTTGGGTGATCCTTGAA (SEQ ID NO: 37) |
| Pstpip1 | N_003978.3 | PSTPIP1-Hs00182777_m1 | A_44_P180717 | NM_011193 | ID:19200 | TGGTGTGATAAAGAGGTTCTCTGGGCTGC- TACATGGAAGTCCCAAGACCACACCTTCTCA (SEQ ID NO: 38) |
| Ubd | NM_006398.3 | UBD-Hs00197374_m1 | A_42_P602724 | NM_053299 | ID:29168 | GTGACTACGGGAGTGGGGTGATGAGAAGCTCAAAAC- CGACTTCCTTTAATCAATTAACCA (SEQ ID NO: 39) |
| C2 | NM_006987.2 | C2-Hs00163794_m1 | A_44_P332606 | NM_172222 | ID:12263 | CCTGGTGAGTTGGGGTCTTTTTGACCCTTGTCAC- GGTTCCTCCAACAAAAACTTGCGCAG (SEQ ID NO: 40) |
| Lst1 | NM_001166538 | LST1-Hs00394683_m1 | A_43_P12274 | NM_022634 | ID:64569 | AGGCAGAGGAGAAGGTGAAGGCGTAAAAGAAGACGCCAGCAC- TGACTATGCCTGCATCGT (SEQ ID NO: 41) |
| Aif1 | NM_001623.3 | AIF1-Hs00610419_g1 | A_44_P421534 | NM_019467 | ID:11629 | TTTCTCAGAATGATGCTGGGCAAGAGATCTGCCATCTTGA- GAATGATTCTGATGTATGAG (SEQ ID NO: 42) |
| C1QTNF7 | NM_001135170.1 | C1QTNF7-Hs00230467_m1 | A_44_P248172 | NM_175425 | ID:109323 | GGTTTCTCCTCTATGTTGATACAGATTACCTGGATTCTA- TATCAGAAGACGATGAGTTGT (SEQ ID NO: 43) |
| MME | NM_002426.4 | MME-Hs00153519_m1 | A_43_P11484 | NM_012608 | ID:24590 | ATCATATTGCTGAAAATCTTCAAACACAAACTCTGGGGTGAG- CATTACCATTGAACAGTT (SEQ ID NO: 45) |
| IGFBP5 | NM_000599.3 | IGFBP5-Hs01052296_m1 | A_44_P285534 | NM_012817 | ID:16011 | ACCCCGGAAACGTATTCCTATTTGAAGCAAGTTGAACGGACAGA- GAAGGGAAGAAGAGAA (SEQ ID NO: 46) |
| CARD11 | NM_032415.3 | CARD11-Hs01060620_m1 | A_44_P421727 | XM_001073551 | ID:108723 | GAGATGAGTACCTCCGGAAACAGAAGACGGAGAC- CATCATCTACTCCCGAGAAAAGAACC (SEQ ID NO: 47) |

**Table 11**

| gene | p value | log2-fold changes | regulation in human clinical GVHD biopsies | Gene Seq. Ref. (human) |
|---|---|---|---|---|
| ANP32A | 0,022 | -2,03 | Down | NM_012903 |
| CARD11 | 0,0015 | 2,68 | Up | NM_032415.3 |
| C1QTNF7 | 0,0002 | -3,26 | Down | NM_001135170.1 |
| CEACAM4 | 0,003 | 4,86 | Up | NM_001817.2 |
| HCLS1 | 0,0006 | 2,53 | Up | NM_008225 |
| HTRA1 | 0,02 | -1,01 | Down | NM_031721 |
| LGALS7 | 0,0172 | -0,82 | Down | NM_022582 |
| LST1 | 0,0138 | -0,75 | Down | N_001166538 |
| MSR1 | 0,0133 | 3,94 | Up | NM_138715.2 |
| PIK3AP1 | 0,0279 | 3,39 | Up | NM_152309.2 |
| PSTPIP1 | 0,0057 | 2,40 | Up | NM_003978.3 |
| PTGER2 | 0,0435 | 1,99 | Up | NM_031088 |
| PTPN7 | 0,0003 | 4,14 | Up | NM_177081 |
| TAP1 | 0,0174 | 3,83 | Up | NM_032055 |
| TGM2 | 0,003 | 5,12 | Up | NM_019386 |
| TREM2 | 0,001 | 4,23 | Up | NM_031254 |
| UBD | 0,0441 | 2,38 | Up | NM_006398.3 |
| CTGF | 0,036 | -1,90 | Down | NM_001901.2 |

The following embodiments are particularly preferred:
1. A method of predicting the risk of a subject to develop graft versus host reaction (GvHR) or graft versus host disease (GvHD), comprising
   (a) determining the expression level of one or more prognostic RNA transcripts, or their corresponding cDNAs, or their expression products, in a sample obtained from said subject, wherein said transcript(s) or expression products is/are the transcript or expression product of one or more genes selected from the group consisting of:
      (i) Msr1, Pik3ap1, Pstpip1, Ctss, Pbx2, Grem1, Ly6g6e, Olr1, Spr1, Spic, Nfe2, Tnfaip8l2, and Ier3; or
      (ii) Msr1, Ctss, Pbx2, Grem1, Ly6g6e, Olr1, Spr1, Spic, and Nfe2; or
      (iii) Pik3ap1, Pstpip1, Tnfaip8l2, and Ier3;
   (b) comparing the expression level of said one or more prognostic RNA transcript, or its corresponding cDNA, or its expression product with a corresponding baseline value;
      wherein
      (i) for every unit of increased expression of Olr1, Msr1, Pik3ap1, and/or Pstpip1; or the corresponding cDNA or expression product, said subject is expected to develop GvHR or GvHD; and
      (ii) for every unit of decreased expression of Ctss, Pbx2, Grem1, Ly6g6e, Spr1, Spic, Nfe2, Tnfaip8l2, and/or Ier3; or the corresponding cDNA or expression product, said subject is expected to develop GvHR or GvHD.
2. The method of embodiment 1, wherein the expression level is determined by
   a) DNA microarray analysis or quantitative PCR and subsequent calculation of the mRNA copy number normalized to the amount of total RNA or to the expression level of one or more housekeeping genes; and/or
   b) wherein the expression level of the corresponding expression product(s) is determined by ELISA, Western blotting, protein microarray, immunohistochemistry, flow cytometry or surface plasmon resonance.
3. The method of embodiment 1 or 2, wherein the sample is a biopsy sample or a sample of Peripheral Blood Mononuclear Cells (PBMC).
4. The method of any of embodiments 1 to 3, wherein the subject is a mammal, preferably a human.
5. The method of any one of embodiments 1 to 4, wherein the baseline value is the expression level of said at least one gene in at least one healthy subject.
6. The method of any one of embodiments 1 to 5, further comprising determining the prognostic transcript of one or more genes selected from the group of genes consisting of Ubd, C2, Lst1, Aif1, C1QTNF7, CEACAM4, MME, IGFBP5, Tap1, Ctgf, ANP32A, HCLS1, HTRA1, LGALS7, PTGER2, PTPN7, TGM2, TREM2 and CARD11; or their corresponding cDNAs, or their expression products, wherein
   (i) for every unit of increased expression of one or more of Ubd, C2, Aif1, CEACAM4, Tap1, PTGER2, PTPN7, TGM2, TREM2, HCLS1 and/or CARD11; or the corresponding cDNA or expression product, said patient is expected to develop GvHR or GvHD; and
   (ii) for every unit of decreased expression of one or more of Lst1, C1QTNF7, MME, Ctgf, ANP32A, HTRA1, LGALS7 and/or IGFBP5; or the corresponding cDNAs or expression product(s), said patient is expected to develop GvHR or GvHD.
7. A method of diagnosing graft versus host reaction (GvHR) or graft versus host disease (GvHD) in a subject following transplantation, comprising:
   a) determining the expression level of one or more prognostic RNA transcripts, or their corresponding cDNAs, or their expression products, in a sample obtained from said subject, wherein said transcript(s) or expression products is/are the transcript or expression product of one or more genes selected from the group consisting of:
      (i) Msr1, Pik3ap1, Pstpip1, Ctss, Pbx2, Grem1, Ly6g6e, Olr1, Spr1, Spic, Nfe2 Tnfaip8l2, and Ier3; or
      (ii) Msr1, Ctss, Pbx2, Grem1, Ly6g6e, Olr1, Spr1, Spic, and Nfe2; or
      (iii) Pik3ap1, Pstpip1, Tnfaip8l2, and Ier3;
   b) comparing the expression level of said one or more prognostic RNA transcript, or its corresponding cDNA, or its expression product with a corresponding baseline value;
      wherein
      (i) every unit of increased expression of Olr1, Msr1, Pik3ap1, and/or Pstpip1, or the corresponding cDNA or expression product, is indicative of GvHR or GvHD; and
      (ii) every unit of decreased expression of Ctss, Pbx2, Grem1, Ly6g6e, Spr1, Spic, Nfe2, Tnfaip8l2, and/or Ier3, or the corresponding cDNA or expression product, is indicative of GvHR or GvHD.
8. The method of embodiment 7, wherein the method is further defined as in any one of embodiments 2 to 4.
9. The method of embodiment 7 or 8, further comprising determining the prognostic transcript of one or more genes selected from the group of genes consisting of Ubd, C2, Lst1, Aif1, C1QTNF7, CEACAM4, MME, IGFBP5, Tap1, Ctgf, ANP32A, HCLS1, HTRA1, LGALS7, PTGER2, PTPN7, TGM2, TREM2 and CARD11; or their corresponding cDNAs, or their expression products, wherein
   (i) every unit of increased expression of Ubd, C2, Aif1, CEACAM4, Tap1, PTGER2, PTPN7, TGM2, TREM2, HCLS1 and/or CARD11; or the corresponding cDNA or expression product, is indicative of GvHR or GvHD; and
   (ii) every unit of decreased expression of Lst1, C1QTNF7, MME, Ctgf, ANP32A, HTRA1, LGALS7 and/or IGFBP5; or the corresponding cDNA or expression product, is indicative of GvHR or GvHD.
10. The method of any one of embodiments 7 to 9, wherein the baseline value is the expression level of said at least one gene in said subject prior to said transplantation and/or in at least one healthy subject.
11. A method of monitoring the efficacy of treatment of graft versus host reaction (GvHR) or graft versus host disease (GvHD) in a subject following transplantation, comprising:
   (a) determining the expression level of one or more prognostic RNA transcripts, or their corresponding cDNAs, or their expression products, in a sample obtained from said subject at a first time point T1, and a later second time point T2, wherein said transcript(s) or expression products is/are the transcript or expression product of one or more genes selected from the group consisting of:
      (i) Msr1, Pik3ap1, Pstpip1, Ctss, Pbx2, Grem1, Ly6g6e, Olr1, Spr1, Spic, Nfe2, Tnfaip8l2, and Ier3; or
      (ii) Msr1, Ctss, Pbx2, Grem1, Ly6g6e, Olr1, Spr1, Spic, and Nfe2; or
      (iii) Pik3ap1, Pstpip1, Tnfaip8l2, and Ier3;
   (b) comparing the expression level of said one or more prognostic RNA transcript, or its corresponding cDNA, or its expression product at time point T1 (Δ1) and time point T2 (Δ2) with a corresponding baseline value; wherein
      (i) a decline in units of an increased expression of Olr1, Msr1, Pik3ap1, and/or Pstpip1; or the corresponding cDNA or expression product at time point T2 in comparison with the increased expression of said at least one gene at the time point T1 (ΔΔ=Δ1-Δ2), is indicative of effective treatment of GvHR or GvHD; and
      (ii) a decline in units of a decreased expression of Ctss, Pbx2, Grem1, Ly6g6e, Spr1, Spic, Nfe2, Tnfaip8l2, and/or Ier3; or the corresponding cDNA or expression product at time point T2 in comparison with the decreased expression of said at least one gene at the time point T1(ΔΔ=Δ1-Δ2), is indicative of effective treatment of GvHR or GvHD.
12. The method of embodiment 11, wherein the method is further defined as in any one of embodiments 2 to 4 or embodiment 10.
13. The method of embodiment 11 or 12, further comprising determining the prognostic transcript of one or more genes selected from the group of genes consisting of Ubd, C2, Lst1, Aif1, C1QTNF7, CEACAM4, MME, IGFBP5, Tap1, Ctgf, ANP32A, HCLS1, HTRA1, LGALS7, PTGER2, PTPN7, TGM2, TREM2 and CARD11; or their corresponding cDNAs, or their expression products, wherein
   (i) a decline in units of an increased expression of Ubd, C2, Aif1, CEACAM4, Tap1, PTGER2, PTPN7, TGM2, TREM2, HCLS1 and/or CARD11; or the corresponding cDNA or expression product at time point T2 in comparison with the increased expression of said at least one gene at the time point T1 (ΔΔ=Δ1-Δ2), is indicative of effective treatment of GvHR or GvHD; and
   (ii) a decline in units of a decreased expression of Lst1, C1QTNF7, MME, Ctgf, ANP32A, HTRA1, LGALS7 and/or IGFBP5; or the corresponding cDNA or expression product at time point T2 in comparison with the decreased expression of said at least one gene at the time point T1(ΔΔ=Δ1-Δ2), is indicative of effective treatment of GvHR or GvHD.
14. A method of screening for a candidate substance for treatment of graft versus host reaction (GvHR) or graft versus host disease (GvHD), comprising:
   (a) monitoring the efficacy of treatment by said candidate substance by using the method according to any one of embodiments 11 to 13 in
      (i) a non-human animal model which suffers from GvHR or GvHD and to which the candidate substance has been administered, or
      (ii) in an ex vivo model, including but not limited to cell-based and/or tissue-based GvHR or HvHD assay such as the Skin Explant Assay, wherein said cells and/or tissue have been contacted with said candidate substance; and
   (b) selecting a candidate substance which shows effective treatment of GvHR or GvHD.
15. Use of a kit in a method of predicting the risk of developing graft versus host reaction (GvHR) or graft versus host disease (GvHD) according to any one of embodiments 1 to 6, or in a method of diagnosing GvHR or GvHD according to any one of embodiments 7 to 10, or in a method of monitoring the efficacy of treatment of GvHR or GvHD, wherein the kit comprises at least one isolated polynucleotide, wherein each isolated polynucleotide independently comprises
   (i) at least 20 contiguous nucleotides of the nucleotide sequence selected from SEQ ID NO: 1, 3, 5, 7, 8, 10, 12, 14, 16, 18, 20, 22, and/or 24; or SEQ ID NO: 26-47, or
   (ii) a nucleotide sequence having at least 90% identity to (i), or
   (iii) the coding region of a gene comprising a nucleotide sequence according to (i) or (ii), or
   (iv) a nucleotide sequence that can specifically hybridize, under conditions of high stringency, to a polynucleotide having a nucleotide sequence according to (i), (ii) or (iii); and
   wherein the kit comprises no more than 9000 isolated polynucleotides in total.
16. The use of embodiment 15, wherein the isolated polynucleotides comprise at least 25, preferably at least 30, more preferably at least 35, even more preferably at least 40, most preferably 50, in particular 60 contiguous nucleotides.
17. The use of embodiment 15 or 16, wherein the isolated polynucleotides are arranged in an array, in particular wherein the kit comprises no more than 8000, preferably no more than 7000, more preferably no more than 6000, even more preferably no more than 5000 or even no more than 4000, most preferably no more than 3000 or even no more than 2000, in particular no more than 1000 or even no more than 500 or no more than 100 isolated polynucleotides in total.

### References

Arora M, Weisdorf DJ, Spellman SR, Haagenson MD, Klein JP, et al. (2009) HLA-identical sibling compared with 8/8 matched and mismatched unrelated donor bone marrow transplant for chronic phase chronic myeloid leukemia. J Clin Oncol 27: 1644-1652.
Yakoub-Agha I, Mesnil F, Kuentz M, Boiron JM, Ifrah N, et al. (2006) Allogeneic marrow stem-cell transplantation from human leukocyte antigen-identical siblings versus human leukocyte antigen-allelic-matched unrelated donors (10/10) in patients with standard-risk hematologic malignancy: a prospective study from the French Society of Bone Marrow Transplantation and Cell Therapy. J Clin Oncol 24: 5695-5702.
Consortium TMS (1999) Complete sequence and gene map of a human major histocompatibility complex. The MHC sequencing consortium. Nature 401: 921-923.
Petersdorf EW, Malkki M, Gooley TA, Martin PJ, Guo Z (2007) MHC haplotype matching for unrelated hematopoietic cell transplantation. PLoS Med 4: e8.
Dickinson AM, Harrold JL, Cullup H (2007) Haematopoietic stem cell transplantation: can our genes predict clinical outcome? Expert Rev Mol Med 9: 1-19.
Hurt P, Walter L, Sudbrak R, Klages S, Müller I, et al. (2004) The genomic sequence and comparative analysis of the rat major histocompatibility complex. Genome Res 14: 631-639.
Naper C, Ryan JC, Kirsch R, Butcher GW, Rolstad B, et al. (1999) Genes in two major histocompatibility complex class I regions control selection, phenotype, and function of a rat Ly-49 natural killer cell subset. Eur J Immunol 29: 2046-2053.
Naper C, Dai KZ, Kveberg L, Rolstad B, Niemi EC, et al. (2005) Two structurally related rat Ly49 receptors with opposing functions (Ly49 stimulatory receptor 5 and Ly49 inhibitory receptor 5) recognize nonclassical MHC class Ib-encoded target ligands. J Immunol 174: 2702-2711.
Sviland L, Hromadnikova I, Sedlacek P, Cermakova M, Stechova K, et al. (2001) Histological correlation between different centers using the skin explant model to predict graft-versus-host disease following bone marrow transplantation. Hum Immunol 62: 1277-1281.
Dickinson AM, Wang XN, Sviland L, Vyth-Dreese FA, Jackson GH, et al. (2002) In situ dissection of the graft-versus-host activities of cytotoxic T cells specific for minor histocompatibility antigens. Nat Med 8: 410-414.
Novota P, Sviland L, Zinöcker S, Stocki P, Balavarca Y, et al. (2008) Correlation of Hsp70-1 and Hsp70-2 gene expression with the degree of graft-versus-host reaction in a rat skin explant model. Transplantation 85: 1809-1816.
Thomas PD, Campbell MJ, Kejariwal A, Mi H, Karlak B, et al. (2003) PANTHER: a library of protein families and subfamilies indexed by function. Genome Res 13: 2129-2141.
Pfaffl MW (2001) A new mathematical model for relative quantification in real-time RT-PCR. Nucleic Acids Res 29: e45.
Lerner KG, Kao GF, Storb R, Buckner CD, Clift RA, et al. (1974) Histopathology of graft-vs.-host reaction (GvHR) in human recipients of marrow from HL-A-matched sibling donors. Transplant Proc 6: 367-371.
Landgrebe J, Bretz F, Brunner E (2004) Efficient two-sample designs for microarray experiments with biological replications. In Silico Biol 4: 461-470.
Yang YH, Dudoit S, Luu P, Lin DM, Peng V, et al. (2002) Normalization for cDNA microarray data: a robust composite method addressing single and multiple slide systematic variation. Nucleic Acids Res 30: e15.
Benjamini Y, Hochberg Y (1995) Controlling the false discovery rate: A practical and powerful approach to multiple testing. J Roy Statist Soc Ser B 57: 289-300.
Thomas PD, Kejariwal A, Guo N, Mi H, Campbell MJ, et al. (2006) Applications for protein sequence-function evolution data: mRNA/protein expression analysis and coding SNP scoring tools. Nucleic Acids Res 34: W645-650.
Livak KJ, Schmittgen TD (2001) Analysis of relative gene expression data using real-time quantitative PCR and the 2(-Delta Delta C(T)) Method. Methods 25: 402-408.
Twigger SN, Pruitt KD, Fernandez-Suarez XM, Karolchik D, Worley KC et al. (2008) What everybody should know about the rat genome and its online resources. Nat. Genet. 40: 523-527.

## Claims

1. A method of predicting the risk of a subject to develop graft versus host reaction (GvHR) or graft versus host disease (GvHD), comprising
(a) determining the expression level of one or more prognostic RNA transcripts, or their corresponding cDNAs, or their expression products, in a sample obtained from said subject, wherein one of said transcript(s) or expression products is the transcript or expression product of Pstpip1;
(b) comparing the expression level of said one or more prognostic RNA transcript, or its corresponding cDNA, or its expression product with a corresponding baseline value;
wherein for every unit of increased expression of Pstpip1; or the corresponding cDNA or expression product, said subject is expected to develop GvHR or GvHD.

2. The method of claim 1, wherein the expression level is determined by
(a) DNA microarray analysis or quantitative PCR and subsequent calculation of the mRNA copy number normalized to the amount of total RNA or to the expression level of one or more housekeeping genes; and/or
(b) wherein the expression level of the corresponding expression product(s) is determined by ELISA, Western blotting, protein microarray, immunohisto-chemistry, flow cytometry or surface plasmon resonance.

3. The method of claim 1 or 2, wherein the sample is a biopsy sample or a sample of Peripheral Blood Mononuclear Cells (PBMC).

4. The method of any of claims 1 to 3, wherein the subject is a mammal, preferably a human.

5. The method of any one of claims 1 to 4, wherein the baseline value is the expression level of said at least one gene in at least one healthy subject.

6. The method of any one of claims 1 to 5, further comprising determining the prognostic transcript of one or more genes selected from the group of genes consisting of
(a) ANP32A, C1QTNF7, HTRA1, LGALS7, Ubd, C2, Lst1, Aif1, CEACAM4, MME, IGFBP5, Tap1, Ctgf, HCLS1, PTGER2, PTPN7, TGM2, TREM2 and CARD11, preferably ANP32A, C1QTNF7, HTRA1, LGALS7; or their corresponding cDNAs, or their expression products, wherein
(i) for every unit of increased expression of one or more of Ubd, C2, Aif1, CEACAM4, Tap1, PTGER2, PTPN7, TGM2, TREM2, HCLS1 and/or CARD11; or the corresponding cDNA or expression product, said patient is expected to develop GvHR or GvHD; and
(ii) for every unit of decreased expression of one or more of Lst1, C1QTNF7, MME, Ctgf, ANP32A, HTRA1, LGALS7 and/or IGFBP5; or the corresponding cDNAs or expression product(s), said patient is expected to develop GvHR or GvHD; and/or
(b) Msr1, Pik3ap1, Ctss, Pbx2, Grem1, Ly6g6e, Olr1, Spr1, Spic, Nfe2, Tnfaip8l2, and Ier3; or their corresponding cDNAs, or their expression products, wherein
(i) for every unit of increased expression of Olr1, Msr1, and/or Pik3ap1; or the corresponding cDNA or expression product, said subject is expected to develop GvHR or GvHD; and
(ii) for every unit of decreased expression of Ctss, Pbx2, Grem1, Ly6g6e, Spr1, Spic, Nfe2, Tnfaip8l2, and/or Ier3; or the corresponding cDNA or expression product, said subject is expected to develop GvHR or GvHD.

7. A method of diagnosing graft versus host reaction (GvHR) or graft versus host disease (GvHD) in a subject following transplantation, comprising:
(a) determining the expression level of one or more prognostic RNA transcripts, or their corresponding cDNAs, or their expression products, in a sample obtained from said subject, wherein one of said transcript(s) or expression products is the transcript or expression product of Pstpip1
(b) comparing the expression level of said one or more prognostic RNA transcript, or its corresponding cDNA, or its expression product with a corresponding baseline value;
wherein every unit of increased expression of Pstpip1, or the corresponding cDNA or expression product, is indicative of GvHR or GvHD.

8. The method of claim 7, wherein the method is further defined as in any one of claims 2 to 4; and/or
wherein the baseline value is the expression level of said at least one gene in said subject prior to said transplantation and/or in at least one healthy subject.

9. The method of claim 7 or 8, further comprising determining the prognostic transcript of one or more genes selected from the group of genes consisting of
(a) ANP32A, C1QTNF7, HTRA1, LGALS7, Ubd, C2, Lst1, Aif1, CEACAM4, MME, IGFBP5, Tap1, Ctgf, HCLS1, PTGER2, PTPN7, TGM2, TREM2 and CARD11; or their corresponding cDNAs, or their expression products, wherein
(i) every unit of increased expression of Ubd, C2, Aif1, CEACAM4, Tap1, PTGER2, PTPN7, TGM2, TREM2, HCLS1 and/or CARD11; or the corresponding cDNA or expression product, is indicative of GvHR or GvHD; and
(ii) every unit of decreased expression of Lst1, C1QTNF7, MME, Ctgf, ANP32A, HTRA1, LGALS7 and/or IGFBP5; or the corresponding cDNA or expression product, is indicative of GvHR or GvHD; and/or
(b) Msr1, Pik3ap1, Ctss, Pbx2, Grem1, Ly6g6e, Olr1, Spr1, Spic, Nfe2, Tnfaip8l2, and Ier3; or their corresponding cDNAs, or their expression products, wherein
(i) for every unit of increased expression of Olr1, Msr1, and/or Pik3ap1; or the corresponding cDNA or expression product, said subject is expected to develop GvHR or GvHD; and
(ii) for every unit of decreased expression of Ctss, Pbx2, Grem1, Ly6g6e, Spr1, Spic, Nfe2, Tnfaip8l2, and/or Ier3; or the corresponding cDNA or expression product, said subject is expected to develop GvHR or GvHD.

10. A method of monitoring the efficacy of treatment of graft versus host reaction (GvHR) or graft versus host disease (GvHD) in a subject following transplantation, comprising:
(a) determining the expression level of one or more prognostic RNA transcripts, or their corresponding cDNAs, or their expression products, in a sample obtained from said subject at a first time point T1, and a later second time point T2, wherein one of said transcript(s) or expression products is the transcript or expression product of Pstpip1;
(b) comparing the expression level of said one or more prognostic RNA transcript, or its corresponding cDNA, or its expression product at time point T1 (Δ1) and time point T2 (Δ2) with a corresponding baseline value;
wherein a decline in units of an increased expression of Pstpip1; or the corresponding cDNA or expression product at time point T2 in comparison with the increased expression of said at least one gene at the time point T1 (ΔΔ=Δ1-Δ2), is indicative of effective treatment of GvHR or GvHD.

11. The method of claim 10, wherein the method is further defined as in any one of claims 2 to 4 or claim 8.

12. The method of claim 10 or 11, further comprising determining the prognostic transcript of one or more genes selected from the group of genes consisting of
(a) ANP32A, C1QTNF7, HTRA1, LGALS7, Ubd, C2, Lst1, Aif1, CEACAM4, MME, IGFBP5, Tap1, Ctgf, HCLS1, PTGER2, PTPN7, TGM2, TREM2 and CARD11; or their corresponding cDNAs, or their expression products, wherein
(i) a decline in units of an increased expression of Ubd, C2, Aif1, CEACAM4, Tap1, PTGER2, PTPN7, TGM2, TREM2, HCLS1 and/or CARD11; or the corresponding cDNA or expression product at time point T2 in comparison with the increased expression of said at least one gene at the time point T1 (ΔΔ=Δ1-Δ2), is indicative of effective treatment of GvHR or GvHD; and
(ii) a decline in units of a decreased expression of Lst1, C1QTNF7, MME, Ctgf, ANP32A, HTRA1, LGALS7 and/or IGFBP5; or the corresponding cDNA or expression product at time point T2 in comparison with the decreased expression of said at least one gene at the time point T1(ΔΔ=Δ1-Δ2), is indicative of effective treatment of GvHR or GvHD; and/or
(b) Msr1, Pik3ap1, Ctss, Pbx2, Grem1, Ly6g6e, Olr1, Spr1, Spic, Nfe2, Tnfaip8l2, and Ier3; or their corresponding cDNAs, or their expression products, wherein
(i) a decline in units of an increased expression of Olr1, Msr1, and/or Pik3ap1; or the corresponding cDNA or expression product at time point T2 in comparison with the increased expression of said at least one gene at the time point T1 (ΔΔ=Δ1-Δ2), is indicative of effective treatment of GvHR or GvHD; and
(ii) a decline in units of a decreased expression of Ctss, Pbx2, Grem1, Ly6g6e, Spr1, Spic, Nfe2, Tnfaip8l2, and/or Ier3; or the corresponding cDNA or expression product at time point T2 in comparison with the decreased expression of said at least one gene at the time point T1(ΔΔ=Δ1-Δ2), is indicative of effective treatment of GvHR or GvHD.

13. A method of screening for a candidate substance for treatment of graft versus host reaction (GvHR) or graft versus host disease (GvHD), comprising:
(a) monitoring the efficacy of treatment by said candidate substance by using the method according to any one of claims 10 to 12 in
(i) a non-human animal model which suffers from GvHR or GvHD and to which the candidate substance has been administered, or
(ii) in an ex vivo model, including but not limited to cell-based and/or tissue-based GvHR or HvHD assay such as the Skin Explant Assay, wherein said cells and/or tissue have been contacted with said candidate substance; and
(b) selecting a candidate substance which shows effective treatment of GvHR or GvHD.

14. Use of a kit in a method of predicting the risk of developing graft versus host reaction (GvHR) or graft versus host disease (GvHD) according to any one of claims 1 to 6, or in a method of diagnosing GvHR or GvHD according to any one of claims 7 to 9, or in a method of monitoring the efficacy of treatment of GvHR or GvHD, wherein the kit comprises at least one isolated polynucleotide, wherein each isolated polynucleotide independently comprises
(i) at least 20 contiguous nucleotides of the nucleotide sequence selected from SEQ ID NO: 1, 3, 5, 7, 8, 10, 12, 14, 16, 18, 20, 22, and/or 24; or SEQ ID NO: 26-47, or
(ii) a nucleotide sequence having at least 90% identity to (i), or
(iii) the coding region of a gene comprising a nucleotide sequence according to (i) or (ii), or
(iv) a nucleotide sequence that can specifically hybridize, under conditions of high stringency, to a polynucleotide having a nucleotide sequence according to (i), (ii) or (iii); and
wherein the kit comprises no more than 9000 isolated polynucleotides in total.

15. The use of claim 14, wherein the isolated polynucleotides comprise at least 25, preferably at least 30, more preferably at least 35, even more preferably at least 40, most preferably 50, in particular 60 contiguous nucleotides; and/or wherein the isolated polynucleotides are arranged in an array, in particular wherein the kit comprises no more than 8000, preferably no more than 7000, more preferably no more than 6000, even more preferably no more than 5000 or even no more than 4000, most preferably no more than 3000 or even no more than 2000, in particular no more than 1000 or even no more than 500 or no more than 100 isolated polynucleotides in total.
